# EUROPEAN PATENT SPECIFICATION

(11) **EP 0 506 369 B1**
(45) Date of publication and mention of the grant of the patent: **22.07.1998**
(21) Application number: 92302580.3
(22) Date of filing: 25.03.1992
(51) Int. Cl.: C07D 403/04, C07D 413/04, C07D 417/04, C07D 405/04, C07D 409/04, C07D 401/04, A61K 31/40

(54) **6-Heterocyclic-4-amino-1,2,2a,3,4,5-hexahydrobenz[CD]indoles**
6-Heterocyclisch-4-amino-1,2,2a,3,4,5-Hexahydrobenz[CD]indole
6-Hétérocyclique-4-amino-1,2,2a,3,4,5-hexahydrobenz[CD]indoles

(30) Priority: 28.03.1991 US 676679
(43) Date of publication of application: 30.09.1992
(73) Proprietor: ELI LILLY AND COMPANY, Indianapolis, Indiana 46285 (US)
(72) Inventor: Booher, Richard Nolan, Indianapolis, Indiana 46227 (US); Lawhorn, David Ernest, Greenfield, Indiana 46140 (US); Martinelli, Michael John, Indianapolis, Indiana 46254 (US); Paget, Charles Johnson, Jr., Indianapolis, Indiana 46217 (US); Schaus, John Mehnert, Zionsville, Indiana 46077 (US)
(74) Representative: Tapping, Kenneth George

(56) References cited:
- EP-A- 399 982
- US-A- 4 576 959

## Description

This invention relates to 6-heterocyclic-4-amino-1,2,2a,3,4,5-hexahydrobenz[cd]indoles, their use in modifying the function of serotonin in a mammal, pharmaceutical formulations thereof and processes for preparing same.

Flaugh in U.S. Patent No. 4,576,959 (issued 1986) disclosed a family of 6-substituted-4-dialkylamino-1,3,4,5-tetrahydrobenz[cd]indoles which are described as central serotonin agonists. Leander in U.S. Patent 4,745,126 (1988) disclosed a method for treating anxiety in humans employing a 4-substituted-1,3,4,5-tetrahydrobenz[cd]indole-6-carboxamide derivative.

European Patent Application 399,982 discloses certain heterocyclic-substituted aminotetralins. These compounds are disclosed as being serotonin agonists, partial agonists or antagonists.

Despite the progress of science as representated above, many mammals, both humans and animals, continue to be afflicted with diseases which can be cured or ameliorated with compounds capable of modifying serotonin function in the body. Accordingly, the need continues for safer, more selective, drugs which can be used to modify such function. As such, it is an object of the present invention to provide certain 6-heterocyclic-substituted hexahydrobenz[cd]indoles which are useful in treating conditions requiring modification of the serotonin function in the body.

The present invention provides compounds of the Formula 1 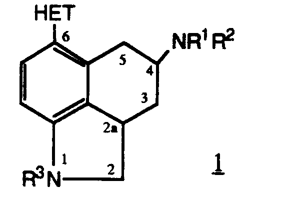 wherein:
R¹ is hydrogen, C₁-C₄ alkyl, C₃-C₄ alkenyl, cyclopropylmethyl, phenyl C₁-C₄ alkyl, phenyl(C₁-C₄)alkyl substituted with one or two substituents selected from (C₁-C₃) alkoxy, halo, hydroxy, (C₁-C₃) thioalkyl, nitro, (C₁-C₃) alkyl and trifluoromethyl, -(CH₂)ₙS(C₁-C₄ alkyl), -C(O)R⁴, -(CH₂)ₙC(O)NR⁵R⁶;
R² is hydrogen, C₁-C₄ alkyl, cyclopropylmethyl or C₃-C₄ alkenyl,
R³ is hydrogen, C₁-C₄ alkyl or an amino-blocking group;
n is 1-4;
R⁴ is hydrogen, C₁-C₄ alkyl, C₁-C₄ haloalkyl, C₁-C₄ alkoxy or phenyl;
R⁵ and R⁶ are independently hydrogen, a C₁-C₄ alkyl, or a C₅-C₈ cycloalkyl, with the proviso that when one of R⁵ or R⁶ is a cycloalkyl the other is hydrogen;
HET is an aromatic 5- or 6-membered heterocyclic ring, said ring having from one to three heteroatoms which are the same or different and which are selected from the group consisting of sulfur, oxygen, and nitrogen with the proviso that the 6-membered heterocyclic ring can only contain carbon and nitrogen and with the further proviso that the 5-membered ring may contain no more than one oxygen or one sulfur but not both oxygen and sulfur, optionally substituted on one or two carbon atoms with (C₁-C₃) alkyl, halo, hydroxy, (C₁-C₃) alkoxy (C₁-C₃) thioalkyl, NH₂, CN or phenyl.

The invention also provides a pharmaceutical formulation comprising a compound of Formula 1 in combination with a pharmaceutically acceptable excipient therefor.

A further embodiment of the invention is a method for effecting a biological response at the 5HT_{1A} receptor by administering a compound of Formula 1. Another embodiment involves a method for treating a variety of conditions in a mammal which require regulation of serotonin functions by administering a compound of Formula 1.

A final embodiment of this invention is to provide a process suitable for preparing compounds of Formula 1.

As used herein, the term "alkyl" represents a straight or branched alkyl chain having the indicated number of carbon atoms. For example, "C₁-C₄ alkyl" groups are methyl, ethyl, n-propyl, isopropyl, n-butyl, sec.-butyl, isobutyl and tert-butyl.

The term "cycloalkyl" means an aliphatic carbocyclic structure having the indicated number of carbon atoms in the ring. For example, the term "C₃-C₇ cycloalkyl" means cyclopropyl, cyclobutyl, cyclopentyl, cyclohexyl, and cycloheptyl.

The term "phenyl (C₁-C₄ alkyl)" means an aryl structure joined to a C₁-C₄ alkyl group. Examples of such groups are benzyl, phenylethyl, α-methylbenzyl, 3-phenylpropyl, α-naphthylmethyl, β-naphthylmethyl, 4-phenylbutyl, and the like. Similarly the term "phenyl (C₁-C₃ alkyl)" means a phenyl structure joined to a C₁-C₃ alkyl.

In the foregoing, the term "C₁-C₃ alkyl" means any of methyl, ethyl, n-propyl, and isopropyl; the term "C₁-C₃ alkoxy" means any of methoxy, ethoxy, n-propoxy, and isopropoxy; the term "halo" means any of fluoro, chloro, bromo, and iodo; and the term "C₁-C₃ thioalkyl" means any of methylthio, ethylthio, n-propylthio, and isopropylthio.

Examples of substituted phenyl are p-bromophenyl, m-iodophenyl, p-tolyl, o-hydroxyphenyl, β-(4-hydroxy)naphthyl, p-(methylthio)phenyl, m-trifluoromethylphenyl, 2-chloro-4-methoxyphenyl, a-(5-chloro)naphthyl, and the like.

Exemples of the substituted phenyl (C₁-C₄ alkyl) are p-chlorobenzyl, o-methoxybenzyl, m-(methylthio)-a-methylbenzyl, 3-(4′-trifluoromethylphenyl)propyl, o-iodobenzyl, p-methylbenzyl, and the like.

The term "amino-blocking group" is used herein as it is frequently used in synthetic organic chemistry, to refer to a group which will prevent an amino group from participating in a reaction carried out on some other functional group of the molecule, but which can be removed from the amine when it is desired to do so. Such groups are discussed by T. W. Greene in chapter 7 of Protective Groups in Organic Synthesis, John Wiley and Sons, New York, 1981, and by J. W. Barton in chapter 2 of Protective Groups in Organic Chemistry, J. F. W. McOmie, ed., Plenum Press, New York, 1973, which are incorporated herein by reference in their entirety. Examples of such groups include benzyl and substituted benzyl such as 3,4-dimethoxybenzyl, o-nitrobenzyl, and triphenylmethyl; those of the formula -COOR where R includes such groups as methyl, ethyl, propyl, isopropyl, 2,2,2-trichloroethyl, 1-methyl-1-phenylethyl, isobutyl, t-butyl, t-amyl, vinyl, allyl, phenyl, benzyl, p-nitrobenzyl, o-nitrobenzyl, and 2,4-dichlorobenzyl; acyl groups and substituted acyl such as formyl, acetyl, chloroacetyl, dichloroacetyl, trichloroacetyl, trifluoroacetyl, benzoyl, and p-methoxybenzoyl; and other groups such as methanesulfonyl, p-toluenesulfonyl, p-bromobenzenesulfonyl, p-nitrophenylethyl, and p-toluenesulfonylaminocarbonyl. Preferred amino-blocking groups are benzyl (-CH₂C₆H₅), aryl [C(O)R] or SiR₃ where R is C₁-C₄ alkyl, halomethyl, or 2-halo-substituted-(C₂-C₄ alkoxy).

The term "aromatic 5- or 6-membered heterocyclic ring" refers to a ring containing from one to three heteroatoms which can be nitrogen, oxygen or sulfur. The 5-membered heterocyclic rings can contain carbon and nitrogen atoms and up to one oxygen or one sulfur but not one of each. The 6-membered heterocyclic rings can contain carbon and nitrogen atoms only. The 5- or 6-membered rings can have one or two of the carbon atoms in the ring substituted independently with C₁-C₃ alkyl, halogen, OH, C₁-C₃ alkoxy, C₁-C₃ alkylthio, NH₂, CN or phenyl.

These aromatic 5- or 6-membered heterocyclic rings can be either substituted or unsubstituted and include furan, thiophene, thiazole, oxazole, isoxazole, isothiazole, oxadiazole, thiadiazole, pyridine, pyridazine, pyrimidine, pyrazine, pyrrole, pyrazole, imidazole, and triazole. The heterocyclic ring can be attached to the benzene ring by any carbon in the heterocyclic ring, for example, 2- or 3-furan.

As used herein the following terms refer to the structure indicated and includes all of the structural isomers:

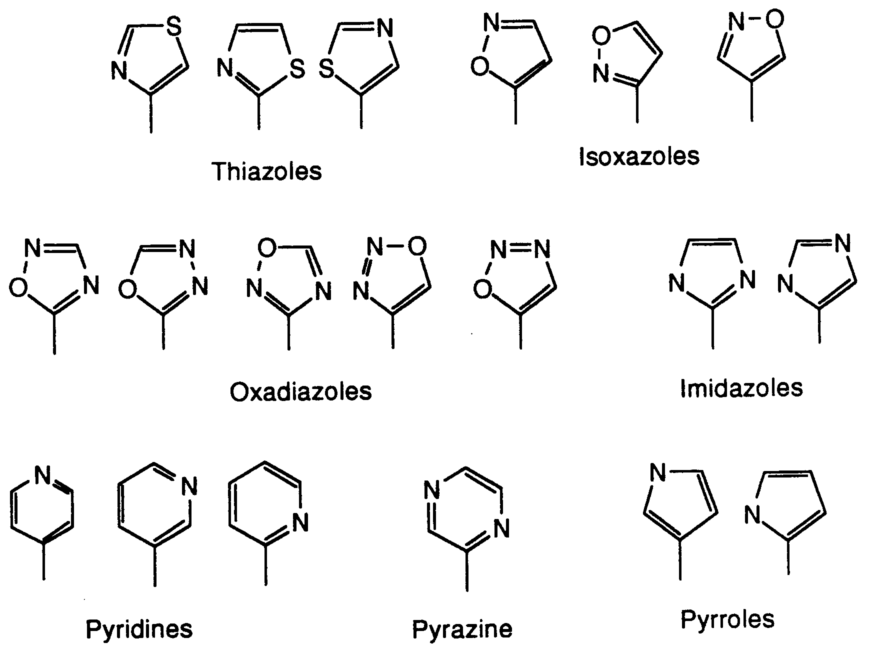

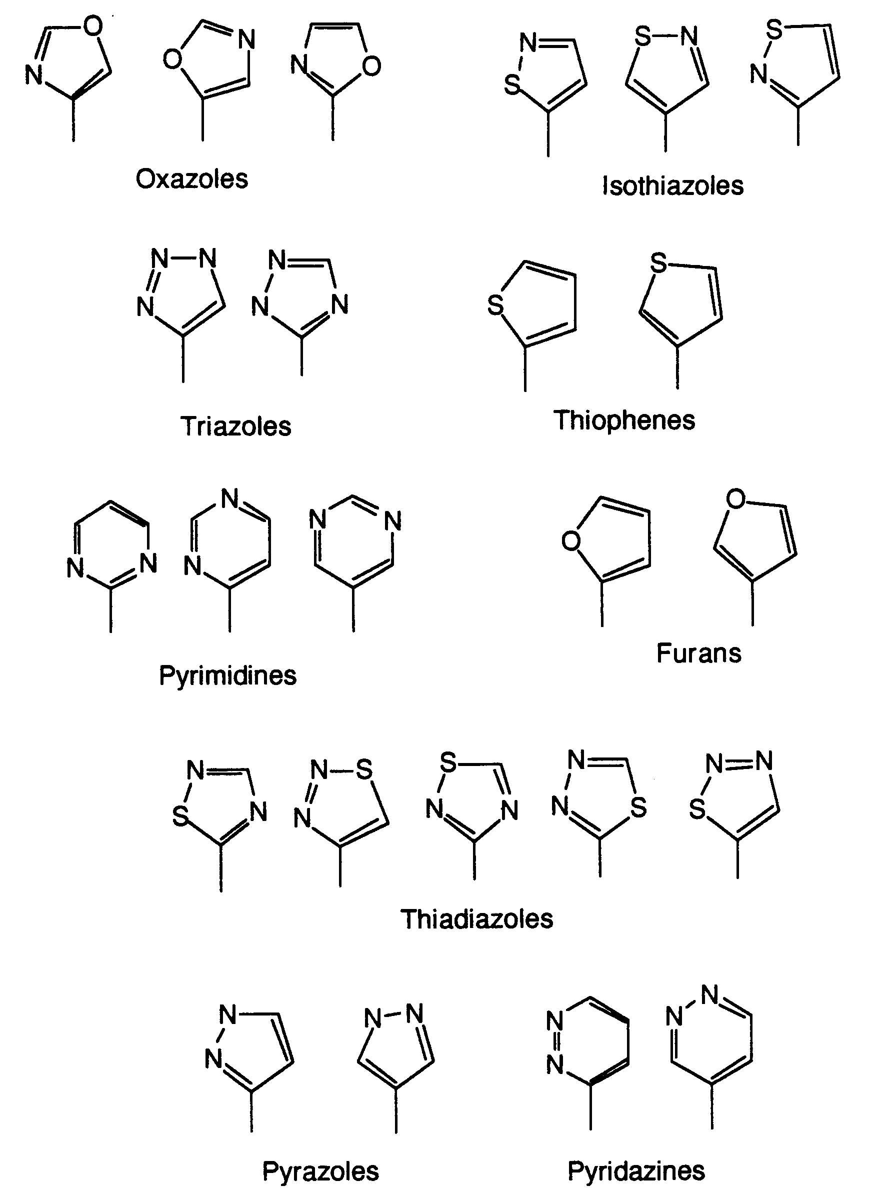

While all of the compounds of the invention are useful for the purposes taught herein, certain of the present compounds are preferred for such uses. Preferably R¹ and R² are both C₁-C₄ alkyl, particularly n-propyl, R³ is hydrogen, and HET is one of the following isoxazole, pyrazole, pyridine, thiazole, furan, thiophene or oxadiazole. Other preferred aspects of the present invention are noted hereinafter.

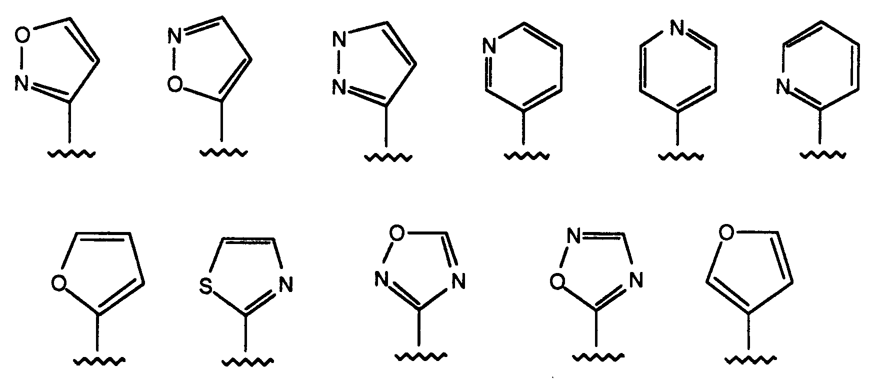

The compounds of the instant invention have at least two chiral centers and therefore at least four stereoisomers can exist for each. Chiral centers exist at positions 2a and 4 of Formula 1. If a substitutent group contains a chiral center, then additional stereoisomers can exist. Racemic mixtures as well as the substantially pure stereoisomers of Formula 1 are contemplated as within the scope of the present invention. By the term "substantially pure", it is meant that at least about 90 mole percent, more preferably at least about 95 mole percent and most preferably at least 98 mole percent of the desired stereoisomer is present compared to other possible stereoisomers. Particularly preferred stereoisomers of Formula 1 are those in which the configuration of the chiral center at position 2a is S and at position 4 is R, i.e., 2aS, 4R.

The terms "R" and "S" are used herein as comonly used in organic chemistry to denote specific configuration of a chiral center. The term "R" refers to "right" and refers that configuration of a chiral center with a clockwise relationship of group priorities (highest to second lowest) when viewed along the bond toward the lowest priority group. The term "S" or "left" refers to that configuration of a chiral center with a counterclockwise relationship of group priorities (highest to second lowest) when viewed along the bond toward the lowest priority group. The priority of groups is based upon their atomic number (heaviest isotope first). A partial list of priorities and a discussion of stereo chemistry is contained in the book: The Vocabulary of Organic Chemistry, Orchin, et al., John Wiley and sons Inc., publishers, page 126, which is incorporated herein by reference.

As set forth above, this invention includes the pharmaceutically-acceptable salts of the compounds of Formula 1. Since the compounds of this invention are amines, they are basic in nature and accordingly react with any number of inorganic and organic acids to form pharmaceutically acceptable salts such as hydrochloric acid, nitric acid, phosphoric acid, sulfuric acid, hydrobromic acid, hydroiodic acid, phosphorous acid and others, as well as salts derived from nontoxic organic acids such as aliphatic mono and dicarboxylic acids, amino acids, phenyl-substituted alkanoic acids, hydroxyalkanoic and hydroxyalkandioic acid, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, acrylate, formate, tartrate isobutyrate, caprate, heptanoate, propiolate, oxalate, malonate, succinate, suberate, sebacate, fumarate, maleate, mandelate, butyne-1,4-dioate, hexyne-1,6-dioate, hippurate, benzoate, chlorobenzoate, methylbenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, naphthalene-1-sulfonate, naphthalene-2-sulfonate and mesylate.

Particularly preferred compounds of Formula 1 include the compounds in which R³ is hydrogen, R¹ and R² are both either n-propyl or methyl and HET is 3-isoxazolyl, 5-isoxazolyl, 3-isothiazolyl, 5-isothiazolyl, 2-imidazolyl or 4-imidazolyl. These compounds include the racemic mixtures of possible stereoisomers as well as the substantially pure stereoisomers with different configurations at positions 2a and 4, i.e., 2aR, 4R or 2aR, 4S or 2aS, 4R or 2aS, 4S.

As depicted in Scheme I, the compounds of the present invention can be prepared by reacting a 4-amino-6-metallo-substituted hexahydrobenz[cd]indole as represented by structure 2 with a heterocyclic compound represented by structure 4. In structure 2, M represents a metallo moiety such as lithium, magnesian, zinc, tin, mercury, boronic acid(-BO₂H₂) and the like while Z is an amino-blocking group. When the metallo moiety is multivalent, it is normally associated with other moieties such as, for example, halo for magnesium (Grignard reagent) and alkyl groups for tin (trialkyltin). The heterocycle represented by structure 4 containing a leaving group "L", such as a chloro, bromo, or trifluoromethylsulfonoxy group, which can be displaced by the metallo-indole. The heterocycle can be substituted as set forth hereinabove.

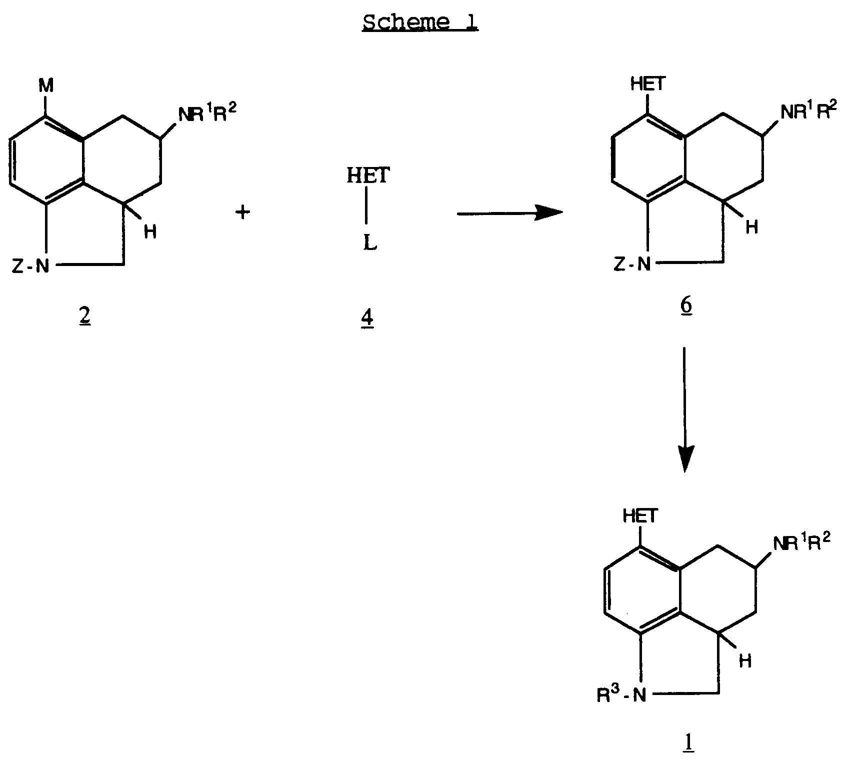

The reaction of the metallo-indoline 2 and heterocycle 4 is accomplished in the presence of a palladian or nickel catalyst such as Pd[P(C₆H₅)₃]₄, PdCl₂, Pd[P(C₆H₅)₃]₂Cl₂, Ni(acac)₂, NiCl₂[P(C₆H₅)₃]₂ and the like, wherein "acac" represents acetylacetonate and "C₆H₅" represents a phenyl group. The organometallic reagent 2 is prepared by methods comonly used in the art for such preparations, for example, the lithium or magnesium reagents can be prepared by contacting the appropriate 6-chloro-, 6-bromo- or 6-iodo-substituted hexahydrobenzindole with an organolithium reagent or magnesian metal in a solvent such as ether or tetrahydrofuran. Other organometailic derivatives can be used such as zinc, tin, mercury or boronic acid (-BO₂H₂). The zinc, tin and mercury reagents can be prepared by reaction of the lithiated benzindole with a zinc, tin or mercury derivative such as zinc chloride, chlorotrialkylstannane, or mercuric chloride. The boronic acid derivative can be prepared by reacting the lithian reagent with trimethylborate followed by hydrolysis of the resulting boronate ester. Mercuric acetate can be contacted directly with the hexahydrobenzindole to provide the mercurated derivative.

The 1-nitrogen of the hexahydro benzindole is preferably protected with a group such as triphenylmethyl (trityl), benzyl, or benzoyl. These protecting groups are represented by Z in structures 2. The protecting group can be removed after the coupling reaction is accomplished to provide the 1-hydrobenzindole compound.

An alternative method of preparing the compounds of the instant invention involves contacting an organometailic reagent prepared from a heterocyclic compound with a 6-bromo or 6-iodo-4-aminobenzindole. The reaction is accomplished in the presence of a catalyst such as that used in reaction Scheme I. The metal in the organometailic derivative of the heterocycle can be lithium, magnesium (Grignard reagent), zinc, tin, mercury, or a boronic acid (-BO₂H₂). These organometallic compounds can be prepared by standard methods, as described above for the benzindoles. Alternatively, the lithiated heterocycles can be prepared by treating a heterocycle with a strong base such as an alkyllithium or a lithian dialkylamide.

Unless otherwise indicated, in the following preparation procedures, Rₐ and Rₐ′ may independently be hydrogen, C₁-C₃ alkyl, halogen, OH, O (C₁-C₃ alkyl), S(C₁-C₃ alkyl), NH₂, CN, or phenyl. Rb may be hydrogen, C₁-C₃ alkyl, phenyl, or (C₁-C₃ alkyl) phenyl. R_{c} may be a hydrogen or C₁-C₃ alkyl. R_{d} may be OH, O(C₁-C₃ alkyl), O(phenyl), O(C₁-C₃ alkylphenyl), halo, S(C₁-C₃ alkyl), S(phenyl), S(C₁-C₃ alkylphenyl), NH₂, NH(C₁-C₃ alkyl), N(C₁-C₃ alkyl)₂, OCO(C₁-C₃ alkyl), OCO(phenyl), OCO(C₁-C₃ alkylphenyl) or the like.

In an alternative preparation procedure, compounds of the instant invention having a 5-membered heterocyclic ring in the 6-position can be prepared by the cycloaddition of a compound of the type represented in structure 8 wherein R¹ and R² are as defined above and B is an amino-protecting group or hydrogen, 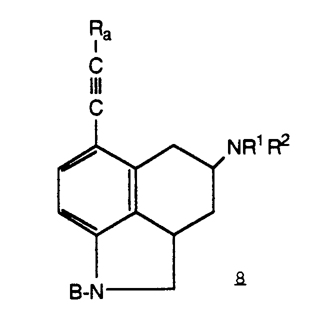 with a 1,3-dipole of the type ${\text{}}^{\text{+}} {\text{T=U-V}}^{\text{-}}$ in which T, U, and V can be selected from the following list of (a) through (i).

| | T | U | V |
|---|---|---|---|
| (a) | CRₐ | N | CHRₐ |
| (b) | CRₐ | N | NR_{b} |
| (c) | CRa | N | O |
| (d) | N | N | O |
| (e) | CRₐ | CRₐ' | NR_{b} |
| (f) | CRₐ | CRₐ' | O |
| (g) | N | CRₐ' | CHRₐ |
| (h) | N | CRₐ' | NR_{b} |
| (i) | N | CRₐ' | O |

In this list Rₐ and Rₐ' are not OH or NH₂, N represents nitrogen and O represents oxygen. This cycloaddition provides products of the structure 10, wherein R¹ and R² are as defined above and B is an amino protecting group or hydrogen. 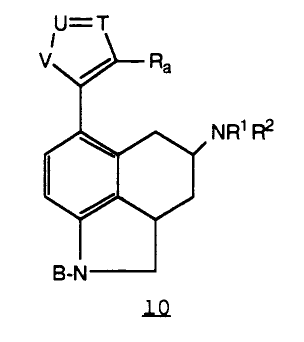 The 1-nitrogen of structures 8 and 10 can be protected using standard protecting groups preferably (C₂H₅)₂NC(O)-, triisopropylsilyl, benzoyl, or benzenesulfonyl.

Alternatively, the 6-alkyne-substituted indole of structure 8 can be reacted with a dipole of the type ${\text{}}^{\text{+}} {\text{T- U=V}}^{\text{-}}$ in which T, U, and V are selected from the following list for (j) and (k):

| | T | U | V |
|---|---|---|---|
| (j) | CHRₐ | N | N |
| (k) | NR_{b} | N | N |

In this list Rₐ is not OH or NH₂ and N is nitrogen. This reaction provides products of structure 12, 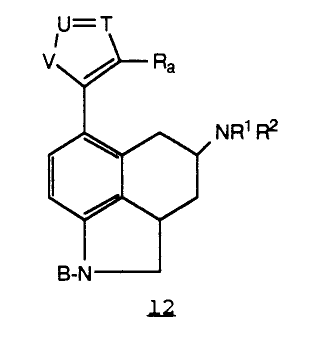 wherein R¹, R², Rₐ and B are as defined above.

Alternative procedures for preparing certain of the instant compounds are set forth hereinbelow in Schemes 2 through 18. As used in these reaction Schemes, "Ar" refers to the 1,2,2a,3,4,5-hexanydrobenz[cd]indole, with the indicated substituent in the 6-position. In these schemes, "Me" is methyl, "Et" is ethyl, "NBS" represents n-bromosuccinimide, Rₐ, R_{b}, R_{c} and R_{d} are defined above, "MsCl" represents methanesulfonyl chloride, "△" represents heat, "ø" and "Ph" each represent phenyl, "DMF" represents dimethylformamide, "DMS" represents dimethyl sulfide, "TMS" represents trimethylsilyl, "[O]" represents an oxidant, Lawesson's reagent is p-methoxyphenylthionophosphine sulfide dimer, "Ac" represents acetyl, "NCS" represents N-chlorosuccinimide, "DCC" represents dicyclohexylcarbodiimide, "Im" represents 1-imidazolyl, and "[H]" represents a reductant. As set forth hereinabove, the 1-nitrogen of the benz[cd]indole is normally protected with an aminoblocking group, preferably triisopropylsilyl.

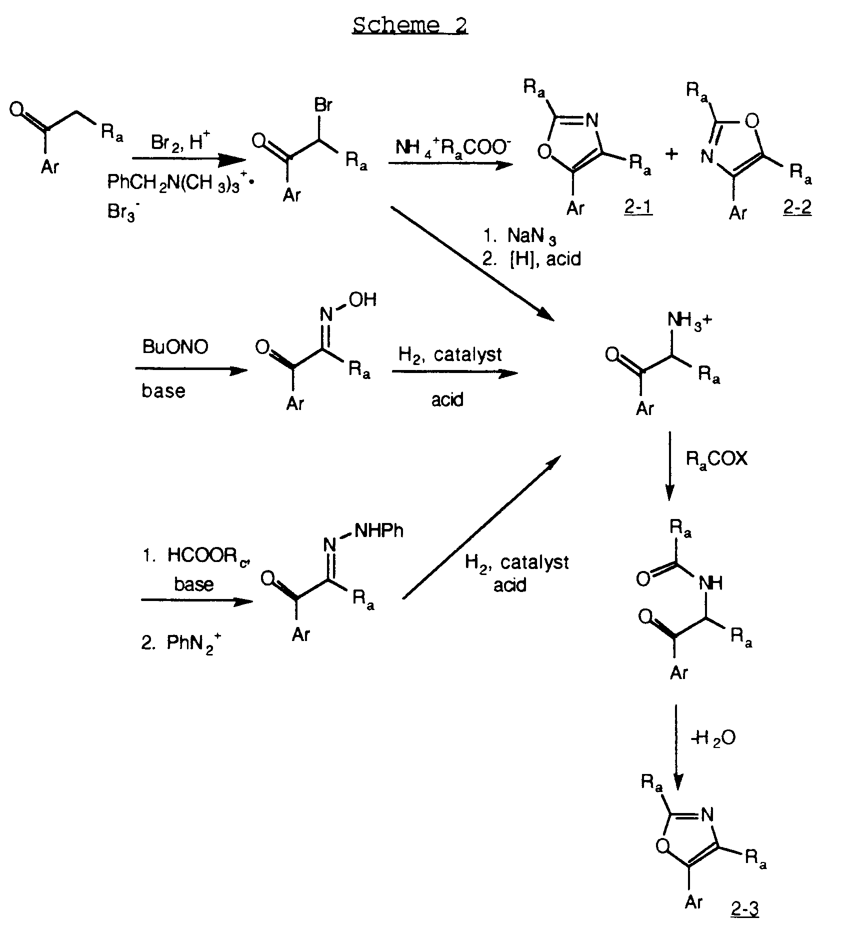

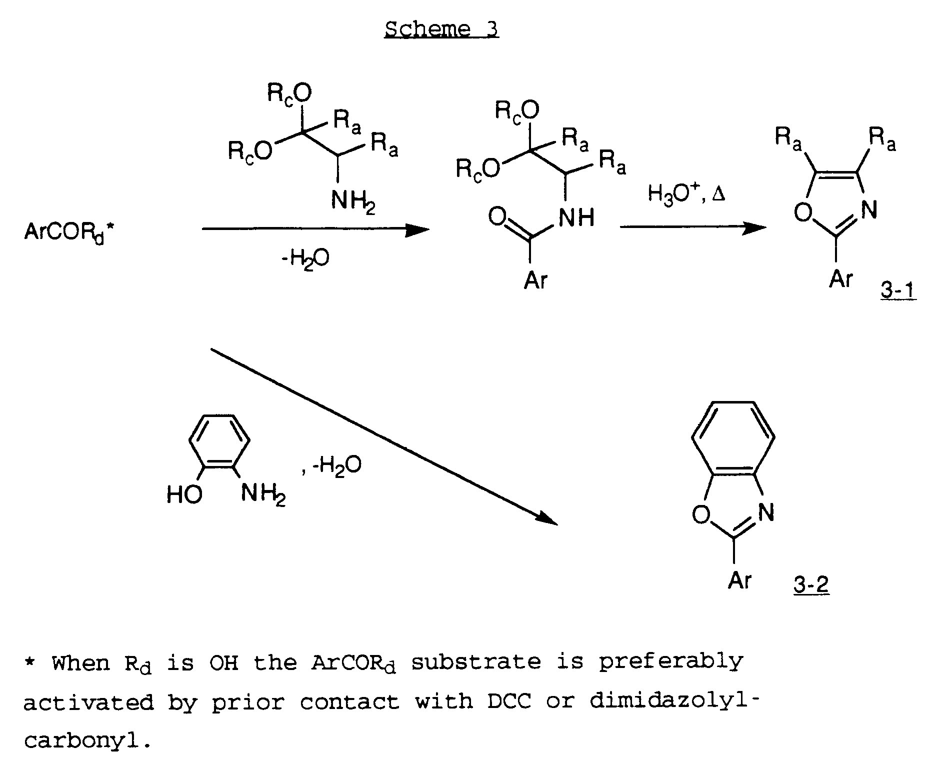

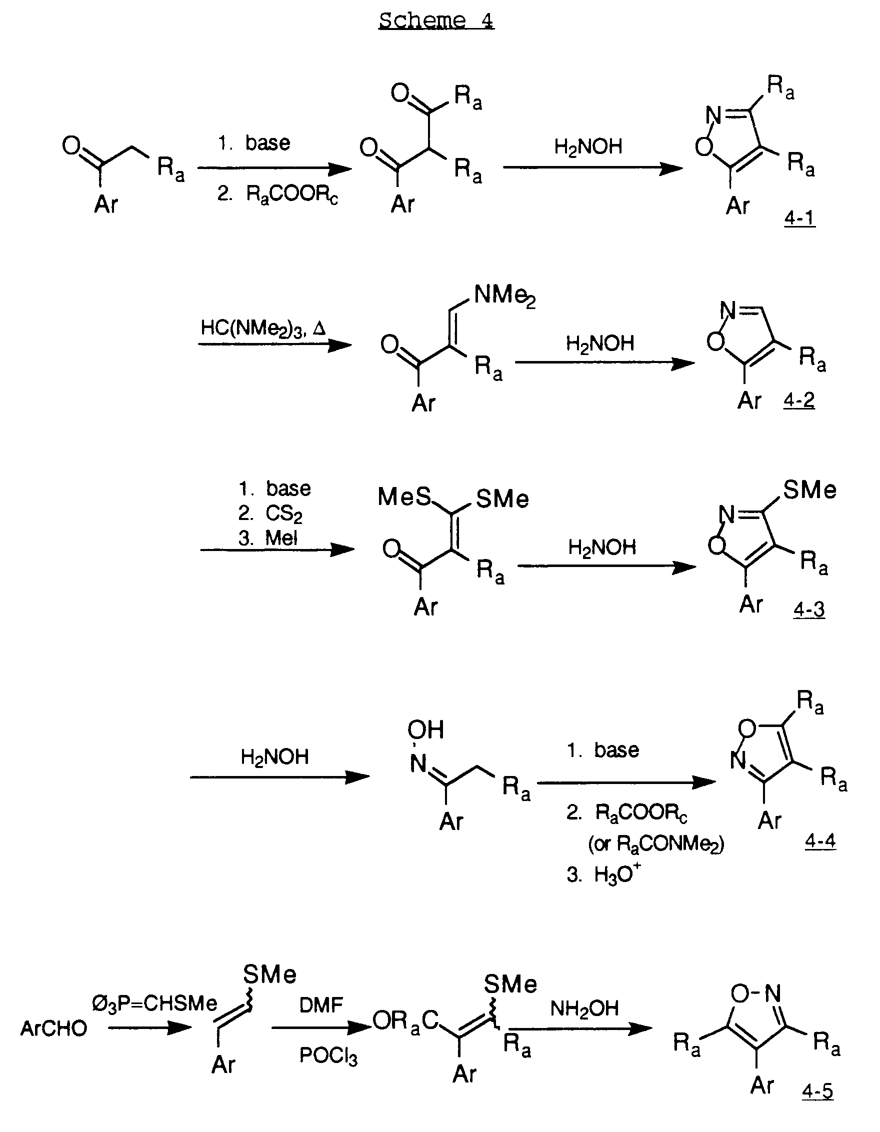

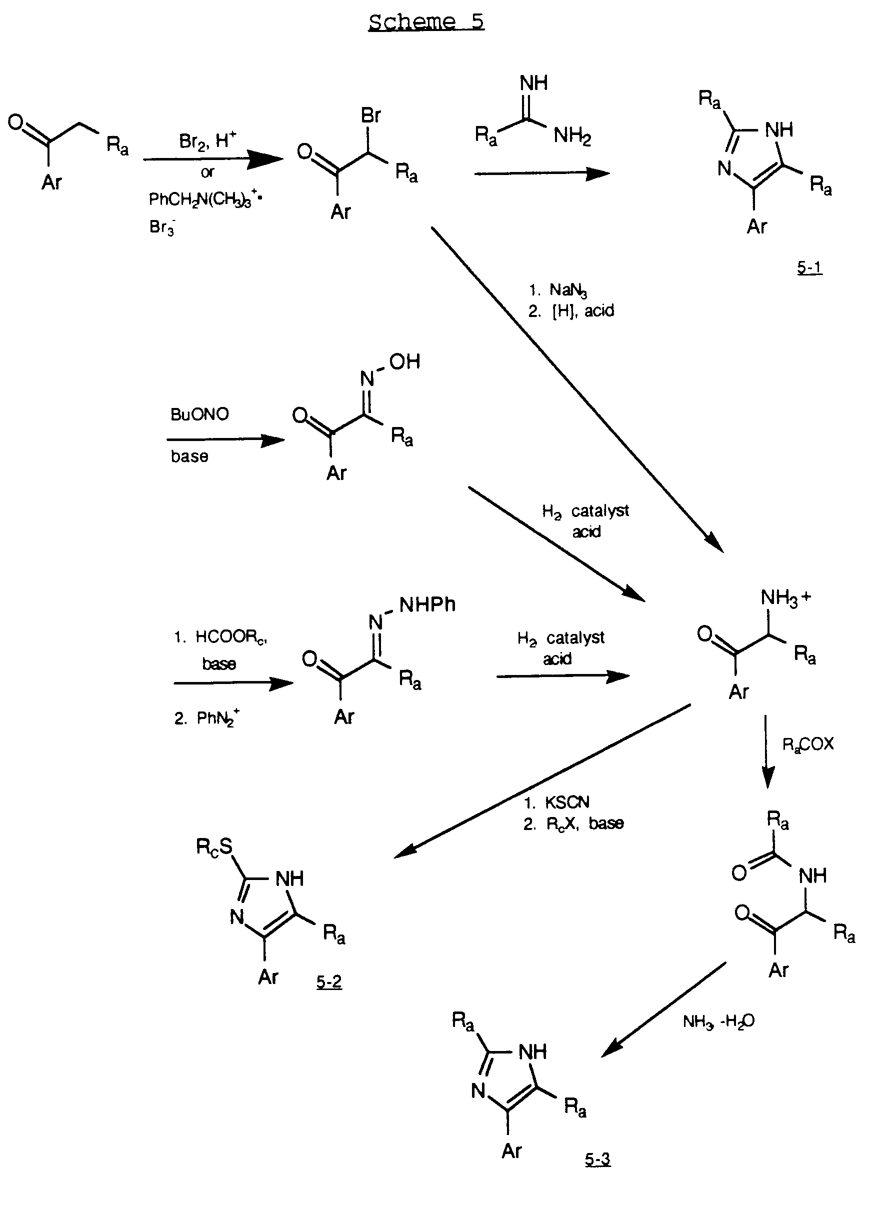

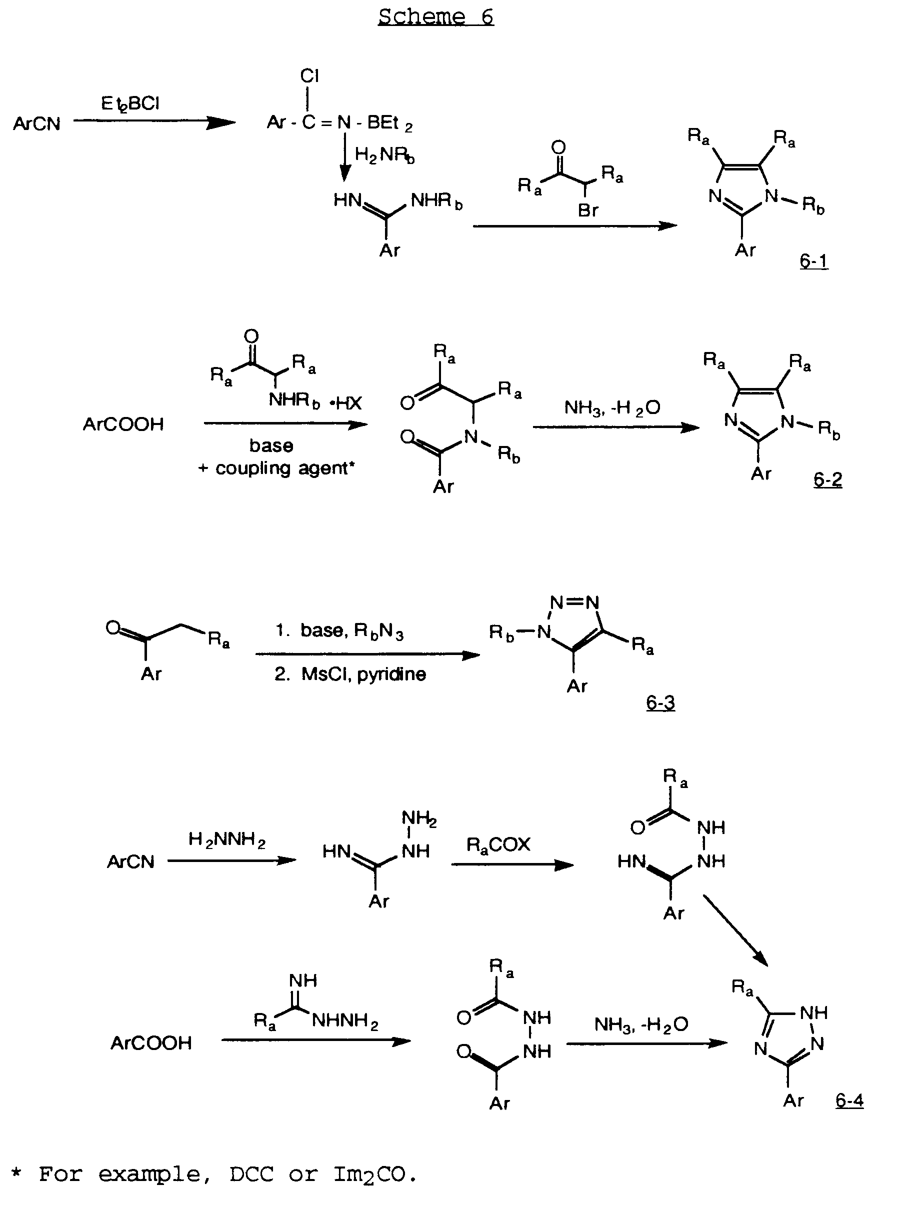

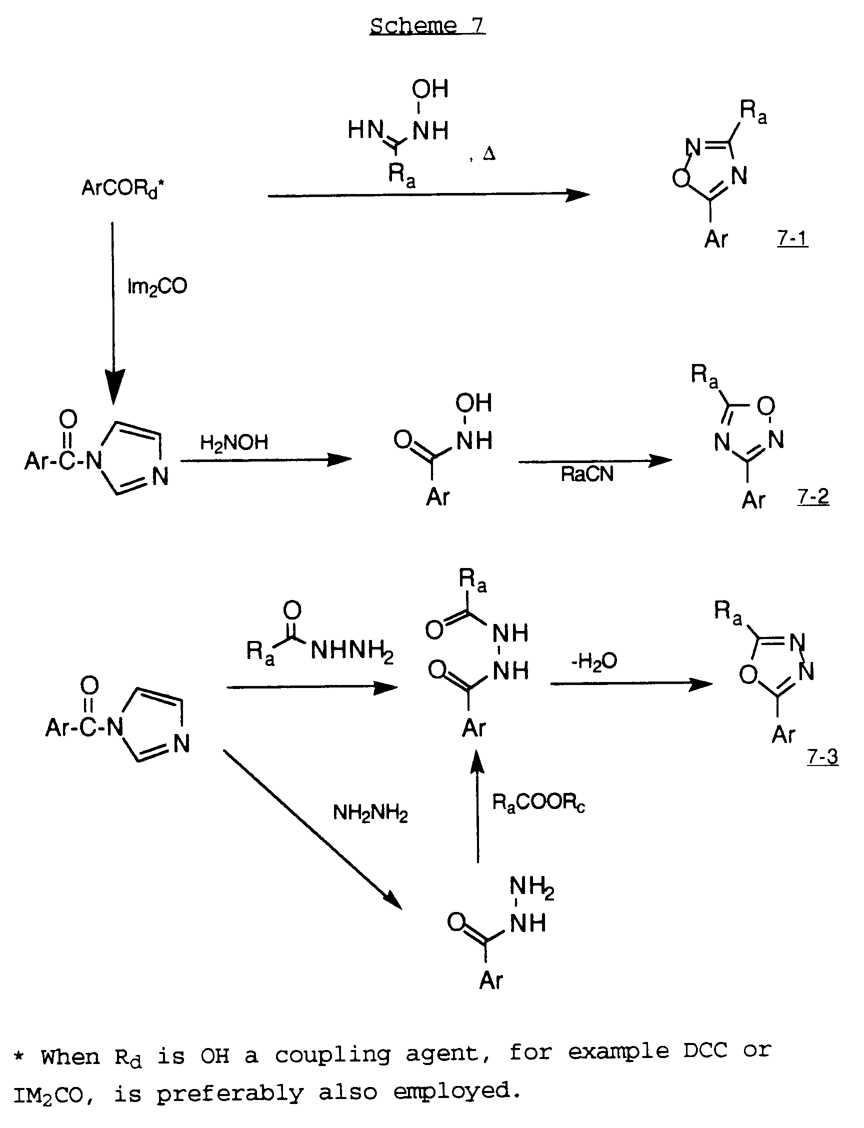

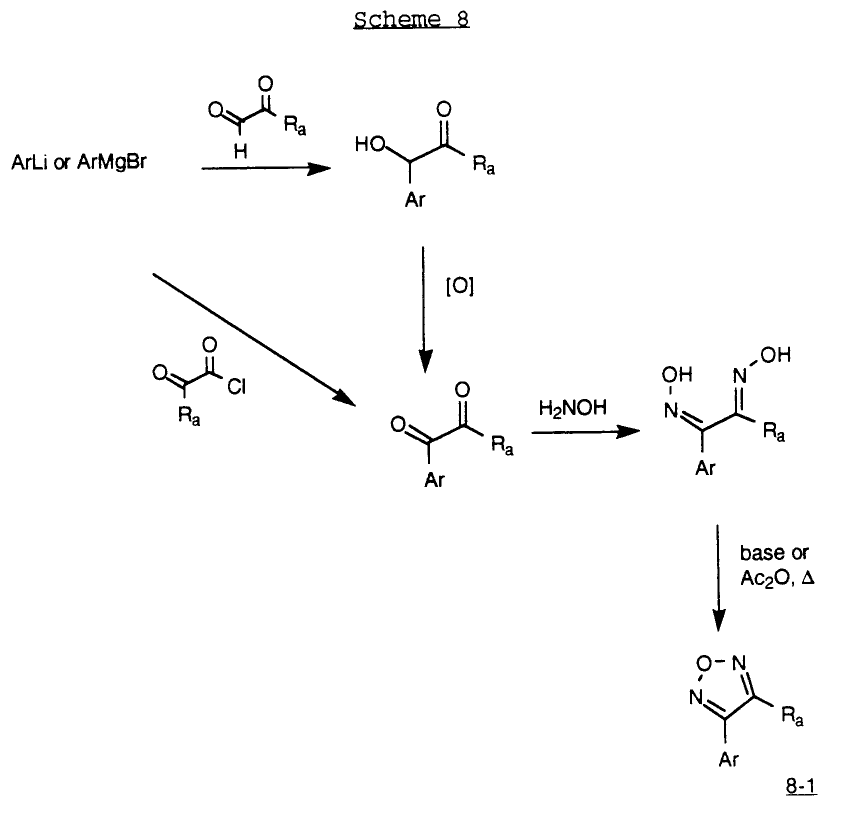

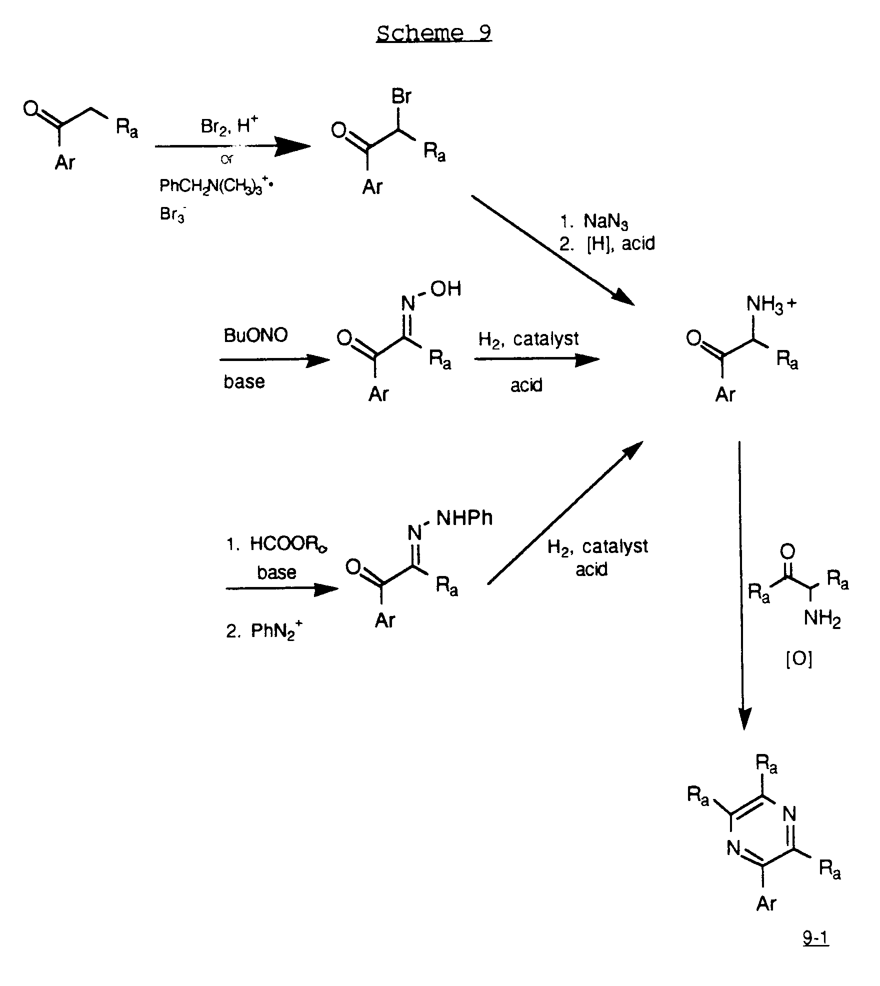

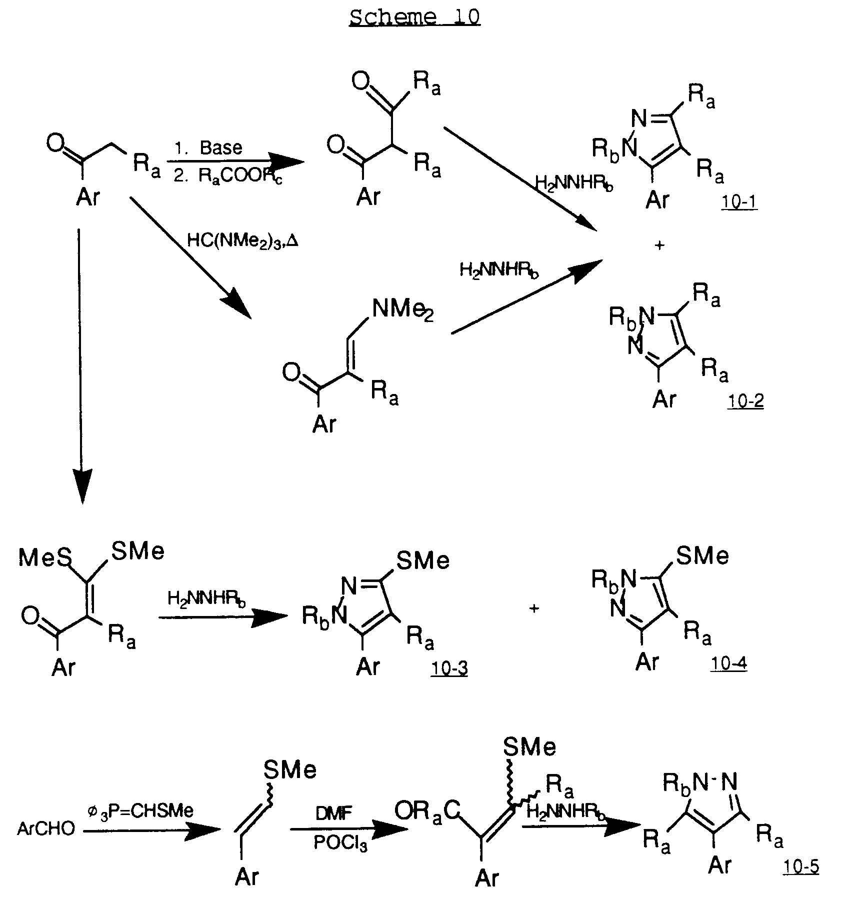

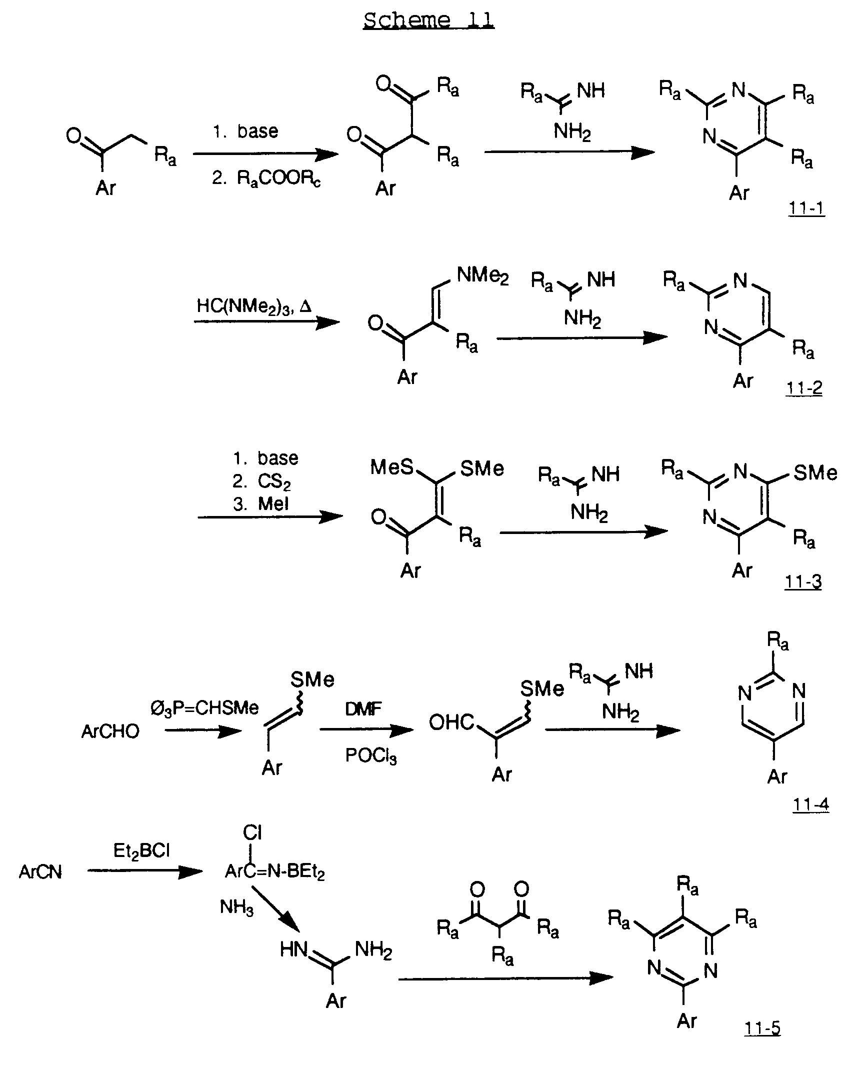

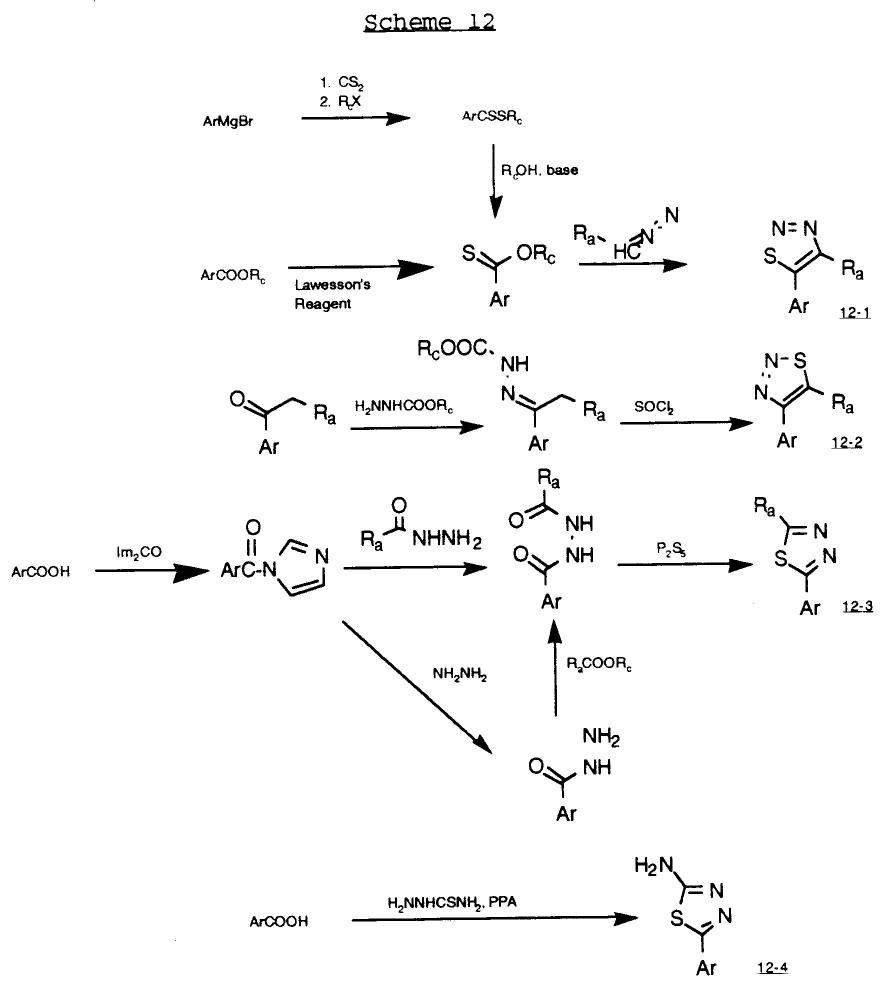

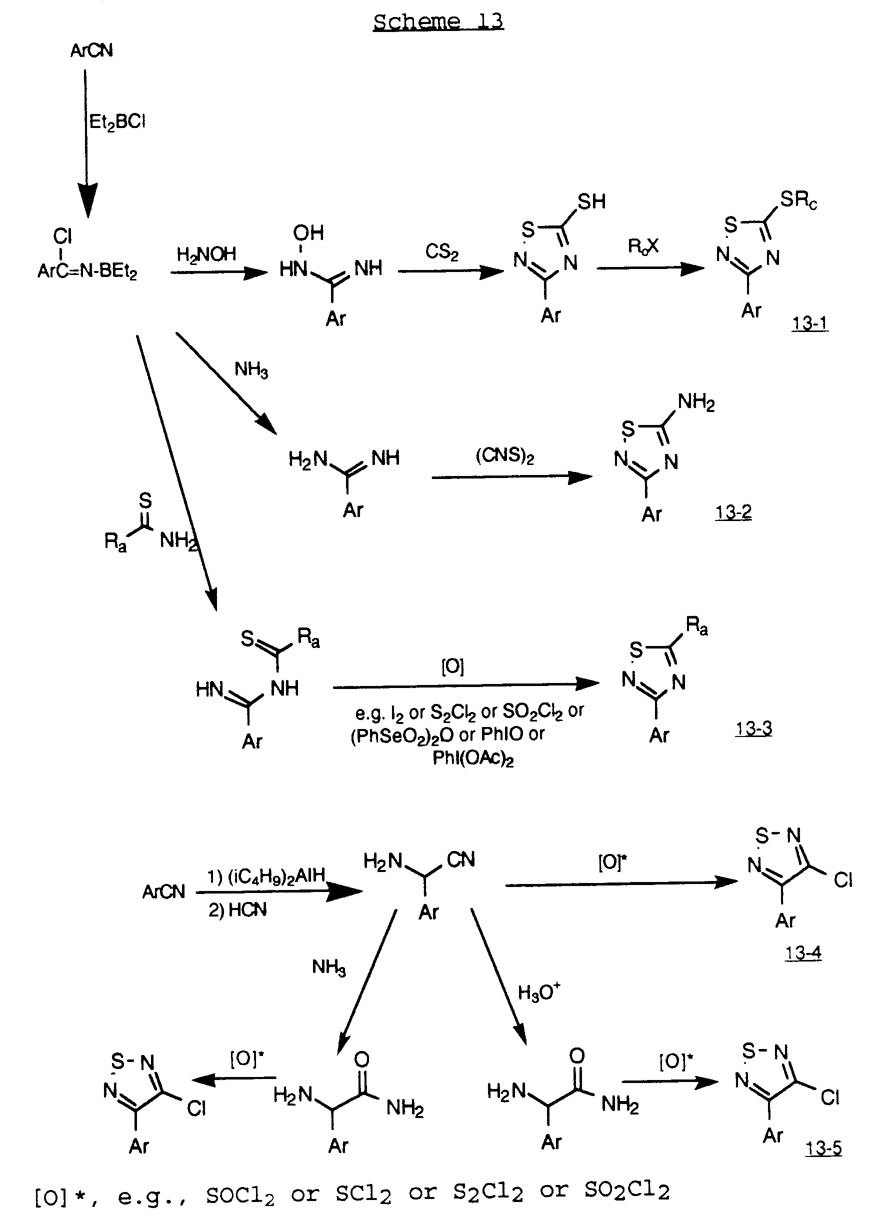

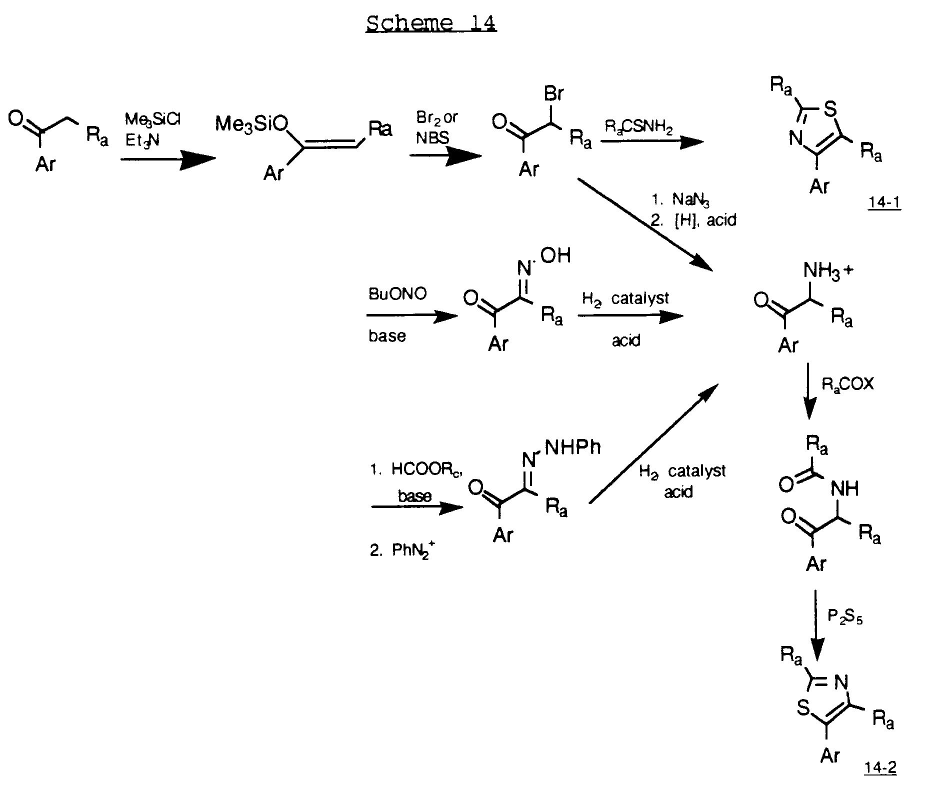

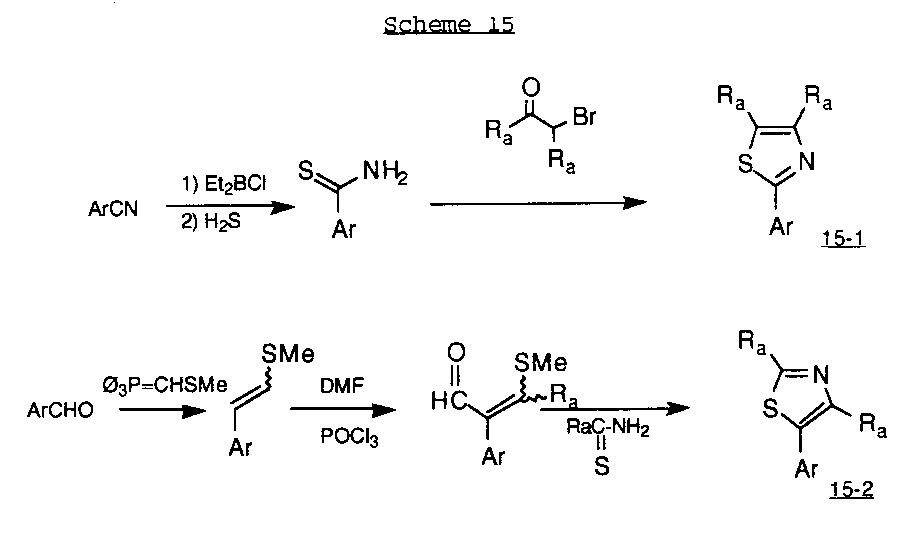

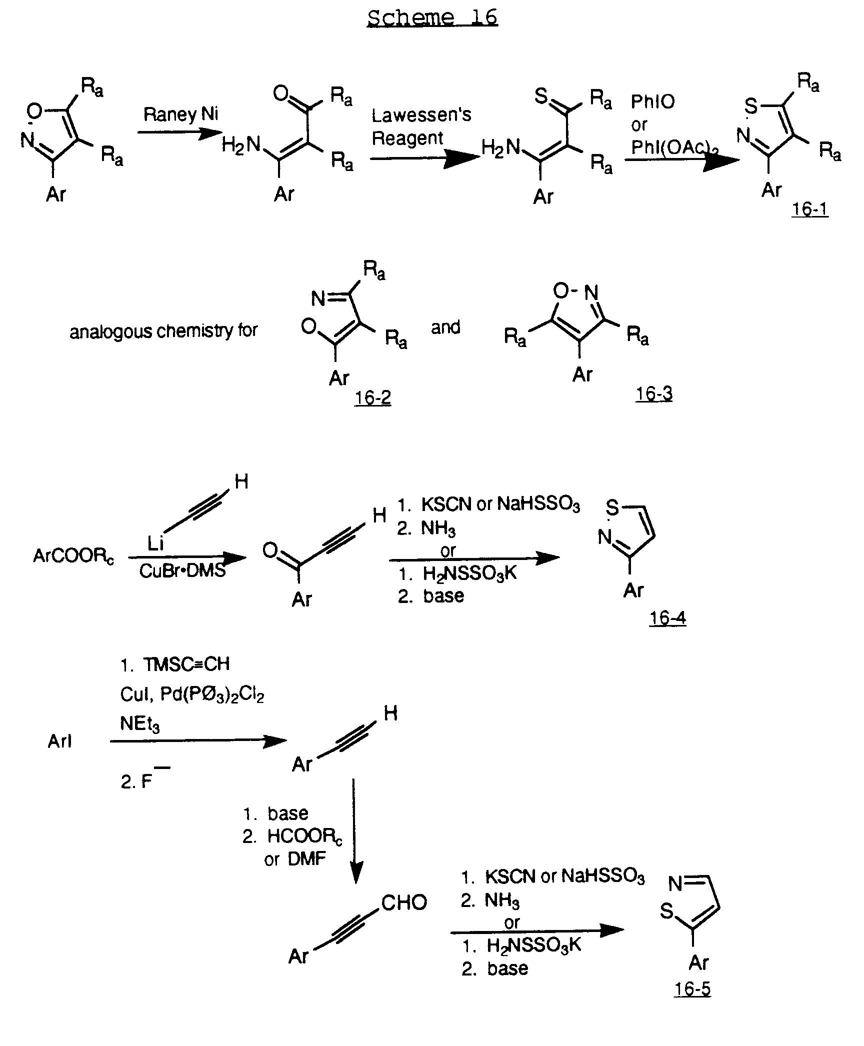

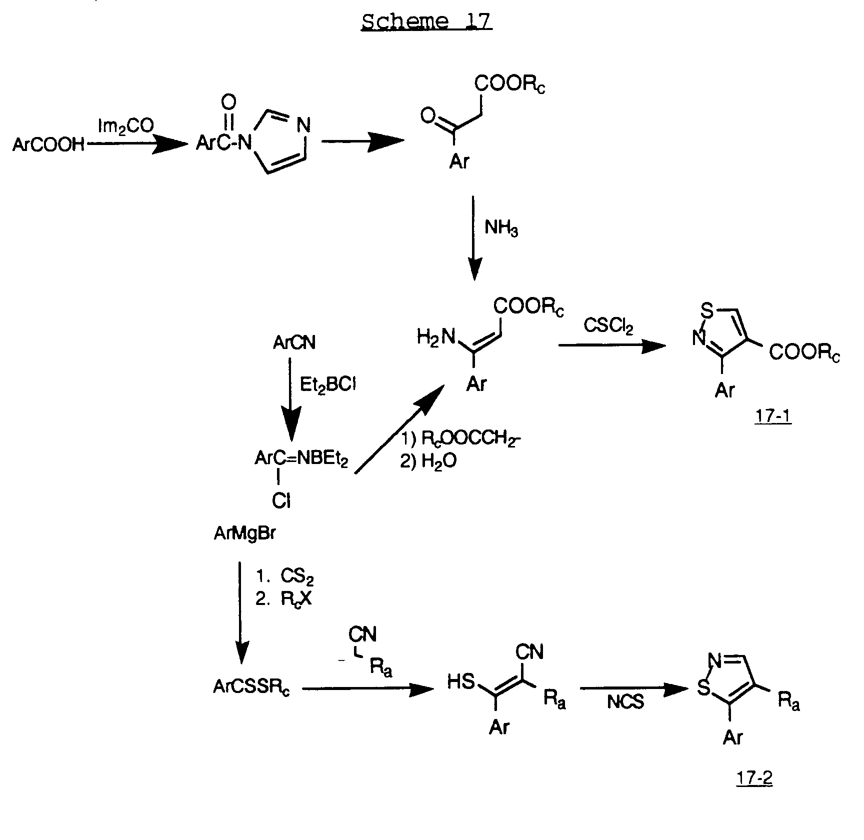

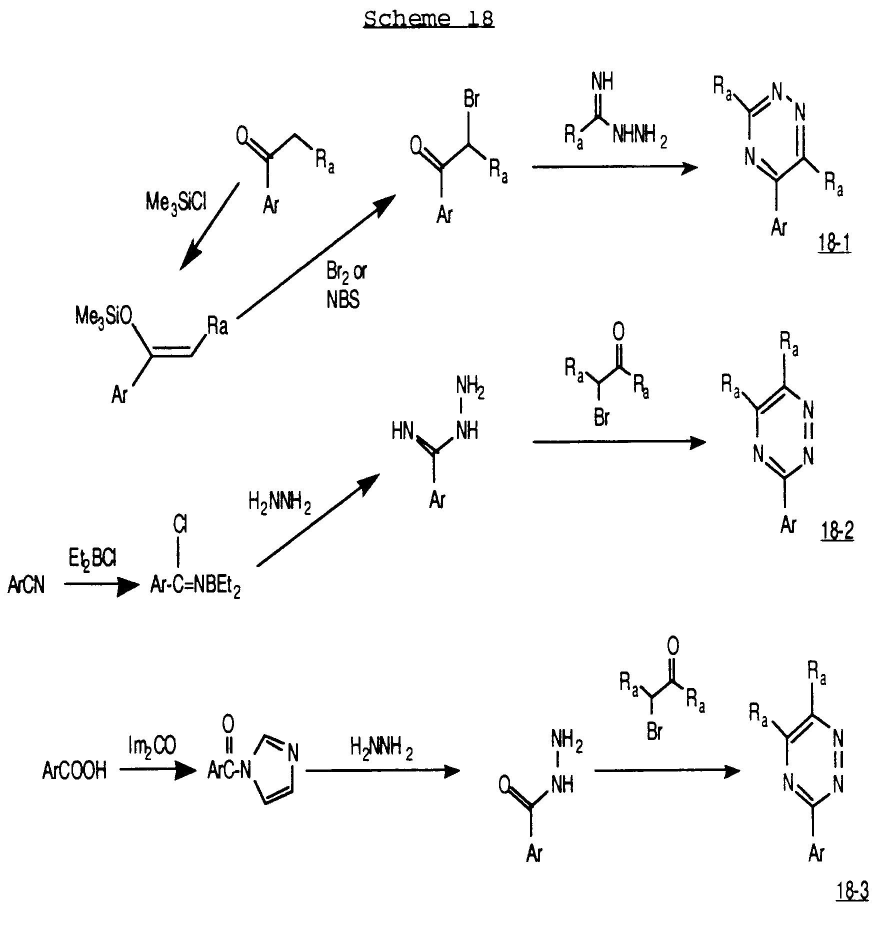

Scheme 19 illustrates a preparation of a starting material for reaction Scheme 1.

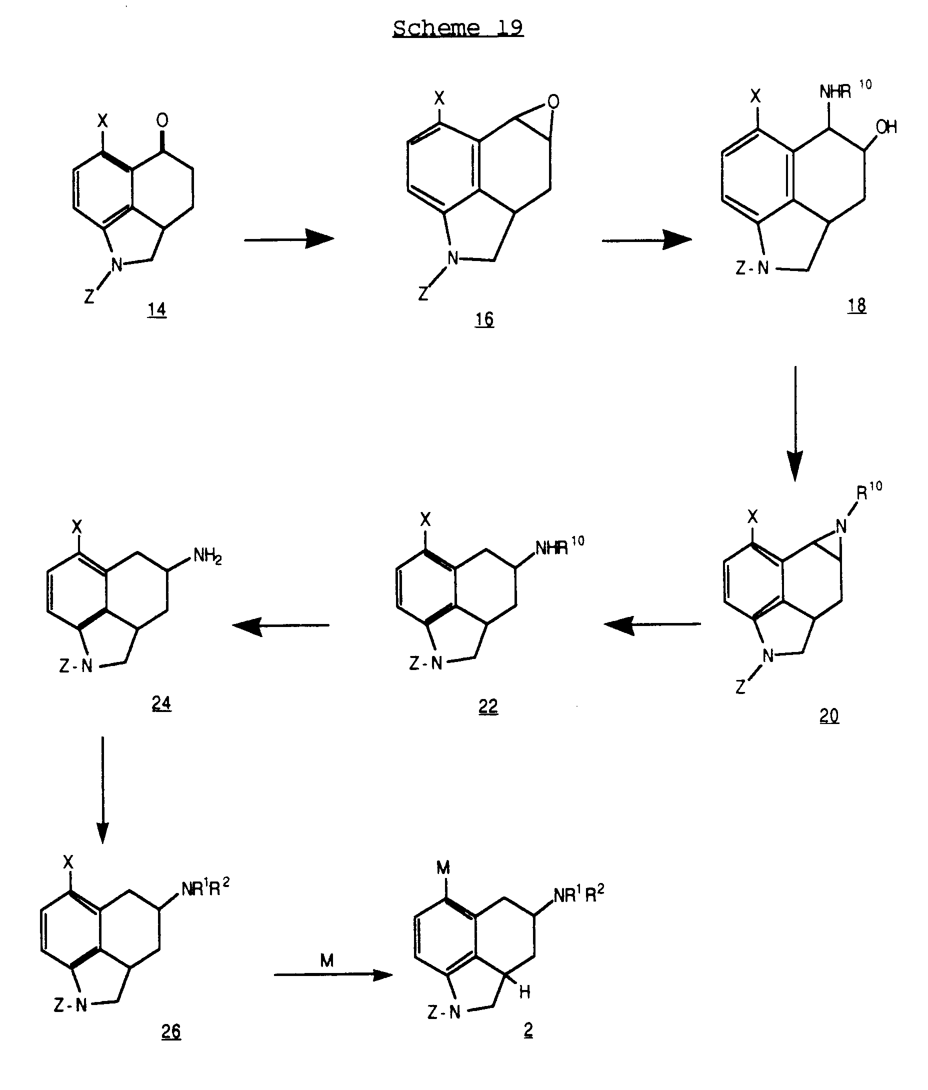

In Scheme 19, epoxides of Formula 16 are known to the art or can be prepared from compounds known to the art using common reagents and techniques. For example, Flaugh, et al., J. Med. Chem. , 31, 1746 (1988); Nichols et al., Org. Prep. and Proc., Int., 9, 277 (1977); and Leanna et al., Tet. Lett., 30, No. 30, 3935 (1989), teach methods of preparation of various embodiments of compounds of structures 16. Those skilled in the art of organic chemistry will recognize that there are four stereoisomers of structure 16: 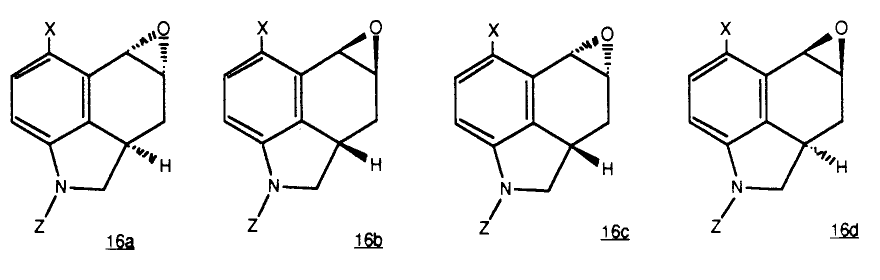 Structures 16a and 16b are herein referred to collectively as the exo-isomers; similarly, structures 16c and 16d are the endo-isomers. Leanna et al., supra, teach the preparation of epoxides of structures 16 which are substantially exo or substantially endo, as desired. The preferred starting material is the compound of structure 16 wherein Z is benzoyl and X is hydrogen; the most preferred starting material is the mixture of substantially the exo-isomers thereof.

Amino alcohols of structure 18 are formed by reacting an epoxide of structure 16 with an amine of formula R¹⁰NH₂

. Such amines are readily available. Opening of the epoxide ring proceeds substantially regiospecifically with the amino group at the 5-position and the hydroxyl group at the 4-position. The reaction is also stereospecific in the sense that stereoisomers of structure 18a-d are formed from, respectively, stereoisomers of structure 16a-d,

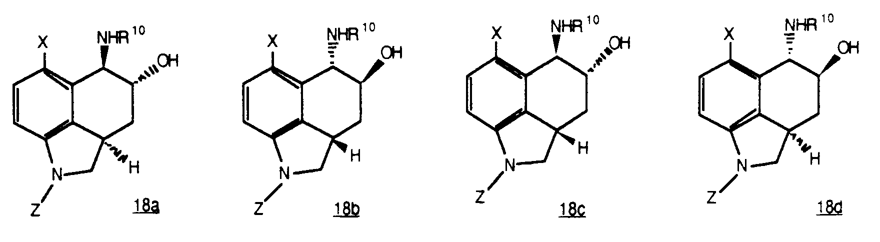

A stereoselective synthesis of the amino alcohol of structure 18, and hence of all the subsquent intermediates and products of Scheme 19, can be effected by using a substantially pure enantiomer of an amine of the formula R¹⁰NH₂ wherein R¹⁰ contains at least one chiral center. The diastereomers of the resulting amino alcohol can then be separated by a number of means known in the art, for example by chromatography or crystallization. Suitable solvents for recrystallization include those such as diethyl ether, n-butanol, and mixtures of hexane and ethyl acetate. An alternative method of achieving a stereospecific synthesis is depicted in Scheme 19 and comprises conversion of all the diastereomers of structure 18 to corresponding diastereomers of structure 20, followed by the separation of said diastereomers of structure 20; that alternative method is discussed below. If a stereoselective synthesis is not desired, then separation of the stereoisomers of the amino alcohol of structure 18 is not required and the amine R¹⁰NH₂ need not be optically active.

A particularly efficient stereoselective process for a highly preferred compound of structure 18, 1-benzoyl-4-hydroxy-5-(1-phenylethyl)amino-1,2,2a,3,4,5-hexahydrobenz[cd]indole, comprises the reaction of a mixture of substantially the exo-isomers of the corresponding epoxide of structure 16, or a mixture of substantially the endo-isomers of the corresponding epoxide of structure 16, with a substantially pure enantiomer of 1-phenethylamine in the solvent n-butanol and the subsequent selective crystallization of one of the two isomers of the amino alcohol. The temperature of the reaction can be from about 50° to about 150°C, preferably about 80° to about 100°C.

After the reaction is complete, as determined for example by thin layer chromatography or liquid chromatography, the desired amino alcohol is crystallized at about -20° to about 40°C; the preferred temperature for the crystallization is about 0° to about 15°C. Therefore this process has the valuable attribute that the reaction and the separation of stereoisomers occur efficiently in a single step. By the proper selection of the epoxide isomers, exo or endo, and the enantiomer of 1-phenyl-ethylamine, R or S, one can determine which of the stereoisomers of the compound of structure 18 precipitate from the reaction mixture.

A number of methods of forming aziridines such as those of structure 20 from amino alcohols such as those of Formula 18 are known to the art. Two examples are the use of diethyl azodicarboxylate and triphenylphosphine (O. Mitsunobu, Synthesis, January, 1981, page 1), and the use of bromine and triphenylphosphine (J. P. Freemer and P. J. Mondron, Synthesis, December, 1974, page 894).

A particularly efficient alternative to the above methods involving treating a compound of structure 18 with a tertiary amine in an inert solvent followed by the addition of methanesulfonyl chloride. The following stereoisomers of the aziridine of structure 20, 20a-d, arise respectively from the stereoisomers of structure 18a-d, with retention of configuration at any chiral center in the substituents Z, R¹⁰ or X: 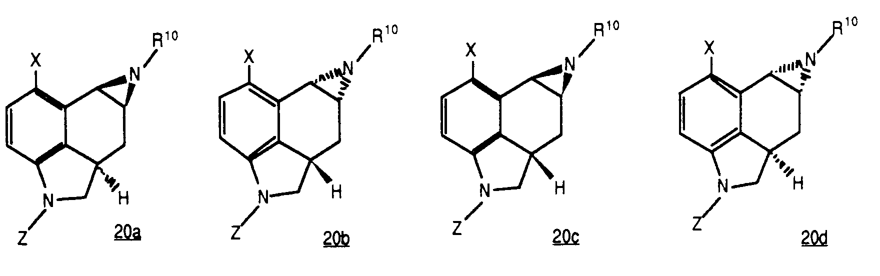 suitable tertiary amines are those of the formula (R¹¹)₃N, where the R¹¹ groups are independently C₁-C₄ alkyl. Suitable solvents are chlorinated hydrocarbons such as methylene chloride, chloroform, carbon tetrachloride, and dichloroethane; aromatic hydrocarbons such as benzene, toluene, and the xylenes; and ethers such as tetrahydrofuran, diethyl ether, and methyl t-butyl ether. The reaction can be conducted at a temperature from about -35° to about 45°C. In the preferred embodiment, the amino alcohol is treated with triethylamine in methylene chloride at about -20° to about 0°C, then the reaction mixture is warmed to about 15° to about 35°C for the completion of the reaction. If desired, the product, an aziridine of structure 20, can be crystallized from an appropriate solvent such as acetonitrile or isopropanol after an aqueous workup. In the event that Z contains at least one chiral center in substantially a single stereo-configuration and that the aziridine of structure 20 is prepared as a mixture of stereoisomers, said stereoisomers can be separated by methods such as chromatography and crystallization, thereby providing a stereospecific synthesis of the aziridine of structure 20 and subsequent products.

The aziridine ring can be opened to form an intermediate secondary amine of structure 22. A number of methods of opening aziridines are comonly known. It is, however, crucial that the method used for opening the aziridine to form a secondary amine of structure 22 be substantially regiospecific; the aziridine must be opened to form substantially the 4-amino compound rather than the 5-amino compound. One such method is catalytic hydrogenolysis as taught by Y. Sugi and S. Mitsui, Bull. Chem. Soc. Jap., 43, pp. 1489-1496 (1970). Catalysts which are suitable are the usual hydrogenation and hydrogenolysis catalysts, such as the noble metal catalysts; the preferred catalyst is palladium. Suitable solvents include hydrocarbons such as hexanes and heptanes; aromatic hydrocarbons such as benzene, toluene, xylenes, ethylbenzene, and t-butylbenzene; alcohols such as methanol, ethanol, and isopropanol; and mixtures of solvents such as acetic acid mixed with said alcohols. The preferred solvent for preparing the compound of structure 22, wherein Z is benzoyl, X is hydrogen, and R¹⁰ is 1-phenylethyl, is a mixture of methanol and phosphoric acid or acetic acid. The source of hydrogen can be an atmosphere of elemental hydrogen supplied at a pressure of about 1 atmosphere or higher, or the source of hydrogen can be compounds which are suitable to serve as hydrogen donors in a catalytic transfer hydrogenolysis reaction, such as formic acid, hydrazine, or cyclohexene. The preferred hydrogen source is an atmosphere of hydrogen gas supplied at about 1 to about 10 atmospheres pressure. The temperature of the reaction may be from about -20° to about 80°C; the preferred temperature for the hydrogenolysis of the aziridine wherein Z is benzoyl, X is hydrogen, and R¹⁰ is 1-phenylethyl is about -20° to about 0°C.

The conversion of compounds of structure 20 to compounds of structure 22 proceeds without disturbing the stereochemical configuration of the chiral centers at the 2a- or 4-positions of the structure 22 or of the chiral centers that may be present in any of the substituents.

If desired, the compound of structure 22 can be isolated by the usual methods such as crystallization. The secondary amine of structure 22 can be converted to a primary amine of structure 24 by a number of methods known to the art of organic chemistry, or alternatively the secondary amine itself can be isolated.

However, the preferred method is to convert the secondary amine of structure 22 to the primary amine of structure 24 without isolating the secondary amine, but rather by simply continuing without interruption the hydrogenolysis reaction that produced the compound of structure 22. Therefore, the preferred solvent and catalyst are the same as those for the preparation of the secondary amine of structure 22. It may be desirable to conduct the hydrogenolysis of the secondary amine of structure 22 at a different temperature or a different pressure or different temperature and pressure than the hydrogenolysis of the aziridine of structure 20. For the hydrogenolysis of the preferred compound of structure 22 wherein Z is benzoyl, X is hydrogen, and R¹⁰ is 1-phenylethyl, the preferred temperature and pressure are about 50° to about 60°C and about 1 to about 20 atmospheres. Under these condistions, he hydrogenolysis of compounds of structure 22 to compounds of structure 24 proceeds without disturbing the stereochemical configuration of the chiral center at the 4-postion.

The isolation of the compound of structure 24 can be accomplished by the usual methods such as crystallization. If desired, the compound of structure 24 can be further purified, for example by recrystallization.

Of course, as those skilled in the art will recognize, variations of Scheme 10 will be desirable or necessary for certain embodiments of the invention. For example, it may be undesirable to subject a compound in which X is halo to the catalytic hydrogenolysis steps of Scheme 19 because the undesired displacement of the halogen may compete with the desired hydrogenolysis of the carbon nitrogen bonds. One alternative strategy is to postpone the halogenation until after the hydrogenolysis. Another alternative strategy is to use a milder means of reduction that would leave the halogen in place. A third alternative, useful in the instance when the halogen is to serve as a leaving group, is to perform the desired displacement of halogen before the hydrogenolysis step.

Compounds of Formula 1 can be prepared from the compound of structure 24, whether it exists as a mixture of stereoisomers or as a substantially pure enantiomer, using common reagents and methods well known in the art. A preferred intermediate to the compounds of the instant invention is the 6-bromo-derivative. Preferably Z is an aminoblocking group such as benzoyl. A preferred method of introducing the bromo substituent at the 6-position is by reaction with bromine in glacial acetic acid, buffered with sodian acetate. Amino blocking groups can be added, if desired, to the 4-amino substituent using such methods as those disclosed by Greene, supra, and Barton, supra. Alkyl groups can be added, if desired, to the 4-amino substituent using such comon methods as amonolysis of the appropriate halide as discussed by Morrison and Boyd, Chapter 22, Organic Chemistry, Third Edition, Allyn and Bacon, Boston, 1973, to provide a compound of structure 26 wherein R¹ and R² are defined hereinabove. If desired, the benzoyl group can be removed from the 1-position using known methods and optionally replaced with other amino-protecting groups. Preferably the benzoyl group represented by Z is replaced with a triphenylmethyl group prior to the metallating step to form structure 2. The amino-protecting groups and alkyl groups can be added either before or after the bromination, as desired.

The 4-amino-6-bromohexahydrobenz[cd]indole starting materials used to prepare the compounds of the invention can be readily prepared by other processes such as depicted as Reaction Scheme 2 disclosed in United states Patent No. 4,576,959 of Flaugh, incorporated herein by reference in its entirety.

The procedure of Scheme 19 using the 4,5-epoxide provides a convenient way to prepare the optically active isomers of the compounds of the present invention. Such isomers can also be isolated by resolving racemic mixtures. This resolution can be carried out in the presence of a resolving agent, by chromatography or by repeated crystallization. Particularly useful resolving agents are d- and l-tartaric acids, d- and l-ditoluoyltartaric acids, and the like.

The methods of preparation described in Schemes 2-18 provide compounds in which the heteroaromatic ring may or may not be substituted. The general reactions provided below set forth methodology for incorporating, interconverting, and removing substituents on the heteroaromatic ring. Additional methods for performing these transformations are cited in Comprehensive Organic Transformations By Richard C. Larock, VCH Publishers, Inc., New York (1989) which is incorporated herein by reference. "HET" refers to the heteroaromatic attached to the hexahydrobenz[cd]indole at position C-6.

### 1. Halogen substituents (X) :

$\text{HET-OH → HET-X}$POX₃, PX₃, SOX₂, PPh₃·X₂, or P(OR)₃·X₂ ${\text{HET-NH}}_{\text{2}} \text{→ HET-X}$1. HONO; 2. CuX, or KI, or HBF₄, △

### 2. O(C₁ - C₃ alkyl), i.e., [OR]

$\text{HET-X → HET-OR}$RO-, CuI, (DMF, or DMAc, or NMP), △ $\text{HET-OH → HET-OR}$Base, RX; or CH₂N₂

### 3. Hydroxy substituent:

${\text{HET-NH}}_{\text{2}} \text{→ HET-OH}$1.HONO; 2. H₃O+, △ $\text{HET-OMe → HET-OH}$48% HBr, △; or BBr₃

### 4. Cyano substituent:

${\text{HET-NH}}_{\text{2}} \text{→ HET-CN}$1. HONO; 2. CuCN $\text{HET-X → HET-CN}$CuCN, (DMF, or DMAc, or NMP), △; or CN⁻, △

### 5. S(C₁ - C₃ alkyl); i.e., [SR]

${\text{HET-NH}}_{\text{2}} \text{→ HET-SR}$1. HONO; 2. RSH, base $\text{HET-X → HET-SR}$RS⁻, CuI, (DMF, or DMAc, or NMP), △

### 6. Amino substituent:

${\text{HET-NO}}_{\text{2}} {\text{→ HET-NH}}_{\text{2}}$H₂, catalyst (i.e., Pt or Pd)

### 7. Hydrogen substituent:

$\text{HET-X → HET-H}$H₂, catalyst; or R₃SnH, 2,2'-azobis(2-methyl)propionitrile), △ $\text{HET-OH → HET-H}$1. 5-chloro-1-phenyltetrazole, 2. H₂, catalyst ${\text{HET-NH}}_{\text{2}} \text{→ HET-H}$1. HONO, 2. H₃PO₂ ${\text{HET-CH}}_{\text{2}} \text{Ph → HET-H}$H₂, catalyst (ie Pd) (This applies if the benzyl group is attached to a nitrogen in the heterocyclic ring.) $\text{HET-SR → HET-H}$Raney Ni

6-acyl-substituted-hexahydrobenz[cd]indoles are preferred intermediates in the preparation of certain of the compounds of the instant invention, particularly 6-isoxazole-indoles and 6-pyrazole-indoles. The 6-acyl substituted indolines can be prepared by several routes using the 6-iodo-substituted indolines of structure 30 as depicted in Scheme 20 where R¹, R² and Z are as defined hereinabove.

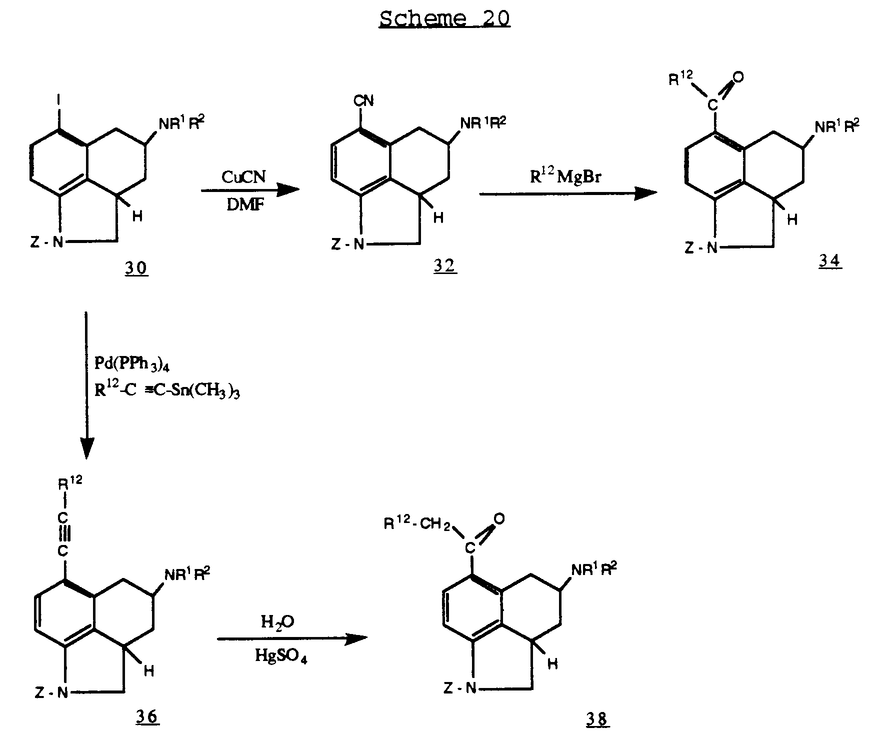

In a preferred method of preparation as depicted in Scheme 20, the nitrile 32 is contacted with an organometallic reagent such as a Grignard reagent under standard conditions to provide the 6-acyl derivative 34. For this reaction Z is preferably benzoyl or trityl. Alternatively, a 6-alkyne intermediate of structure 36 can be prepared and then hydrolyzed to provide the acyl derivative 38. This method provides a methylene group adjacent to the carbonyl group. In this method Z can be an amino protecting group such as benzoyl although the unprotected 1-nitrogen is preferred, i.e., Z is hydrogen. Compounds of structure 30 can be contacted with a palladian catalyst Pd(PPh₃)₄ [where Ph is phenyl] and the tin alkyne compound R¹²-C≡C-Sn(CH₃)₃ wherein R¹² is a C₁-C₇ alkyl, substituted C₁-C₇ alkyl, aryl (C₁-C₃ alkyl), substituted aryl (C₁-C₃ alkyl), or C₃-C₇ cycloalkyl. This reaction is normally conducted in a solvent such as toluene at an elevated temperature, for example at about 100°C. Typically an excess of the tin alkyne is used along with about 0.25 equivalents of the palladium compound based on compound 30. The 6-alkyne 36 is then contacted with HgSO₄ in water to provide the ketone 38.

In another preparation method depicted in Scheme 21, the 6-iodo derivative 30 can be used to prepare certain 6-acyl compounds directly. This is accomplished by contacting the 6-iodo compound with a trialkyltinalkyl complex and carbon monoxide in the presence of a palladium catalyst Pd(PPh₃)₄ [where Ph is phenyl] as described in the literature for arylhalides. [A. Schoenberg and R. F. Heck, J. Org. Chem., 39, p. 3327 (1974); and A. Schoenberg, I. Bartoletti, and R. F. Heck, J. Org. Chem., 39, p. 3318 (1974)]. Although a blocking group Z such as diethylcarbamoyl can be used for this method, the method can also be accomplished when Z is hydrogen or the blocking group can be removed to provide compounds of structure 40 where R¹, R² and R¹² are as defined above.

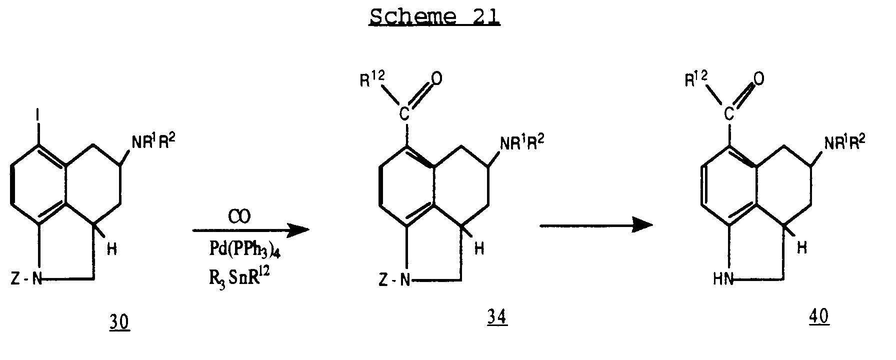

The following examples further illustrate the preparation of the compounds of this invention. The examples are provided for purposes of illustration only and are not to be construed as limiting the scope of the instant invention in any way.

The terms and abbreviations used in the instant examples have their normal meaning unless otherwise designated, for example, "°C" refers to degrees celsius; "N" refers to normal or normality; "mmol" referes to millimole; "g" refers to gram; "mL" means milliliter; "M" refers to molar; "min" refers to minutes; "hr" refers to hours; "NMR" refers to nuclear magnetic resonance; "IR" refers to infrared spectroscopy; "U.V. " refers to ultraviolet spectroscopy; and "MS " refers to mass spectrometry.

### Example 1

### A. Preparation of (±)-1-Benzoyl-6-cyano-4-(di-n-propylamino)-1,2,2a,3,4,5-hexahydrobenz[c,d]indole

To a solution of (±)-1-benzoyl-6-bromo-4-(di-n-propyl-amino)hexahydrobenz[cd]indole (5.5 g, 12.5 mol) in DMF (100 mL) under a N₂ atmosphere was added 3.4g (37.5 mol) of CuCN and 7.1 g (37.5 mol) of Cul. The reaction mixture was then stirred at 140°C. for 6 hr. The reaction mixture was poured onto ice, diluted with water, CH₂Cl₂ added and stirred for 30 minutes. The mixture was filtered through a Celite pad and the filtrate was extracted twice with CH₂Cl₂. The organic solution was washed twice with saturated NaCl solution. The CH₂Cl₂ solution was dried over MgSO₄ and then evaporated to provide 4 g of a solid. Chromatography of this crude product over silica gel with 1:19 MeOH/CH₂Cl₂ as eluent gave 3 g (62%) of product.
mp = 122-124°C.

### B. Preparation of (-)(2aR,4S)-1-Benzoyl-6-cyano-4-(di-n-propylamino)-1,2,2a,3,4,5-hexahydrobenz[cd]indole.

To a solution of (-)6-bromo compound (30.0 g; 0.068 mol) in 500 ml of DMF was added CuCN (18.3 g; 0.2 mol) and Cul (38.0 g; 0.2 mol). The reaction mixture was then stirred at 140°C for 6 hr. The reaction mixture was poured into 4L of water. The ppt was collected and washed several times with water. The ppt was suspended in dil NH₄OH and slurred with ethyl acetate. The whole mixture was filtered through a celite pad. The ethyl acetate solution was separated and washed with brine solution. The ethyl acetate solution was dried (MgSO₄) and concentrated to dryness to provide 21.3 g of the (-)-6-nitrile.

### C. Preparation of (+)(2aS,4R)-6-cyano counterpart of Example 1B.

In a similar manner as in Example 1B above, the (+)-6-bromo compound (17.1 g, 0.039 mol) was contacted with CuCN (10.75 g; 0.12 mol) and Cul (22.8 g; 0.12 mol) in 300 ml DMF to give 11.6 g of (+)-6-cyano compound.

### Example 2

### Preparation of (±)-6-cyano-4-(di-n-propylamino)-1,2,2a,3,4,5-hexahydrobenz[cd]indole.

To a stirred solution of 4.8 g (0.0124 mol) of (±)-1-benzoyl-6-cyano-4-(di-n-propylamino)-1,2,2a,3,4,5-hexahydrobenz[cd]indole in 200 mL of THF cooled to -78°C under a N₂ atmosphere were added 16 mL (0.025 mol) of 1.6 M solution of n-butyl lithium in hexane. The reaction mixture was stirred at -78° C. for 30 minutes and then allowed to warm to -20° C. To the reaction mixture was added 100 mL of 1N HCl. The mixture was extracted once with ethyl ether. The acidic solution was made alkaline with the addition of cold 5N NaOH. The basic mixture was extracted twice with CH₂Cl₂. The combined organic solution was washed with saturated NaCl solution. The CH₂Cl₂ solution was dried over MgSO₄ and evaporated to give 4 g of an oil. Chromatogrphy of this oil over silica gel with ethyl acetate as eluent gave 3 g (85%) of product as an oil which upon standing solidified.

### Example 3

### Prepration of (+)(2aS,4R)-1-trityl-6-cyano-4-(di-n-propylamino)-1,2,2a,3,4,5-hexahydrobenz[cd]indole.

To a solution of (+)(2aS,4R)-6-cyano-4-(di-n-propylamino)-1,2,2a,3,4,5-hexahydrobenz[cd]indole (12.8 g, 0.045 mol) and triethylamine (4.5 g, 0.045 mol) in 400 mL of methylene chloride was added a solution of triphenylmethyl chloride (trityl chloride) (12.6 g, 0.045 mol) in 100 mL of methylene chloride dropwise at RT. The reaction mixture was stirred for 16 hr at RT. The reaction mixture was extracted water and cold 1N HCl. The organic solution was washed with saturated NaHCO₃ solution and with saturated brine solution. The organic layer was dried (MgSO₄) and concentrated to dryness *in vacu*o to give a residue. The residue was slurried with warm hexanes, cooled and filtered to remove insolubles. The filtrate was concentrated to an oil. The oil was chromatographed (silica gel, 20% ethyl acetate in hexanes) to provide 20.6 g of the (+)-trityl nitrile.

### Example 4

### Preparation of (+)(2aS,4R)-6-acetyl-4-(di-n-propylamino)-1,2,2a,3,4,5-hexahydrobenz[cd]indole.

A solution of 2.4 g (4.6 mol) (+)-1-trityl-6-cyano-4-(di-n-propylamino)-1,2,2a,3,4,5-hexahydrobenz[cd]indole in 100 mL of THF was treated with 25 mL of 2.0M methylmagnesium bromide in diethyl ether. The reaction mixture was refluxed for 16 hr. The reaction mixture was cooled and excess Grignard reagent was decomposed with addition of saturated NH₄Cl solution. The reaction mixture was extracted with ethyl acetate. The organic solution was evaporated to an oil. The oil was dissolved in 25 mL of 5N HCl and the solution was stirred at room temperature for 30 min. The acidic solution was made alkaline with the addition of excess concentrated NH₄OH solution. The basic mixture was extracted twice with ethyl acetate. The combined organic solution was washed once with saturated NaCl solution and dried over MgSO₄. The ethyl acetate solution was evaporated to yield 1.4 g of an oil. Chromatography of this oil over silicia gel with ethyl acetate as eluent gave 1.2 g (87%) of product. Recrystallization from hexanes yielded 840 mg of the product (+) ketone.
mp = 121-122°C

### Example 5

### Preparation of (±)-6-Acetyl-4-(di-n-propylamino)-1,2,2a,3,4,5-hexahydrobenz[cd]indole.

A solution of 0.5 g (1.8 mol) of (±)-6-cyano-4-(di-n-propylamino)-1,2,2a,3,4,5-hexahydrobenz[cd]indole in 75 mL of benzene was treated with 5 mL of 2.0M methymagnesium bromide in diethyl ether. The reaction mixture was refluxed for 2 days. The reaction mixture was cooled and excess Grignard reagent was decomposed with addition of saturated NH₄Cl solution. The benzene layer was separated and washed once with saturated NaCl solution. The organic solution was evaporated to an oil. The oil was dissolved in 25 mL of 5N HCl and the solution was stirred at room temperature for 30 min. The acidic solution was made alkaline with the addition of excess concentrated NH₄OH solution. The basic mixture was extracted twice with CH₂Cl₂. The combined organic solution was washed once with saturated NaCl solution and dried over MgSO₄. The CH₂Cl₂ solution was evaporated to yield 0.5 g of an oil. Chromatography of this oil over silicia gel with ethyl acetate as eluent gave 0.4 g (75%) of product as an oil which upon standing solidified.
mp = 76-77° C

### Example 6

### Preparation of (+)(2aS,4R)-6-(3-pyrazolyl)-4-(di-n-propylamino)-1,2,2a,3,4,5-hexahydrobenz[cd]indole·2HCL.

A solution of (+)-1-triphenymethyl-6-acetyl-4-(di-n-propylamino)-1,2,2a,3,4,5-hexahydrobenz[cd]indole (1.67 g, 3 mol) and 3 mL of tris(dimethylamino)methane in 50 mL of toluene was refluxed for 5 hr. The reaction was concentrated *in vacu*o and the residue was dissolved in 100 mL of CH₃OH. To the CH₃OH solution was 2 mL of 85% hydrazine and the reaction mixture was stirred at RT for 16 hours. To the reaction mixture was added 50 ml of 1N HCl and stirred for an additional 1 hr. The solution was concentrated in vacuo to remove CH₃OH and the acidic solution was extracted with ethyl acetate. The acidic solution was separated and made alkaline with addition of excess concentrated NH₄OH. The basic mixture was extracted with ethyl acetate. The ethyl acetate solution was washed with brine solution, dried (MgSO₄) and concentrated *in vacuo* provide 900 mg of an oil. The crude product was chromatographed through silica gel (flash column, ethyl acetate) to yield 700 mg of pyrazole compound. The oil was dissolved in 50 mL of CH₃OH and 2 equivalents of 0.1 N HCL was added to the solution. The solution was concentrated *in vacuo* and the residue was crystallized from ethanol/ethyl ether. Yield - 400 mg
mp = 260 d
MS m/e 324(FD)

| Analysis calculated for C₂₀H₂₈N₄·2HCl | | | |
|---|---|---|---|
| Theory: | C, 60.45; | H, 7.61; | N, 14.1; |
| Found: | C, 60.21; | H, 7.60; | N, 14.26. |

### Example 7

### Preparation of (±)-6-(5-isoxazolyl)-4-(di-n-propylamino)-1,2,2a,3,4,5-hexahydrobenz[cd]indole·2HCl.

To a solution of (±)-6-acetyl-4- (di-n-propylamino)-1,2,2a,3,4,5-hexahydrobenz[cd]indole (2.3 g, 7.7 mol) and triethylamine (1.1 ml, 8 mol) in 90 ml CH₂Cl₂ under N₂ was added dropwise a solution of 2,2,2-trichloroethyl chloroformate. The reaction mixture was stirred at RT for 1 hr. The CH₂Cl₂ solution was extracted with water and 1N HCl. The organic solution was washed with saturated NaHCO₃ solution and with brine solution. The CH₂Cl₂ solution was dried (MgSO₄) and concentrated *in vacu*o to give 3.3 g of the 1-carbamylindoline.

A solution of this 1-carbamylindoline (3.3 g, 7.7 mol) and tris(dimethylamino)-methane (5 mL) in 70 mL of toluene was stirred at reflux for 16 hr. The reaction mixture was concentrated to dryness *in vacuo*. The residue was dissolved in 50 mL of acetic acid and hydroxylamine hydrochloride (2.5 g, 36 mol) was added. The reaction mixture was stirred at RT for 16 hr and then concentrated *in vacuo* to dryness. The residue was suspended in water and excess concentrated NH₄OH was added to the mixture. The basic mixture was extracted with CH₂Cl₂. The organic solution was washed with brine solution, dried (MgSO₄) and concentrated *in vacuo* to give 3.1 g of an oil. The crude product was chromatographed (flash column, silical gel 20% hexanes in ethyl acetate) to yield 2.0 g of (±)-1-carbamyl-6-isoxazolylindoline.

This isoxazole carbamate was dissolved in 20 mL of acetic acid and 1 g of zinc dust was added at once. The reaction mixture was stirred at RT for 4 hr. The reaction mixture was filtered through a celite pad and the filtrate was concentrated to dryness *in vacuo*. The residue was suspended in saturated NaHCO₃ solution and extracted with CH₂Cl₂. The organic solution was washed with brine solution, dried (MgSO₄) and concentrated to an oil. The crude material was chromatographed (flash column, silica gel, ethyl acetate) to give 500 mg of isoxazole indoline. The product was dissolved in 50 mL of CH₃OH and 2 equivalents of 0.1N HCl were added. The solution was concentrated to dryness and the residue was crystallized from ethanol/ethyl ether to give 85 mg of isoxazole substituted product as the dihydrochloride.
mp = 226°C d
MS m/e 325(FD)

| Analysis calculated for C₂₀H₂₇N₃O·2HCl | | | |
|---|---|---|---|
| Theory: | C, 60.30; | H, 7.34; | N, 10.55; |
| Found: | C, 58.83; | H, 7.18; | N, 10.01. |

### Example 8

### Preparation of (+)(2aS,4R)-6-(3-isoxazolyl)-4-(di-n-propylamino)-1,2,2a,3,4,5-hexahydrobenz[cd]indole · 2 HCl.

A solution of (+)-1-triphenylmethyl-6-acetyl-4-(di-n-propylamino)-1,2,2a,3,4,5-hexahydrobenz[cd]indole (3.33 g, 6 mol), 5 g hydroxylamine hydrochloride, 20 mL pyridine and 30 mL of ethanol was refluxed for 16 hr. The reaction mixture was concentrated to dryness *in vacu*o and the residue was dissolved in 5N HCl. The acidic mixture was extracted with ethyl acetate. The acidic solution was made alkaline with excess NH₄OH solution and extracted with ethyl acetate. The ethyl acetate solution was washed with brine solution, dried (MgSO₄) and concentrated *in vacuo* to give 1.5 g of crude product which was chromatographed (flash column, silica gel, ethyl acetate) give to 1.2 g of oxime.
mp = 129-130°C.

To a solution of this oxime (1.2 g, 3.8 mol) in 100 mL of THF cooled to -5°C under a N₂ atmosphere was added 7.5 mL n-butyllithium (1.6 M in hexanes) dropwise with stirring. The reaction mixture was stirred with continued cooling for 1 hr. To the reaction mixture was added 2 mL (26 mol) of DMF at once and then stirred for 1 hr at RT. The reaction mixture was poured into 50 mL of 1N H₂SO₄ and the acidic solution was warmed on a steam bath for 1 hr. The acidic solution was cooled, extracted with ethyl ether, and then made alkaline with excess 5N NaOH. The basic mixture was extracted with ethyl acetate. The organic was layer was washed with brine solution, dried (MgSO₄) and concentrated *in vacu*o to give 1 g of an oil. The oil was chromatographed (flash column, silica gel, ethyl acetate) to yield 500 mg of product as an oil. The oil was dissolved in 50 mL of CH₃OH and 2 equivalents of 0.1N HCL was added. The solution was concentrated to dryness *in vacuo* and the residue was crystallized from ethanol/ethyl ether. Crystallization gave 300 mg of the dihydrochloride of the 6-isoxazolyl product.
mp = 215°C d
MS m/e 325(FD)

### Example 9

### Preparation of (±)-1-benzoyl-6-[4-(2-aminothiazolyl)]-4-(di-n-propylamino)-1,2,2a,3,4,5-hexahydrobenz[cd]indole.

To a solution of (±)-6-acetyl-4-(di-n-propylamino)-1,2,2a,3,4,5-hexahydrobenz[cd]indole (205 mg, 0.7 mol) and triethyl amine (81 mg, 0.8 mol) in 20 mL of CH₂Cl₂ was added a solution of benzoyl chloride (112 mg, 0.8 mol) in 20 mL of CH₂Cl₂. The reaction mixture was stirred at RT for 2 hr. The reaction mixture was sucessively washed with water, saturated NaHCO₃ solution, brine solution and dried (MgSO₄). The organic layer was concentrated to dryness *in vacuo* to give 200 mg of the 1-benzoyl derivative.

A solution of this N-benzoyl compound (200 mg, 0.5 mol) in 20 mL of acetic acid was saturated with HBr(gas). To the solution was added dropwise a solution of bromine (0.2 mL) in 5 mL of acetic acid. The reaction was stirred at RT for 30 min and then concentrated to dryness *in vacuo*. The residue was dissolved in 30 mL of ethanol then 500 mg of thiourea were added and the mixture refluxed for 16 hr. The reaction was concentrated to dryness *in vacuo* and the residue dissolved in water. The acidic solution was made alkaline with the addition of excess concentrated NH₄OH. The basic mixture was extracted with CH₂Cl₂. The organic solution was washed with brine solution, dried (MgSO₄) and evaporated to dryness to give 200 mg of an oil. The oil was chromatographed (flash column, silica gel, ethyl acetate) to provide 140 mg of the named 6-aminothiazolyl compound.
MS m/e 460(FD)

### Example 10

### Preparation of (+)(2aS,4R)-6-(5-isoxazolyl)-4-(di-n-propylamino)-1,2,2a,3,4,5-hexahydrobenz[cd]indole · 2 HCl

To a solution of (+)(2aS,4R)-6-acetyl-4-(di-n-propylamino)-1,2,2a,3,4,5-hexahydrobenz[cd]indole (1.7 g, 5.7 mol) and triethylamine (0.8 ml, 6 mol) in 90 ml CH₂Cl₂ was added dropwise a solution of 2,2,2-trichloroethylchloroformate (1.3 g, 6 mol) in 10 ml CH₂Cl₂. The reaction mixture was stirred at room temperature for one hour and then extracted with water and 1N HCl. The organic solution was washed with a saturated NaHCO₃ solution, a saturated brine solution, dried over MgSO₄ and then concentrated to dryness in vacuo to give 2.5 g of the 1-carbamylindoline.

A solution of the 1-carbamylindoline (2.5 g, 5.7 mol) and tris(dimethylamino)methane (5 ml) in 100 ml of toluene was stirred at reflux for 16 hours. After 16 hours the reaction mixture was concentrated to dryness in vacuo. The resulting residue was dissolved in 50 ml of acetic acid and 1.5 g (22 mol) of a hydroxylamine hydrochloride solution were added. The resulting reaction mixture was stirred at room temperature for 16 hours and then concentrated to dryness in vacuo. The resulting residue was suspended in water and an excess of a concentrated NH₄OH solution was added to basicify the mixture. The basic mixture was then extracted with CH₂Cl₂ and the resulting organic extract was washed with a saturated brine solution, dried over MgSO₄ and then concentrated in vacuo to give 2.1 g of an oil. This oil was chromatographed (flash column, silica gel, EtOAc) to yield 1.9 g of (+)(2aS,4R)-6-isoxazolylindoline. The above compound was dissolved in 30 ml of acetic acid and 1.5 g of zinc dust were added all at once. The resulting reaction mixture was stirred at room temperature for four hours and then filtered through a celite pad. The filtrate thus obtained was then concentrated to dryness in vacuo. The resulting residue was suspended in a saturated NaHCO₃ solution, which was then extracted with CH₂Cl₂. The organic extract was then washed with a saturated brine solution, dried over MgSO₄ and concentrated in vacuo to an oil. This oil was chromatographed (flash column, silica gel, EtOAc) to give 400 mg of isoxazolylindoline. Such compound was dissolved in 50 ml of methanol and two equivalents of 0.1N HCl were added. The resulting solution was concentrated to dryness in vacuo and the resulting residue was then crystallized from ethanol/diethyl ether to give 170 mg of title compound.
mp = 235°C d
MS m/e 325(FD)
[α]_{D} + 27.29° (MeOH)

| Analysis calculated for C₂₀H₂₇N₃O·2HCl | | | |
|---|---|---|---|
| Theory: | C, 60.30; | H, 7.34; | N, 10.55; |
| Found: | C, 60.53; | H, 7.54; | N, 10.26. |

### Example 11

### Preparation of (-)(2aR,4S)-6-(5-isoxazolyl)-4-(di-n-propylamino)-1,2,2a,3,4,5-hexahydrobenz[cd]indole · 2 HCl

The title compound was prepared substantially in accordance with the method described in Example 10, above, utilizing 2.5 g (8.3 mol) of (-)(2aR,4S)-6-acetyl-4-(di-n-propylamino)-1,2,2a,3,4,5-hexahydrobenz[cd]indole (prepared substantially in accordance with the method described in Example 4) and 1.5 g (22 mol) of a hydroxylamine hydrochloride solution. Such reaction sequence provided 500 mg of title compound.
m.p. 235°C d
MS m/e 325(FD)
[α]_{D}-29.18°(MeOH)

| Analysis calculated for C₂₀H₂₇N₃O·2HCl | | | |
|---|---|---|---|
| Theory: | C, 60.30; | H, 7.34; | N, 10.55; |
| Found: | C, 60.11; | H, 7.41; | N, 10.43. |

### Example 12

### Preparation of (-)(2aR,4S)-6-(3-phenyloxadiazol-5-yl)-4-(di-n-propylamino)-1,2,2a,3,4,5-hexahydrobenz[cd]indole

A sodium ethoxide solution was prepared by dissolving 49 mg (2.1 mmol) of sodium in 35 ml of ethanol. Phenylhydroxamidine (1.73 g, 12.71 mmol) and 6-ethoxycarbonyl-4-(di-n-propylamino)-1,2,2a,3,4,5-hexahydrobenz[cd]indole (890 mg, 2.1 mmol) were added to the ethoxide solution and the resulting solution was heated to reflux and stirred at that temperature for 6.25 hours and then stirred overnight at room temperature. The next morning additional sodium ethoxide solution (50 mg of sodium in 10 ml of ethanol) was added and the reaction mixture was again stirred at reflux overnight. The next morning water was added to the reaction mixture and the resulting solution was then extracted with ethyl acetate. The organic extract was washed sequentially with water and a saturated brine solution, dried over sodian sulfate and then concentrated in vacuo to provide 2.33 g of a brown oil. This oil was purified by flash chromatography [2.5% isopropanol in chloroform (NH₄OH)] to provide 260 mg of title product as a light yellow solid. Such product was purified by recrystallization from hexane.

| Analysis calculated for C₂₅H₃₀N₄O | | | |
|---|---|---|---|
| Theory: | C, 74.59; | H, 7.51; | N, 13.92; |
| Found: | C, 74.59; | H, 7.52; | N, 13.90. |

### Example 13

### Preparation of (-)(2aR,4S)-6-(2-furyl)-4-(di-n-propylamino)-1,2,2a,3,4,5-hexahydrobenz[cd]indole

To a sealed tube with threads containing 13 ml of dry tetrahydrofuran were added 1.2 g (2.46 mol) of (+)(2aS,4R)-1-benzyl-6-iodo-4-(di-n-propylamino)-1,2,2a,3,4,5-hexahydrobenz[cd]indole, 968 mg (2.71 mol) of 2-(tributylstannyl)furan and 200 mg of bis(triphenylphosphine)palladium(II) chloride. The resulting mixture was then deaerated with argon for 15 minutes. After deaeration, the tube was sealed with a teflon cap and the contents thereof were heated at reflux temperature for 24 hours. After 24 hours, the reaction mixture was cooled, filtered through a celite pad and the resulting filtrate was then concentrated in vacuo to provide a viscous orange oil. Flash chromatography of this oil over silica gel with 60% ethyl acetate/hexane plus 0.5% ammonium hydroxide as eluent gave the protected analog of the title compound in 61% yield.

The above-mentioned protected analog (635 mg, 1.4 mol) was dissolved in 10 ml of dry tetrahydrofuran and the resulting solution was chilled to a -78°C. Once chilled, 1.5 ml (2.39 mol) of a 1.7M solution of n-butyllithium in hexane was added dropwise via syringe. Once n-butyllithium addition was complete the reaction mixture was warmed to room temperature. The reaction mixture was quenched with a saturated NaHCO₃ solution and then partitioned between ethyl acetate and water. The aqueous layer was extracted with ethyl acetate, and the organic layers were combined, washed with a saturated brine solution, dried over sodian sulfate and then concentrated in vacuo to provide a viscous orange oil. This oil was chromatographed over silica gel (elution with 20% ethyl acetate/hexane plus 0.5% ammonium hydroxide) to provide 161 mg of title compound as a pale yellow oil.
MS m/e 324(FD)
[α]_{D} -45.63°(MeOH)

| Analysis calculated for C₂₁H₂₈N₂O: | | | |
|---|---|---|---|
| Theory: | C, 77.74; | H, 8.70; | N, 8.63; |
| Found: | C, 78.74; | H, 8.82; | N, 8.27. |

### Example 14

### Preparation of (+)(2aS,4R)-6-(2-furyl)-4-(di-n-propylamino)-1,2,2a,3,4,5-hexahydrobenz[cd]indole

The title compound was prepared substantially in accordance with the method set forth in Example 13, above, utilizing 1.5 g (3.07 mol) of (-)(2aR,4s)-1-benzyl-6-iodo-4-(di-n-propylamino)-1,2,2a,3,4,5-hexahydrobenz[cd]indole, 250 mg of bis(triphenylphosphine)palladium(II) chloride and 1.21 g (3.38 mol) of 2-(tributylstannyl)furan to provide 592 mg of title compound as a viscous brown oil.
MS m/e 325.22(FD)
[α]_{D} +42.0°(MeOH)

| Analysis calculated for C₂₁H₂₈N₂O: | | | |
|---|---|---|---|
| Theory: | C, 77.74; | H, 8.70; | N, 8.63; |
| Found: | C, 77.59; | H, 8.10; | N, 8.83. |

### Example 15

### Preparation of (-)(2aR,4S)-6-(3-furyl)-4-(di-n-propylamino)-1,2,2a,3,4,5-hexahydrobenz[cd]indole

The title compound was prepared substantially in accordance with the method described in Example 13, above, utilizing 1.50 g (3.07 mol) of (+)(2aS,4R)-1-benzyl-6-iodo-4-(di-n-propylamino)-1,2,2a,3,4,5-hexahydrobenz[cd]indole, 1.21 g (3.38 mol) of 3-(tributylstannyl)furan and 250 mg of bis(triphenylphosphine)palladium(II) chloride to provide 711 mg of title product as a pale yellow viscous oil.
MS m/e 324(FD)

| Analysis calculated for C₂₁H₂₈N₂O | | | |
|---|---|---|---|
| Theory: | C, 77.24; | H, 8.70; | N, 8.63; |
| Found: | C, 77.49; | H, 8.68; | N, 8.45. |

### Example 16

### Preparation of (+)(2aS,4R)-6-(2-thienyl)-4-(di-n-propylamino)1,2,2a,3,4,5-hexahydrobenz[cd]indole

The title compound was prepared substantially in accordance with the method set forth in Example 13, above, utilizing 1.5 g (3.1 mol) of (-)(2aR,4S)-1-benzyl-6-iodo-4-(di-n-propylamino)-1,2,2a,3,4,5-hexahydrobenz[cd]indole, 150 mg of bis(triphenylphosphine)palladium(II) chloride and 1.27 g (3.41 mol) of 2-(tributylstannyl)thiophene to provide 719 mg of title compound as a light brown viscous oil.
MS m/e 341(FD)

| Analysis calculated for C₂₁H₂₈N₂S | | | | |
|---|---|---|---|---|
| Theory: | C, 74.07; | H, 8.29; | N, 18.60; | S, 9.42; |
| Found: | C, 74.24; | H, 8.60; | N, 7.52; | S, 9.15. |

### Example 17

### Preparation of (+)(2aS,4R)-6-(2-pyridyl)-4-(di-n-propylamino)-1,2,2a,3,4,5-hexahydrobenz[cd]indole

The title compound was prepared substantially in accordance with the method set forth in Example 13, above, utilizing 1.50 g (3.07 mol) of (-)(2aR,4S)-1-benzyl-6-iodo-4-(di-n-propylamino)-1,2,2a,3,4,5-hexahydrobenz[cd]indole, 250 mg of bis(triphenylphosphine)palladium(II) chloride and 1.24 g (3.38 mol) of 2-(tributylstannyl)pyridine to produce 474 mg of title compound as a colorless foam. The hydrochloride salt of the title compound was prepared by dissolving the foam in diethyl ether and then treating the resulting solution with a saturated hydrochloric acid in methanol solution. A yellow foam comprised of such salt was afforded after concentration in vacuo.
MS m/e 336.24(FD)

| Analysis calculated for C₂₂H₂₉N₃·HCl | | | |
|---|---|---|---|
| Theory: | C, 71.04; | H, 8.13; | N, 11.30; |
| Found: | C, 70.60; | H, 8.46; | N, 10.58. |

### Example 18

### Preparation of (+)(2aS,4R)-6-(3-pyridyl)-4-(di-n-propylamino)-1,2,2a,3,4,5-hexahydrobenz[cd]indole

The title compound was prepared substantially in accordance with the procedure set forth in Example 13, above, utilizing 1.50 g (3.07 mol) of (-)(2aR,4S)-1-benzyl-6-iodo-4-(di-n-propylamino)-1,2,2a,3,4,5-hexahydrobenz[cd]indole, 250 mg of bis(triphenylphosphine)palladium(II) chloride and 1.24 g (3.38 mol) of 3-(tributylstannyl)pyridine to produce 475 mg of title compound as a pale yellow oil. The bishydrochloric acid salt of the title compound was prepared by dissolving the oil in diethyl ether and then adding a saturated hydrochloric acid in methanol solution dropwise. Once an excess of hydrochloric acid had been added the mixture was concentrated in vacuo to provide a pale yellow foam.
MS m/e 336.24(FD)

| Analysis calculated for C₂₂H₂₉N₃·2HCl: | | | |
|---|---|---|---|
| Theory: | C, 64.70; | H, 7.65; | N, 10.29; |
| Found: | C, 65.84; | H, 7.55; | N, 9.76. |

### Example 19

### Preparation of (-)(2aR,4S)-6-(2-oxazolyl)-4-(di-n-propylamino)-1,2,2a,3,4,5-hexahydrobenz[cd]indole

### A. 2-tributylstannyloxazole

A solution of 1.00 g (14.5 mol) of oxazole in 25 ml of THF at -78°C was treated with 10.2 ml (14.6 mol) of 1.43M butyllithium in hexane. After stirring for 30 minutes, an addition of 3.93 ml (14.5 mol) of tributyltin chloride was made, and the solution was allowed to warm to room temperature. Stirring was continued for another hour after which most of the solvents were evaporated in vacuo. The resulting residue was taken up in 50 ml of hexane, and the resulting precipitate was separated by filtration through filtercel. Evaporation of the solvent from the filtrate provided 5.13 g of a colorless oil which was identified by NMR as the 2-stannyl derivative plus a small amount of tetrabutylstannane.

### B. (-)(2aR,4S)-1-benzoyl-6-(2-oxazolyl)-4-(di-n-propylamino)-1,2,2a,3,4,5-hexahydrobenz[cd]indole

A solution of 5.0 g (13.8 mol) of the crude 2-tributylstannyloxazole prepared above and 6.8 g (13.9 mol) of (+) (2aS,4R)-1-benzoyl-6-iodo-4-(di-n-propylamino)-1,2,2a,3,4,5-hexahydrobenz[cd]indole in 100 ml of toluene was treated with 0.7 g (0.6 mol) of tetrakis(triphenyl-phosphine)palladium then refluxed under nitrogen for 20 hours. After cooling the reaction mixture was washed with a saturated brine solution and then dried over Na₂SO₄. Concentration in vacuo provided a viscous oil which was chromatographed over a silica gel column using a solvent gradient progressing from toluene to 1:1 toluene/EtOAc. The product from the column was dissolved in 1M HCl. This solution was then washed with ether, basicified with 5M NaOH, and extracted with CH₂Cl₂. Concentration of the extract in vacuo gave about 4 g of a brown oil. When this oil was dissolved in pentane a small amount of a red/brown resin separated leaving a clear, yellow solution. The resin was separated and the pentane was evaporated to provide a residue. This residue was crystallized by dissolving it in a small amount of CH₂Cl₂ and slowly adding isoctane. The crystalline (-)(2aR,4S)-1-benzoyl-6-(2-oxazolyl)-4-(di-n-propylamino)-1,2,2a,3,4,5-hexahydrobenz[cd]indole, obtained in four crops, weighed 2.63 g. mp 103-4°C.

### C. (-)(2aR,4S)-6-(2-oxazolyl)-4-(di-n-propylamino)-1,2,2a,3,4,5-hexahydrobenz[cd]indole

A solution of 1.00 g (2.33 mol) of the above 1-benzoyl compound in 25 ml of THF was stirred at -78°C as 3.0 ml (4.29 mol) of 1.43M butyllithium in hexane was added. The resulting solution was allowed to warm to 0°C, then poured into water and extracted with CH₂Cl₂. The CH₂Cl₂ extract was then, in turn, extracted with 1M HCl. The resulting aqueous extract was basicified with 1M NaOH, and, in turn, extracted with CH₂Cl₂. After drying over Na₂SO₄, the extract was concentrated in vacuo to provide title compound as a viscous oil.
MS m/e 326(FD)
[α]_{D} = -60°(MeOH).

| Analysis calculated for C₂₀H₂₇N₃O: | | | |
|---|---|---|---|
| Theory: | C, 73.81; | H, 8.36; | N, 12.91; |
| Found: | C, 73.37; | H, 8.26; | N, 12,09. |

### Example 20

### Preparation of (-)(2aR,4S)-6-(5-isoxazolyl)-4-[di-(cyclopropylmethyl)amino]-1,2,2a,3,4,5-hexahydrobenz[cd]indole

To a solution of (-)(2aR,4S)-6-acetyl-4-[di-(cyclopropylmethyl)amino]-1,2,2a,3,4,5-hexahydrobenz[cd]indole (2.5 g, 7.7 mol) and triethylamine (1.1 ml, 8 mol) in 90 ml CH₂Cl₂ was added dropwise a solution of 2,2,2-trichloroethylchloroformate (1.7 g, 8 mol) in 10 ml CH₂Cl₂. The reaction mixture was stirred at room temperature for one hour and then extracted with water and 1N HCl. The organic solution was washed with a saturated NaHCO₃ solution, a saturated brine solution, dried over MgSO₄ and then concentrated to dryness in vacuo to give 3.1 g of the 1-carbamylindoline.

A solution of the 1-carbamylindoline (3.1 g, 6.2 mmol) and tris(dimethylamino)methane (5 ml) in 100 ml of toluene was stirred at reflux for 16 hours. After 16 hours the reaction mixture was concentrated to dryness in vacuo. vacuo. The resulting residue was dissolved in 50 ml of acetic acid and 2.0 g (29 mol) of a hydroxylamine hydrochloride solution were added. The resulting reaction mixture was stirred at room temperature for 16 hours and then concentrated to dryness in vacuo. The resulting residue was suspended in water and an excess of a concentrated NH₄OH solution was added to basicify the mixture. The basic mixture was then extracted with CH₂Cl₂ and the resulting organic extract was washed with a saturated brine solution, dried over MgSO₄ and then concentrated in vacuo to give 2.1 g of an oil. This oil was chromatographed (flash column, silica gel, EtOAc) to yield 1.7 g of the protected (-) (2aR,4S)-6-isoxazolylindoline.

The above compound (1.7 g, 3.2 mol) was dissolved in 30 ml of acetic acid and 1.5 g of zinc dust were added all at once. The resulting reaction mixture was stirred at room temperature for four hours and then filtered through a celite pad. The filtrate thus obtained was then concentrated to dryness in vacuo. The resulting residue was suspended in a saturated NaHCO₃ solution, which was extracted with CH₂Cl₂. The organic extract was then washed with a saturated brine solution, dried over MgSO₄ and concentrated in vacuo to an oil. This oil was chromatographed (flash column, silica gel, EtOAc) to give 660 mg of title compound.

The present compounds of Formula 1 have been found to have selective affinity for the 5HT receptors in the brain with much less affinity for other receptors. Because of their ability to selectively bind to 5HT receptors, the compounds of Formula 1 are useful in treating disease states which require alteration of 5-HT receptor function, particularly 5-HT_{1A}, and/or 5HT_{1D} but without the side effects which may be associated with less selective compounds. This alteration may involve reproducing (an agonist) or inhibiting (an antagonist) the function of serotonin. These disease states include anxiety, depression, gastric acid secretion, hypertension, nausea, sexual dysfunction, cognition, senile dementia, migraine, consumptive disorders such as appetite disorders, alcoholism and smoking. The foregoing conditions are treated with a pharmaceutically effective amount of a compound of Formula 1 or a pharmaceutically acceptable salt thereof.

The term "pharmaceutically effective amount", as used herein, represents an amount of a compound of the invention which is capable of diminishing the adverse symptoms of the particular disease. The particular dose of compound administered according to this invention of course be determined by the particular circumstances surrounding the case, including the compound administered, the route of administration, the particular condition being treated, and similar considerations. The compounds can be administered by a variety of routes including the oral, rectal, transdermal, subcutaneous, intravenous, intramuscular or intranasal routes. A typical single dose for prophylactic treatment, however, will contain from about 0.01 mg/kg to about 50 mg/kg of the active compound of this invention when administered orally. Preferred oral doses will be about 0.01 to about 3.0 mg/kg, ideally about 0.01 to about 0.1 mg/kg. When a present compound is given orally it may be necessary to administer the compound more than once each day, for example about every eight hours. For IV administration by bolus, the dose will be from about 10 µg/kg to about 300 µg/kg, preferably about 20 µg/kg to about 50 µg/kg.

The following experiments were conducted to demonstrate the ability of the compounds of Formula 1 to bind to 5-HT receptors. Such experiments demonstrate the utility of the compounds of Formula 1 in treating disease states (such as those noted above) which require alteration of 5-HT receptor function.

The affinities of certain of the compounds of Formula 1 at the central 5-HT_{1A} receptors were determined using a modification of the binding assay described by Taylor et al., J. Pharmacol. Exp. Ther., 236, 118-125 (1986). Membranes for the binding assaywere prepared from male Sprague-Dawley rats (150-250 g). The animals were killed by decapitation, and the brains were rapidly chilled and dissected to obtain the hippocampi. Membranes from the hippocampi were either prepared that day, or the hippocampi were stored frozen (-70°C) until the day of preparation. The membranes were prepared by homogenizing the tissue in 40 volumes of ice-cold Tris-HCl buffer (50 mM, pH 7.4 at 22°C) using a Techmar Tissumizer (setting 65 for 15 sec), and the homogenate was centrifuged at 39800xg for 10 minutes. The resulting pellet was then resuspended in the same buffer, and the centrifugation and resuspension process was repeated three additional times to wash the membranes. Between the second and third washes the resuspended membranes were incubated for 10 minutes at 37°C to facilitate the removal of endogenous ligands. The final pellet was resuspended in 67 mM Tris-HCl, pH 7.4 to a concentration of 2 mg of tissue original wet weight/200 µl. This homogenate was stored frozen (-70°C) until the day of the binding assay. Each tube for the binding assay had a final volume of 800 µl and contained the following: Tris-HCl (50 mM), pargyline, (10 µm), CaCl₂ (3mM), [³H]8-OH-DPAT (1.0 nM), appropriate dilutions of the drugs of interest, and membrane resuspension equivalent to 2 mg of original tissue wet weight, for a final pH of 7.4. The assay tubes were incubated for 10 minutes at 37°C, and the contents were then rapidly filtered through GF/B filters (pretreated with 0.6% polyethylenimine), followed by four 1 ml washes with ice-cold buffer. The radioactivity trapped by the filters were quantitated by liquid scintillation spectrometry, and specific [³H]8-OH-DPAT binding to the 5-HT_{1A} sites was defined as the difference between [³H]8-OH-DPAT bound in the presence and absence of 10 µM 5-HT.

The results of the evaluation of various compounds of Formula 1 in the test system described above are set forth in Table 1, below. In Table 1, the first column provides the Example Number of the compound evaluated while the second column provides the amount of test compound (expressed in nanomolar concentration) required to inhibit the binding of [³H]8-OH-DPAT by 50% (indicated as IC₅₀).

**Table 1**

| IN VITRO BINDING ACTIVITY AT THE 5-HT1A RECEPTOR | |
|---|---|
| Example No. | 5-HT1A in vitro binding (IC₅₀, nM) |
| 6 | 6.37 |
| 7 | 1.95 |
| 8 | 0.91 |
| 10 | 0.73 |
| 11 | 2.08 |
| 12 | 105.00 |
| 13 | 21.09 |
| 14 | 5.30 |
| 15 | 2.74 |
| 17 | 17.34 |
| 18 | 1.92 |

The affinities of certain of the compounds of Formula 1 at the central 5-HT_{1D} binding sites were determined using a modification of the binding assay described by Heuring and Peroutka, J. Neurosci., 7, 894 (1987). Bovine brains were obtained and the caudate nuclei were dissected out and frozen at -70°C until the time that the membranes were prepared for the binding assays. At that time the tissues were homogenized in 40 volumes of ice-cold Tris-HCl buffer (50mM, pH 7.4 at 22°C) with a Techmar Tissumizer (setting 65 for 15 sec), and the homogenate was centrifuged at 39,800xg for 10 minutes. The resulting pellet was then resuspended in the same buffer, and the centrifugation and resuspension process was repeated three additional times to wash the membranes. Between the second and third washes the resuspended membranes were incubated for 10 minutes at 37°C to facilitate the removal of endogenous 5-HT. The final pellet was resuspended in the buffer to a concentration of 25 mg of original tissue wet weight/ml for use in the binding assay. Each tube for the binding assay had a final volume of 800 µl and contained the following: Tris-HCl (50mM), pargyline (10 µM), ascorbate (5.7 mM), CaCl₂ (3mM), 8-OH-DPAT (100 nM to mask 5-HT_{1A} receptors), mesulergine (100 nM to mask 5-HT_{1C} receptors), [³H]5-HT (1.7-1.9 nM), appropriate dilutions of the drugs of interest, and membrane resuspension equivalent to 5 mg of original tissue wet weight, for a final pH of 7.4. The assay tubes were incubated for 10 minutes at 37°C, and the contents were then rapidly filtered through GF/B filters (pretreated with 0.5% polyethylenimine), followed by four 1 ml washes with ice-cold buffer. The radioactivity trapped by the filters was quantitated by liquid scintillation spectrometry, and specific [³H]5-HT binding to the 5-HT_{1D} sites was defined as the difference between [³H]5-HT bound in the presence of 10 µM 5-HT.

The results of the evaluation of various compounds of Formula 1 in the test system described above are set forth in Table 2, below. In Table 2, the first column provides the Example Number of the compound evaluated while the second column provides the amount of test compound (expressed in nanomolar concentration) rehired to inhibit the binding of [³H]5-HT by 50% (indicated as IC₅₀).

**Table 2**

| IN VITRO BINDING ACTIVITY AT THE 5-HT1D RECEPTOR | |
|---|---|
| Example No. | 5-HT1D in vitro binding (IC₅₀, nM) |
| 6 | 30.00 |
| 7 | 23.58 |
| 8 | 9.12 |
| 10 | 11.24 |
| 11 | 1375.00 |
| 13 | 1887.62 |
| 14 | 43.14 |
| 15 | 19.40 |
| 17 | 163.02 |
| 18 | 40.29 |

The compounds of the present invention are preferably formulated prior to administration. Therefore, yet another embodiment of the present invention is a pharmaceutical formulation comprising a compound of the invention and a pharmaceutically acceptable excipient therefor. The present pharmaceutical formulations are prepared by known procedures using well known and readily available ingredients. In making the compositions of the present invention, the active ingredient will usually be mixed with an excipient, diluted by an excipient or enclosed within an excipient serving as a carrier which can be in the form of a capsule, sachet, paper or other container. When the excipient serves as a diluent, it can be a solid, semi-solid or liquid material which acts as a vehicle, carrier or median for the active ingredient. Thus, the compositions can be in the form of tablets, pills, powders, lozenges, sachets, cachets, elixirs, suspensions, emulsions, solutions, syrups, aerosols (as a solid or in a liquid median), ointments containing for example up to 10% by weight of the active compound, soft and hard gelatin capsules, suppositories, sterile injectable solutions and sterile packaged powders.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include lubricating agents such as talc, magnesium stearate and mineral oil, wetting agents, emulsifying and suspending agents, preserving agents such as methyl and propylhydroxybenzoates, sweetening agents or flavoring agents. The compositions of the invention may be formulated so as to provide quick, sustained or delayed release of the active ingredient after administration to the patient by employing procedures well known in the art.

The compositions are preferably formulated in a unit dosage form, each dosage containing from about 0.5 to about 50 mg, more usually about 1 to about 10 mg of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The following formulation examples are illustrative only and are not intended to limit the scope of the invention in any way.

### Formulation 1

Hard gelatin capsules are prepared using the following ingredients:

| | Quantity (mg/capsule) |
|---|---|
| (+)-6-(3-isoxazolyl)-4-(di-n-propylamino)-1,2,2a,3,4,5-hexahydrobenz[cd]indole | 25 |
| Starch, dried | 425 |
| Magnesium stearate | 10 |
| Total | 460 mg |

The above ingredients are mixed and filled into hard gelatin capsules in 460 mg quantities.

### Formulation 2

A tablet formula is prepared using the ingredients below:

| | Quantity (mg/tablet) |
|---|---|
| (±)-6-[3-(5-aminothiazolyl)]-4-(di-n-propylamino)-1,2,2a,3,4,5-hexahydrobenz[cd]indole | 25 |
| Cellulose, microcrystalline | 625 |
| Colloidal Silicon dioxide | 10 |
| Stearic acid | 5 |

The components are blended and compressed to form tablets each weighing 665 mg.

### Formulation 3

A dry powder inhaler formulation is prepared containing the following components:

| | Weight % |
|---|---|
| (±)-6-(5-isoxazolyl)-4-(di-n-propylamino)-1,2,2a,3,4,5-hexahydrobenz[cd]indole | 5 |
| Lactose | 95 |

The active compound is mixed with the lactose and the mixture added to a dry powder inhaling applicance.

### Formulation 4

Tablets each containing 60 mg of active ingredient are made up as follows:

| | |
|---|---|
| (+)-6-(2-pyrazolyl)-4-(di-n-propylamino)-1,2,2a,3,4,5-hexahydrobenz[cd]indole | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch and cellulose are passed through a No. 20 mesh U.S. sieve and mixed thoroughly. The solution of polyvinylpyrrolidone is mixed with the resultant powders which are then passed through a No. 4 mesh U.S. sieve. The granules so produced are dried at 50-60°C and passed through a No. 16 mesh U.S. sieve. The sodium carboxymethyl starch, magnesium stearate and talc, previously passed through a No. 30 mesh U.S. sieve, are then added to the granules which, after mixing, are compressed on a tablet machine to yield tablets each weighing 150 mg.

### Formulation 5

Capsules each containing 20 mg of medicament are made as follows:

| | |
|---|---|
| (±)-6-(5-oxadiazolyl)-4-(di-methylamino)-1,2,2a,3,4,5-hexahydrobenz[cd]indole | 20 mg |
| Starch | 169 mg |
| Magnesium stearate | 1 mg |
| Total | 190 mg |

The active ingredient, cellulose, starch and magnesium stearate are blended, passed through a No. 20 mesh U.S. sieve, and filled into hard gelatin capsules in 190 mg quantities.

### Formulation 6

Suppositories each containing 225 mg of active ingredient are made as follows:

| | |
|---|---|
| (+)-6-(4-pyridinyl)-4-(di-n-propylamino)-1,2,2a,3,4,5-hexahydrobenz[cd]indole | 225 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

### Formulation 7

Suspensions each containing 50 mg of medicament per 5 ml dose are made as follows:

| | |
|---|---|
| (±)-6-(2-thiazolyl)-4-(di-n-propylamino)-1,2,2a,3,4,5-hexahydrobenz[cd]indole | 50 mg |
| Xanthan Gum | 4 mg |
| Sodium carboxymethyl cellulose (11%) | |
| Microcrystalline Cellulose (89%) | 50 mg |
| Sucrose | 1.75 g |
| Sodium Benzoate | 10 mg |
| Flavor | q.v. |
| Color | q.v. |
| Purified water to | 5 ml |

The medicament, sucrose and xanthan gum are blended, passed through a No. 10 mesh U.S. sieve, and then mixed with a previously made solution of the microcrystalline cellulose and sodium carboxymethylcellulose in water. The sodium benzoate, flavor and color are diluted with some of the water and added with stirring. Sufficient water is then added to produce the required volume.

### Formulation 8

Capsules each containing 50 mg of medicament are made as follows:

| | |
|---|---|
| (+)-6-(5-isoxazolyl)-4-(di-methylamino)-1,2,2a,3,4,5-hexanydrobenz[cd]indole | 50 mg |
| Starch | 507 mg |
| Magnesium stearate | 3 mg |
| Total | 560 mg |

The active ingredient, cellulose, starch and magnesium stearate are blended, passed through a No. 20 mesh U.S. sieve, and filled into hard gelatin capsules.

## Claims (Claims for the following Contracting State(s): AT, BE, CH, DE, DK, FR, GB, IT, LI, LU, NL, PT, SE)

1. A compound of the formula 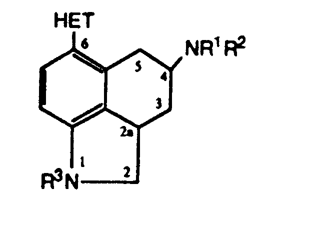 wherein
R¹ is hydrogen, C₁-C₄ alkyl, C₃-C₄ alkenyl, cyclopropylmethyl, phenyl(C₁-C₄)alkyl, phenyl(C₁-C₄)alkyl substituted with one or two substituents selected from (C₁-C₃) alkoxy, halo, hydroxy, (C₁-C₃) thioalkyl, nitro, (C₁-C₃) alkyl and trifluoromethyl, -(CH₂)ₙS(C₁-C₄ alkyl), -C(O)R⁴, -(CH₂)ₙC(O)NR⁵R⁶;
R² is hydrogen, C₁-C₄ alkyl, cyclopropylmethyl or C₃-C₄ alkenyl;
R³ is hydrogen, C₁-C₄ alkyl or an amino protecting group;
n is 1-4;
R⁴ is hydrogen, C₁-C₄ alkyl, C₁-C₄ haloalkyl, C₁-C₄ alkoxy or phenyl;
R⁵ and R⁶ are independently hydrogen, a C₁-C₄ alkyl, or a C₅-C₈ cycloalkyl with the proviso that when one of R⁵ or R⁶ is a cycloalkyl the other is hydrogen;
HET is an aromatic 5- or 6-membered heterocyclic ring, said ring having from one to three heteroatoms which are the same or different and which are selected from the group consisting of sulfur, oxygen, and nitrogen with the proviso that the 6-membered heterocyclic ring can only contain carbon and nitrogen and with the further proviso that the 5-membered ring contains no more than one oxygen or one sulfur but not both oxygen and sulfur, optionally substituted on one or two carbon atoms with (C₁-C₃) alkyl, halo, hydroxy, (C₁-C₃) alkoxy (C₁-C₃) thioalkyl, NH₂, CN or phenyl; or pharmaceutically acceptable salts thereof.

2. The compound of Claim 1 wherein HET is an isoxazole, a pyrazole, a pyridine, a thiazole, a furan, a thiophene, an oxadiazole or a pharmaceutically acceptable salt thereof.

3. The compound of Claim 1 or Claim 2 wherein R¹ and R² are independently C₁-C₃ alkyl or a pharmaceutically acceptable salt thereof.

4. The compound of any one of Claims 1 to 3 wherein R³ is hydrogen or a pharmaceutically acceptable salt thereof.

5. The compound of Claim 1 or Claim 2 wherein R¹ is -(CH₂)ₙC(O)NR⁵R⁶ wherein n is 2, R⁵ is hydrogen, R⁶ is cyclohexyl, R² is C₁-C₃ alkyl, and R³ is hydrogen or C₁-C₄ alkyl or a pharmaceutically acceptable salt thereof.

6. The substantially pure stereoisomer of a compound of any one of Claims 1 to 5 or a pharmaceutically acceptable salt thereof.

7. The stereoisomer of Claim 6 wherein the configuration at position 2a is S and at position 4 is R or a pharmaceutically acceptable salt thereof.

8. A compound of Claim 1 selected from the group consisting of 6-(3-isoxazolyl)-4-(di-n-propylamino)-1,2,2a,3,4,5-hexahydrobenz[cd]indole; 6-(5-isoxazolyl)-4-(di-n-propylamino)-1,2,2a,3,4,5-hexahydrobenz[cd]indole; 6-(3-pyrazolyl)-4-(di-n-propylamino)-1,2,2a,3,4,5-hexanydrobenz[cd]indole; 6-(4-pyrazolyl)-4-(dimethylamino)-1,2,2a,3,4,5-hexahydrobenz[cd]indole; 6-(4-pyridinyl)-4-(di-n-propylamino)-1,2,2a,3,4,5-hexanydrobenz[cd]indole; 6-(2-pyridinyl)-4-(di-n-propylamino)-1,2,2a,3,4,5-hexahydrobenz[cd]indole; 6-(3-pyridinyl)-4-(di-n-propylamino)-1,2,2a,3,4,5-hexahydrobenz[cd]indole; 6-(2-thiazolyl)-4-(di-n-propylamino)-1,2,2a,3,4,5-hexahydrobenz[cd]indole; 6-(5-thiazolyl)-4-(di-n-propylamino)-1,2,2a,3,4,5-hexanydrobenz[cd]indole; 6-(2-oxadiazolyl)-4-(di-n-propylamino)-1,2,2a,3,4,5-hexanydrobenz[cd]indole; 6-(3-furyl)-4-(di-n-propylamino)-1,2,2a,3,4,5-hexahydrobenz[cd]indole or a pharmaceutically acceptable salt thereof.

9. A pharmaceutical formulation comprising as active ingredient a compound as claimed in any one of Claims 1 to 8, or a pharmaceutically acceptable salt thereof, associated with one or more pharmaceutically acceptable carriers, diluents or excipients therefor.

10. A compound as claimed in any one of Claims 1 to 8 for use in treating serotonin related disorders.

11. A process for preparing a compound of the formula 1 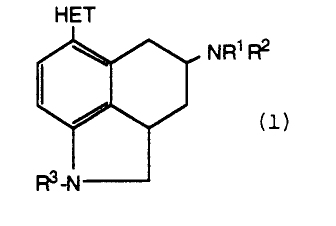 wherein
R¹ is hydrogen, C₁-C₄ alkyl, C₃-C₄ alkenyl, cyclopropylmethyl, phenyl(C₁-C₄)alkyl, phenyl(C₁-C₄)alkyl substituted with one or two substituents selected from (C₁-C₃) alkoxy, halo, hydroxy, (C₁-C₃) thioalkyl, nitro, (C₁-C₃) alkyl and trifluoromethyl, -(CH₂)ₙS(C₁-C₄ alkyl), -C(O)R⁴, -(CH₂)ₙC(O)NR⁵R⁶;
R² is hydrogen, C₁-C₄ alkyl, cyclopropylmethyl or C₃-C₄ alkenyl;
R³ is hydrogen, C₁-C₄ alkyl or an amino protecting group;
n is 1-4;
R⁴ is hydrogen, C₁-C₄ alkyl, C₁-C₄ haloalkyl, C₁-C₄ alkoxy or phenyl;
R⁵ and R⁶ are independently hydrogen, a C₁-C₄ alkyl, or a C₅-C₈ cycloalkyl with the proviso that when one of R⁵ or R⁶ is a cycloalkyl the other is hydrogen;
HET is an aromatic 5- or 6-membered heterocyclic ring, said ring having from one to three heteroatoms which are the same or different and which are selected from the group consisting of sulfur, oxygen, and nitrogen with the proviso that the 6-membered heterocyclic ring can only contain carbon and nitrogen and with the further proviso that the 5-membered ring contains no more than one oxygen or one sulfur but not both oxygen and sulfur, optionally substituted on one or two carbon atoms with (C₁-C₃) alkyl, halo, hydroxy, (C₁-C₃) alkoxy (C₁-C₃) thioalkyl, NH₂, CN or phenyl; or a pharmaceutically acceptable salt thereof, which comprises:
1) reacting a 4-amino-6-metallo-substituted hexahydrobenz[cd]indole of the formula 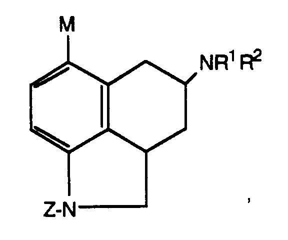 wherein R¹ and R² are as set forth above; Z is an amino protecting group and M is a metallo moiety, with a heterocyclic compound of the formula
HET-L,
wherein HET is as defined above and L is a leaving group;
2) deprotecting a compound of the formula 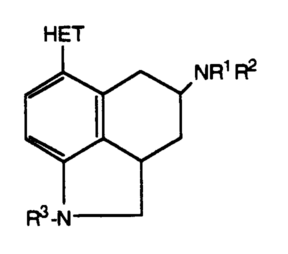 wherein HET, R¹ and R² are as defined above and R³ is an amino protecting group so as to provide a compound of the formula 1 wherein R³ is hydrogen;
3) reacting a 4-amino-6-halo-substituted hexahydrobenz[cd]indole of the formula 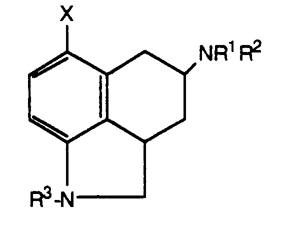 wherein R¹, R² and R³ are as defined above and X is halo with an organometailic derivative of the formula
M-HET
wherein HET is as defined above and M is lithium, magnesium, zinc, tin, mercury or boronic acid;
4) reacting a compound of the formula 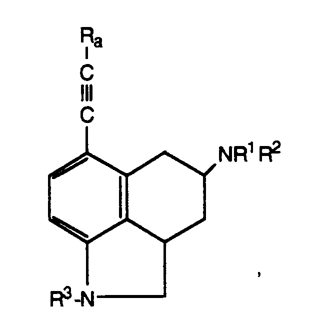 where R¹, R² and R³ are as defined above and Rₐ is hydrogen, C₁-C₃ alkyl, halogen, O(C₁-C₃ alkyl), S(C₁-C₃ alkyl), CN or phenyl, with a 1,3-dipole of the formula
⁺T=U-V⁻
in which T, U and V can be selected from the following list
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
where Rₐ is as set forth above, Rₐ' is hydrogen, C₁-C₃ alkyl, halogen, O(C₁-C₃ alkyl), S(C₁-C₃ alkyl), CN or phenyl and R_{b} is hydrogen, C₁-C₃ alkyl, phenyl or (C₁-C₃ alkyl)phenyl, so as to provide a compound of the formula 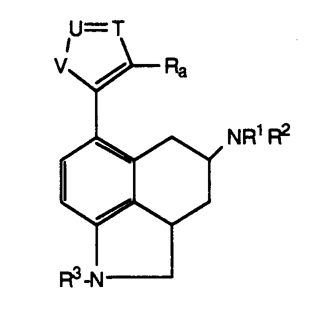 wherein R¹, R², R³, Rₐ, T, U and V are as set forth above;
5) reacting a compound of the formula 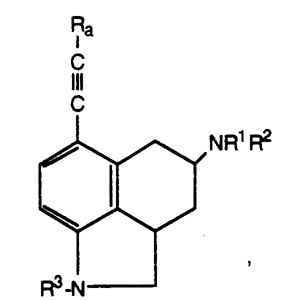 wherein R¹, R², and R³ are as defined above and Rₐ is hydrogen, C₁-C₃ alkyl, halogen, O(C₁-C₃ alkyl), S(C₁-C₃ alkyl), CN or phenyl, with a 1,3-dipole of the formula
⁺T-U=V⁻
in which T, U and V are selected from the following
| T | U | V |
|---|---|---|
| CHRₐ | N | N |
| NR_{b} | N | N |
where Rₐ is as set forth above and R_{b} is hydrogen, C₁-C₃ alkyl, phenyl or (C₁-C₃ alkyl)phenyl, so as to provide a compound of the formula 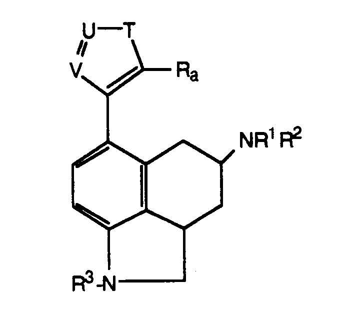 wherein R¹, R², R³, Rₐ, V, U and T are as defined above;
6) reacting a compound of the formula 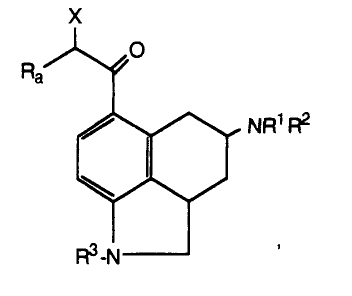 wherein R¹, R² and R³ are as set forth above, X is halo and Rₐ is hydrogen, C₁-C₃ alkyl, halogen, OH, O(C₁-C₃ alkyl), S(C₁-C₃ alkyl), NH₂, CN or phenyl, with
NH₄⁺RₐCOO- (where Rₐ is as defined above)
so as to provide a mixture of compounds of the formula 1 wherein HET is 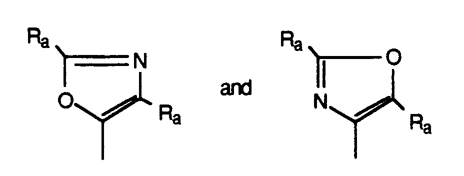 and then, optionally, separating the compounds from each other;
7) dehydrating a compound of the formula 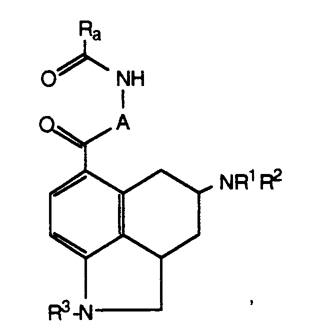 wherein R¹, R² and R³ are as set forth above, A is C-Rₐ or NH and each Rₐ is independently hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 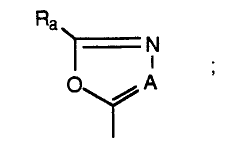
8) cyclizing and dehydrating a compound of the formula 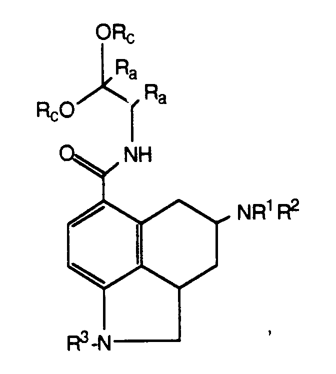 wherein R¹, R² and R³ are as set forth above, Rₐ is hydrogen, C₁-C₃ alkyl, halogen, OH, O(C₁-C₃ alkyl), S(C₁-C₃ alkyl), NH₂, CN or phenyl and R_{c} is hydrogen or C₁-C₃ alkyl, so as to provide a compound of the formula 1 wherein HET is 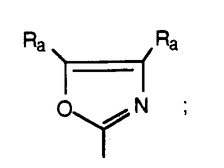
9) reacting a compound of the formula 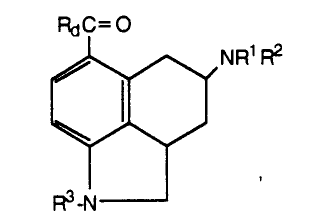 where R¹, R² and R³ are as set forth above and R_{d} is OH, O(C₁-C₃ alkyl), O(phenyl), O(C₁-C₃ alkylphenyl), halo, S(C₁-C₃ alkyl), S(phenyl), S(C₁-C₃ alkylphenyl), NH₂, NH(C₁-C₃ alkyl), N(C₁-C₃ alkyl)₂, OCO(C₁-C₃ alkyl), OCO(phenyl), or OCO(C₁-C₃ alkylphenyl), with 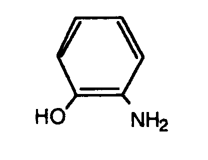 so as to provide a compound of the formula 1 wherein HET is 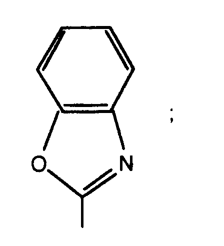
10) cyclizing and dehydrating a compound of the formula 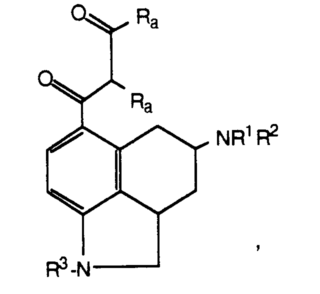 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 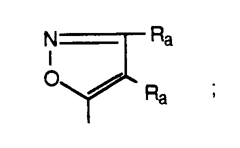
11) cyclizing and dehydrating a compound of the formula 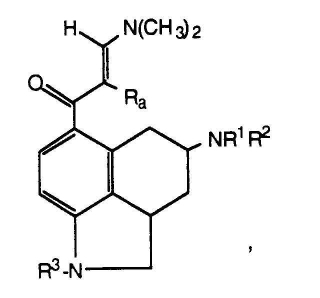 wherein R¹, R² and R³ are as set forth above and Rₐ is hydrogen, C₁-C₃ alkyl, halogen, OH, O(C₁-C₃ alkyl), S(C₁-C₃ alkyl ), NH₂, CN or phenyl, with hydroxylamine so as to provide a compound of the formula 1 wherein HET is 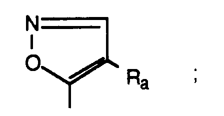
12) cyclizing and dehydrating a compound of the formula 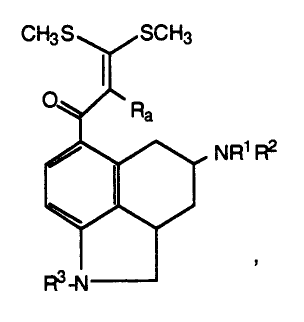 wherein R¹, R² and R³ are as set forth above and Rₐ is hydrogen, C₁-C₃ alkyl, halogen, OH, O(C₁-C₃ alkyl), S(C₁-C₃ alkyl ), NH₂, CN or phenyl, with hydroxylamine so as to provide a compound of the formula 1 wherein HET is 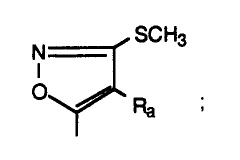
13) cyclizing and dehydrating a dianion of the formula 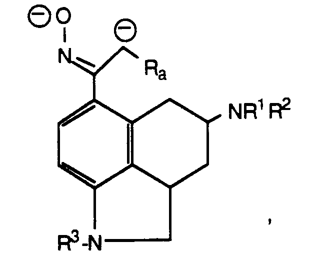 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a carbonyl derivative of the formula RₐCOOR_{c} (where Rₐ is as defined above and R_{c} is hydrogen or C₁-C₃ alkyl) or RₐCON(CH₃)₂ (where Rₐ is as defined above) so as to provide a compound of the formula 1 wherein HET is 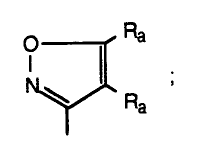
14) cyclizing and dehydrating a compound of the formula 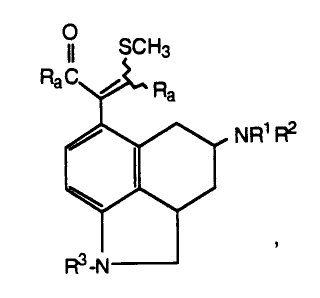 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 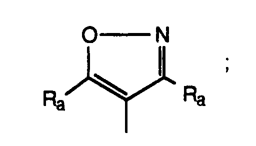
15) reacting a compound of the formula 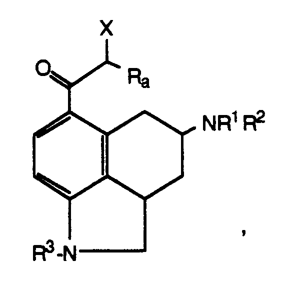 wherein R¹, R² and R³ are as set forth above, X is halo and Rₐ is hydrogen, C₁-C₃ alkyl, halogen, OH, O(C₁-C₃ alkyl ), S(C₁-C₃ alkyl), NH₂, CN or phenyl, with 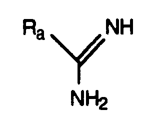 (where Rₐ is as set forth above)
so as to provide a compound of the formula 1 wherein HET is 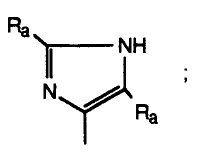
16) reacting a compound of the formula 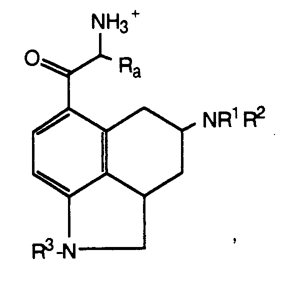 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with KSCN, and then R_{c}X (where R_{c} is hydrogen or C₁-C₃ alkyl and X is halogen) in the presence of a base so as to provide a compound of the formula 1 wherein HET is 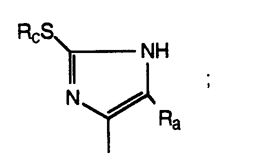
17) dehydrating a compound of the formula 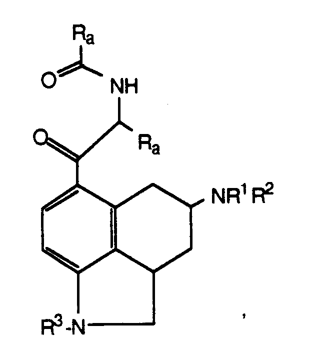 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, in the presence of ammonia or ammonium hydroxide so as to provide a compound of the formula 1 wherein HET is 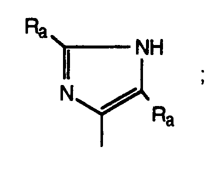
18) reacting a compound of the formula 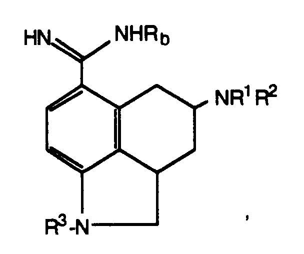 wherein R¹, R² and R³ are as set forth above and R_{b} is hydrogen, C₁-C₃ alkyl, phenyl or (C₁-C₃ alkyl)phenyl, with a compound of the formula 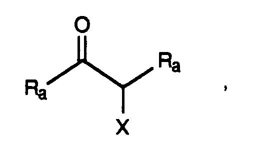 wherein Rₐ is hydrogen, C₁-C₃ alkyl, halogen, OH, O(C₁-C₃ alkyl), S(C₁-C₃ alkyl), NH₂, CN or phenyl, and X is halo, so as to provide a compound of the formula 1 wherein HET is 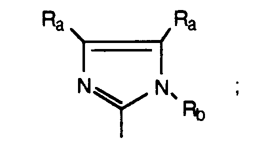
19) dehydrating a compound of the formula 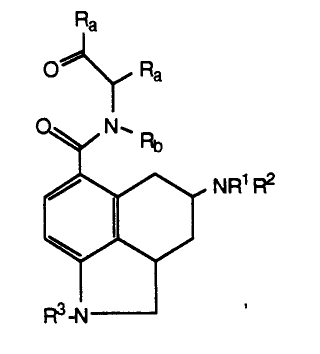 wherein R¹, R² and R³ are as set forth above, Rₐ is hydrogen, C₁-C₃ alkyl, halogen, OH, O(C₁-C₃ alkyl), S(C₁-C₃ alkyl), NH₂, CN or phenyl, and R_{b} is hydrogen, C₁-C₃ alkyl, phenyl or (C₁-C₃ alkyl) phenyl, in the presence of ammonia or ammonium hydroxide so as to provide a compound of the formula 1 wherein HET is 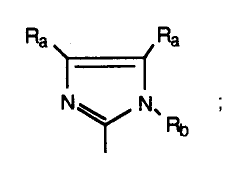
20) reacting a compound of the formula 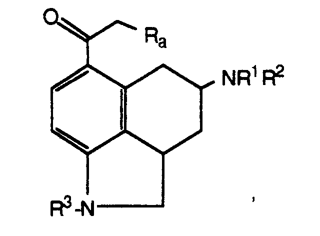 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an azide of the formula R_{b}N₃, where R_{b} is hydrogen, C₁-C₃ alkyl, phenyl or (C₁-C₃ alkyl)phenyl, followed by dehydration of the resulting compound so as to provide a compound of the formula 1 wherein HET is 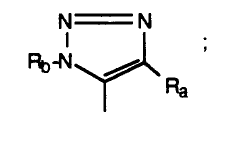
21) cyclizing and dehydrating a compound of the formula 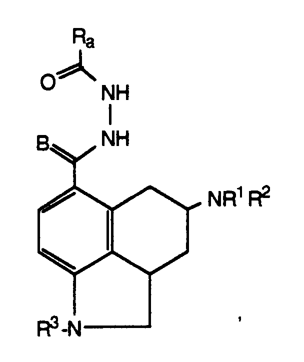 wherein R¹, R² and R³ are as set forth above, Rₐ is hydrogen, C₁-C₃ alkyl, halogen, OH, O(C₁-C₃ alkyl), S(C₁-C₃ alkyl), NH₂, CN or phenyl, and B is O or NH, so as to provide a compound of the formula 1 wherein HET is 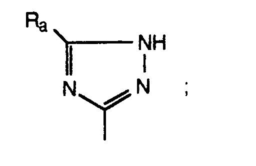 with the proviso that when B is O said cyclization reaction is run in the presence of ammonia or ammonium hydroxide;
22) reacting a compound of the formula 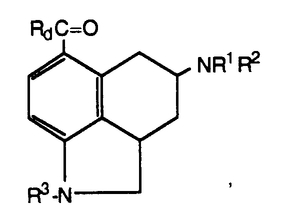 wherein R¹, R² and R³ are as set forth above and R_{d} is OH, O(C₁-C₃ alkyl), O(phenyl), O(C₁-C₃ alkylphenyl), halo, S(C₁-C₃ alkyl), S(phenyl), S(C₁-C₃ alkylphenyl, NH₂, NH(C₁-C₃ alkyl), N(C₁-C₃ alkyl)₂, OCO(C₁-C₃ alkyl), OCO(phenyl) or OCO(C₁-C₃ alkylphenyl), with a compound of the formula 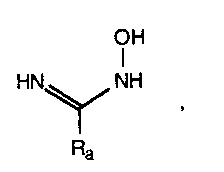 wherein Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 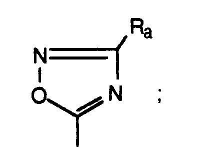
23) reacting a compound of the formula 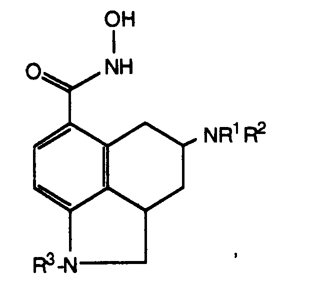 wherein R¹, R² and R³ are as set forth above, with RₐCN, where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 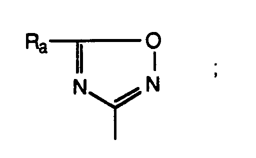
24) cyclizing and dehydrating a compound of the formula 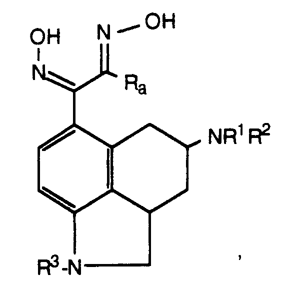 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 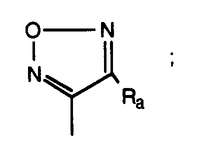
25) reacting a compound of the formula 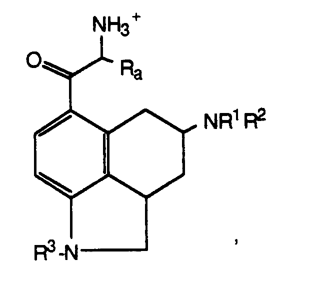 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 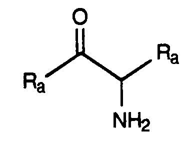 (where Rₐ is as set forth above) in the presence of an oxidizing agent, so as to provide a compound of the formula 1 wherein HET is 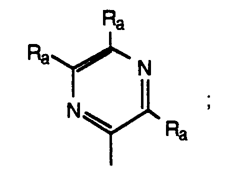
26) reacting a compound of the formula 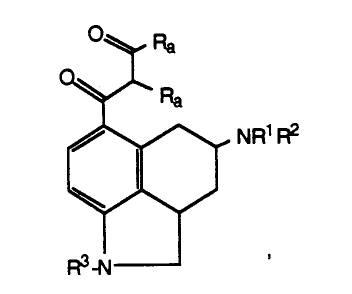 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 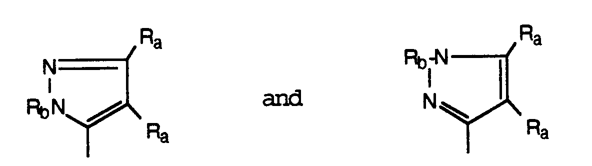 and then, optionally, separating the compounds from each other;
27) reacting a compound of the formula 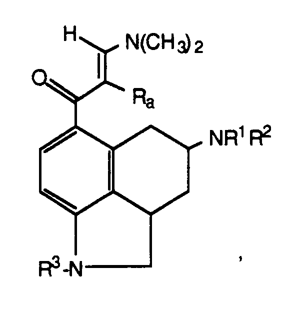 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 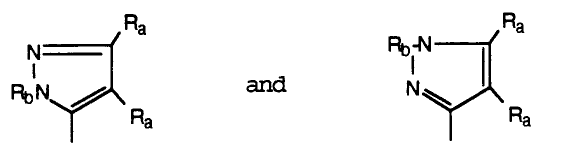 and then, optionally, separating the compounds from each other;
28) reacting a compound of the formula 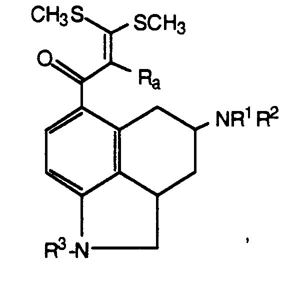 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 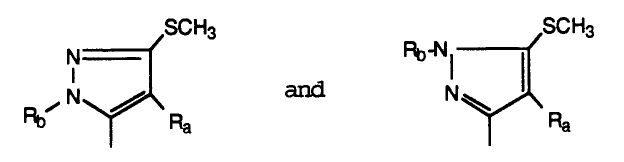 and then, optionally, separating the compounds from each other;
29) reacting a compound of the formula 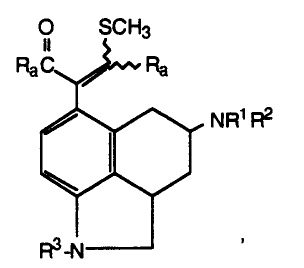 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHRb, where R_{b} is hydrogen, C₁-C₃ alkyl, phenyl or (C₁-C₃ alkyl)phenyl, so as to provide a compound of the formula 1 wherein HET is 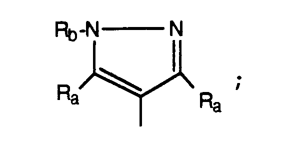
30) reacting a compound of the formula 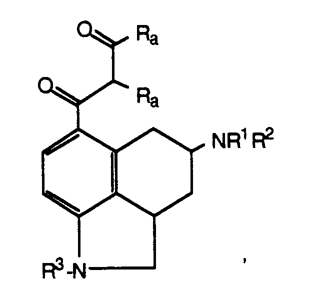 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 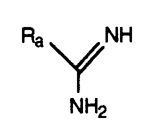 (where Rₐ is as set forth above)
so as to provide a compound of the formula 1 wherein HET is 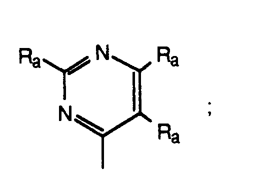
31) reacting a compound of the formula 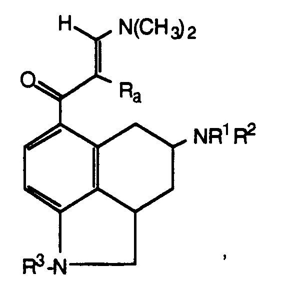 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 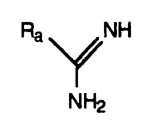 (where Rₐ is as set forth above)
so as to provide a compound of the formula 1 wherein HET is 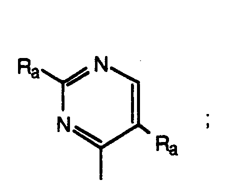
32) reacting a compound of the formula 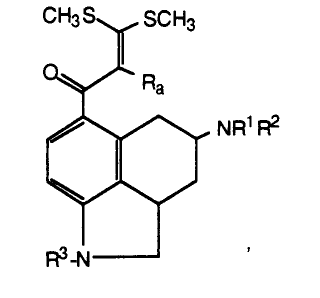 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 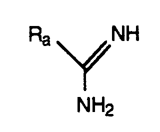 (where Rₐ is as set forth above)
so as to provide a compound of the formula 1 wherein HET is 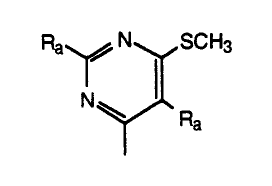
33) reacting a compound of the formula 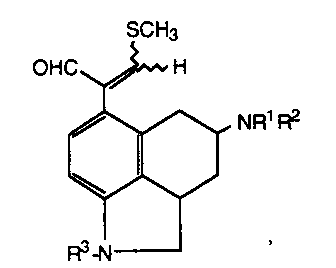 where R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 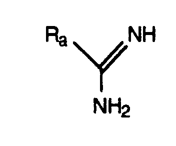 (where Rₐ is as set forth above)
so as to provide a compound of the formula 1 wherein HET is 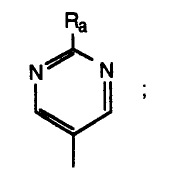
34) reacting a compound of the formula 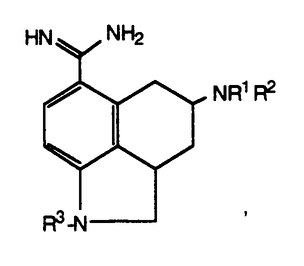 wherein R¹, R² and R³ are as set forth above, with a compound of the formula 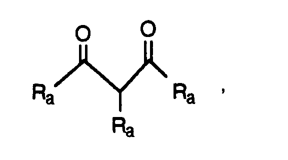 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 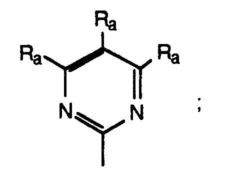
35) reacting a compound of the formula 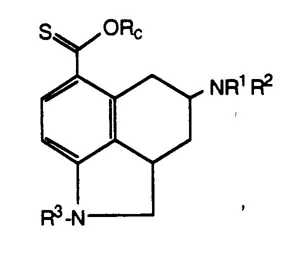 wherein R¹, R² and R³ are as set forth above and R_{c} is hydrogen or C₁-C₃ alkyl, with a compound of the formula RₐCH₂NNH, where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 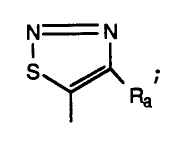
36) reacting a compound of the formula 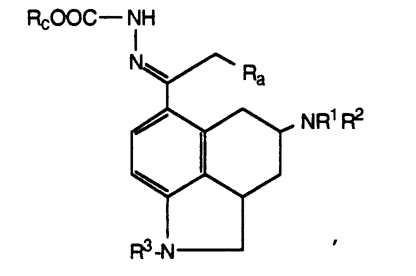 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 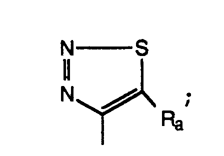
37) reacting a compound of the formula 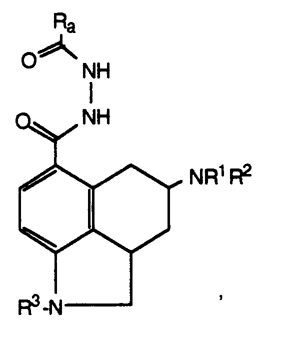 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with P₂S₅ so as to provide a compound of the formula 1 wherein HET is 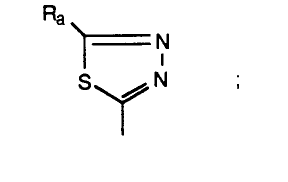
38) reacting a compound of the formula 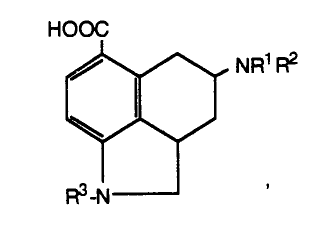 wherein R¹, R² and R³ are as set forth above, with a compound of the formula H₂NNHCSNH₂, in the presence of polyphosphoric acid, so as to provide a compound of the formula 1 wherein HET is 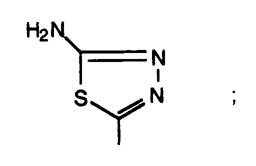
39) reacting a compound of the formula 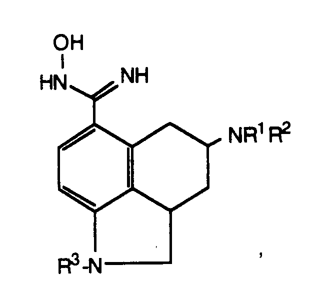 wherein R¹, R² and R³ are as set forth above, with carbon disulfide so as to provide a compound of the formula 1 wherein HET is 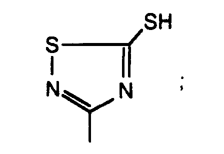
40) reacting a compound of the formula 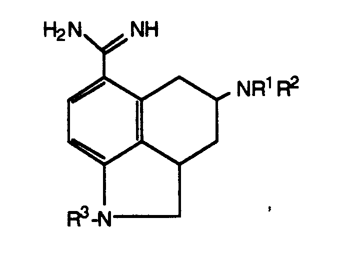 wherein R¹, R² and R³ are as set forth above, with (CNS)₂ so as to provide a compound of the formula 1 wherein HET is 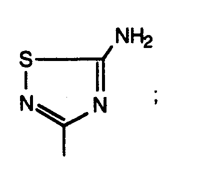
41) oxidizing a compound of the formula 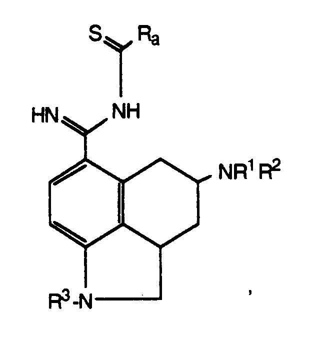 wherein R¹, R² and R³ are as set forth above, and Rₐ is hydrogen, C₁-C₃ alkyl, halogen, OH, O (C₁-C₃ alkyl), S(C₁-C₃ alkyl), NH₂, CN or phenyl, so as to provide a compound of the formula 1 wherein HET is 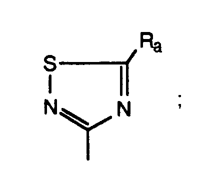
42) reacting a compound of the formula 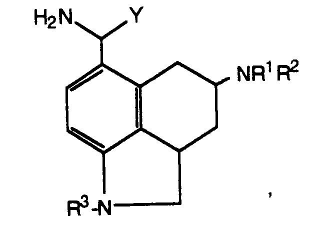 wherein R¹, R² and R³ are as set forth above and Y is -CN or -C(O)NH₂, with an oxidizing agent such as SOCl₂, SCl₂, S₂Cl₂ or SO₂Cl₂ so as to provide a compound of the formula 1 wherein HET is 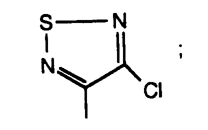
43) reacting a compound of the formula 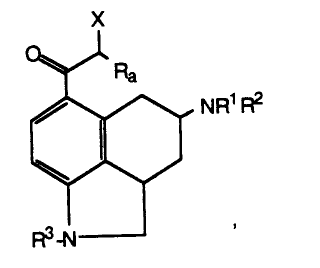 wherein R¹, R² and R³ are as set forth above, X is halogen and Rₐ is hydrogen, C₁-C₃ alkyl, halogen, OH, O(C₁-C₃ alkyl), S(C₁-C₃ alkyl), NH₂, CN or phenyl, with RₐCSNH₂, where Rₐ is as set forth above, so as to provide a compound of the formula 1 wherein HET is 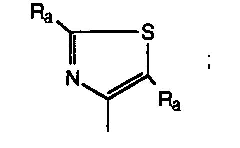
44) reacting a compound of the formula 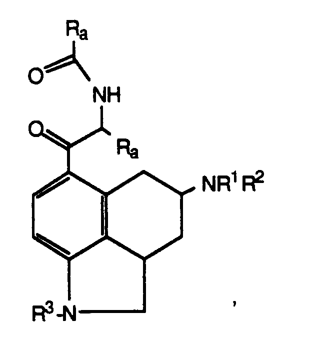 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with P₂S₅ so as to provide a compound of the formula 1 wherein HET is 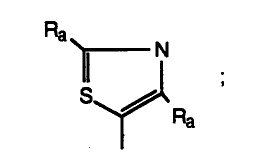
45) reacting a compound of the formula 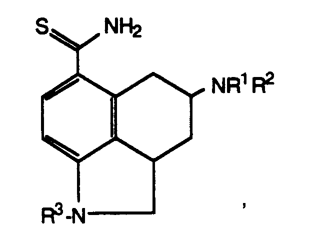 wherein R¹, R² and R³ are as set forth above, with a compound of the formula 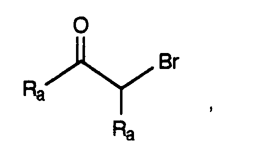 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 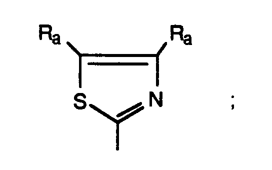
46) reacting a compound of the formula 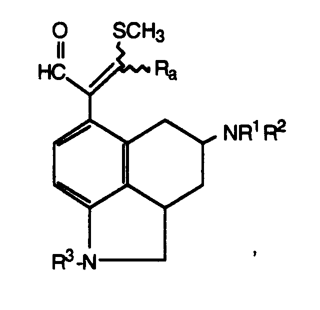 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with RₐC(S)NH₂, where Rₐ is as set forth above, so as to provide a compound of the formula 1 wherein HET is 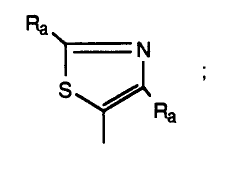
47) reacting a compound of the formula 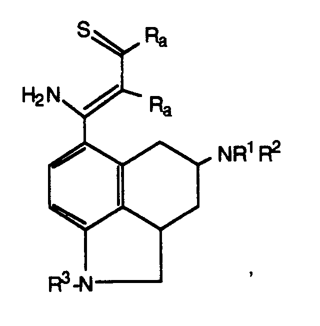 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to prepare a compound of the formula 1 wherein HET is 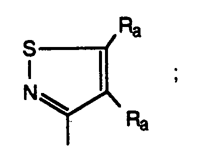
48) reacting a compound of the formula 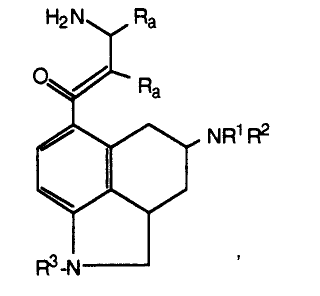 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to provide a compound of the formula 1 wherein HET is 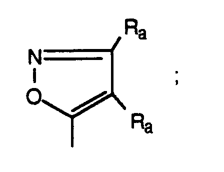
49) reacting a compound of the formula 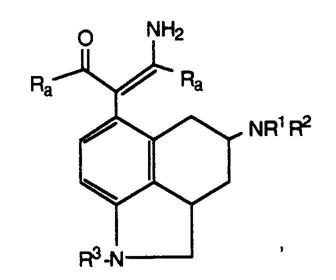 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to provide a compound of the formula 1 wherein HET is 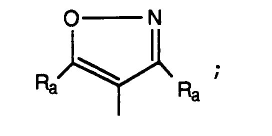
50) reacting a compound of the formula 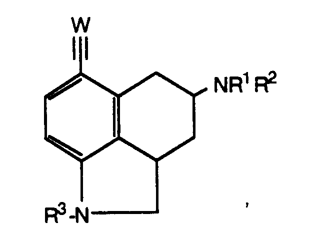 wherein R¹, R² and R³ are as set forth above and W is hydrogen or CHO, with KSCN or NaHSSO₃ and then reacting the intermediate formed thereby with ammonia or ammonium hydroxide so as to provide a compound of the formula 1 wherein HET is 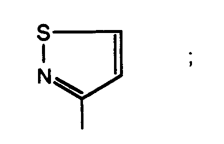
51) reacting a compound of the formula 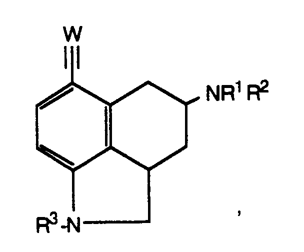 wherein R¹, R² and R³ are as set forth above and W is hydrogen or CHO, with H₂NSSO₃K and then reacting the intermediate formed thereby with a base so as to provide a compound of the formula 1 wherein HET is 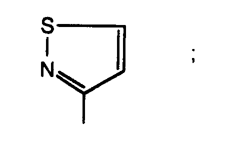
52) reacting a compound of the formula 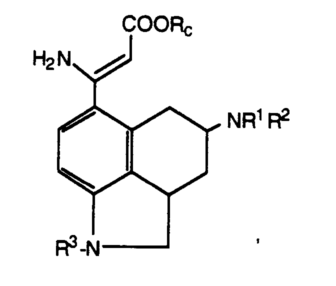 wherein R¹, R² and R³ are as set forth above and R_{c} is hydrogen or C₁-C₃ alkyl, with CSCl₂ so as to provide a compound of the formula 1 wherein HET is 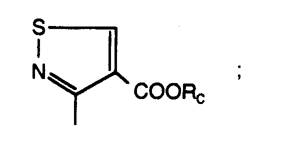
53) reacting a compound of the formula 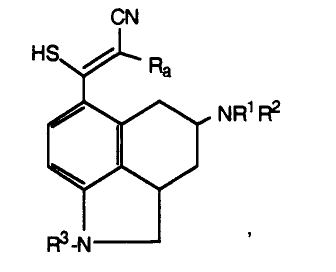 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with NCS so as to provide a compound of the formula 1 wherein HET is 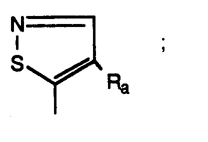
54) reacting a compound of the formula 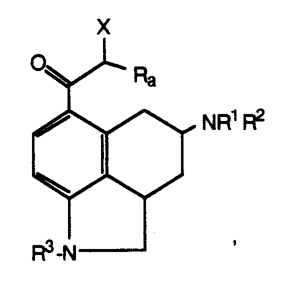 wherein R¹, R² and R³ are as set forth above, X is halogen and Rₐ is hydrogen, C₁-C₃ alkyl, halogen, OH, O(C₁-C₃ alkyl), S(C₁-C₃ alkyl), NH₂, CN or phenyl, with 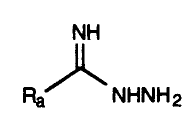 (where Rₐ is as set forth above)
so as to provide a compound of the formula 1 wherein HET is 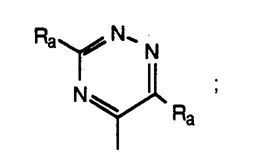
55) reacting a compound of the formula 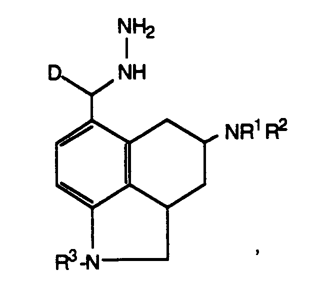 wherein R₁, R₂ and R₃ are as set forth above and D is =NH or =O, with a compound of the formula 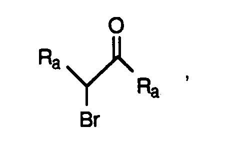 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 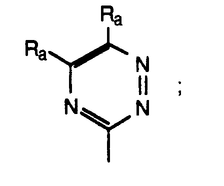
56) reacting a compound of the formula 1 wherein HET is substituted with at least one hydroxy group with POX₃, PX₃, SOX₂, P(phenyl)₃·X₂ or P(C₁-C₃ alkoxy)₃·X₂, where each X is a halogen, so as to convert the hydroxy group to a halogen substituent;
57) reacting a compound of the formula 1 where HET is substituted with at least one amino group with HONO so as to form the corresponding diazonium ion, followed by conversion of such ion to the corresponding halide substituted compound with CuX (where X is halogen), KI or HBF₄ with heat;
58) reacting a compound of the formula 1 wherein HET is substituted with at least one halogen substituent with an alkoxide anion of the formula
(C₁-C₃ alkyl)-O^{⊖}
so as to convert the halogen substituent to an alkoxy substituent;
59) reacting a compound of the formula 1 wherein HET is substituted with at least one hydroxy group with a (C₁-C₃ alkyl)halide so as to convert the hydroxy group to an alkoxy substituent;
60) reacting a compound of the formula 1 wherein HET is substituted with at least one hydroxy substituent with a diazo compound of the formula (C₁-C₃ alkyl)N₂ so as to convert the hydroxy group to an alkoxy substituent;
61) reacting a compound of the formula 1 wherein HET is substituted with at least one amino group with HONO so as to form the corresponding diazonium ion, followed by conversion of such ion to the corresponding hydroxy substituted compound using water or an acid;
62) reacting a compound of the formula 1 wherein HET is substituted with at least one C₁-C₃ alkoxy group with concentrated hydroiodic acid, concentrated hydrobromic acid or a Lewis Acid so as to convert the alkoxy group to a hydroxy substituent;
63) reacting a compound of the formula 1 wherein HET is substituted with at least one amino substituent with HONO so as to form the corresponding diazonium ion, followed by conversion of such ion to the corresponding nitrile substituted compound using CuCN;
64) reacting a compound of the formula 1 wherein HET is substituted with at least one amino substituent with HONO so as to form the corresponding diazonium ion, followed by conversion of such ion to the corresponding C₁-C₃ alkylthio substituted compound using a C₁-C₃ alkyl mercaptan;
65) reacting a compound of the formula 1 wherein HET is substituted with at least one halogen substituent with an alkylthio anion of the formula
(C₁-C₃ alkyl)-S^{⊖}
so as to convert the halogen substituent to an alkylthio moiety;
66) reacting a compound of the formula 1 wherein HET is substituted with at least one -SH moiety with a (C₁-C₃ alkyl)halide so as to provide the corresponding compound of the formula 1 having an alkylthio moiety;
67) reducing a compound of the formula 1 wherein HET is substituted with at least one nitro substituent, so as to provide a compound of the formula 1 wherein the nitro substituent is converted to an amino moiety;
68) reducing a compound of the formula 1 wherein HET is substituted with at least one halogen substituent so as to convert the halogen substituent to a hydrogen atom;
69) reducing a compound of the formula 1 wherein HET is substituted with at least one hydroxy group so as to convert the hydroxy substituent to a hydrogen atom;
70) reacting a compound of the formula 1 wherein HET is substituted with at least one amino moiety with HONO so as to form the corresponding diazonium ion, followed by conversion of such ion to the corresponding unsubstituted compound using H₃PO₂;
71) reducing a compound of the formula 1 wherein HET is substituted on one of the nitrogen atoms in such heterocyclic ring with a C₁-C₃ alkyl phenyl group so as to provide the corresponding compound of the formula 1 wherein the nitrogen atom is unsubstituted;
72) reducing a compound of the formula 1 wherein HET is substituted with at least one C₁-C₃ alkylthio moiety so as to provide the corresponding unsubstituted compound of the formula 1 or
73) reacting a compound of the formula 1 with a pharmaceutically acceptable organic or inorganic acid so as to form a pharmaceutically acceptable acid addition salt of such compound.

## Claims (Claims for the following Contracting State(s): ES, GR)

1. A process for preparing a compound of the formula 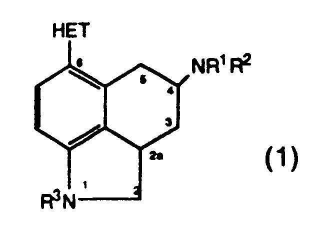 wherein
R¹ is hydrogen, C₁-C₄ alkyl, C₃-C₄ alkenyl, cyclopropylmethyl, phenyl(C₁-C₄)alkyl, phenyl(C₁-C₄)alkyl substituted with one or two substituents selected from (C₁-C₃) alkoxy, halo, hydroxy, (C₁-C₃) thioalkyl, nitro, (C₁-C₃) alkyl and trifluoromethyl, -(CH₂)ₙS(C₁-C₄ alkyl), -C(O)R⁴, -(CH₂)ₙC(O)NR⁵R⁶;
R² is hydrogen, C₁-C₄ alkyl, cyclopropylmethyl or C₃-C₄ alkenyl;
R³ is hydrogen, C₁-C₄ alkyl or an amino protecting group;
n is 1-4;
R⁴ is hydrogen, C₁-C₄ alkyl, C₁-C₄ haloalkyl, C₁-C₄ alkoxy or phenyl;
R⁵ and R⁶ are independently hydrogen, a C₁-C₄ alkyl, or a C₅-C₈ cycloalkyl with the proviso that when one of R⁵ or R⁶ is a cycloalkyl the other is hydrogen;
HET is an aromatic 5- or 6-membered heterocyclic ring, said ring having from one to three heteroatoms which are the same or different and which are selected from the group consisting of sulfur, oxygen, and nitrogen with the proviso that the 6-membered heterocyclic ring can only contain carbon and nitrogen and with the further proviso that the 5-membered ring contains no more than one oxygen or one sulfur but not both oxygen and sulfur, optionally substituted on one or two carbon atoms with (C₁-C₃) alkyl, halo, hydroxy, (C₁-C₃) alkoxy (C₁-C₃) thioalkyl, NH₂, CN or phenyl; or a pharmaceutically acceptable salt thereof, which comprises:
1) reacting a 4-amino-6-metallo-substituted hexahydrobenz[cd]indole of the formula 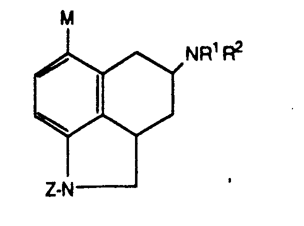 wherein R¹ and R² are as set forth above; Z is an amino protecting group and M is a metallo moiety, with a heterocyclic compound of the formula
HET-L,
wherein HET is as defined above and L is a leaving group;
2) deprotecting a compound of the formula 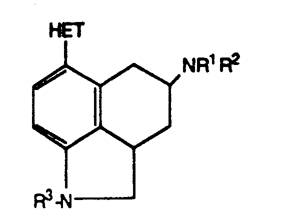 wherein HET, R¹ and R² are as defined above and R³ is an amino protecting group so as to provide a compound of the formula 1 wherein R³ is hydrogen;
3) reacting a 4-amino-6-halo-substituted hexahydrobenz[cd]indole of the formula 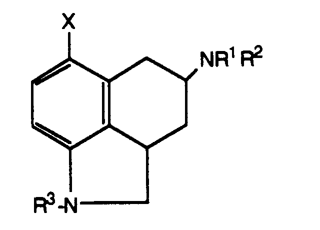 wherein R¹, R² and R³ are as defined above and X is halo with an organometailic derivative of the formula
M-HET
where HET is as defined above and M is lithium, magnesium, zinc, tin, mercury or boronic acid;
4) reacting a compound of the formula 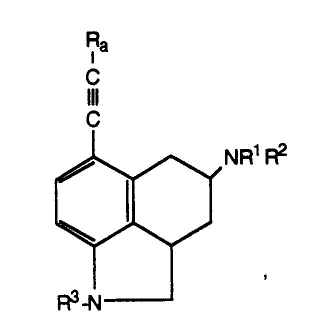 where R¹, R² and R³ are as defined above and Rₐ is hydrogen, C₁-C₃ alkyl, halogen, O(C₁-C₃ alkyl), S(C₁-C₃ alkyl), CN or phenyl, with a 1,3-dipole of the formula
⁺T=U-V⁻
in which T, U and V can be selected from the following list.
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
where Rₐ is as set forth above, Rₐ' is hydrogen, C₁-C₃ alkyl, halogen, O(C₁-C₃ alkyl), S(C₁-C₃ alkyl), CN or phenyl and R_{b} is hydrogen, C₁-C₃ alkyl, phenyl or (C₁-C₃ alkyl)phenyl, so as to provide a compound of the formula 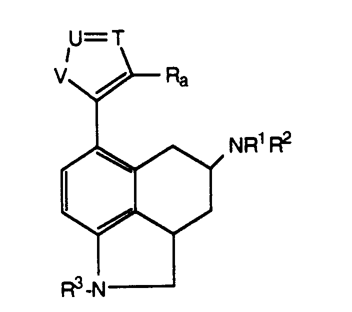 wherein R¹, R², R³, Rₐ, T, U and V are as set forth above;
5) reacting a compound of the formula 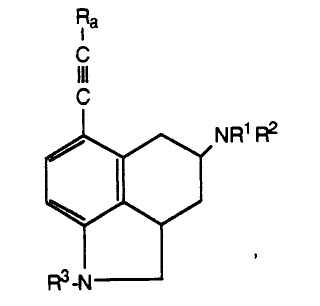 wherein R¹, R², and R³ are as defined above and Rₐ is hydrogen, C₁-C₃ alkyl, halogen, O(C₁-C₃ alkyl), S(C₁-C₃ alkyl), CN or phenyl, with a 1,3-dipole of the formula
⁺T-U=V⁻
in which T, U and V are selected from the following
| T | U | V |
|---|---|---|
| CHRₐ | N | N |
| NR_{b} | N | N |
where Rₐ is as set forth above and R_{b} is hydrogen, C₁-C₃ alkyl, phenyl or (C₁-C₃ alkyl)phenyl, so as to provide a compound of the formula 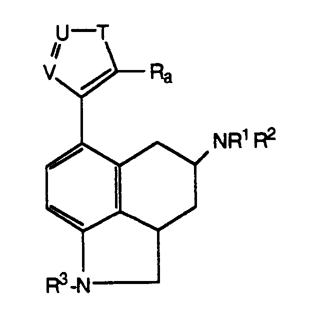 wherein R¹, R², R³, Rₐ, V, U and T are as defined above;
6) reacting a compound of the formula 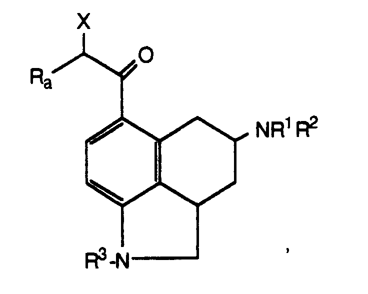 wherein R¹, R² and R³ are as set forth above, X is halo and Rₐ is hydrogen, C₁-C₃ alkyl, halogen, OH, O(C₁-C₃ alkyl), S(C₁-C₃ alkyl), NH₂, CN or phenyl, with NH₄⁺RₐCOO- (where Rₐ is as defined above) so as to provide a mixture of compounds of the formula 1 wherein HET is 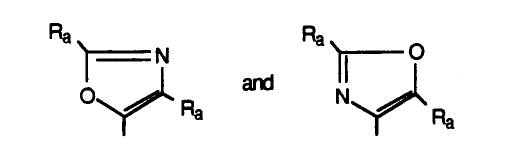 and then, optionally, separating the compounds from each other;
7) dehydrating a compound of the formula 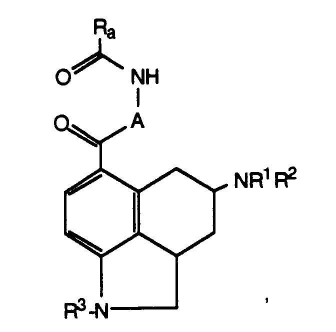 wherein R¹, R² and R³ are as set forth above, A is C-Rₐ or NH, and each Rₐ is independently hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 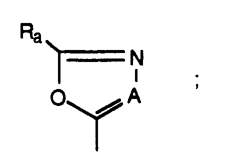
8) cyclizing and dehydrating a compound of the formula 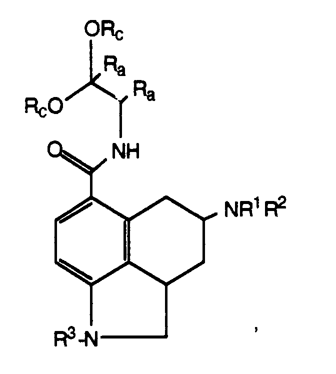 wherein R¹, R² and R³ are as set forth above, Rₐ is hydrogen, C₁-C₃ alkyl, halogen, OH, O(C₁-C₃ alkyl), S(C₁-C₃ alkyl), NH₂, CN or phenyl and R_{c} is hydrogen or C₁-C₃ alkyl, so as to provide a compound of the formula 1 wherein HET is 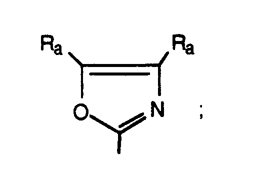
9) reacting a compound of the formula 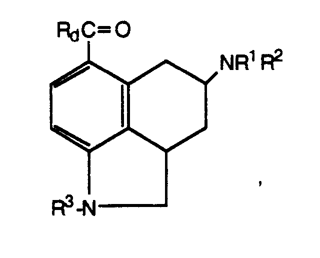 where R¹, R² and R³ are as set forth above and R_{d} is OH, O(C₁-C₃ alkyl), O(phenyl), O(C₁-C₃ alkylphenyl), NH₂, halo, S(C₁-C₃ alkyl), S(phenyl), S(C₁-C₃ alkylphenyl), NH(C₁-C₃ alkyl), N(C₁-C₃ alkyl)₂, OCO(C₁-C₃ alkyl), OCO(phenyl), or OCO(C₁-C₃ alkylphenyl), with 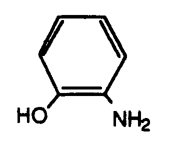 so as to provide a compound of the formula 1 wherein HET is 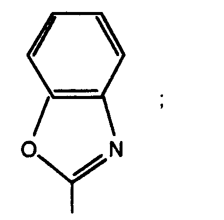
10) cyclizing and dehydrating a compound of the formula 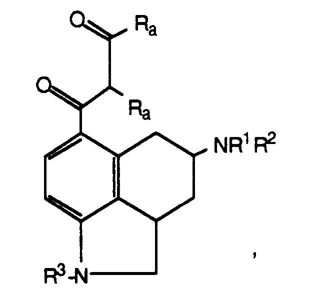 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 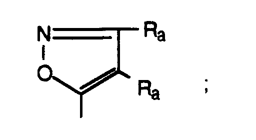
11) cyclizing and dehydrating a compound of the formula 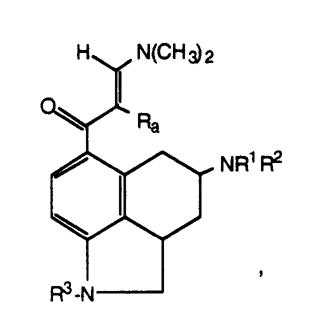 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 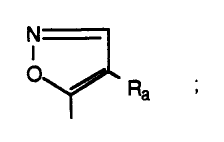
12) cyclizing and dehydrating a compound of the formula 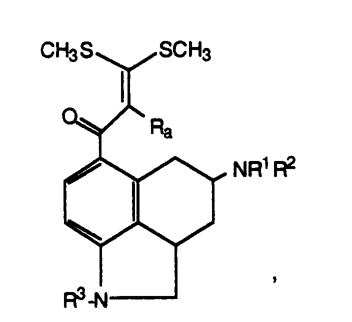 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 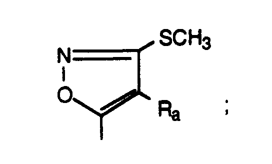
13) cyclizing and dehydrating a dianion of the formula 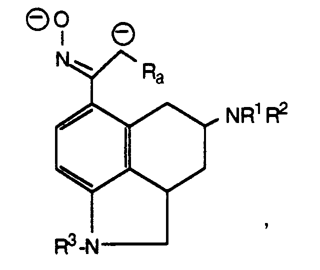 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a carbonyl derivative of the formula RₐCOOR_{c} (where Rₐ is as defined above and R_{c} is hydrogen or C₁-C₃ alkyl) or RₐCON(CH₃)₂ (where Rₐ is as defined above so as to provide a compound of the formula 1 wherein HET is 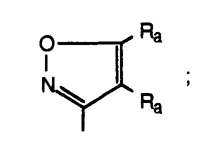
14) cyclizing and dehydrating a compound of the formula 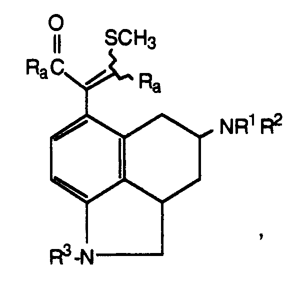 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with hydroxylamine so as to provide a compound of the formula 1 wherein HET is 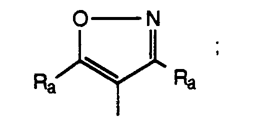
15) reacting a compound of the formula 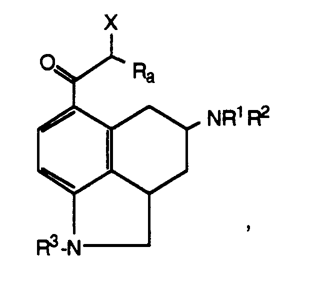 wherein R¹, R² and R³ are as set forth above, X is halo and Rₐ is hydrogen, C₁-C₃ alkyl, halogen, OH, O(C₁-C₃ alkyl), S(C₁-C₃ alkyl), NH₂, CN or phenyl, with 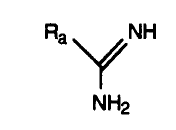 (where Rₐ is as set forth above)
so as to provide a compound of the formula 1 wherein HET is 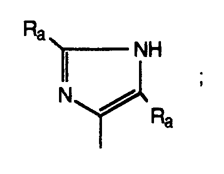
16) reacting a compound of the formula 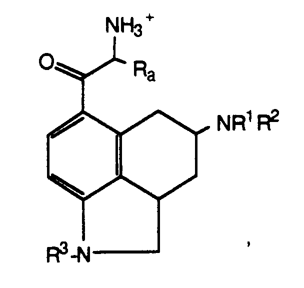 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with KSCN, and then R_{c}X (where R_{c} is hydrogen or C₁-C₃ alkyl and X is halogen) in the presence of a base so as to provide a compound of the formula 1 wherein HET is 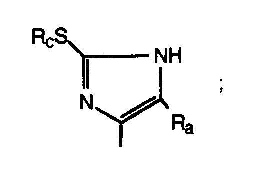
17) dehydrating a compound of the formula 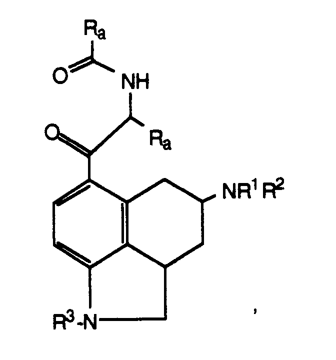 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, in the presence of ammonia or ammonium hydroxide so as to provide a compound of the formula 1 wherein HET is 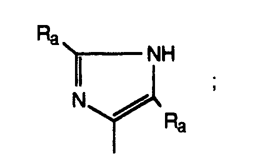
18) reacting a compound of the formula 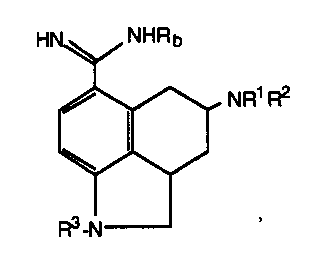 wherein R¹, R² and R³ are as set forth above and R_{b} is hydrogen, C₁-C₃ alkyl, phenyl or (C₁-C₃ alkyl)phenyl, with a compound of the formula 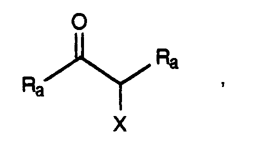 wherein Rₐ is hydrogen, C₁-C₃ alkyl, halogen, OH, O(C₁-C₃ alkyl), S(C₁-C₃ alkyl), NH₂, CN or phenyl, and X is halo, so as to provide a compound of the formula 1 wherein HET is 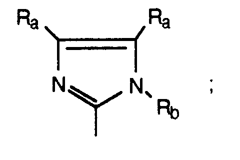
19) dehydrating a compound of the formula 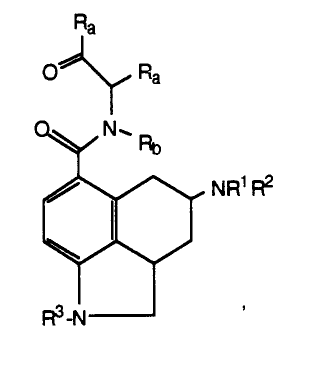 wherein R¹, R² and R³ are as set forth above, Rₐ is hydrogen, C₁-C₃ alkyl, halogen, OH, O(C₁-C₃ alkyl), S(C₁-C₃ alkyl), NH₂, CN or phenyl, and R_{b} is hydrogen, C₁-C₃ alkyl, phenyl or (C₁-C₃ alkyl)phenyl, in the presence of ammonia or ammonium hydroxide so as to provide a compound of the formula 1 wherein HET is 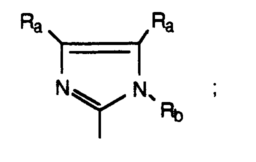
20) reacting a compound of the formula 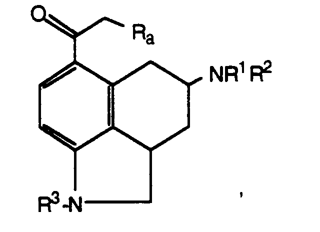 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an azide of the formula R_{b}N₃, where R_{b} is hydrogen, C₁-C₃ alkyl, phenyl or (C₁-C₃ alkyl)phenyl, followed by dehydration of the resulting compound so as to provide a compound of the formula 1 wherein HET is 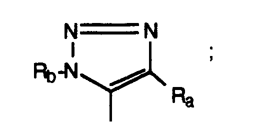
21) cyclizing and dehydrating a compound of the formula 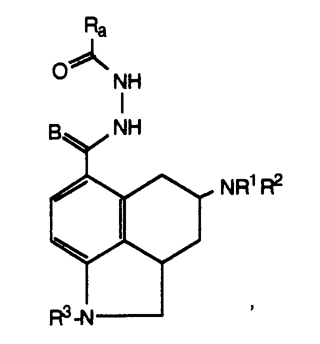 wherein R¹, R² and R³ are as set forth above, Ra is hydrogen, C₁-C₃ alkyl, halogen, OH, O(C₁-C₃ alkyl), S(C₁-C₃ alkyl), NH₂, CN or phenyl, and B is O or NH, so as to provide a compound of the formula 1 wherein HET is 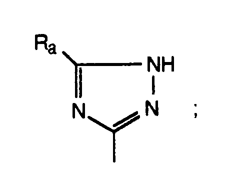 with the proviso that when B is O said cyclization reaction is run in the presence of ammonia or ammonium hydroxide;
22) reacting a compound of the formula 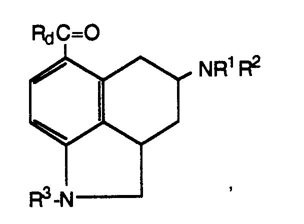 wherein R¹, R² and R³ are as set forth above and R_{d} is OH, O(C₁- C₃ alkyl), O(phenyl), O(C₁-C₃ alkylphenyl), halo, S(C₁-C₃ alkyl), S(phenyl), S(C₁-C₃ alkylphenyl, NH₂, NH(C₁-C₃ alkyl ), N(C₁-C₃ alkyl)₂, OCO(C₁-C₃ alkyl), OCO(phenyl) or OCO(C₁-C₃ alkylphenyl), with a compound of the formula 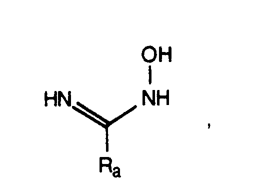 wherein Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 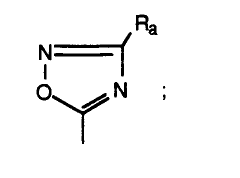
23) reacting a compound of the formula 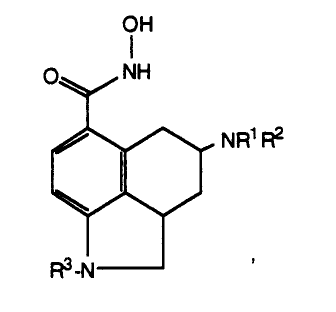 wherein R¹, R² and R³ are as set forth above, with RₐCN, where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 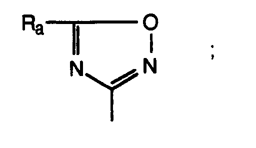
24) cyclizing and dehydrating a compound of the formula 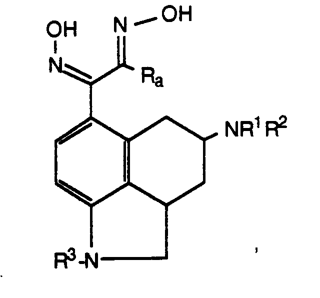 wherein R¹, R² and R³ are as set forth above and Rₐ is hydrogen, C₁-C₃ alkyl, halogen, OH, O(C₁-C₃ alkyl), S(C₁- C₃ alkyl), NH₂, CN or phenyl, so as to provide a compound of the formula 1 wherein HET is 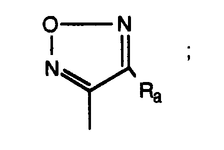
25) reacting a compound of the formula 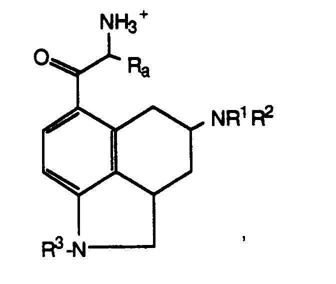 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 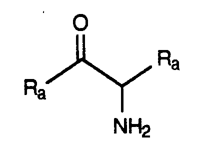 (where Rₐ is as set forth above) in the presence of an oxidizing agent, so as to provide a compound of the formula 1 wherein HET is 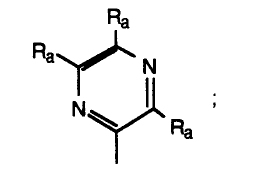
26) reacting a compound of the formula 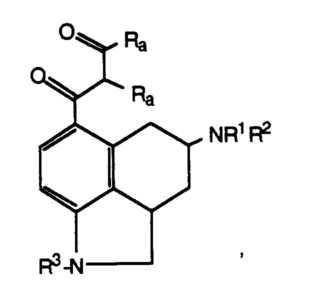 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 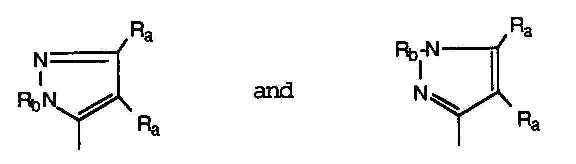 and then, optionally, separating the compounds from each other;
27) reacting a compound of the formula 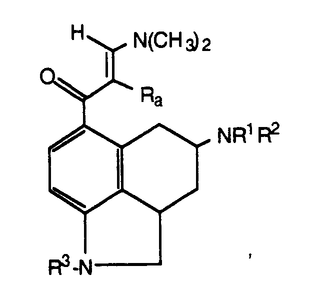 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 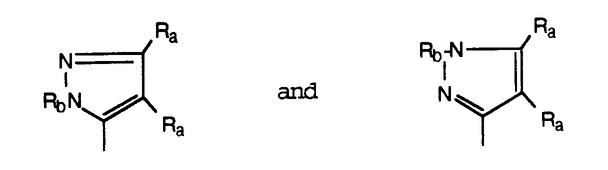 and then, optionally, separating the compounds from each other;
28) reacting a compound of the formula 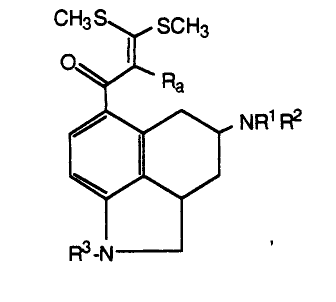 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide compounds of the formula 1 wherein HET is 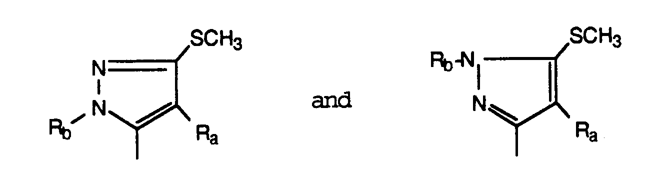 and then, optionally, separating the compounds from each other;
29) reacting a compound of the formula 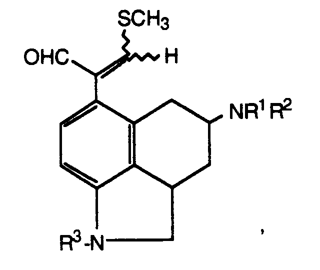 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula H₂NNHR_{b}, where R_{b} is hydrogen, C₁-C₃ alkyl, phenyl or (C₁-C₃ alkyl)phenyl, so as to provide a compound of the formula 1 wherein HET is 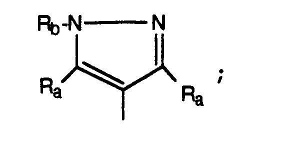
30) reacting a compound of the formula 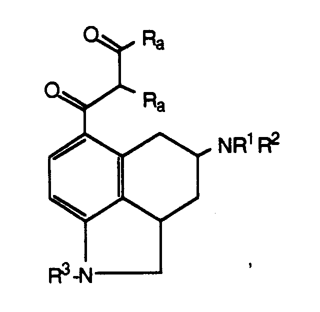 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 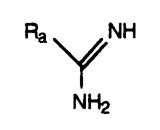 (where Rₐ is as set forth above)
so as to provide a compound of the formula 1 wherein HET is 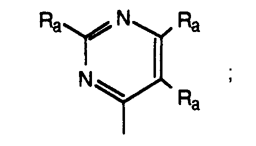
31) reacting a compound of the formula 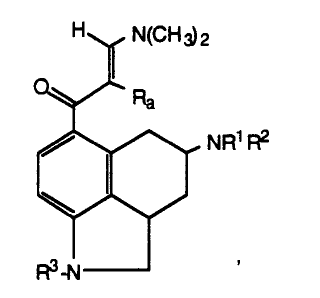 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 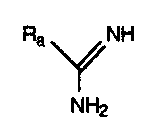 (where Rₐ is as set forth above)
so as to provide a compound of the formula 1 wherein HET is 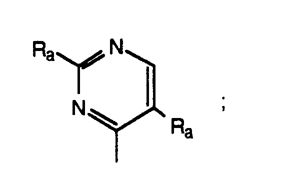
32) reacting a compound of the formula 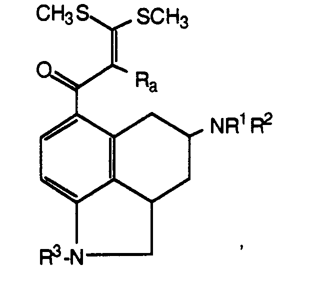 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 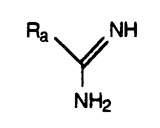 (where Rₐ is as set forth above)
so as to provide a compound of the formula 1 wherein HET is 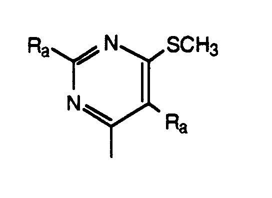
33) reacting a compound of the formula 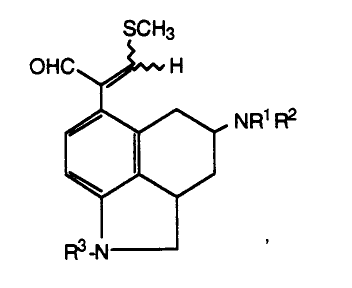 where R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with a compound of the formula 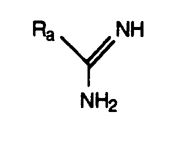 (where Rₐ is as set forth above)
so as to provide a compound of the formula 1 wherein HET is 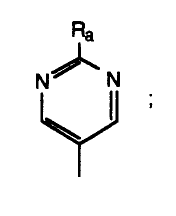
34) reacting a compound of the formula 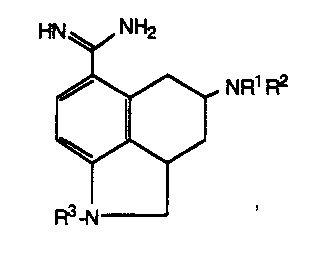 wherein R¹, R² and R³ are as set forth above, with a compound of the formula 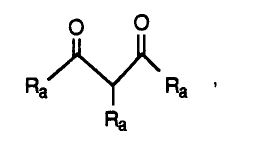 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 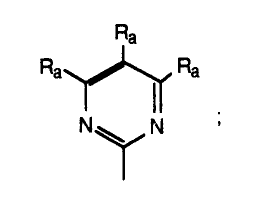
35) reacting a compound of the formula 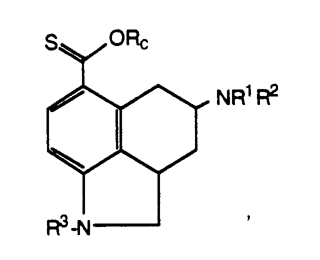 wherein R¹, R² and R³ are as set forth above and R_{c} is hydrogen or C₁-C₃ alkyl, with a compound of the formula RₐCH₂NNH, where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 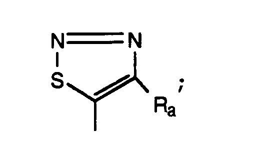
36) reacting a compound of the formula 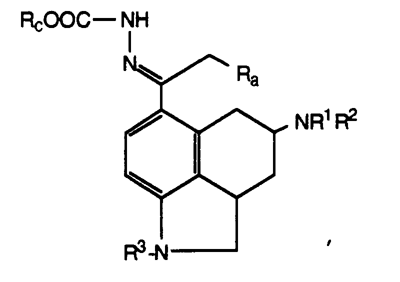 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 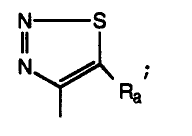
37) reacting a compound of the formula 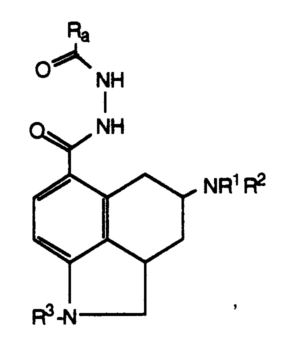 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with P₂S₅ so as to provide a compound of the formula 1 wherein HET is 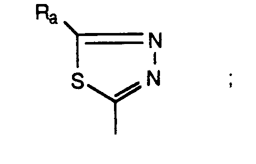
38) reacting a compound of the formula 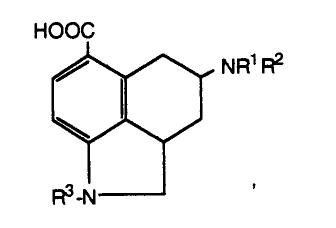 wherein R¹, R² and R³ are as set forth above, with a compound of the formula H₂NNHCSNH₂, in the presence of polyphosphoric acid, so as to provide a compound of the formula 1 wherein HET is 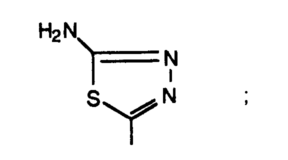
39) reacting a compound of the formula 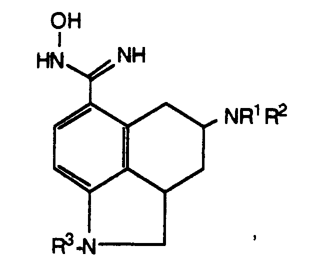 wherein R¹, R² and R³ are as set forth above, with carbon disulfide so as to provide a compound of the formula 1 wherein HET is 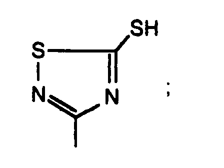
40) reacting a compound of the formula 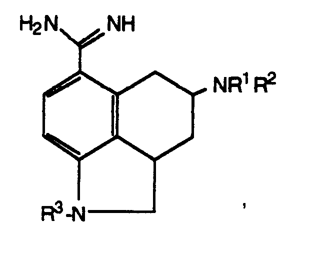 wherein R¹, R² and R³ are as set forth above, with (CNS)₂ so as to provide a compound of the formula 1 wherein HET is 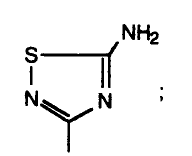
41) oxidizing a compound of the formula 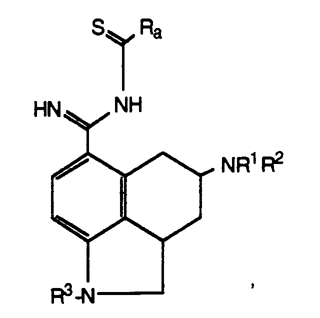 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 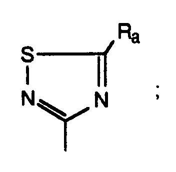
42) reacting a compound of the formula 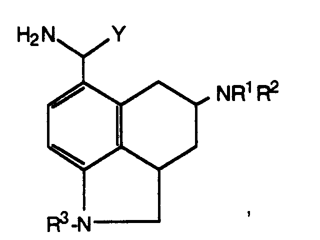 wherein R¹, R² and R³ are as set forth above and Y is -CN or -C(O)NH₂, with an oxidizing agent such as SOCl₂, SCl₂, S₂Cl₂ or SO₂Cl₂ so as to provide a compound of the formula 1 wherein HET is 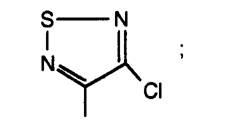
43) reacting a compound of the formula 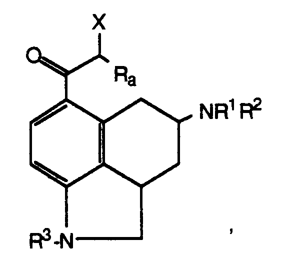 wherein R¹, R² and R³ are as set forth above, X is halogen and Rₐ is hydrogen, C₁-C₃ alkyl, halogen, OH, O(C₁-C₃ alkyl), S(C₁-C₃ alkyl), NH₂, CN or phenyl, with RₐCSNH₂, where Rₐ is as set forth above, so as to provide a compound of the formula 1 wherein HET is 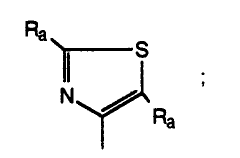
44) reacting a compound of the formula 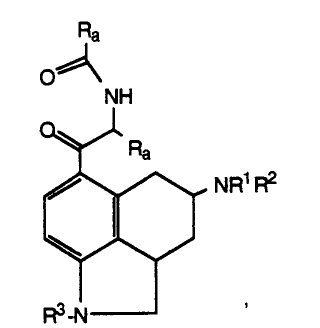 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with P₂S₅ so as to provide a compound of the formula 1 wherein HET is 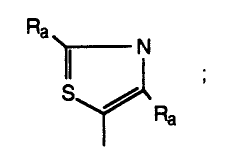
45) reacting a compound of the formula 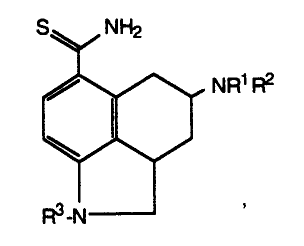 wherein R¹, R² and R³ are as set forth above, with a compound of the formula 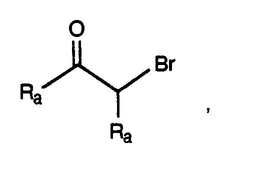 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 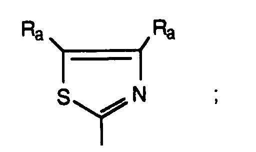
46) reacting a compound of the formula 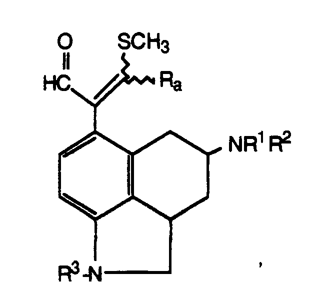 wherein R¹, R² and R³ are as set forth above, and Rₐ is hydrogen, C₁-C₃ alkyl, halogen, OH, O(C₁-C₃ alkyl), S(C₁-C₃ alkyl), NH₂, CN or phenyl, with RₐC(S)NH₂, where Rₐ is as set forth above, so as to provide a compound of the formula 1 wherein HET is 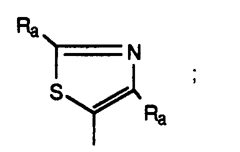
47) reacting a compound of the formula 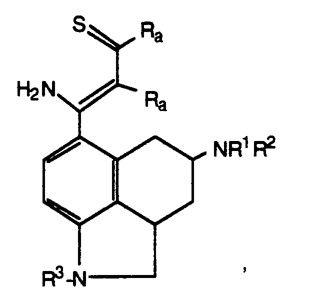 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to prepare a compound of the formula 1 wherein HET is 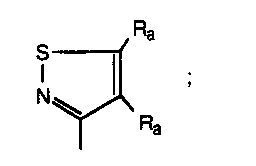
48) reacting a compound of the formula 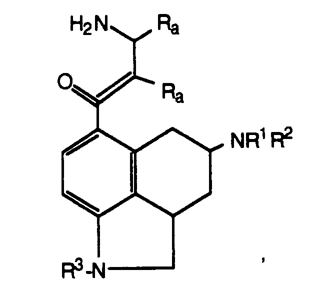 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to provide a compound of the formula 1 wherein HET is 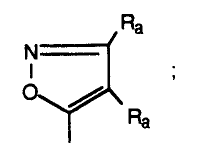
49) reacting a compound of the formula 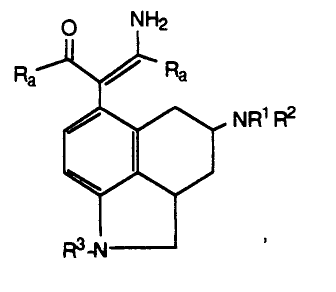 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with an oxidizing agent so as to provide a compound of the formula 1 wherein HET is 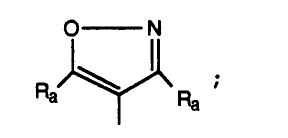
50) reacting a compound of the formula 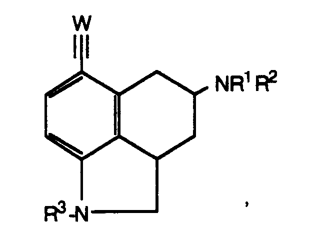 wherein R¹, R² and R³ are as set forth above and W is hydrogen or CHO, with KSCN or NaHSSO₃ and then reacting the intermediate formed thereby with ammonia or ammonium hydroxide so as to provide a compound of the formula 1 wherein HET is 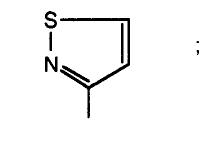
51) reacting a compound of the formula 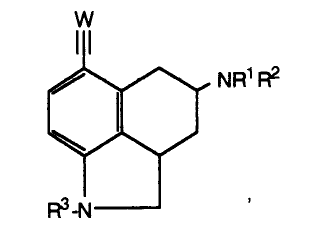 wherein R¹, R² and R³ are as set forth above and W is hydrogen or CHO, with H₂NSSO₃K and then reacting the intermediate formed thereby with a base so as to provide a compound of the formula 1 wherein HET is 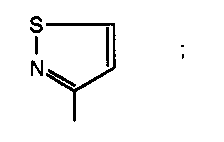
52) reacting a compound of the formula 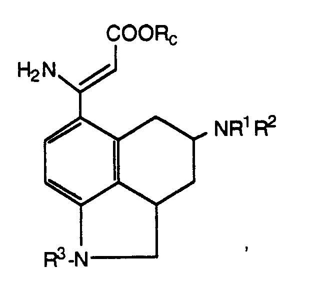 wherein R¹, R² and R³ are as set forth above and R_{c} is hydrogen or C₁-C₃ alkyl, with CSCl₂ so as to provide a compound of the formula 1 wherein HET is 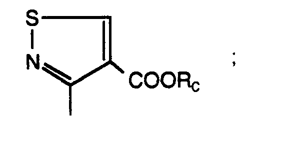
53) reacting a compound of the formula 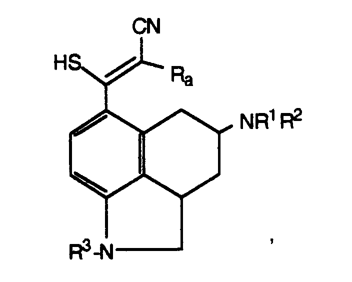 wherein R¹, R² and R³ are as set forth above and Rₐ is hydrogen, C₁-C₃ alkyl, halogen, OH, O(C₁-C₃ alkyl), S(C₁-C₃ alkyl), NH₂, CN or phenyl, with NCS so as to provide a compound of the formula 1 wherein HET is 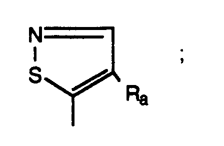
54) reacting a compound of the formula 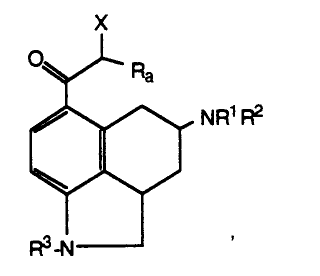 wherein R¹, R² and R³ are as set forth above, X is halogen and Rₐ is hydrogen, C₁-C₃ alkyl, halogen, OH, O(C₁-C₃ alkyl), S(C₁-C₃ alkyl), NH₂, CN or phenyl, with 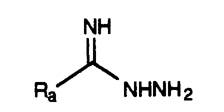 (where Rₐ is as set forth above)
so as to provide a compound of the formula 1 wherein HET is 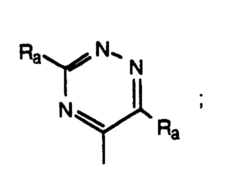
55) reacting a compound of the formula 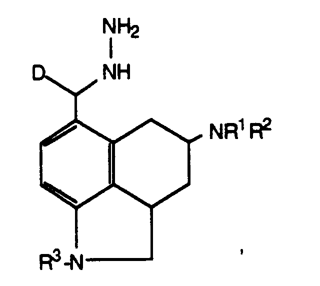 wherein R₁, R₂ and R₃ are as set forth above and D is =NH or =O, with a compound of the formula 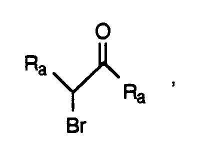 where Rₐ is hydrogen, C₁-C₃ alkyl, halogen, OH, O(C₁-C₃ alkyl), S(C₁-C₃ alkyl), NH₂, CN or phenyl, so as to provide a compound of the formula 1 wherein HET is 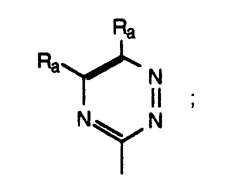
56) reacting a compound of the formula 1 wherein HET is substituted with at least one hydroxy group with POX₃, PX₃, SOX₂, P(phenyl)₃·X₂ or P(C₁-C₃ alkoxy)₃·X₂, where each X is a halogen, so as to convert the hydroxy group to a halogen substituent;
57) reacting a compound of the formula 1 where HET is substituted with at least one amino group with HONO so as to form the corresponding diazonium ion, followed by conversion of such ion to the corresponding halide substituted compound with CuX (where X is halogen), KI or HBF₄ with heat;
58) reacting a compound of the formula 1 wherein HET is substituted with at least one halogen substituent with an alkoxide anion of the formula
(C₁-C₃ alkyl)-O^{⊖}
so as to convert the halogen substituent to an alkoxy substituent;
59) reacting a compound of the formula 1 wherein HET is substituted with at least one hydroxy group with a (C₁-C₃ alkyl)halide so as to convert the hydroxy group to an alkoxy substituent;
60) reacting a compound of the formula 1 wherein HET is substituted with at least one hydroxy substituent with a diazo compound of the formula (C₁-C₃ alkyl)N₂ so as to convert the hydroxy group to an alkoxy substituent;
61) reacting a compound of the formula 1 wherein HET is substituted with at least one amino group with HONO so as to form the corresponding diazonium ion, followed by conversion of such ion to the corresponding hydroxy substituted compound using water or an acid;
62) reacting a compound of the formula 1 wherein HET is substituted with at least one C₁-C₃ alkoxy group with concentrated hydroiodic acid, concentrated hydrobromic acid or a Lewis Acid so as to convert the alkoxy group to a hydroxy substituent;
63) reacting a compound of the formula 1 wherein HET is substituted with at least one amino substituent with HONO so as to form the corresponding diazonium ion, followed by conversion of such ion to the corresponding nitrile substituted compound using CuCN;
64) reacting a compound of the formula 1 wherein HET is substituted with at least one amino substituent with HONO so as to form the corresponding diazonium ion, followed by conversion of such ion to the corresponding C₁-C₃ alkylthio substituted compound using a C₁-C₃ alkyl mercaptan;
65) reacting a compound of the formula 1 wherein HET is substituted with at least one halogen substituent with an alkylthio anion of the formula
(C₁-C₃ alkyl)-S^{⊖}
so as to convert the halogen substituent to an alkylthio moiety;
66) reacting a compound of the formula 1 wherein HET is substituted with at least one -SH moiety with a (C₁-C₃ alkyl)halide so as to provide the corresponding compound of the formula 1 having an alkylthio moiety;
67) reducing a compound of the formula 1 wherein HET is substituted with at least one nitro substituent, so as to provide a compound of the formula 1 wherein the nitro substituent is converted to an amino moiety;
68) reducing a compound of the formula 1 wherein HET is substituted with at least one halogen substituent so as to convert the halogen substituent to a hydrogen atom;
69) reducing a compound of the formula 1 wherein HET is substituted with at least one hydroxy group so as to convert the hydroxy substituent to a hydrogen atom;
70) reacting a compound of the formula 1 wherein HET is substituted with at least one amino moiety with HONO so as to form the corresponding diazonium ion, followed by conversion of such ion to the corresponding unsubstituted compound using H₃PO₂;
71) reducing a compound of the formula 1 wherein HET is substituted on one of the nitrogen atoms in such heterocyclic ring with a C₁-C₃ alkyl phenyl group so as to provide the corresponding compound of the formula 1 wherein the nitrogen atom is unsubstituted;
72) reducing a compound of the formula 1 wherein HET is substituted with at least one C₁-C₃ alkylthio moiety so as to provide the corresponding unsubstituted compound of the formula 1; or
73) reacting a compound of the formula 1 with a pharmaceutically acceptable organic or inorganic acid so as to form a pharmaceutically acceptable acid addition salt of such compound.

2. The process of Claim 1 wherein HET is an isoxazole, a pyrazole, a pyridine, a thiazole, a furan, a thiophene, an oxadiazole or a pharmaceutically acceptable salt thereof.

3. The process of Claim 1 or Claim 2 wherein R¹ and R² are independently C₁-C₃ alkyl or a pharmaceutically acceptable salt thereof.

4. The process of any one of Claims 1 to 3 wherein R³ is hydrogen or a pharmaceutically acceptable salt thereof.

5. The process of Claim 1 or Claim 2 wherein R¹ is -(CH₂)ₙC(O)NR⁵R⁶ wherein n is 2, R⁵ is hydrogen, R⁶ is cyclohexyl, R² is C₁-C₃ alkyl, and R³ is hydrogen or C₁-C₄ alkyl or a pharmaceutically acceptable salt thereof.

6. A process for preparing a substantially pure stereoisomer of a compound of Claim 1 or a pharmaceutically acceptable salt thereof which comprises the process of any one of Claims 1 to 5.

7. The process of Claim 6 wherein the stereoisomer's configuration at position 2a is S and at position 4 is R or a pharmaceutically acceptable salt thereof.

8. A process of Claim 1 which is used to prepare a compound selected from the group consisting of 6-(3-isoxazolyl)-4-(di-n-propylamino)-1,2,2a,3,4,5-hexahydrobenz[cd]indole; 6-(5-isoxazolyl)-4-(di-n-propylamino)-1,2,2a,3,4,5-hexahydrobenz[cd]indole; 6-(3-pyrazolyl)-4-(di-n-propylamino)-1,2,2a,3,4,5-hexahydrobenz[cd]indole; 6-(4-pyrazolyl)-4-(dimethylamino)-1,2,2a,3,4,5-hexahydrobenz[cd]indole; 6-(4-pyridinyl)-4-(di-n-propylamino)-1,2,2a,3,4,5-hexahydrobenz[cd]indole; 6-(2-pyridinyl)-4-(di-n-propylamino)-1,2,2a,3,4,5-hexahydrobenz[cd]indole; 6-(3-pyridinyl)-4-(di-n-propylamino)-1,2,2a,3,4,5-hexahydrobenz[cd]indole; 6-(2-thiazolyl)-4-(di-n-propylamino)-1,2,2a,3,4,5-hexahydrobenz[cd]indole; 6-(5-thiazolyl)-4-(di-n-propylamino)-1,2,2a,3,4,5-hexahydrobenz[cd]indole; 6-(2-oxadiazolyl)-4-(di-n-propylamino)-1,2,2a,3,4,5-hexahydrobenz[cd]indole; 6-(3-furyl)-4-(di-n-propylamino)-1,2,2a,3,4,5-hexahydrobenz[cd]indole or a pharmaceutically acceptable salt thereof.

9. A process for preparing a pharmaceutical formulation which comprises admixing a compound of the formula 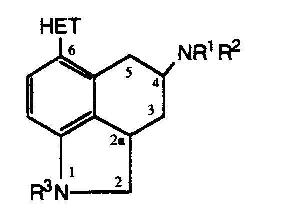 wherein
R¹ is hydrogen, C₁-C₄ alkyl, C₃-C₄ alkenyl, cyclopropylmethyl, phenyl(C₁-C₄)alkyl, phenyl(C₁-C₄)alkyl substituted with one or two substituents selected from (C₁-C₃) alkoxy, halo, hydroxy, (C₁-C₃) thioalkyl, nitro, (C₁-C₃) alkyl and trifluoromethyl, -(CH₂)ₙS(C₁-C₄ alkyl), -C(O)R⁴, -(CH₂)ₙC(O)NR⁵R⁶;
R² is hydrogen, C₁-C₄ alkyl, cyclopropylmethyl or C₃-C₄ alkenyl;
R³ is hydrogen, C₁-C₄ alkyl or an amino protecting group;
n is 1-4;
R⁴ is hydrogen, C₁-C₄ alkyl, C₁-C₄ halo alkyl, C₁-C₄ alkoxy or phenyl;
R⁵ and R⁶ are independently hydrogen, a C₁-C₄ alkyl, or a C₅-C₈ cycloalkyl with the proviso that when one of R⁵ or R⁶ is a cycloalkyl the other is hydrogen;
HET is an aromatic 5- or 6-membered heterocyclic ring, said ring having from one to three heteroatoms which are the same or different and which are selected from the group consisting of sulfur, oxygen, and nitrogen with the proviso that the 6-membered heterocyclic ring can only contain carbon and nitrogen and with the further proviso that the 5-membered ring contains no more than one oxygen or one sulfur but not both oxygen and sulfur, optionally substituted on one or two carbon atoms with (C₁-C₃) alkyl, halo, hydroxy, (C₁-C₃) alkoxy (C₁-C₃) thioalkyl, NH₂, CN or phenyl; or a pharmaceutically acceptable salt thereof, with one or more pharmaceutically acceptable carriers, diluents or excipients therefor.

## Patentansprüche (Patentansprüche für folgende(n) Vertragsstaat(en): AT, BE, CH, DE, DK, FR GB, IT, LI, LU, NL, PT, SE)

1. Verbindung der Formel 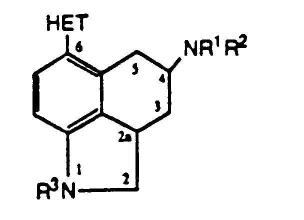 worin
R¹ steht für Wasserstoff, C₁-C₄ Alkyl, C₃-C₄ Alkenyl, Cyclopropylmethyl, Phenyl(C₁-C₄)alkyl, Phenyl(C₁-C₄)alkyl, das mit einem oder mehreren Substituenten substituiert ist, ausgewählt aus (C₁-C₃) Alkoxy, Halogen, Hydroxy, (C₁-C₃) Thioalkyl, Nitro, (C₁-C₃) Alkyl und Trifluormethyl, -(CH₂)ₙS(C₁-C₄ Alkyl), -C(O)R⁴, -(CH₂)ₙC(O)NR⁵R⁶,
R² für Wasserstoff, C₁-C₄ Alkyl, Cyclopropylmethyl oder C₃-C₄ Alkenyl steht,
R³ für Wasserstoff, C₁-C₄ Alkyl oder eine Aminoschutzgruppe steht,
n für 1-4 steht,
R⁴ für Wasserstoff, C₁-C₄ Alkyl, C₁-C₄ Halogenalkyl, C₁-C₄ Alkoxy oder Phenyl steht,
R⁵ und R⁶ unabhängig für Wasserstoff, C₁-C₄ Alkyl oder C₅-C₈ Cycloalkyl stehen mit der Maßgabe, daß wenn eines von R⁵ und R⁶ für ein Cycloalkyl steht, das andere für Wasserstoff steht,
HET für einen aromatischen fünf- oder sechsgliedrigen heterocyclischen Ring steht, wobei der Ring ein bis drei Heteroatome aufweist, die gleich oder verschieden sind und ausgewählt sind aus der Gruppe, die besteht aus Schwefel, Sauerstoff und Stickstoff, mit der Maßgabe, daß der sechsgliedrige heterocyclische Ring nur Kohlenstoff und Stickstoff enthalten kann und mit der weiteren Maßgabe, daß der fünfgliedrige Ring nicht mehr als einen Sauerstoff oder einen Schwefel aber nicht sowohl Sauerstoff als auch Schwefel enthält, und wahlweise an einem oder zwei Kohlenstoffatomen substituiert ist durch (C₁-C₃) Alkyl, Halogen, Hydroxy, (C₁-C₃) Alkoxy, (C₁-C₃) Thioalkyl, NH₂, CN oder Phenyl,
oder pharmazeutisch annehmbare Salze hiervon.

2. Verbindung nach Anspruch 1, worin HET für ein Isoxazol, ein Pyrazol, ein Pyridin, ein Thiazol, ein Furan, ein Thiophen, ein Oxadiazol steht oder ein pharmazeutisch annehmbares Salz hiervon.

3. Verbindung nach Anspruch 1 oder Anspruch 2, worin R¹ und R² unabhängig für C₁-C₃ Alkyl stehen oder ein pharmazeutisch annehmbares Salz hiervon.

4. Verbindung nach einem der Ansprüche 1 bis 3, worin R³ für Wasserstoff steht oder ein pharmazeutisch annehmbares Salz hiervon.

5. Verbindung nach Anspruch 1 oder Anspruch 2, worin R¹ für -(CH₂)ₙC(O)NR⁵R⁶ steht, worin n für 2 steht, R⁵ für Wasserstoff steht, R⁶ für Cyclohexyl steht, R² für C₁-C₃ Alkyl steht und R³ für Wasserstoff oder C₁-C₄ Alkyl steht oder ein pharmazeutisch annehmbares Salz hiervon.

6. Im wesentlichen reines Stereoisomer einer Verbindung nach einem der Ansprüche 1 bis 5 oder ein pharmazeutisch annehmbares Salz hiervon.

7. Stereoisomer nach Anspruch 6, worin die Konfiguration an der Position 2a S ist und an der Position 4 R ist oder ein pharmazeutisch annehmbares Salz hiervon.

8. Verbindung nach Anspruch 1, ausgewählt aus der Gruppe, die besteht aus 6-(3-Isoxazolyl)-4-(di-n-propylamino)-1,2,2a,3,4,5-hexahydrobenz[cd]indol, 6-(5-Isoxazolyl)-4-(di-n-propylamino)-1,2,2a,3,4,5-hexahydrobenz[cd]indol, 6-(3-Pyrazolyl)-4-(di-n-propylamino)-1,2,2a,3,4,5-hexahydrobenz[cd]indol, 6-(4-Pyrazolyl)-4-(dimethylamino)-1,2,2a,3,4,5-hexahydrobenz[cd]indol, 6-(4-Pyridinyl)-4-(di-n-propylamino)-1,2,2a,3,4,5-hexahydrobenz[cd]indol,6-(2-Pyridinyl)-4-(di-n-propylamino)-1,2,2a,3,4,5-hexahydrobenz[cd]indol, 6-(3-Pyridinyl)-4-(di-n-propylamino)-1,2,2a,3,4,5-hexahydrobenz[cd]indol, 6-(2-Thiazolyl)-4-(di-n-propylamino)-1,2,2a,3,4,5-hexahydrobenz[cd]indol, 6-(5-Thiazolyl)-4-(di-n-propylamino)-1,2,2a,3,4,5-hexahydrobenz[cd]indol, 6-(2-Oxadiazolyl)-4-(di-n-propylamino)-1,2,2a,3,4,5-hexahydrobenz[cd]indol, 6-(3-Furyl)-4-(di-n-propylamino)-1,2,2a,3,4,5-hexahydrobenz[cd]indol oder ein pharmazeutisch annehmbares Satz hiervon.

9. Pharmazeutische Formulierung, die als Wirkstoff eine Verbindung nach einem der Ansprüche 1 bis 8 oder ein pharmazeutisch annehmbares Salz hiervon zusammen mit einem oder mehreren pharmazeutisch annehmbaren Trägern, Verdünnungsmitteln oder Hilfsstoffen hierfür enthält.

10. Verbindung nach einem der Ansprüche 1 bis 8 zur Verwendung bei der Behandlung von Störungen, die mit Serotonin zusammenhängen.

11. Verfahren zur Herstellung einer Verbindung der Formel 1 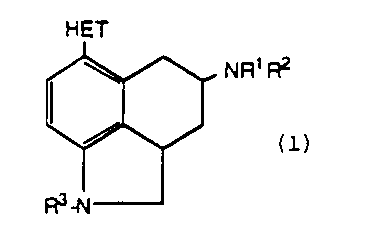 worin
R¹ steht für Wasserstoff, C₁-C₄ Alkyl, C₃-C₄ Alkenyl, Cyclopropylmethyl, Phenyl(C₁-C₄)alkyl, Phenyl(C₁-C₄)alkyl, das mit einem oder mehreren Substituenten substituiert ist, ausgewählt aus (C₁-C₃) Alkoxy, Halogen, Hydroxy, (C₁-C₃) Thioalkyl, Nitro, (C₁-C₃) Alkyl und Trifluormethyl, -(CH₂)ₙS(C₁-C₄ Alkyl), -C(O)R⁴, -(CH₂)ₙC(O)NR⁵R⁶,
R² für Wasserstoff, C₁-C₄ Alkyl, Cyclopropylmethyl oder C₃-C₄ Alkenyl steht,
R³ für Wasserstoff, C₁-C₄ Alkyl oder eine Aminoschutzgruppe steht,
n für 1-4 steht,
R⁴ für Wasserstoff C₁-C₄ Alkyl, C₁-C₄ Halogenalkyl, C₁-C₄ Alkoxy oder Phenyl steht,
R⁵ und R⁶ unabhängig für Wasserstoff, C₁-C₄ Alkyl oder C₅-C₈ Cycloalkyl stehen mit der Maßgabe, daß wenn eines von R⁵ und R⁶ für ein Cycloalkyl steht, das andere für Wasserstoff steht,
HET für einen aromatischen fünf- oder sechsgliedrigen heterocyclischen Ring steht, wobei der Ring ein bis drei Heteroatome aufweist, die gleich oder verschieden sind und ausgewählt sind aus der Gruppe, die besteht aus Schwefel, Sauerstoff und Stickstoff, mit der Maßgabe, daß der sechsgliedrige heterocyclische Ring nur Kohlenstoff und Stickstoff enthalten kann und mit der weiteren Maßgabe, daß der fünfgliedrige Ring nicht mehr als einen Sauerstoff oder einen Schwefel aber nicht sowohl Sauerstoff als auch Schwefel enthält, und wahlweise an einem oder zwei Kohlenstoffatomen substituiert ist durch (C₁-C₃) Alkyl, Halogen, Hydroxy, (C₁-C₃) Alkoxy, (C₁-C₃) Thioalkyl, NH₂, CN oder Phenyl,
oder eines pharmazeutisch annehmbaren Salzes hiervon,
gekennzeichnet durch
1) Umsetzung eines 4-amino-6-metall-substituierten Hexahydrobenz[cd]indols der Formel 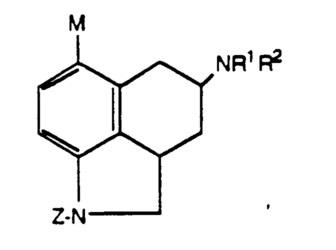 worin R¹ und R² wie vorher definiert sind, Z für eine Aminoschutzgruppe steht und M für einen Metallrest steht, mit einer heterocyclischen Verbindung der Formel
HET-L
worin HET wie vorher definiert ist und L für eine Abgangsgruppe steht,
2) Abspalten der Schutzgruppen von einer Verbindung der Formel 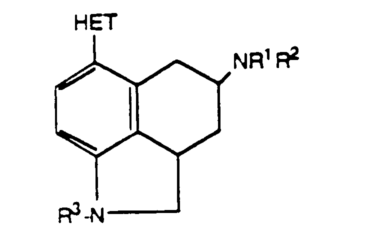 worin HET, R¹ und R² wie oben definiert sind und R³ für eine Aminoschutzgruppe steht unter Bereitstellung einer Verbindung der Formel 1, worin R³ für Wasserstoff steht,
3) Umsetzung eines 4-amino-6-halogen-substituierten Hexahydrobenz[cd]indols der Formel 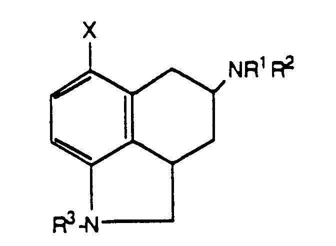 worin R¹, R² und R³ wie oben definiert sind und X für Halogen steht,
mit einem Organometallderivat der Formel
M-HET
worin HET wie oben definiert ist und M für Lithium, Magesium, Zink, Zinn, Quecksilber oder Borsäure steht,
4) Umsetzung einer Verbindung der Formel 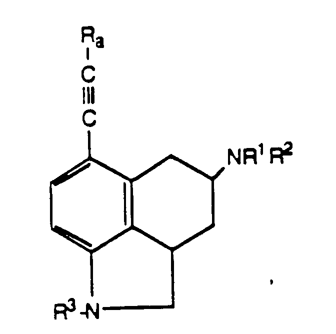 worin R¹, R² und R³ wie oben definiert sind und Rₐ für Wasserstoff, C₁-C₃ Alkyl, Halogen, O(C₁-C₃ Alkyl), S(C₁-C₃ Alkyl), CN oder Phenyl steht, mit einem 1,3-Dipol der Formel
⁺T=U-V⁻
worin T, U und V aus der folgenden Liste ausgewählt sein können
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
worin Rₐ wie oben definiert ist, Rₐ' für Wasserstoff, C₁-C₃ Alkyl, Halogen, O(C₁-C₃ Alkyl), S(C₁-C₃ Alkyl), CN oder Phenyl steht und R_{b} für Wasserstoff, C₁-C₃ Alkyl, Phenyl oder (C₁-C₃ Alkyl)phenyl steht, unter Bildung einer Verbindung der Formel 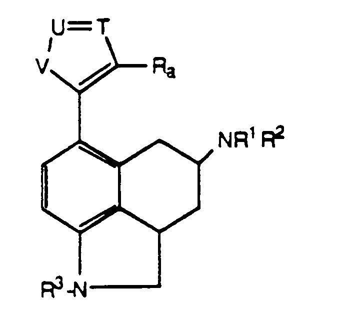 worin R¹, R², R³, Rₐ, T, U und V wie oben definiert sind,
5) Umsetzung einer Verbindung der Formel 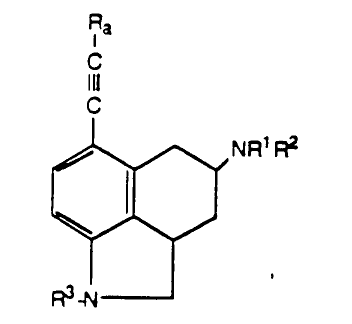 worin R¹, R² und R³ wie oben definiert sind und Rₐ für Wasserstoff, C₁-C₃ Alkyl, Halogen, O(C₁-C₃ Alkyl), S(C₁-C₃ Alkyl), CN oder Phenyl steht, mit einem 1,3-Dipol der Formel
⁺T-U=V⁻
worin T, U und V aus folgenden ausgewählt sind
| T | U | V |
|---|---|---|
| CHRₐ | N | N |
| NR_{b} | N | N |
worin Rₐ wie oben definiert ist und R_{b} für Wasserstoff, C₁-C₃ Alkyl, Phenyl oder (C₁-C₃ Alkyl)phenyl steht, unter Bildung einer Verbindung der Formel 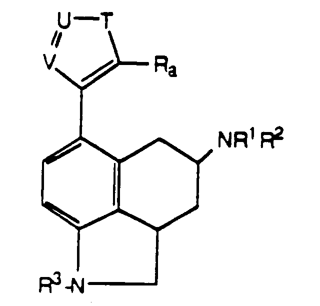 worin R¹, R², R³, Rₐ, V, U und T wie oben definiert sind,
6) Umsetzung einer Verbindung der Formel 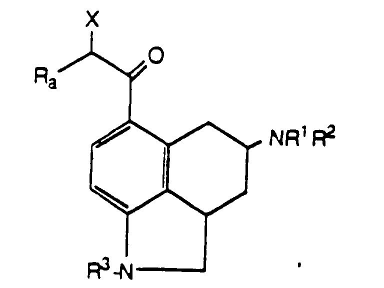 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht,
mit NH₄⁺RₐCOO⁻ (worin Rₐ wie oben definiert ist) unter Bildung eines Gemisches der Verbindungen der Formel 1, worin HET steht für 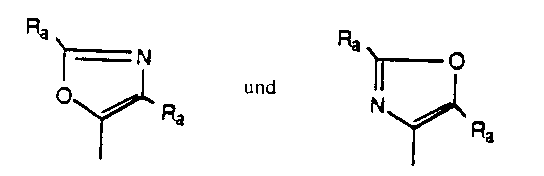 und anschließend wahlweise Trennung der Verbindungen voneinander,
7) Dehydrierung einer Verbindung der Formel 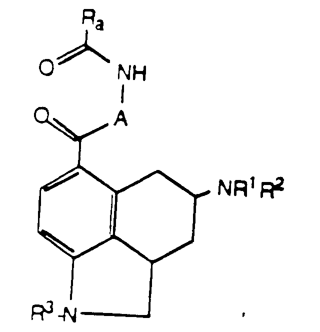 worin R¹, R² und R³ wie oben definiert sind, A für C-Rₐ oder NH steht und jedes Rₐ unabhängig stellt für Wasserstoff, C₁-C₃ Alkyl, Halogen, OH, O(C₁-C₃ Alkyl), S(C₁-C₃ Alkyl), NH₂, CN oder Phenyl, unter Bildung einer Verbindung der Formel 1, worin HET steht für 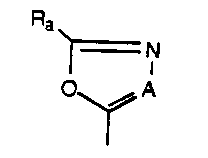
8) Cyclisierung und Dehydrierung einer Verbindung der Formel 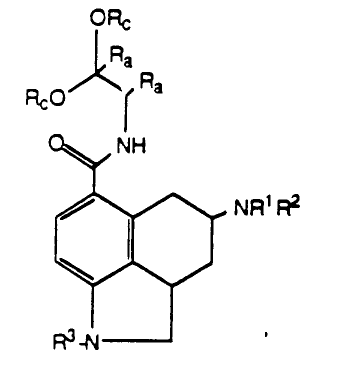 worin R¹, R² und R³ wie oben definiert sind, Rₐ steht für Wasserstoff C₁-C₃ Alkyl, Halogen, OH, O(C₁-C₃ Alkyl), S(C₁-C₃Alkyl), NH₂, CN oder Phenyl und R_{c} für Wasserstoff oder C₁-C₃ Alkyl steht, unter Bildung einer Verbindung der Formel 1, worin HET steht für 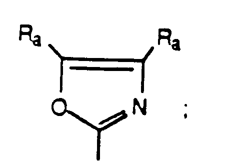
9) Umsetzung einer Verbindung der Formel 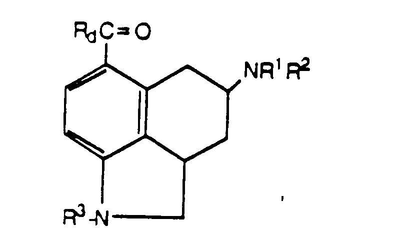 worin R¹, R² und R³ wie oben definiert sind und R_{d} steht für OH, O(C₁-C₃ Alkyl), O(Phenyl), O(C₁-C₃ Alkylphenyl), Halogen, S(C₁-C₃ Alkyl), S(Phenyl), S(C₁-C₃ Alkylphenyl), NH₂, NH(C₁-C₃ Alkyl), N(C₁-C₃ Alkyl)₂, OCO(C₁-C₃ Alkyl), OCO(Phenyl) oder OCO(C₁-C₃ Alkylphenyl) mit 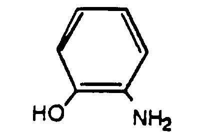 unter Bildung einer Verbindung der Formel 1, worin HET steht für 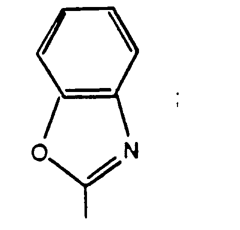
10) Cyclisierung und Dehydrierung einer Verbindung der Formel 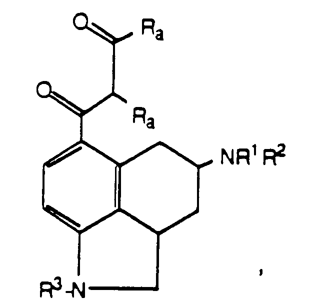 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 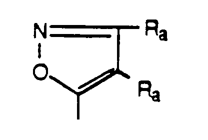
11) Cyclisierung und Dehydrierung einer Verbindung der Formel 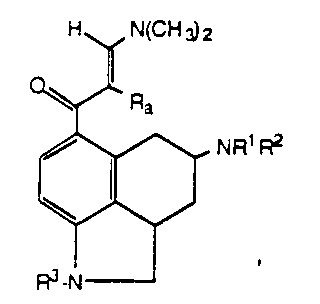 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 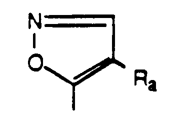
12) Cyclisierung und Dehydrierung einer Verbindung der Formel 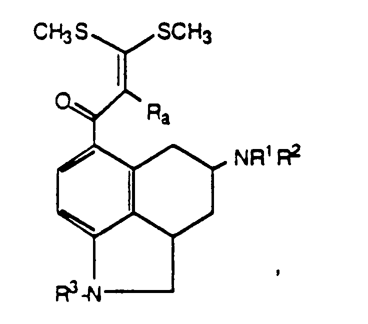 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 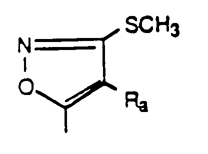
13) Cyclisierung und Dehydrierung eines Dianions der Formel 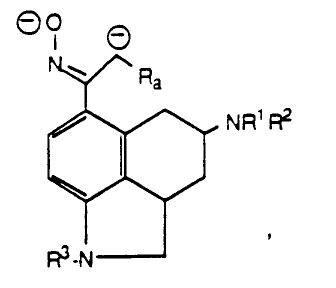 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht,
mit einem Carbonylderivat der Formel RₐCOOR_{c} (worin Rₐ wie oben definiert ist und R_{c} für Wasserstoff oder C₁-C₃ Alkyl steht) oder RₐCON(CH₃)₂ (worin Rₐ wie oben definiert ist) unter Bildung einer Verbindung der Formel 1, worin HET steht für 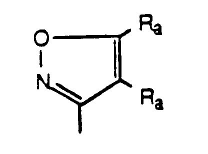
14) Cyclisierung und Dehydrierung einer Verbindung der Formel 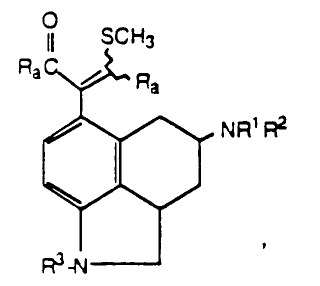 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht,
mit Hydroxylamin unter Bildung einer Verbindung der Formel 1, worin HET steht für 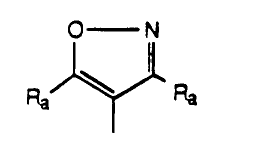
15) Umsetzung einer Verbindung der Formel 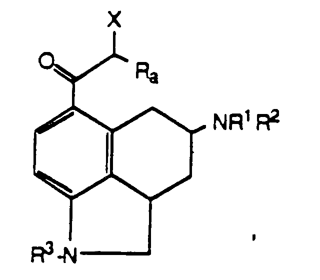 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht mit 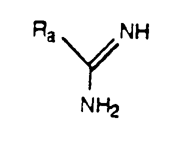 (worin Rₐ wie oben definiert ist)
unter Bildung einer Verbindung der Formel 1, worin HET steht für 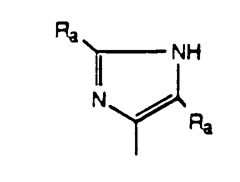
16) Umsetzung einer Verbindung der Formel 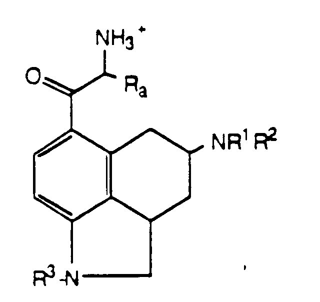 worin R¹, R² und R³ wie oben deinen sind und Rₐ für Wasserstoff C₁-C₃ Alkyl, Halogen, OH, O(C₁-C₃ Alkyl), S(C₁-C₃ Alkyl), NH₂, CN oder Phenyl steht,
mit KSCN und anschließend R_{c}X (worin R_{c} für Wasserstoff oder C₁-C₃ Alkyl steht und X für Halogen steht) in Gegenwart einer Base unter Bildung einer Verbindung der Formel 1, worin HET steht für 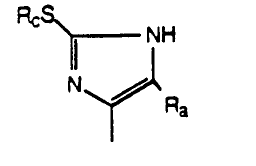
17) Dehydrierung einer Verbindung der Formel 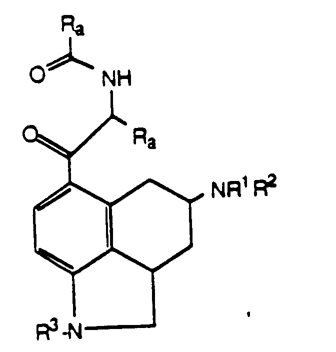 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht in Gegenwart von Ammoniak oder Ammoniumhydroxid unter Bildung einer Verbindung der Formel 1, worin HET steht für 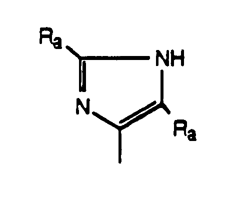
18) Umsetzung einer Verbindung der Formel 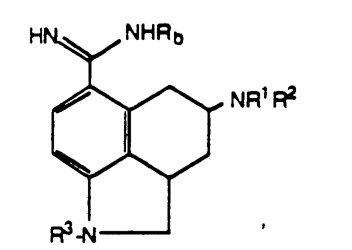 worin R¹, R² und R³ wie oben definiert sind und R_{b} steht für Wasserstoff, C₁-C₃ Alkyl, Phenyl oder (C₁-C₃ Alkyl)phenyl mit einer Verbindung der Formel 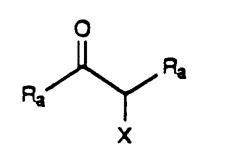 worin Rₐ für Wasserstoff, C₁-C₃ Alkyl, Halogen, OH, O(C₁-C₃ Alkyl), S(C₁-C₃ Alkyl), NH₂, CN oder Phenyl steht und X für Halogen steht, unter Bildung einer Verbindung der Formel 1, worin HET steht für 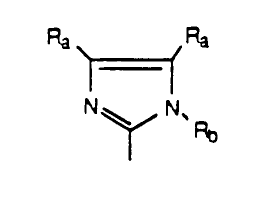
19) Dehydrierung einer Verbindung der Formel 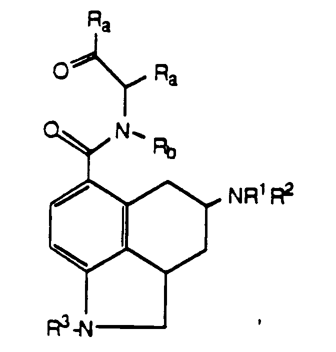 worin R₁, R₂ und R₃ wie oben definiert sind, Rₐ für Wasserstoff, C₁-C₃ Alkyl, Halogen, OH, O(C₁-C₃ Alkyl), S(C₁-C₃ Alkyl), NH₂, CN oder Phenyl stellt und R_{b} für Wasserstoff, C₁-C₃ Alkyl, Phenyl oder (C₁-C₃ Alkyl)phenyl steht in Gegenwart von Ammoniak oder Ammoniumhydroxid unter Bildung einer Verbindung der Formel 1, worin HET steht für 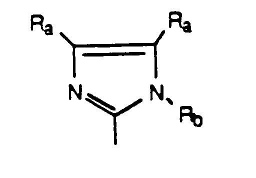
20) Umsetzung einer Verbindung der Formel 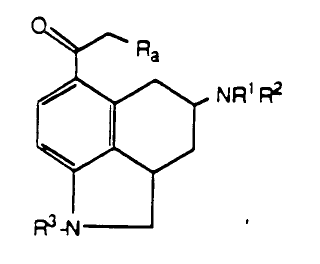 worin R¹, R² und R³ wie oben definiert sind und Rₐ für Waserstoff, C₁-C₃ Alkyl, Halogen, OH, O(C₁-C₃ Alkyl), S(C₁-C₃ Alkyl), NH₂, CN oder Phenyl steht, mit einem Azid der Formel R_{b}N₃, worin R_{b} für Wasserstoff, C₁-C₃ Alkyl, Phenyl oder (C₁-C₃ Alkyl)phenyl steht gefolgt von einer Dehydrierung der entstehenden Verbindung unter Bildung einer Verbindung der Formel 1, worin HET steht für 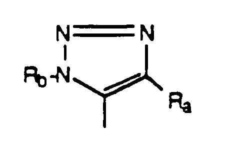
21) Cyclisierung und Dehydrierung einer Verbindung der Formel 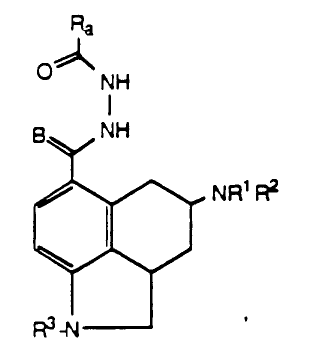 worin R¹, R² und R³ wie oben definiert sind, Rₐ für Wasserstoff, C₁-C₃ Alkyl, Halogen, OH, O(C₁-C₃ Alkyl), S(C₁-C₃ Alkyl), NH₂, CN oder Phenyl steht und B für O oder NH steht unter Bildung einer Verbindung der Formel 1, worin HET stellt für 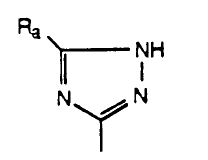 mit der Maßgabe, daß wenn B für O steht, diese Cyclisierungsreaktion in Gegenwart von Ammoniak oder Ammoniumhydroxid durchgeführt wird,
22) Umsetzung einer Verbindung der Formel 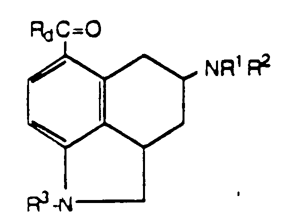 worin R¹, R² und R³ wie oben definiert sind und R_{d} für OH, O(C₁-C₃ Alkyl), O(Phenyl), O(C₁-C₃ Alkylphenyl), Halogen, S(C₁-C₃ Alkyl), S(Phenyl), S(C₁-C₃ Alkylphenyl), NH₂, NH(C₁-C₃ Alkyl), N(C₁-C₃ Alkyl)₂, OCO(C₁-C₃ Alkyl, OCO(Phenyl) oder OCO(C₁-C₃ Alkylphenyl) steht,
mit einer Verbindung der Formel 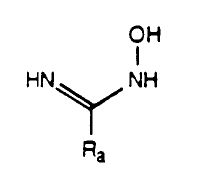 worin Rₐ für Wasserstoff C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 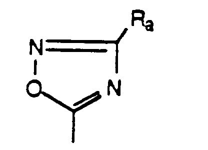
23) Umsetzung einer Verbindung der Formel 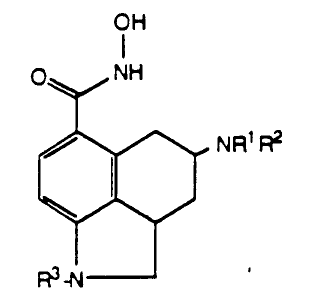 worin R¹, R² und R³ wie oben definiert sind mit RₐCN, worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 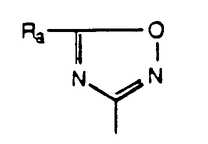
24) Cyclisierung und Dehydrierung einer Verbindung der Formel 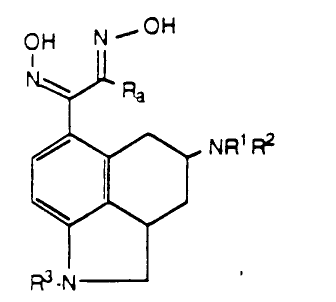 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 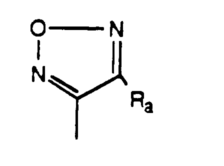
25) Umsetzung einer Verbindung der Formel 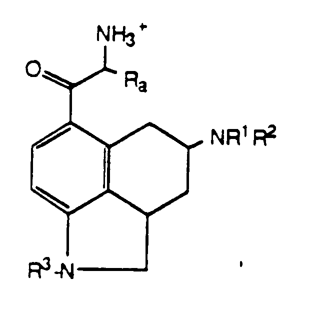 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 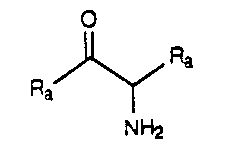 (worin Rₐ wie oben definiert ist)
in Gegenwart eines Oxidationsmittels unter Bildung einer Verbindung der Formel 1, worin HET steht für 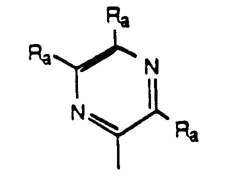
26) Umsetzung einer Verbindung der Formel 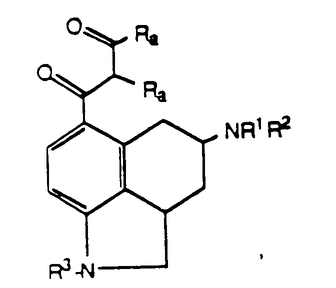 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 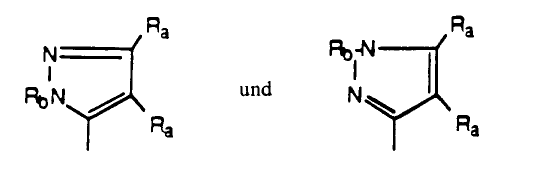 und anschließend wahlweise Trennung der Verbindungen voneinander,
27) Umsetzung einer Verbindung der Formel 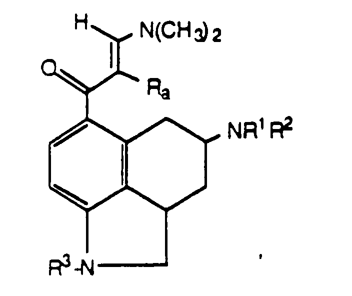 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 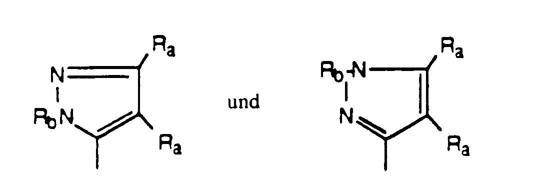 und anschließend wahlweise Trennung der Verbindungen voneinander,
28) Umsetzung einer Verbindung der Formel 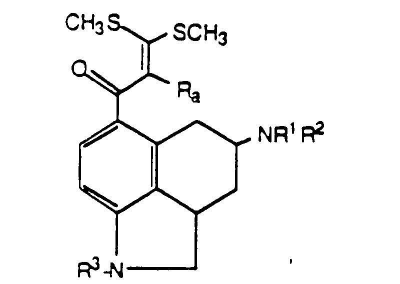 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 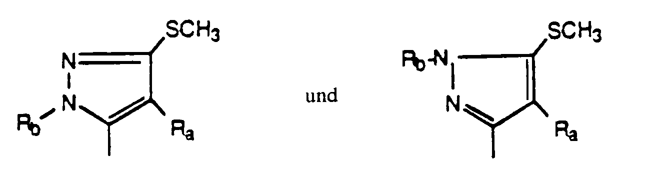 und anschließend wahlweise Trennung der Verbindungen voneinander,
29) Umsetzung einer Verbindung der Formel 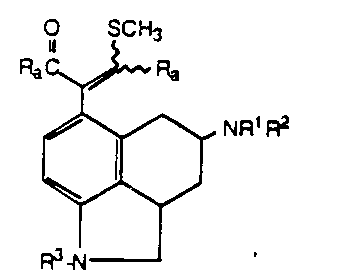 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung einer Verbindung der Formel 1, worin HET steht für 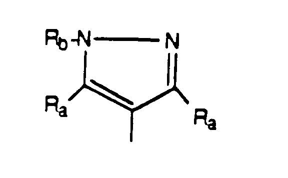
30) Umsetzung einer Verbindung der Formel 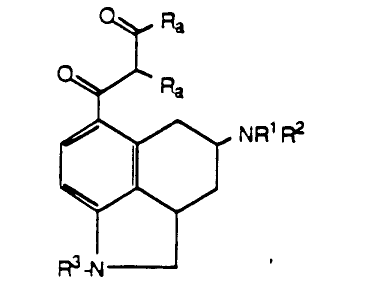 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht, mit einer Verbindung der Formel 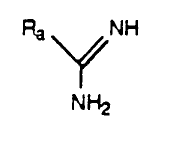 (worin Rₐ wie oben definiert ist)
unter Bildung einer Verbindung der Formel 1, worin HET steht für 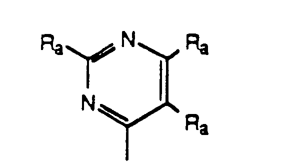
31) Umsetzung einer Verbindung der Formel 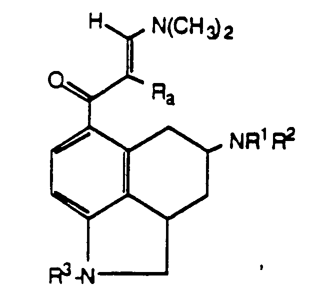 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 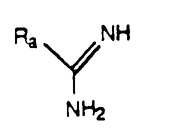 (worin Rₐ wie oben definiert ist)
unter Bildung einer Verbindung der Formel 1, worin HET steht für 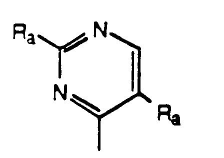
32) Umsetzung einer Verbindung der Formel 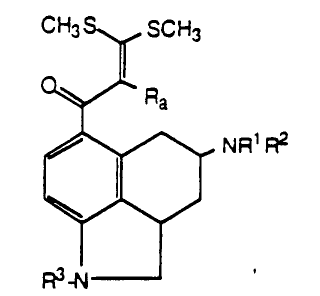 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht, mit einer Verbindung der Formel 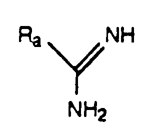 (worin Rₐ wie oben definiert ist)
unter Bildung einer Verbindung der Formel 1, worin HET steht für 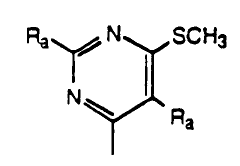
33) Umsetzung einer Verbindung der Formel 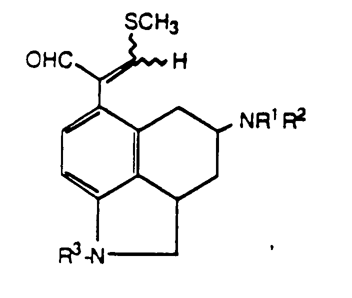 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 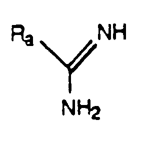 (worin Rₐ wie oben definiert ist)
unter Bildung einer Verbindung der Formel 1, worin HET steht für 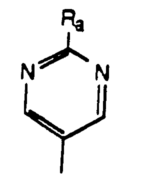
34) Umsetzung einer Verbindung der Formel 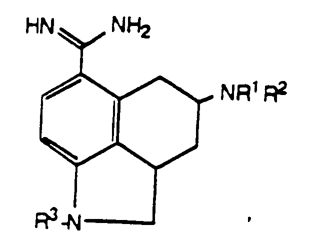 worin R¹, R² und R³ wie oben definiert sind, mit einer Verbindung der Formel 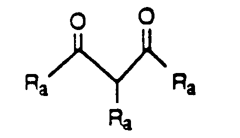 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 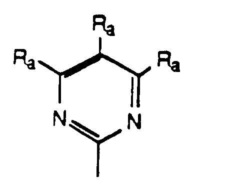
35) Umsetzung einer Verbindung der Formel 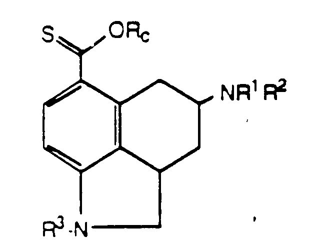 worin R¹, R² und R³ wie oben definiert sind und R_{c} für Wasserstoff oder C₁-C₃ Alkyl steht, mit einer Verbindung der Formel RₐCH₂NNH, worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 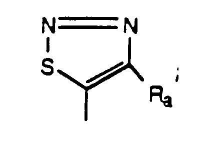
36) Umsetzung einer Verbindung der Formel 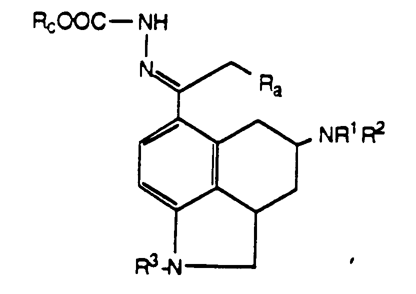 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 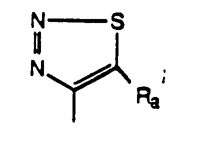
37) Umsetzung einer Verbindung der Formel 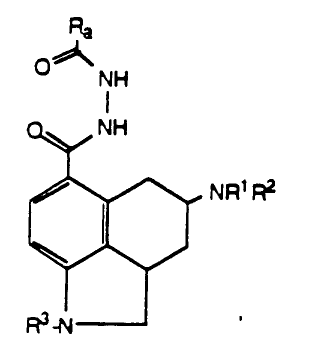 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit P₂S₅ unter Bildung einer Verbindung der Formel 1, worin HET steht für 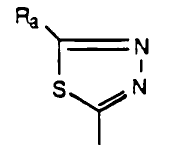
38) Umsetzung einer Verbindung der Formel 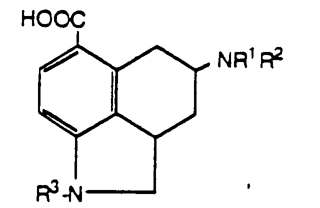 worin R¹, R² und R³ wie oben definiert sind mit einer Verbindung der Formel H₂NNHCSNH₂ in Gegenwart von Polyphosphorsäure unter Bildung einer Verbindung der Formel 1, worin HET steht für 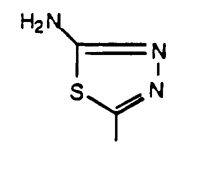
39) Umsetzung einer Verbindung der Formel 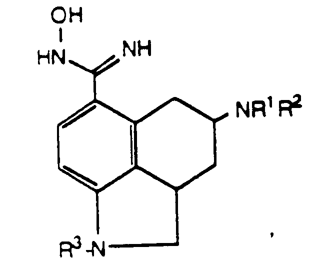 worin R¹, R² und R³ wie oben definiert sind, mit Schwefelkohlenstoff unter Bildung einer Verbindung der Formel 1, worin HET steht für 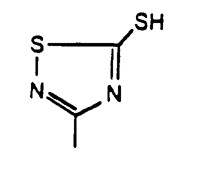
40) Umsetzung einer Verbindung der Formel 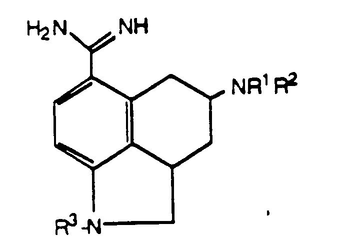 worin R¹, R² und R³ wie oben definiert sind, mit (CNS)₂ unter Bildung einer Verbindung der Formel 1, worin HET steht für 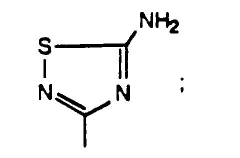
41) Oxidation einer Verbindung der Formel 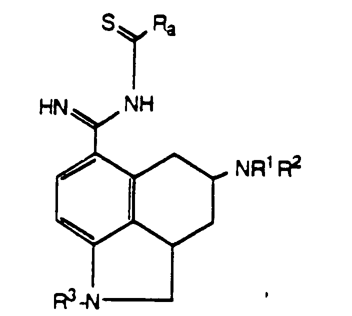 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 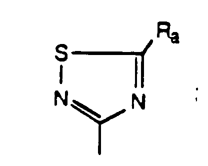
42) Umsetzung einer Verbindung der Formel 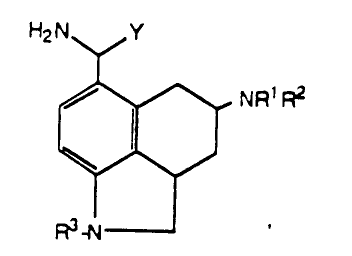 worin R¹, R² und R³ wie oben definiert sind und Y für -CN oder -C(O)NH₂ steht, mit einem Oxidationsmittel, wie SOCl₂, SCl₂, S₂Cl₂ oder SO₂Cl₂ unter Bildung einer Verbindung der Formel 1, worin HET steht für 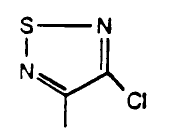
43) Umsetzung einer Verbindung der Formel 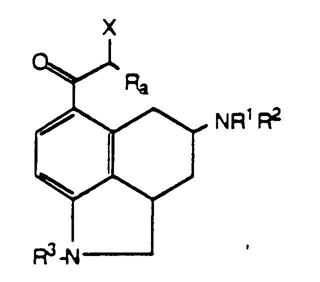 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht, mit RₐCSNH₂, worin Rₐ wie oben definiert ist, unter Bildung einer Verbindung der Formel 1, worin HET steht für 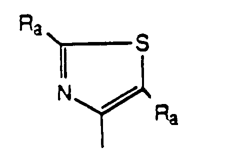
44) Umsetzung einer Verbindung der Formel 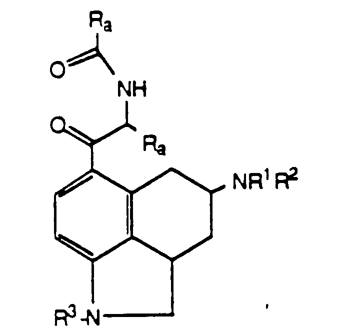 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit P₂S₅ unter Bildung einer Verbindung der Formel 1, worin HET steht für 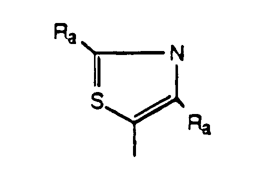
45) Umsetzung einer Verbindung der Formel 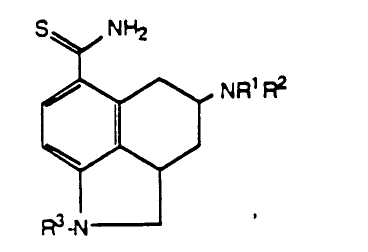 worin R¹, R² und R³ wie oben definiert sind, mit einer Verbindung der Formel 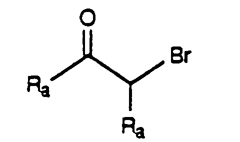 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 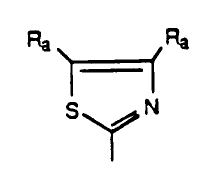
46) Umsetzung einer Verbindung der Formel 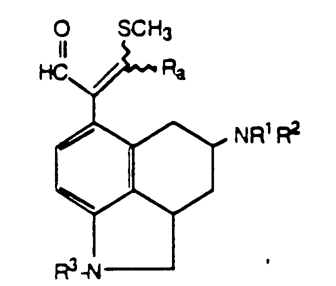 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit RₐC(S)NH₂, worin Rₐ wie oben definiert ist, unter Bildung einer Verbindung der Formel 1, worin HET steht für 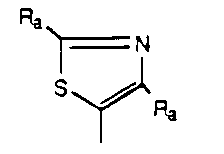
47) Umsetzung einer Verbindung der Formel 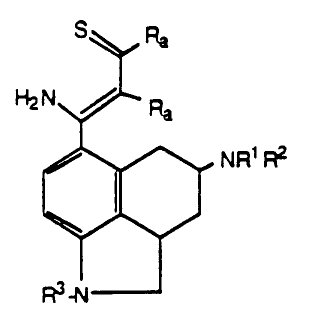 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl stellt, mit einem Oxidationsmittel unter Bildung einer Verbindung der Formel 1, worin HET steht für 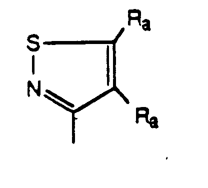
48) Umsetzung einer Verbindung der Formel 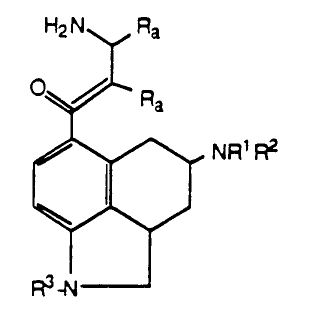 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einem Oxidationsmittel unter Bildung einer Verbindung der Formel 1, worin HET steht für 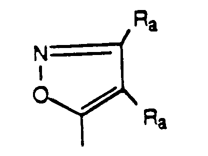
49) Umsetzung einer Verbindung der Formel 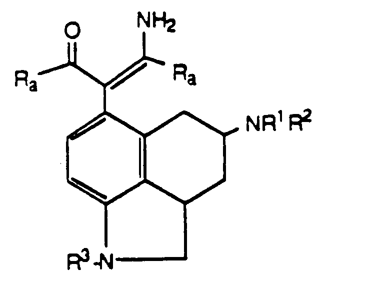 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einem Oxidationsmittel unter Bildung einer Verbindung der Formel 1, worin HET steht für 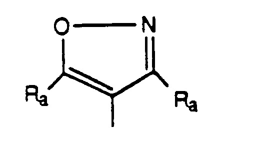
50) Umsetzung einer Verbindung der Formel 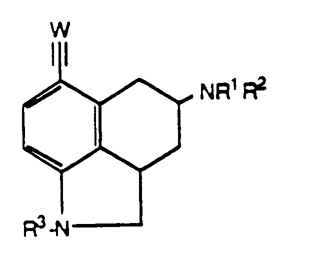 worin R¹, R² und R³ wie oben definiert sind und W für Wasserstoff oder CHO steht, mit KSCN oder NaHSSO₃ und anschließende Umsetzung des hierbei gebildeten Zwischenprodukts mit Ammoniak oder Ammoniumhydroxid unter Bildung einer Verbindung der Formel 1, worin HET steht für 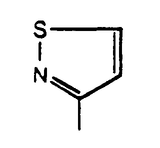
51) Umsetzung einer Verbindung der Formel 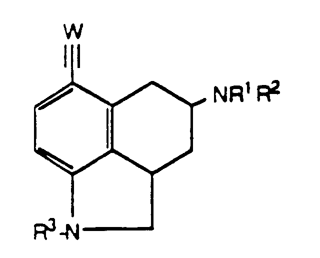 worin R¹, R² und R³ wie oben definiert sind und W für Wasserstoff oder CHO steht, mit H₂NSSO₃K und anschließende Umsetzung des hierbei gebildeten Zwischenprodukts mit einer Base unter Bildung einer Verbindung der Formel 1, worin HET steht für 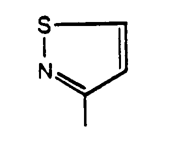
52) Umsetzung einer Verbindung der Formel 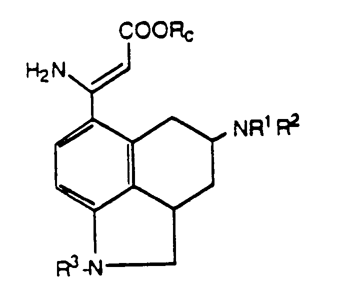 worin R¹, R² und R³ wie oben definiert sind und R_{c} für Wasserstoff oder C₁-C₃ Alkyl steht, mit CSCl₂ unter Bildung einer Verbindung der Formel 1, worin HET steht für 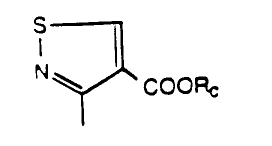
53) Umsetzung einer Verbindung der Formel 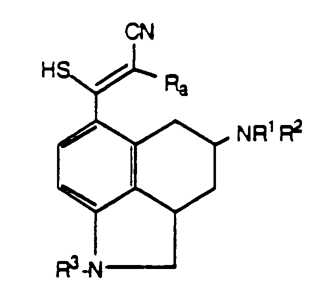 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit NCS unter Bildung einer Verbindung der Formel 1, worin HET steht für 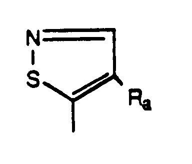
54) Umsetzung einer Verbindung der Formel 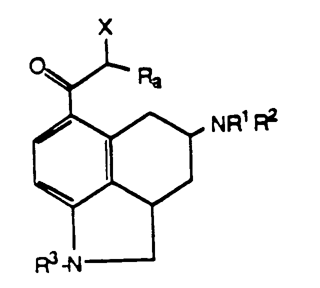 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht, mit 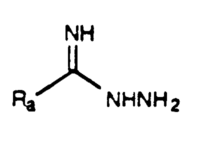 (worin Rₐ wie oben definiert ist)
unter Bildung einer Verbindung der Formel 1, worin HET steht für 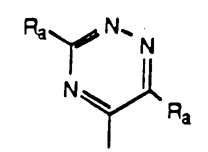
55) Umsetzung einer Verbindung der Formel 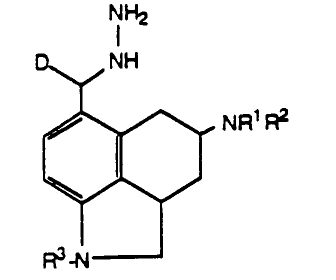 worin R¹, R² und R³ wie oben definiert sind und D für NH oder O steht mit einer Verbindung der Formel 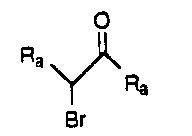 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 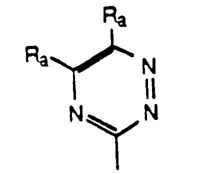
56) Umsetzung einer Verbindung der Formel 1
worin HET an zumindest einer Hydroxygruppe substituiert ist mit POX₃, PX₃, SOX₂ P(Phenyl)₃ · X₂ oder P(C₁-C₃ Alkoxy)₃ · X₂ , worin jedes X für Halogen steht, unter Umwandlung der Hydroxygruppe in einen Halogensubstituenten.
57) Umsetzung einer Verbindung der Formel 1, worin HET an zumindest einer Aminogruppe substituiert ist mit HONO unter Bildung des entsprechenden Diazoniumions, gefolgt von einer Umwandlung eines solchen Ions in die entsprechende halogensubstituierte Verbindung mit CuX (worin X für Halogen steht), KI oder HBF₄ unter Hitze.
58) Umsetzung einer Verbindung der Formel 1, worin HET an zumindest einem Halogensubstituenten mit einem Alkoxidanion der Formel
(C₁-C₃ Alkyl)-O⁻
unter Umwandlung des Halogensubstituenten in einen Alkoxysubstituenten substituiert wird,
59) Umsetzung einer Verbindung der Formel 1, worin HET an zumindest einer Hydroxygruppe mit einem (C₁-C₃ Alkyl)halogenid unter Umwandlung der Hydroxygruppe in einen Alkoxysubstituenten substituiert wird,
60) Umsetzung einer Verbindung der Formel 1, worin HET an zumindest einem Hydroxysubstituenten mit einer Diazoverbindung der Formel (C₁-C₃ Alkyl)N₂ unter Umwandlung der Hydroxygruppe in einen Alkoxysubstituenten substituiert wird,
61) Umsetzung einer Verbindung der Formel 1, worin HET an zumindest einer Aminogruppe mit HONO unter Bildung des entsprechenden Diazoniumions substituiert ist, gefolgt von der Umwandlung eines solchen Ions in die entsprechende hydroxysubstituierte Verbindung mittels Wasser oder einer Säure,
62) Umsetzung einer Verbindung der Formel 1, worin HET an zumindest einer C₁-C₃ Alkoxygruppe mit konzentrierter Iodwasserstoffsäure, konzentrierter Bromwasserstoffsäure oder einer Lewissäure unter Umwandlung der Alkoxygruppe in einen Hydroxysubstituenten substituiert wird,
63) Umsetzung einer Verbindung der Formel 1, worin HET an zumindest einem Aminosubstituenten mit HONO unter Bildung des entsprechenden Diazoniumions substituiert wird, gefolgt von der Umwandlung eines solchen Ions in die entsprechende nitrilsubstituierte Verbindung mittels CuCN,
64) Umsetzung einer Verbindung der Formel 1, worin HET an zumindest einem Aminosubstituenten mit HONO unter Bildung des entsprechenden Diazoniumions substituiert wird, gefolgt von der Umwandlung eines solchen Ions in die entsprechende C₁-C₃ Alkylthio-substituierte Verbindung mittels eines C₁-C₃ Alkylmercaptans,
65) Umsetzung einer Verbindung der Formel 1, worin HET an zumindest einem Halogensubstituenten mit einem Alkylthioanion der Formel
(C₁-C₃ Alkyl)-S⁻
unter Umwandlung des Halogensubstituenten in einen Alkylthiorest substituiert wird,
66) Umsetzung einer Verbindung der Formel 1, worin HET an zumindest einem -SH Rest mit einem (C₁-C₃ Alkyl)halogenid unter Bildung der entsprechenden Verbindung der Formel 1 mit einem Alkylthiorest substituiert wird,
67) Reduktion einer Verbindung der Formel 1, worin HET an zumindest einem Nitrosubstituenten unter Bildung einer Verbindung der Formel 1 substituiert wird, worin der Nitrosubstituent in einen Aminorest umgewandelt wird,
68) Reduktion einer Verbindung der Formel 1, worin HET an zumindest einem Halogensubstituenten unter Umwandlung des Halogensubstituenten in ein Wasserstoffatom substituiert wird,
69) Reduktion einer Verbindung der Formel 1, worin HET an zumindest einer Hydroxygruppe unter Umwandlung des Hydroxysubstituenten in ein Wasserstoffatom substituiert wird,
70) Umsetzung einer Verbindung der Formel 1, worin HET an zumindest einem Aminorest mit HONO unter Bildung des entsprechenden Diazoniumions substituiert wird, gefolgt von der Umwandlung eines solchen Ions in die entsprechende unsubstituierte Verbindung mittels H₃PO₂,
71) Reduktion einer Verbindung der Formel 1, worin HET an zumindest einem der Stickstoffatome in einem solchen heterocyclischen Ring mit einer C₁-C₃ Alkylphenylgruppe unter Bildung der entsprechenden Verbindung der Formel 1 substituiert wird, worin das Stickstoffatom unsubstituiert ist,
72) Reduktion einer Verbindung der Formel 1, worin HET an zumindest einem C₁-C₃ Alkylthiorest unter Bildung der entsprechenden unsubstituierten Verbindung der Formel 1 substituiert wird, oder
73) Umsetzung einer Verbindung der Formel 1 mit einer pharmazeutisch annehmbaren organischen oder anorganischen Säure unter Bildung eines pharmazeutisch annehmbaren Säureadditionssalzes einer solchen Verbindung.

## Patentansprüche (Patentansprüche für folgende(n) Vertragsstaat(en): ES, GR)

1. Verfahren zur Herstellung einer Verbindung der Formel 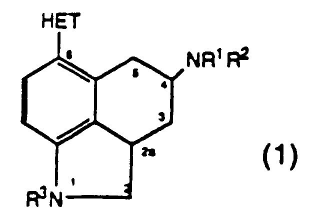 worin
R¹ steht für Wasserstoff, C₁-C₄ Alkyl, C₃-C₄ Alkenyl, Cyclopropylmethyl, Phenyl(C₁-C₄)alkyl, Phenyl(C₁-C₄)alkyl, das mit einem oder mehreren Substituenten substituiert ist, ausgewählt aus (C₁-C₃) Alkoxy, Halogen, Hydroxy, (C₁-C₃) Thioalkyl, Nitro, (C₁-C₃) Alkyl und Trifluormethyl, -(CH₂)ₙS(C₁-C₄ Alkyl), -C(O)R⁴, -(CH₂)ₙC(O)NR⁵R⁶,
R² für Wasserstoff, C₁-C₄ Alkyl, Cyclopropylmethyl oder C₃-C₄ Alkenyl steht,
R³ für Wasserstoff, C₁-C₄ Alkyl oder eine Aminoschutzgruppe steht,
n für 1-4 steht,
R⁴ für Wasserstoff, C₁-C₄ Alkyl, C₁-C₄ Halogenalkyl, C₁-C₄ Alkoxy oder Phenyl steht,
R⁵ und R⁶ unabhängig für Wasserstoff, C₁-C₄ Alkyl oder C₅-C₈ Cycloalkyl stehen mit der Maßgabe, daß wenn eines von R⁵ und R⁶ für ein Cycloalkyl steht, das andere für Wasserstoff steht,
HET für einen aromatischen fünf- oder sechsgliedrigen heterocyclischen Ring steht, wobei der Ring ein bis drei Heteroatome aufweist, die gleich oder verschieden sind und ausgewählt sind aus der Gruppe, die besteht aus Schwefel, Sauerstoff und Stickstoff, mit der Maßgabe, daß der sechsgliedrige heterocyclische Ring nur Kohlenstoff und Stickstoff enthalten kann und mit der weiteren Maßgabe, daß der fünfgliedrige Ring nicht mehr als einen Sauerstoff oder einen Schwefel aber nicht sowohl Sauerstoff als auch Schwefel enthält, und wahlweise an einem oder zwei Kohlenstoffatomen substituiert ist durch (C₁-C₃) Alkyl, Halogen, Hydroxy, (C₁-C₃) Alkoxy, (C₁-C₃) Thioalkyl, NH₂, CN oder Phenyl,
oder pharmazeutisch annehmbarer Salze hiervon,
gekennzeichnet durch
1) Umsetzung eines 4-amino-6-metall-substituierten Hexahydrobenz[cd]indols der Formel 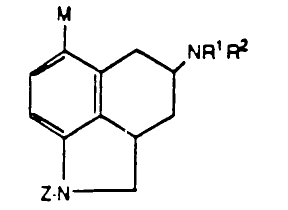 worin R¹ und R² wie vorher definiert sind, Z für eine Aminoschutzgruppe steht und M für einen Metallrest steht, mit einer heterocyclischen Verbindung der Formel
HET-L
worin HET wie vorher definiert ist und L für eine Abgangsgruppe steht,
2) Abspalten der Schutzgruppen von einer Verbindung der Formel 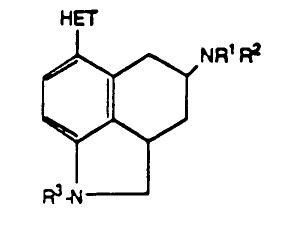 worin HET, R¹ und R² wie oben definiert sind und R³ für eine Aminoschutzgruppe steht unter Bereitstellung einer Verbindung der Formel 1, worin R³ für Wasserstoff steht,
3) Umsetzung eines 4-amino-6-halogen-substituierten Hexahydrobenz[cd]indols der Formel 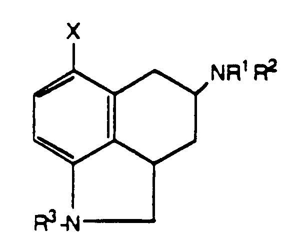 worin R¹, R² und R³ wie oben definiert sind und X für Halogen steht,
mit einem Organometallderivat der Formel
M-HET
worin HET wie oben definiert ist und M für Lithium, Magesium, Zink, Zinn, Quecksilber oder Borsaure steht,
4) Umsetzung einer Verbindung der Formel 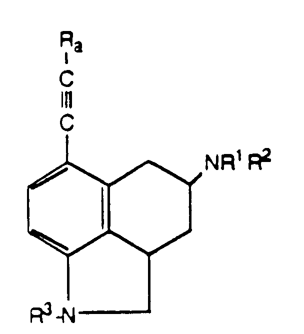 worin R¹, R² und R³ wie oben definiert sind und Rₐ für Wasserstoff, C₁-C₃ Alkyl, Halogen, O(C₁-C₃ Alkyl), S(C₁-C₃ Alkyl), CN oder Phenyl steht,
mit einem 1,3-Dipol der Formel
⁺T=U-V⁻
worin T, U und V aus der folgenden Liste ausgewählt sein können
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
worin Rₐ wie oben definiert ist, Rₐ' für Wasserstoff, C₁-C₃ Alkyl, Halogen, O(C₁-C₃ Alkyl), S(C₁-C₃ Alkyl), CN oder Phenyl steht und R_{b} für Wasserstoff, C₁-C₃ Alkyl, Phenyl oder (C₁-C₃ Alkyl)phenyl steht, unter Bildung einer Verbindung der Formel 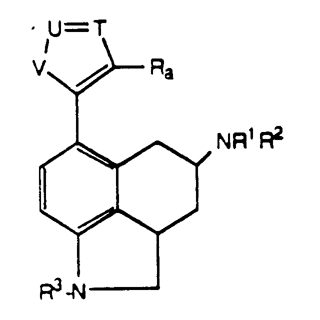 worin R¹, R², R³, Rₐ, T, U und V wie oben definiert sind,
5) Umsetzung einer Verbindung der Formel 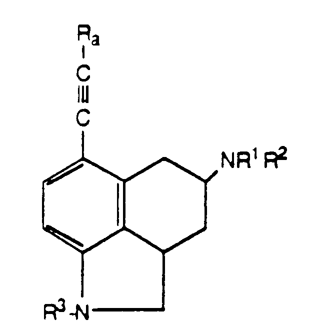 worin R¹, R² und R³ wie oben definiert sind und Rₐ für Wasserstoff, C₁-C₃ Alkyl, Halogen, O(C₁-C₃ Alkyl), S(C₁-C₃ Alkyl), CN oder Phenyl steht, mit einem 1,3-Dipol der Formel
⁺T-U=V⁻
worin T, U und V aus folgenden ausgewählt sind
| T | U | V |
|---|---|---|
| CHRₐ | N | N |
| NR_{b} | N | N |
worin Rₐ wie oben definiert ist und R_{b} für Wasserstoff, C₁-C₃ Alkyl, Phenyl oder (C₁-C₃ Alkyl)phenyl steht, unter Bildung einer Verbindung der Formel 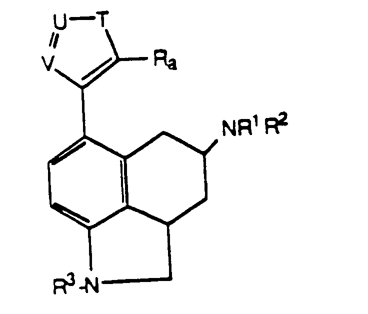 worin R¹, R², R³, Rₐ, V, U und T wie oben definiert sind,
6) Umsetzung einer Verbindung der Formel 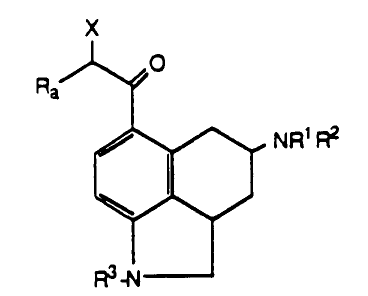 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht,
mit NH₄⁺RₐCOO⁻ (worin Rₐ wie oben definiert ist) unter Bildung eines Gemisches der Verbindungen der Formel 1, worin HET steht für 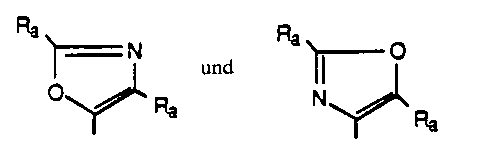 und anschließend wahlweise Trennung der Verbindungen voneinander,
7) Dehydrierung einer Verbindung der Formel 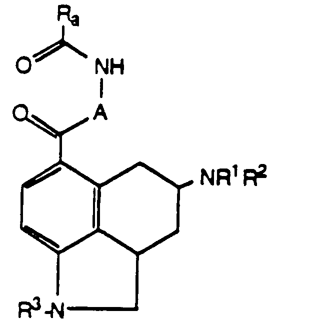 worin R¹, R² und R³ wie oben definiert sind, A für C-Rₐ oder NH steht und jedes Rₐ unabhängig steht für Wasserstoff, C₁-C₃ Alkyl, Halogen, OH, O(C₁-C₃ Alkyl), S(C₁-C₃ Alkyl), NH₂, CN oder Phenyl, unter Bildung einer Verbindung der Formel 1, worin HET steht für 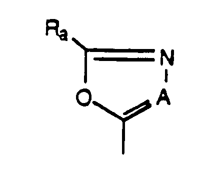
8) Cyclisierung und Dehydrierung einer Verbindung der Formel 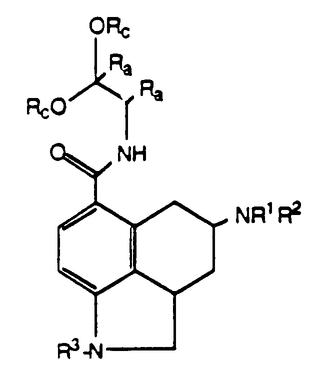 worin R¹, R² und R³ wie oben definiert sind, Rₐ steht für Wasserstoff, C₁-C₃ Alkyl, Halogen, OH, O(C₁-C₃ Alkyl), S(C₁-C₃ Alkyl), NH₂, CN oder Phenyl und R_{c} für Wasserstoff oder C₁-C₃ Alkyl steht, unter Bildung einer Verbindung der Formel 1, worin HET steht für 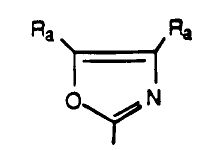
9) Umsetzung einer Verbindung der Formel 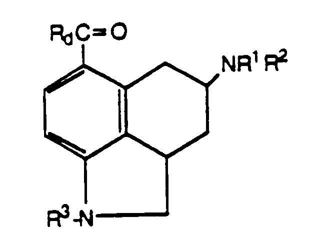 worin R¹, R² und R³ wie oben definiert sind und R_{d} steht für OH, O(C₁-C₃ Alkyl), O(Phenyl), O(C₁-C₃ Alkylphenyl), NH₂, Halogen, S(C₁-C₃ Alkyl), S(Phenyl), S(C₁-C₃ Alkylphenyl), NH(C₁-C₃ Alkyl), N(C₁-C₃ Alkyl)₂, OCO(C₁-C₃ Alkyl), OCO(Phenyl) oder OCO(C₁-C₃ Alkylphenyl) mit 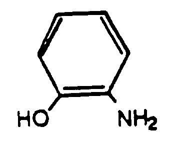 unter Bildung einer Verbindung der Formel 1, worin HET steht für 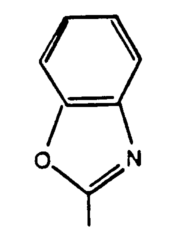
10) Cyclisierung und Dehydrierung einer Verbindung der Formel 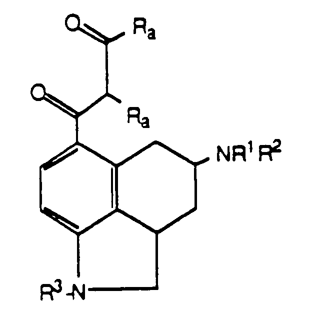 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 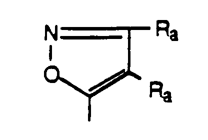
11) Cyclisierung und Dehydrierung einer Verbindung der Formel 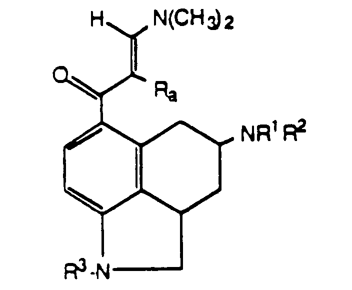 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 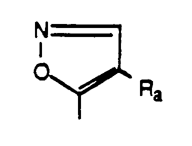
12) Cyclisierung und Dehydrierung einer Verbindung der Formel 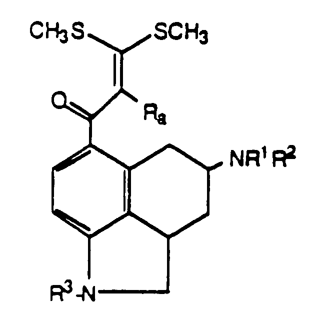 worin R¹, R² und R³ wie oben definiert sind und Rₐ steht für Wasserstoff, C₁-C₃ Alkyl, Halogen, OH, O(C₁-C₃ Alkyl), S(C₁-C₃ Alkyl), NH₂, CN oder Phenyl,
mit Hydroxylamin unter Bildung einer Verbindung der Formel 1, worin HET steht für 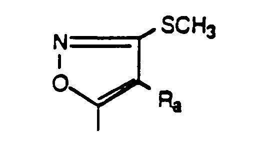 13) Cyclisierung und Dehydrierung eines Dianions der Formel 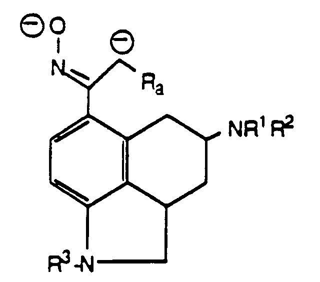 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht,
mit einem Carbonylderivat der Formel RₐCOOR_{c} (worin Rₐ wie oben definiert ist und R_{c} für Wasserstoff oder C₁-C₃ Alkyl steht) oder RₐCON(CH₃)₂ (worin Rₐ wie oben definiert ist) unter Bildung einer Verbindung der Formel 1, worin HET steht für 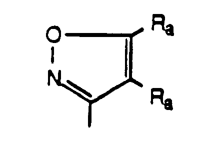
14) Cyclisierung und Dehydrierung einer Verbindung der Formel 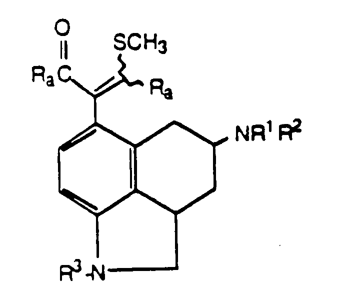 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht,
mit Hydroxylamin unter Bildung einer Verbindung der Formel 1, worin HET steht für 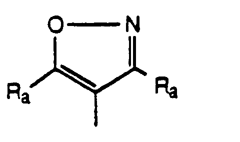
15) Umsetzung einer Verbindung der Formel 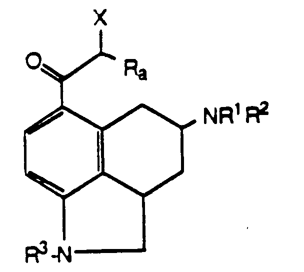 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht mit 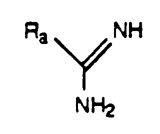 (worin Rₐ wie oben definiert ist)
unter Bildung einer Verbindung der Formel 1, worin HET steht für 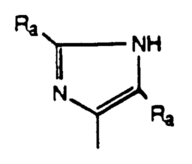
16) Umsetzung einer Verbindung der Formel 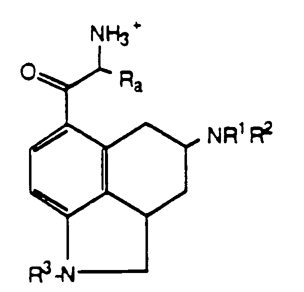 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht,
mit KSCN und anschließend R_{c}X (worin R_{c} für Wasserstoff oder C₁-C₃ Alkyl steht und X für Halogen steht) in Gegenwart einer Base unter Bildung einer Verbindung der Formel 1, worin HET steht für 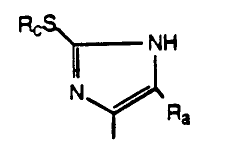
17) Dehydrierung einer Verbindung der Formel 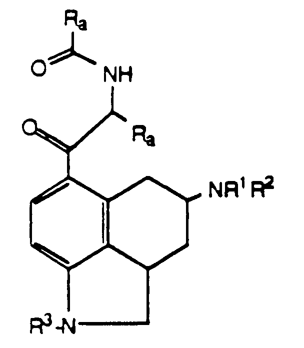 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht in Gegenwart von Ammoniak oder Ammoniumhydroxid unter Bildung einer Verbindung der Formel 1, worin HET steht für 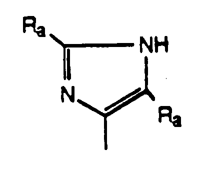
18) Umsetzung einer Verbindung der Formel 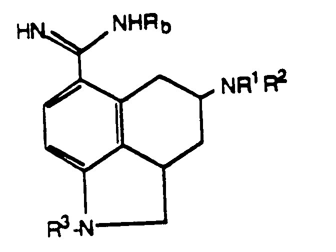 worin R¹, R² und R³ wie oben definiert sind und R_{b} steht für Wasserstoff, C₁-C₃ Alkyl, Phenyl oder (C₁-C₃ Alkyl)phenyl mit einer Verbindung der Formel 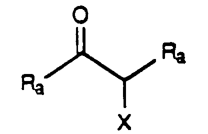 worin Rₐ für Wasserstoff, C₁-C₃ Alkyl, Halogen, OH, O(C₁-C₃ Alkyl), S(C₁-C₃ Alkyl), NH₂, CN oder Phenyl steht und X für Halogen steht, unter Bildung einer Verbindung der Formel 1, worin HET steht für 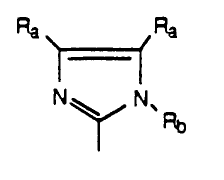
19) Dehydrierung einer Verbindung der Formel 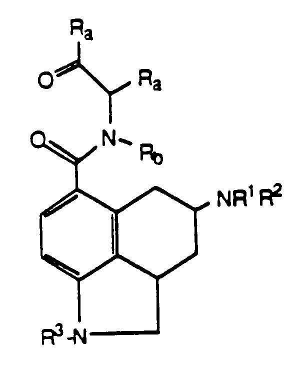 worin R₁, R₂ und R₃ wie oben definiert sind, Rₐ für Wasserstoff, C₁-C₃ Alkyl, Halogen, OH, O(C₁-C₃ Alkyl), S(C₁-C₃ Alkyl), NH₂, CN oder Phenyl steht und R_{b} für Wasserstoff, C₁-C₃ Alkyl, Phenyl oder (C₁-C₃ Alkyl)phenyl steht in Gegenwart von Ammoniak oder Ammoniumhydroxid unter Bildung einer Verbindung der Formel 1, worin HET steht für 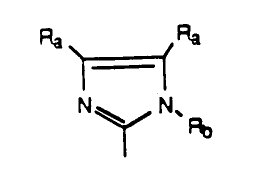
20) Umsetzung einer Verbindung der Formel 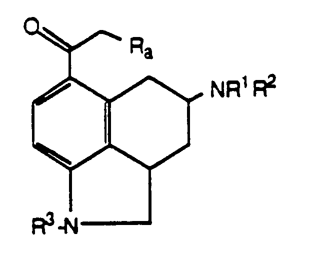 worin R¹, R² und R³ wie oben definiert sind und Rₐ für Waserstoff, C₁-C₃ Alkyl, Halogen, OH, O(C₁-C₃ Alkyl), S(C₁-C₃ Alkyl), NH₂, CN oder Phenyl steht,
mit einem Azid der Formel R_{b}N₃, worin R_{b} für Wasserstoff, C₁-C₃ Alkyl, Phenyl oder (C₁-C₃ Alkyl)phenyl steht gefolgt von einer Dehydrierung der entstehenden Verbindung unter Bildung einer Verbindung der Formel 1, worin HET steht für 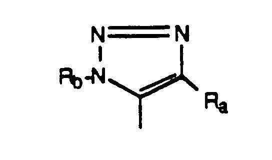
21) Cyclisierung und Dehydrierung einer Verbindung der Formel 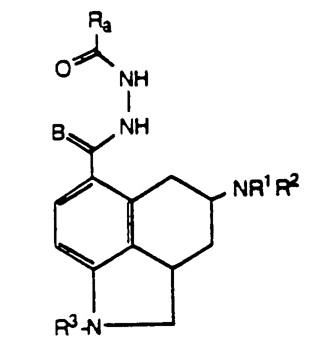 worin R¹, R² und R³ wie oben definiert sind, Rₐ für Wasserstoff, C₁-C₃ Alkyl, Halogen, OH, O(C₁-C₃ Alkyl), S(C₁-C₃ Alkyl), NH₂, CN oder Phenyl steht und B für O oder NH steht unter Bildung einer Verbindung der Formel 1, worin HET steht für 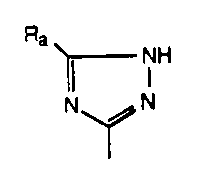 mit der Maßgabe, daß wenn B für O steht, diese Cyclisierungsreaktion in Gegenwart von Ammoniak oder Ammoniumhydroxid durchgeführt wird,
22) Umsetzung einer Verbindung der Formel 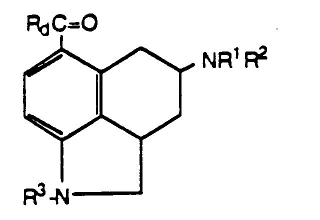 worin R¹, R² und R³ wie oben definiert sind und R_{d} für OH, O(C₁-C₃ Alkyl), O(Phenyl), O(C₁-C₃ Alkylphenyl), Halogen, S(C₁-C₃ Alkyl), S(Phenyl), S(C₁-C₃ Alkylphenyl), NH₂, NH(C₁-C₃ Alkyl), N(C₁-C₃ Alkyl)₂, OCO(C₁-C₃ Alkyl, OCO(Phenyl) oder OCO(C₁-C₃ Alkylphenyl) steht,
mit einer Verbindung der Formel 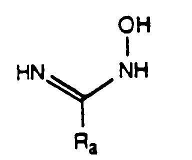 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 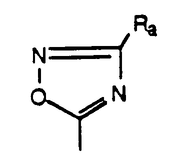
23) Umsetzung einer Verbindung der Formel 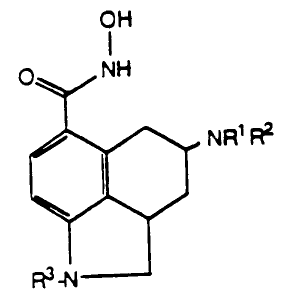 worin R¹, R² und R³ wie oben definiert sind mit RₐCN, worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 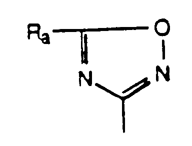
24) Cyclisierung und Dehydrierung einer Verbindung der Formel 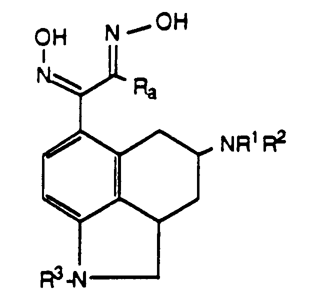 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 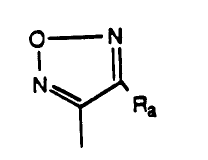
25) Umsetzung einer Verbindung der Formel 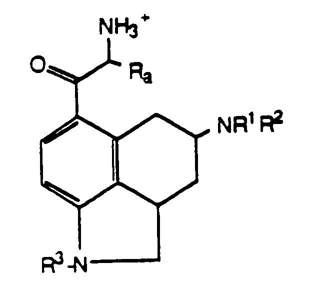 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 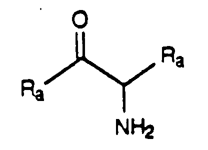 (worin Rₐ wie oben definiert ist)
in Gegenwart eines Oxidationsmittels unter Bildung einer Verbindung der Formel 1, worin HET steht für 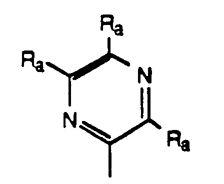
26) Umsetzung einer Verbindung der Formel 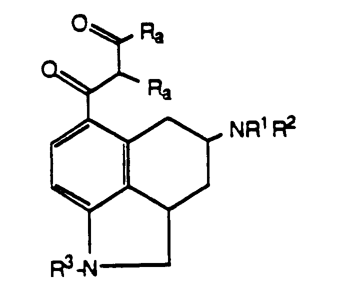 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 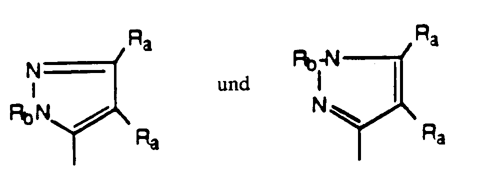 und anschließend wahlweise Trennung der Verbindungen voneinander,
27) Umsetzung einer Verbindung der Formel 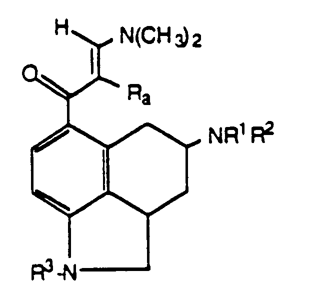 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 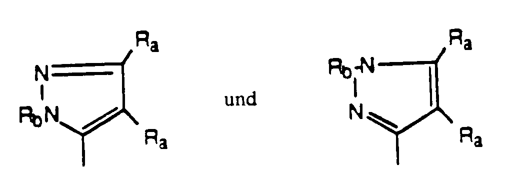 und anschließend wahlweise Trennung der Verbindungen voneinander,
28) Umsetzung einer Verbindung der Formel 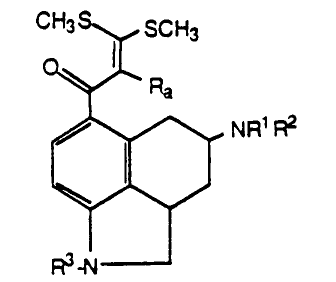 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung von Verbindungen der Formel 1, worin HET steht für 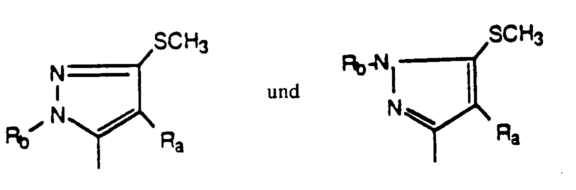 und anschließend wahlweise Trennung der Verbindungen voneinander,
29) Umsetzung einer Verbindung der Formel 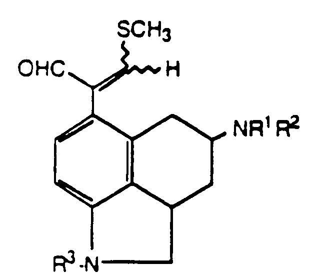 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel H₂NNHR_{b}, worin R_{b} für Wasserstoff, C₁-C₃ Alkyl, Phenyl oder (C₁-C₃ Alkyl)phenyl steht, unter Bildung einer Verbindung der Formel 1, worin HET steht für 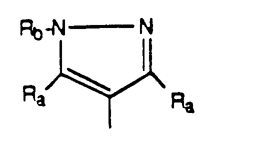
30) Umsetzung einer Verbindung der Formel 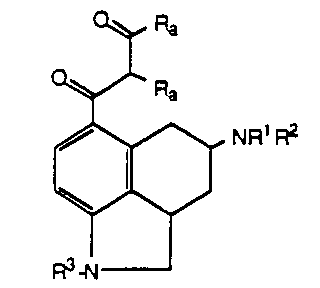 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 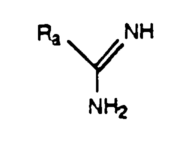 (worin Rₐ wie oben definiert ist)
unter Bildung einer Verbindung der Formel 1, worin HET steht für 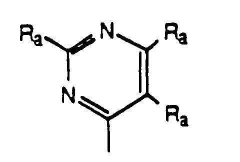
31) Umsetzung einer Verbindung der Formel 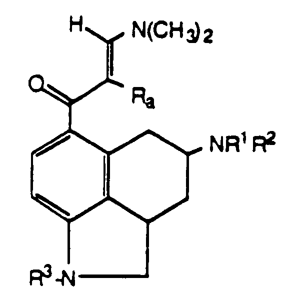 worin R¹, R² und R³ wie oben definiert sind und Rₐ für Wasserstoff C₁-C₃ Alkyl, Halogen, OH O(C₁-C₃ Alkyl), S(C₁-C₃ Alkyl), NH₂, CN oder Phenyl steht, mit einer Verbindung der Formel 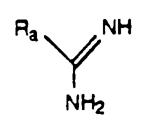 (worin Rₐ wie oben definiert ist)
unter Bildung einer Verbindung der Formel 1, worin HET steht für 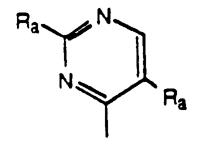
32) Umsetzung einer Verbindung der Formel 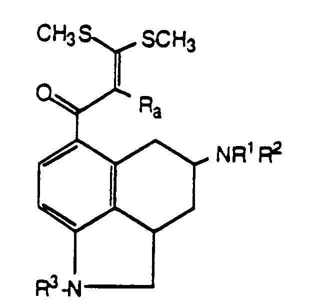 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 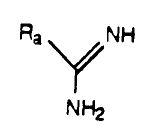 (worin Rₐ wie oben definiert ist)
unter Bildung einer Verbindung der Formel 1, worin HET steht für 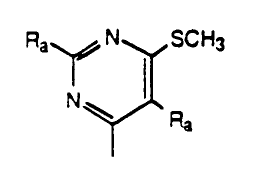
33) Umsetzung einer Verbindung der Formel 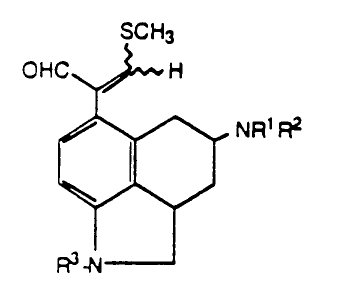 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einer Verbindung der Formel 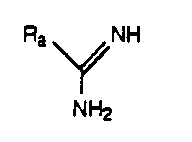 (worin Rₐ wie oben definiert ist)
unter Bildung einer Verbindung der Formel 1, worin HET steht für 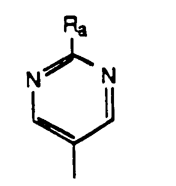
34) Umsetzung einer Verbindung der Formel 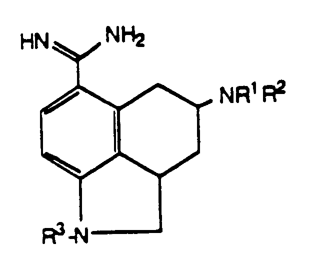 worin R¹, R² und R³ wie oben definiert sind, mit einer Verbindung der Formel 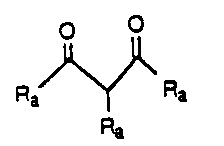 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 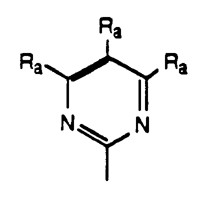
35) Umsetzung einer Verbindung der Formel 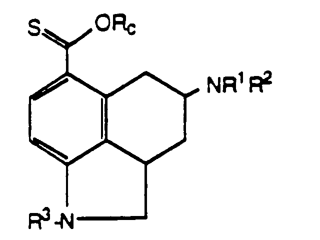 worin R¹, R² und R³ wie oben definiert sind und R_{c} für Wasserstoff oder C₁-C₃ Alkyl steht, mit einer Verbindung der Formel RₐCH₂NNH, worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 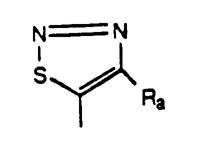
36) Umsetzung einer Verbindung der Formel 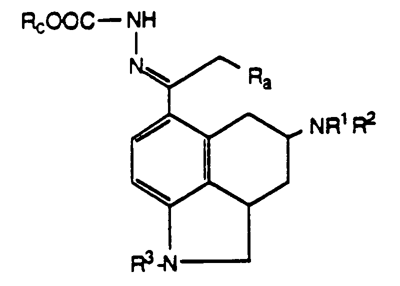 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 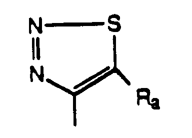
37) Umsetzung einer Verbindung der Formel 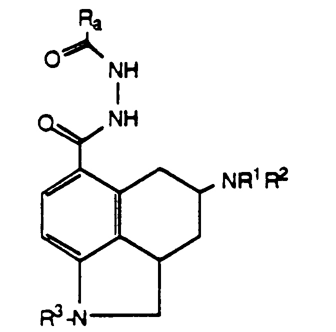 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit P₂S₅ unter Bildung einer Verbindung der Formel 1, worin HET steht für 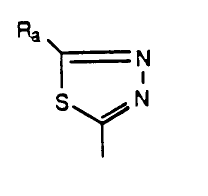
38) Umsetzung einer Verbindung der Formel 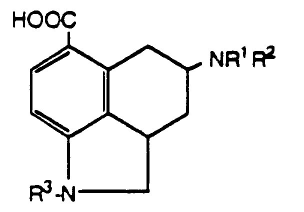 worin R¹, R² und R³ wie oben definiert sind mit einer Verbindung der Formel H₂NNHCSNH₂ in Gegenwart von Polyphosphorsäure unter Bildung einer Verbindung der Formel 1, worin HET steht für 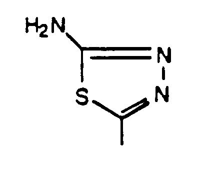
39) Umsetzung einer Verbindung der Formel 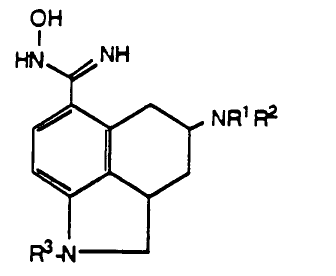 worin R¹, R² und R³ wie oben definiert sind, mit Schwefelkohlenstoff unter Bildung einer Verbindung der Formel 1, worin HET steht für 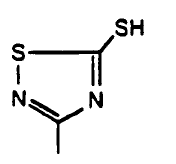
40) Umsetzung einer Verbindung der Formel 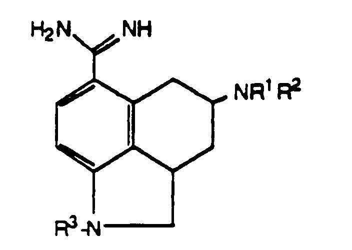 worin R¹, R² und R³ wie oben definiert sind, mit (CNS)₂ unter Bildung einer Verbindung der Formel 1, worin HET steht für 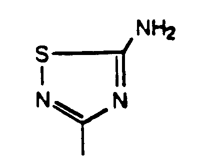
41) Oxidation einer Verbindung der Formel 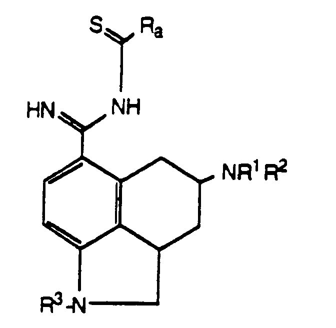 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 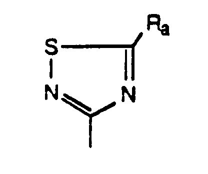
42) Umsetzung einer Verbindung der Formel 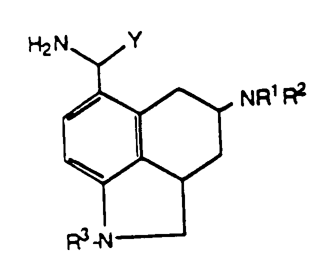 worin R¹, R² und R³ wie oben definiert sind und Y für -CN oder -C(O)NH₂ steht, mit einem Oxidationsmittel, wie SOCl₂, SCl₂, S₂Cl₂ oder SO₂Cl₂ unter Bildung einer Verbindung der Formel 1, worin HET steht für 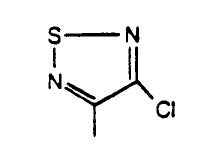
43) Umsetzung einer Verbindung der Formel 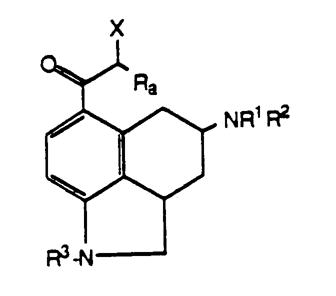 worin R¹, R² und R³ wie oben definiert sind, X für Halogen steht und Rₐ für Wasserstoff C₁-C₃ Alkyl, Halogen, OH, O(C₁-C₃ Alkyl), S(C₁-C₃ Alkyl), NH₂, CN oder Phenyl steht, mit RₐCSNH₂, worin Rₐ wie oben definiert ist, unter Bildung einer Verbindung der Formel 1, worin HET steht für 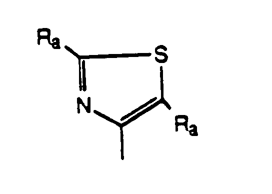
44) Umsetzung einer Verbindung der Formel 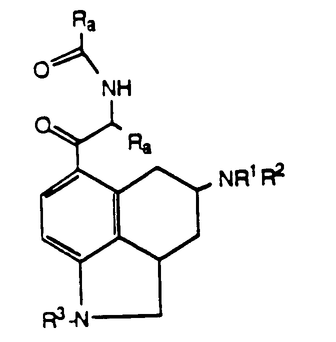 worin R¹, R² und R³ wie oben definiert sind und Rₐ für Wasserstoff C₁-C₃ Alkyl, Halogen, OH, O(C₁-C₃ Alkyl), S(C₁-C₃ Alkyl), NH₂, CN oder Phenyl steht, mit P₂S₅ unter Bildung einer Verbindung der Formel 1, worin HET steht für 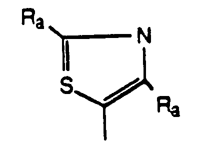
45) Umsetzung einer Verbindung der Formel 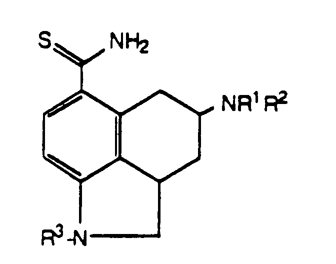 worin R¹, R² und R³ wie oben definiert sind, mit einer Verbindung der Formel 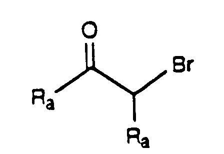 worin Rₐ für Wasserstoff, C₁-C₃ Alkyl, Halogen, OH, O(C₁-C₃ Alkyl), S(C₁-C₃ Alkyl), NH₂, CN oder Phenyl steht unter Bildung einer Verbindung der Formel 1, worin HET steht für 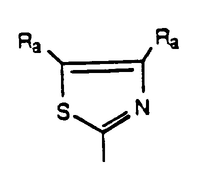
46) Umsetzung einer Verbindung der Formel 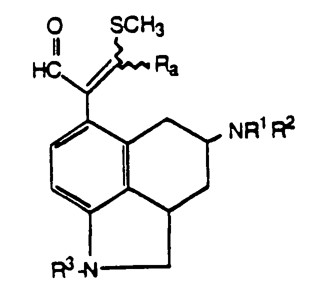 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit RₐC(S)NH₂, worin Rₐ wie oben definiert ist, unter Bildung einer Verbindung der Formel 1, worin HET steht für 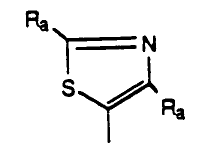
47) Umsetzung einer Verbindung der Formel 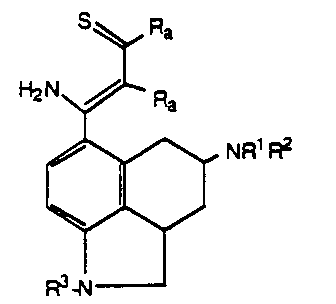 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einem Oxidationsmittel unter Bildung einer Verbindung der Formel 1, worin HET steht für 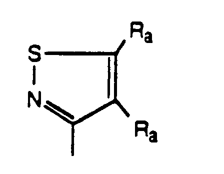
48) Umsetzung einer Verbindung der Formel 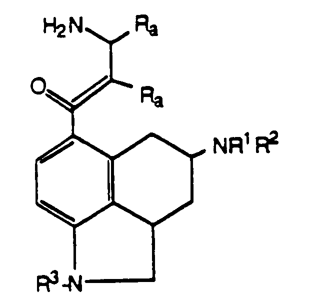 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einem Oxidationsmittel unter Bildung einer Verbindung der Formel 1, worin HET steht für 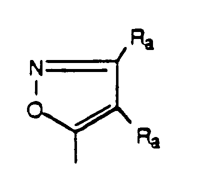
49) Umsetzung einer Verbindung der Formel 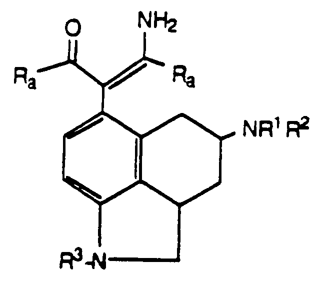 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit einem Oxidationsmittel unter Bildung einer Verbindung der Formel 1, worin HET steht für 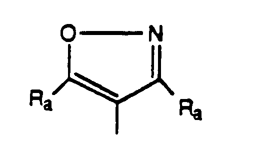
50) Umsetzung einer Verbindung der Formel 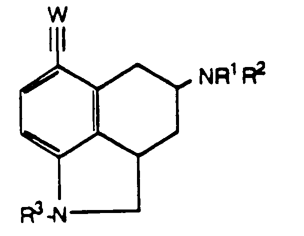 worin R¹, R² und R³ wie oben definiert sind und W für Wasserstoff oder CHO steht, mit KSCN oder NaHSSO₃ und anschließende Umsetzung des hierbei gebildeten Zwischenprodukts mit Ammoniak oder Ammoniumhydroxid unter Bildung einer Verbindung der Formel 1, worin HET steht für 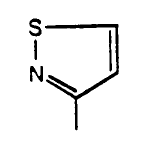
51) Umsetzung einer Verbindung der Formel 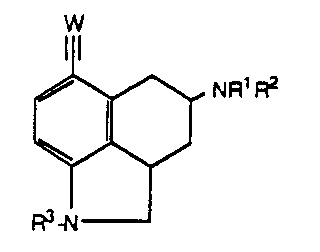 worin R¹, R² und R³ wie oben definiert sind und W für Wasserstoff oder CHO steht, mit H₂NSSO₃K und anschließende Umsetzung des hierbei gebildeten Zwischenprodukts mit einer Base unter Bildung einer Verbindung der Formel 1, worin HET steht für 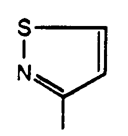
52) Umsetzung einer Verbindung der Formel 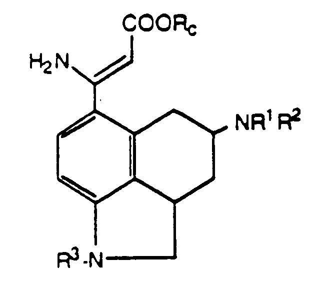 worin R¹, R² und R³ wie oben definiert sind und R_{c} für Wasserstoff oder C₁-C₃ Alkyl steht, mit CSCl₂ unter Bildung einer Verbindung der Formel 1, worin HET steht für 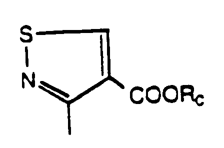
53) Umsetzung einer Verbindung der Formel 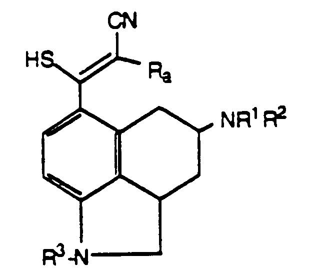 worin R¹, R² und R³ wie oben definiert sind und Rₐ für Wasserstoff, C₁-C₃ Alkyl, Halogen, OH, O(C₁-C₃ Alkyl), S(C₁-C₃ Alkyl), NH₂, CN oder Phenyl steht, mit NCS unter Bildung einer Verbindung der Formel 1, worin HET steht für 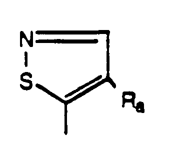
54) Umsetzung einer Verbindung der Formel 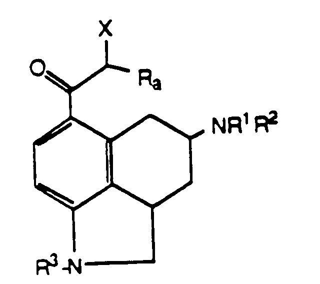 worin R¹, R² und R³ wie oben definiert sind, X für Halogen steht und Rₐ für Wasserstoff, C₁-C₃ Alkyl, Halogen, OH, O(C₁-C₃ Alkyl), S(C₁-C₃ Alkyl), NH₂, CN oder Phenyl steht, mit 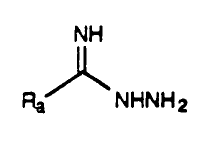 (worin Rₐ wie oben definiert ist)
unter Bildung einer Verbindung der Formel 1, worin HET steht für 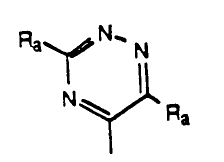
55) Umsetzung einer Verbindung der Formel 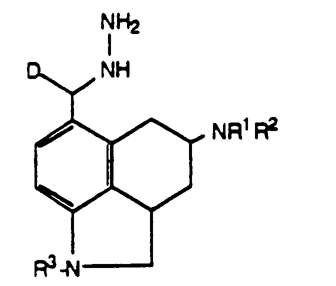 worin R¹, R² und R³ wie oben definiert sind und D für NH oder O steht mit einer Verbindung der Formel 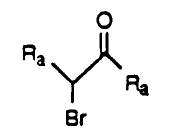 worin Rₐ für Wasserstoff, C₁-C₃ Alkyl, Halogen, OH, O(C₁-C₃ Alkyl), S(C₁-C₃ Alkyl), NH₂, CN oder Phenyl steht, unter Bildung einer Verbindung der Formel 1, worin HET steht für 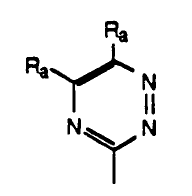
56) Umsetzung einer Verbindung der Formel 1
worin HET an zumindest einer Hydroxygruppe substituiert ist mit POX₃, PX₃, SOX₂ P(Phenyl)₃ · X₂ oder P(C₁-C₃ Alkoxy)₃ · X₂, worin jedes X für Halogen steht, unter Umwandlung der Hydroxygruppe in einen Halogensubstituenten.
57) Umsetzung einer Verbindung der Formel 1, worin HET an zumindest einer Aminogruppe substituiert ist mit HONO unter Bildung des entsprechenden Diazoniumions, gefolgt von einer Umwandlung eines solchen Ions in die entsprechende halogensubstituierte Verbindung mit CuX (worin X für Halogen steht), KI oder HBF₄ unter Hitze,
58) Umsetzung einer Verbindung der Formel 1, worin HET an zumindest einem Halogensubstituenten mit einem Alkoxidenion der Formel
(C₁-C₃ Alkyl)-O⁻
unter Umwandlung des Halogensubstituenten in einen Alkoxysubstituenten substituiert wird,
59) Umsetzung einer Verbindung der Formel 1, worin HET an zumindest einer Hydroxygruppe mit einem (C₁-C₃ Alkyl)halogenid unter Umwandlung der Hydroxygruppe in einen Alkoxysubstituenten substituiert wird,
60) Umsetzung einer Verbindung der Formel 1, worin HET an zumindest einem Hydroxysubstituenten mit einer Diazoverbindung der Formel (C₁-C₃ Alkyl)N₂ unter Umwandlung der Hydroxygruppe in einen Alkoxysubstituenten substituiert wird,
61) Umsetzung einer Verbindung der Formel 1, worin HET an zumindest einer Aminogruppe mit HONO unter Bildung des entsprechenden Diazoniumions substituiert ist, gefolgt von der Umwandlung eines solchen Ions in die entsprechende hydroxysubstituierte Verbindung mittels Wasser oder einer Säure,
62) Umsetzung einer Verbindung der Formel 1, worin HET an zumindest einer C₁-C₃ Alkoxygruppe mit konzentrierter Iodwasserstoffsäure, konzentrierter Bromwasserstoffsäure oder einer Lewissäure unter Umwandlung der Alkoxygruppe in einen Hydroxysubstituenten substituiert wird,
63) Umsetzung einer Verbindung der Formel 1, worin HET an zumindest einem Aminosubstituenten mit HONO unter Bildung des entsprechenden Diazoniumions substituiert wird, gefolgt von der Umwandlung eines solchen Ions in die entsprechende nitrilsubstituierte Verbindung mittels CuCN,
64) Umsetzung einer Verbindung der Formel 1, worin HET an zumindest einem Aminosubstituenten mit HONO unter Bildung des entsprechenden Diazoniumions substituiert wird, gefolgt von der Umwandlung eines solchen Ions in die entsprechende C₁-C₃ Alkylthio-substituierte Verbindung mittels eines C₁-C₃ Alkylmercaptans,
65) Umsetzung einer Verbindung der Formel 1, worin HET an zumindest einem Halogensubstituenten mit einem Alkylthioanion der Formel
(C₁-C₃ Alkyl)-S⁻
unter Umwandlung des Halogensubstituenten in einen Alkylthiorest substituiert wird,
66) Umsetzung einer Verbindung der Formel 1, worin HET an zumindest einem -SH Rest mit einem (C₁-C₃ Alkyl)halogenid unter Bildung der entsprechenden Verbindung der Formel 1 mit einem Alkylthiorest substituiert wird,
67) Reduktion einer Verbindung der Formel 1, worin HET an zumindest einem Nitrosubstituenten unter Bildung einer Verbindung der Formel 1 substituiert wird, worin der Nitrosubstituent in einen Aminorest umgewandelt wird,
68) Reduktion einer Verbindung der Formel 1, worin HET an zumindest einem Halogensubstituenten unter Umwandlung des Halogensubstituenten in ein Wasserstoffatom substituiert wird,
69) Reduktion einer Verbindung der Formel 1, worin HET an zumindest einer Hydroxygruppe unter Umwandlung des Hydroxysubstituenten in ein Wasserstoffatom substituiert wird,
70) Umsetzung einer Verbindung der Formel 1, worin HET an zumindest einem Aminorest mit HONO unter Bildung des entsprechenden Diazoniumions substituiert wird, gefolgt von der Umwandlung eines solchen Ions in die entsprechende unsubstituierte Verbindung mittels H₃PO₂,
71) Reduktion einer Verbindung der Formel 1, worin HET an zumindest einem der Stickstoffatome in einem solchen heterocyclischen Ring mit einer C₁-C₃ Alkylphenylgruppe unter Bildung der entsprechenden Verbindung der Formel 1 substituiert wird, worin das Stickstoffatom unsubstituiert ist,
72) Reduktion einer Verbindung der Formel 1, worin HET an zumindest einem C₁-C₃ Alkylthiorest unter Bildung der entsprechenden unsubstituierten Verbindung der Formel 1 substituiert wird, oder
73) Umsetzung einer Verbindung der Formel 1 mit einer pharmazeutisch annehmbaren organischen oder anorganischen Säure unter Bildung eines pharmazeutisch annehmbaren Säureadditionssalzes einer solchen Verbindung.

2. Verfahren nach Anspruch 1, worin HET für ein Isoxazol, ein Pyrazol, ein Pyradin, ein Thiazol, ein Furan, ein Thiophen, ein Oxadiazol steht oder ein pharmazeutisch annehmbares Salz hiervon.

3. Verfahren nach Anspruch 1 oder Anspruch 2, worin R¹ und R² unabhängig für C₁-C₃ Alkyl stehen, oder ein pharmazeutisch annehmbares Salz hiervon.

4. Verfahren nach einem der Ansprüche 1 bis 3, worin R³ für Wasserstoff steht, oder ein pharmazeutisch annehmbares Salz hiervon.

5. Verfahren nach Anspruch 1 oder Anspruch 2, worin R¹ für -(CH₂)ₙC(O)NR⁵R⁶ steht, worin n für 2 steht, R⁵ für Wasserstoff steht, R⁶ für Cyclohexyl steht, R² für C₁-C₃ Alkyl steht und R³ für Wasserstoff oder C₁-C₄ Alkyl steht, oder ein pharmazeutisch annehmbares Salz hiervon.

6. Verfahren zur Herstellung eines im wesentlichen reinen Stereoisomers einer Verbindung nach Anspruch 1 oder eines pharmazeutisch annehmbaren Salzes hiervon, gekennzichnet durch das Verfahren nach einem der Ansprüche 1-5.

7. Verfahren nach Anspruch 6, worin die Konfiguration des Stereoisomers an der Position 2a S ist und an der Position 4 R ist, oder ein pharmazeutisch annehmbares Salz hiervon.

8. Verfahren nach Anspruch 1, das zur Herstellung einer Verbindung verwendet wird, die ausgewählt ist aus der Gruppe, die besteht aus 6-(3-Isoxazolyl)-4-(di-n-propylamino)-1,2,2a,3,4,5-hexahydrobenz[cd]indol, 6-(5-Isoxazolyl)-4-(di-n-propylamino)-1,2,2a,3,4,5-hexahydrobenz[cd]indol, 6-(3-Pyrazolyl)-4-(di-n-propylamino)-1,2-,2a,3,4,5-hexahydrobenz[cd]indol, 6-(4-Pyrazolyl)-4-(dimethylamino)-1,2,2a,3,4,5-hexahydrobenz[cd]indol,, 6-(4-Pyridinyl)-4-(di-n-propylamino)-1,2,2a,3,4,5-hexahydrobenz[cd]indol,6-(2-Pyridinyl)-4-(di-n-propylamino)-1,2,2a,3,4,5-hexahydrobenz[cd]indol, 6-(3-Pyridinyl)-4-(di-n-propylamino)-1,2,2a,3,4,5-hexahydrobenz[cd]indol, 6-(2-Thiazolyl)-4-(di-n-propylamino)-1,2,2a,3,4,5-hexahydrobenz[cd]indol, 6-(5-Thiazolyl)-4-(di-n-propylamino)-1,2,2a,3,4,5-hexahydrobenz[cd]indol, 6-(2-Oxadiazolyl)-4-(di-n-propylamino)-1,2,2a,3,4,5-hexahydrobenz[cd]indol, 6-(3-Furyl)-4-(di-n-propylamino)-1,2,2a,3,4,5-hexahydrobenz[cd]indol oder eines pharmazeutisch annehmbaren Salzes hiervon.

9. Verfahren zur Herstellung einer pharmazeutischen Formulierung, gekennzeichnet durch Mischen einer Verbindung der Formel 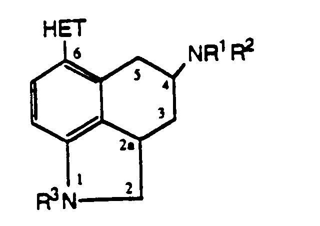 worin
R¹ steht für Wasserstoff, C₁-C₄ Alkyl, C₃-C₄ Alkenyl, Cyclopropylmenthyl, Phenyl(C₁-C₄)alkyl, Phenyl(C₁-C₄)alkyl, das mit einem oder mehreren Substituenten substituiert ist, ausgewählt aus (C₁-C₃) Alkoxy, Halogen, Hydroxy, (C₁-C₃) Thioalkyl, Nitro, (C₁-C₃) Alkyl und Trifluormethyl, -(CH₂)ₙS(C₁-C₄ Alkyl), -C(O)R⁴, -(CH₂)ₙC(O)NR⁵R⁶,
R² für Wasserstoff, C₁-C₄ Alkyl, Cyclopropylmethyl oder C₃-C₄ Alkenyl steht,
R³ für Wasserstoff, C₁-C₄ Alkyl oder eine Aminoschutzgruppe steht,
n für 1-4 steht,
R⁴ für Wasserstoff, C₁-C₄ Alkyl, C₁-C₄ Halogenalkyl, C₁-C₄ Alkoxy oder Phenyl steht,
R⁵ und R⁶ unabhängig für Wasserstoff, C₁-C₄ Alkyl oder C₅-C₈ Cycloalkyl stehen mit der Maßgabe, daß wenn eines von R⁵ und R⁶ für ein Cycloalkyl steht, das andere für Wasserstoff steht,
HET für einen aromatischen fünf- oder sechsgliedrigen heterocylischen Ring steht, wobei der Ring ein bis drei Heteroatome aufweist, die gleich oder verschieden sind und ausgewählt sind aus der Gruppe, die besteht aus Schwefel, Sauerstoff und Stickstoff, mit der Maßgabe, daß der sechsgliedrige heterocyclische Ring nur Kohlenstoff und Stickstoff enthalten kann und mit der weiteren Maßgabe, daß der fünfgliedrige Ring nicht mehr als einen Sauerstoff oder einen Schwefel aber nicht sowohl Sauerstoff als auch Schwefel enthält, und wahlweise an einem oder zwei Kohlenstoffatomen substituiert ist durch (C₁-C₃) Alkyl, Halogen, Hydroxy, (C₁-C₃) Alkoxy, (C₁-C₃) Thioalkyl, NH₂, CN oder Phenyl,
oder eines pharmazeutisch annehmbaren Salzes hiervon zusammen mit einem oder mehreren pharmazeutisch annehmbaren Trägern, Verdünnungsmitteln oder Hilfsstoffen hierfür.

## Revendications (Revendications pour l'(les) Etat(s) contractant(s) suivant(s): AT, BE, CH, DE, DK, FR, GB, IT, LI, LU, NL, PT, SE)

1. Composé répondant à la formule 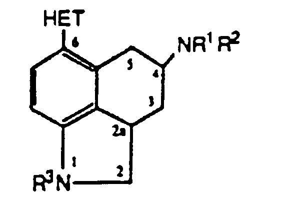 dans laquelle
R¹ représente un atome d'hydrogène, un groupe alkyle en C₁-C₄, un groupe alcényle en C₃-C₄, un groupe cyclopropylméthyle, un groupe phényl(alkyle en C₁-C₄), un groupe phényl(alkyle en C₁-C₄) substitué par un ou deux substituants choisis parmi un groupe alcoxy en C₁-C₃, un atome d'halogène, un groupe hydroxyle, un groupe thio(alkyle en C₁-C₃), un groupe nitro, un groupe alkyle en C₁-C₃ et un groupe trifluorométhyle, un groupe -(CH₂)ₙS(alkyle en C₁-C₄), un groupe -C(O)R⁴, un groupe -(CH₂)ₙC(O)NR⁵R⁶ ;
R² représente un atome d'hydrogène un groupe alkyle en C₁-C₄, un groupe cyclopropylméthyle ou un groupe alcényle en C₃-C₄;
R³ représente un atome d'hydrogène, un groupe alkyle en C₁-C₄ ou un groupe amino-protecteur ;
n vaut 1 à 4 ;
R⁴ représente un atome d'hydrogène un groupe alkyle en C₁-C₄, un groupe halogénoalkyle en C₁-C₄, un groupe alcoxy en C₁-C₄ ou un groupe phényle ;
R⁵ et R⁶ représentent, indépendamment, un atome d'hydrogène, un groupe alkyle en C₁-C₄ ou un groupe cycloalkyle en C₅-C₈, pour autant que lorsqu'un des groupes R⁵ ou R⁶ représente un groupe cycloalkyle, l'autre représente un atome d'hydrogène ;
HET représente un noyau hétérocyclique aromatique à 5 ou à 6 membres, ledit noyau présentant de 1 à 3 hétéroatomes qui sont identiques ou différents et qui sont choisis parmi le groupe constitué du soufre, de l'oxygène et de l'azote, pour autant que le noyau hétérocyclique à 6 membres ne puisse contenir que du carbone et de l'azote et en outre pour autant que le noyau à 5 membres puisse contenir au plus un atome d'oxygène ou un atome de soufre et non simultanément un atome d'oxygène et un atome de soufre, facultativement substitué sur un ou deux atomes de carbone par un groupe alkyle en C₁-C₃, un atome d'halogéne, un groupe hydroxyle, un groupe alcoxy en C₁-C₃, un groupe thio(alkyle en C₁-C₃), un groupe NH₂, un groupe CN ou un groupe phényle ; ou les sels pharmaceutiquement acceptables de celui-ci.

2. Composé selon la revendication 1, dans lequel HET représente un groupe isoxazole, un groupe pyrazole, un groupe pyridine, un groupe thiazole, un groupe furane, un groupe thiophène, un groupe oxadiazole, ou un sel pharmaceutiquement acceptable de celui-ci.

3. Composé selon la revendication 1 ou 2, dans lequel R¹ et R² représentent indépendamment un groupe alkyle en C₁-C₃, ou un sel pharmaceutiquement acceptable de celui-ci.

4. Composé selon l'une quelconque des revendications 1 à 3, dans lequel R³ représente un atome d'hydrogène, ou un sel pharmaceutiquement acceptable de celui-ci.

5. Composé selon la revendication 1 ou 2, dans lequel R¹ représente un groupe -(CH₂)ₙC(O)NR⁵R⁶, dans lequel n vaut 2, R⁵ représente un atome d'hydrogène, R⁶ représente un groupe cyclohexyle, R² représente un groupe alkyle en C₁-C₃ et R³ représente un atome d'hydrogène ou un groupe alkyle en C₁-C₄, ou un sel pharmaceutiquement acceptable de celui-ci.

6. Stéréo-isomère essentiellement pur d'un composé selon l'une quelconque des revendications 1 à 5, ou un sel pharmaceutiquement acceptable de celui-ci.

7. Stéréo-isomère selon la revendication 6, dans lequel la configuration est S à la position 2a et R à la position 4, ou un sel pharmaceutiquement acceptable de celui-ci.

8. Composé selon la revendication 1, choisi parmi le groupe constitué du 6-(3-isoxazolyl)-4-(di-n-propylamino)-1,2,2a,3,4,5-hexahydrobenz[cd]indole ; du 6-(5-isoxazolyl)-4-(di-n-propylamino)-1,2,2a,3,4,5-hexahydrobenz[cd]indole ; du 6-(3-pyrazolyl)-4-(di-n-propylamino)-1,2,2a,3,4,5-hexahydrobenz[cd]indole ; du 6-(4-pyrazolyl)-4-(diméthylamino)-1,2,2a,3,4,5-hexahydrobenz[cd]indole ; du 6-(4-pyridinyl)-4-(di-n-propylamino)-1,2,2a,3,4,5-hexahydrobenz[cd]indole ; du 6-(2-pyridinyl)-4-(di-n-propylamino)-1,2,2a,3,4,5-hexahydrobenz[cd]indole ; du 6-(3-pyridinyl)-4-(di-n-propylamino)-1,2,2a,3,4,5-hexahydrobenz[cd]indole ; du 6-(2-thiazolyl)-4-(di-n-propylamino)-1,2,2a,3,4,5-hexahydrobenz[cd]indole ; du 6-(5-thiazolyl)-4-(di-n-propylamino)-1,2,2a,3,4,5-hexahydrobenz[cd]indole ; du 6-(2-oxadiazolyl)-4-(di-n-propylamino)-1,2,2a,3,4,5-hexahydrobenz[cd]indole ; du 6-(3-furyl)-4-(di-n-propylamino)-1,2,2a,3,4,5-hexahydrobenz[cd]indole, ou un sel pharmaceutiquement acceptable de celui-ci.

9. Formulation pharmaceutique comprenant, comme ingrédient actif, un composé selon l'une quelconque des revendications 1 à 8, ou un sel pharmaceutiquement acceptable de celui-ci, associé à un ou davantage de supports, diluants ou excipients pharmaceutiquement acceptables pour celui-ci.

10. Composé selon l'une quelconque des revendications 1 à 8, pour l'utilisation dans le traitement de troubles liés à la sérotonine.

11. Procédé de préparation d'un composé répondant à la formule 1 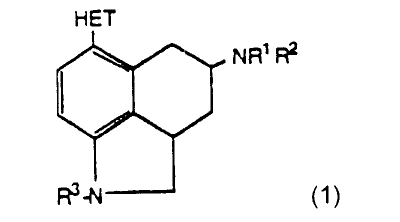 dans laquelle
R¹ représente un atome d'hydrogène, un groupe alkyle en C₁-C₄, un groupe alcényle en C₃-C₄, un groupe cyclopropylméthyle, un groupe phényl(alkyle en C₁-C₄), un groupe phényl(alkyle en C₁-C₄) substitué par un ou deux substituants choisis parmi un groupe alcoxy en C₁-C₃, un atome d'halogène, un groupe hydroxyle, un groupe thio(alkyle en C₁-C₃), un groupe nitro, un groupe alkyle en C₁-C₃ et un groupe trifluorométhyle, un groupe -(CH₂)ₙS(alkyle en C₁-C₄), un groupe -C(O)R⁴, un groupe -(CH₂)ₙC(O)NR⁵R⁶ ;
R² représente un atome d'hydrogène, un groupe alkyle en C₁-C₄, un groupe cyclopropylméthyle ou un groupe alcényle en C₃-C₄ ;
R³ représente un atome d'hydrogène, un groupe alkyle en C₁-C₄ ou un groupe amino-protecteur ;
n vaut 1 à 4 ;
R⁴ représente un atome d'hydrogène, un groupe alkyle en C₁-C₄, un groupe halogénoalkyle en C₁-C₄, un groupe alcoxy en C₁-C₄ ou un groupe phényle ;
R⁵ et R⁶ représentent, indépendamment, un atome d'hydrogène, un groupe alkyle en C₁-C₄ ou un groupe cycloalkyle en C₅-C₈, pour autant que lorsqu'un des groupes R⁵ ou R⁶ représente un groupe cycloalkyle, l'autre représente un atome d'hydrogène ;
HET représente un noyau hétérocyclique aromatique à 5 ou à 6 membres, ledit noyau présentant de 1 à 3 hétéroatomes qui sont identiques ou différents et qui' sont choisis parmi le groupe constitué du soufre, de l'oxygène et de l'azote, pour autant que le noyau hétérocyclique à 6 membres ne puisse contenir que du carbone et de l'azote et en outre pour autant que le noyau à 5 membres puisse contenir au plus un atome d'oxygène ou un atome de soufre et non simultanément un atome d'oxygène et un atome de soufre, facultativement substitué sur un ou deux atomes de carbone par un groupe alkyle en C₁-C₃, un atome d'halogéne, un groupe hydroxyle, un groupe alcoxy en C₁-C₃, un groupe thio(alkyle en C₁-C₃), un groupe NH₂, un groupe CN ou un groupe phényle ; ou un sel pharmaceutiquement acceptable de celui-ci, qui comprend :
1) la réaction d'un hexahydrobenz[cd]indole 4-amino-6-métallo-substitué répondant à la formule 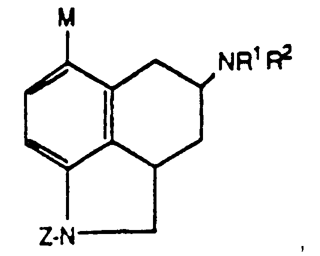 dans laquelle R¹ et R² sont tels que présentés ci-dessus ; Z représente un groupe amino-protecteur et M représente une fraction métallo, avec un composé hétérocyclique répondant à la formule
HET-L,
dans laquelle HET est tel que défini ci-dessus et L représente un groupe sortant ;
2) la déprotection d'un composé répondant à la formule 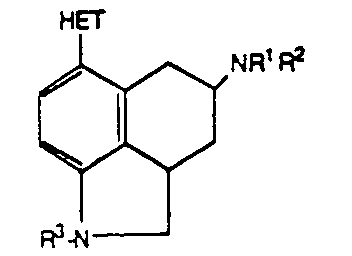 dans laquelle HET, R¹ et R² sont tels que définis ci-dessus et R³ représente un groupe amino-protecteur, de façon à obtenir un composé répondant à la formule 1, dans laquelle R³ représente un atome d'hydrogène ;
3) la réaction d'un hexahydrobenz[cd]indole 4-amino-6-halogéno-substitué répondant à la formule 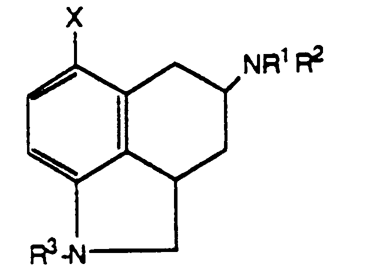 dans laquelle R¹, R² et R³ sont tels que définis ci-dessus et X représente un atome d'halogène, avec un dérivé organo-métallique répondant à la formule
M-HET
dans laquelle HET est tel que défini ci-dessus et M représente le lithium, le magnésium, le zinc, l'étain, le mercure ou l'acide borique ;
4) la réaction d'un composé répondant à la formule 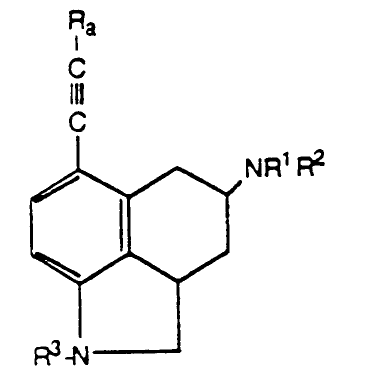 dans laquelle R¹, R² et R³ sont tels que définis ci-dessus et Rₐ représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle, avec un dipôle 1,3 répondant à la formule
⁺T=U-V⁻
dans laquelle T, U et V peuvent être choisis parmi la liste suivante
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
dans laquelle Rₐ est tel que présenté ci-dessus, Rₐ' représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle et R_{b} représente un atome d'hydrogène, un groupe alkyle en C₁-C₃, un groupe phényle ou un groupe (alkyle en C₁-C₃)phényle, de façon à obtenir un composé répondant à la formule 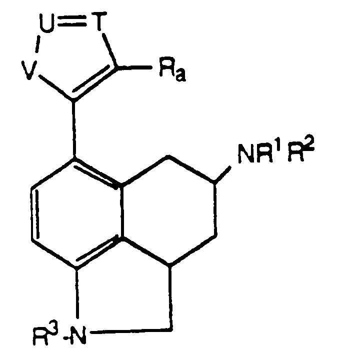 dans laquelle R₁, R₂, R₃, Rₐ, T, U et V sont tels que présentés ci-dessus ;
5) la réaction d'un composé répondant à la formule 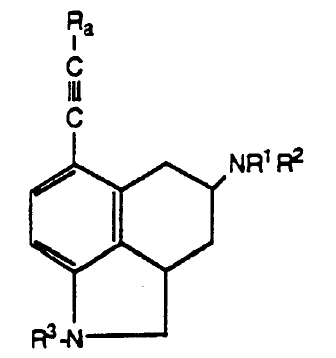 dans laquelle R¹, R² et R³ sont tels que définis ci-dessus et Rₐ représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle, avec un dipôle 1,3 répondant à la formule
⁺T=U-V⁻
dans laquelle T, U et V peuvent être choisis parmi la liste suivante
| T | U | V |
|---|---|---|
| CHRₐ | N | N |
| NR_{b} | N | N |
dans laquelle Rₐ est tel que présenté ci-dessus et R_{b} représente un atome d'hydrogène, un groupe alkyle en C₁-C₃, un groupe phényle ou un groupe (alkyle en C₁-C₃)phényle, de façon à obtenir un composé répondant à la formule 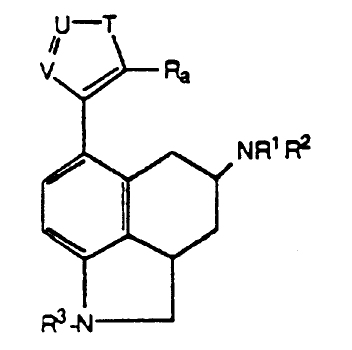 dans laquelle R₁, R₂, R₃, Rₐ, V, U et T sont tels que définis ci-dessus ;
6) la réaction d'un composé répondant à la formule 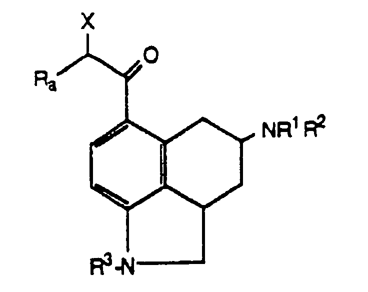 dans laquelle R₁, R₂ et R₃ sont tels que présentés ci-dessus, X représente un atome d'halogène et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec
NH₄⁺RₐCOO⁻ (où Rₐ est tel que défini ci-dessus)
de façon à obtenir un mélange de composés répondant à la formule 1, dans laquelle HET représente les groupes 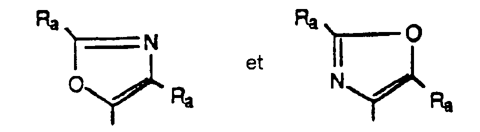 et ensuite, facultativement, la séparation des composés l'un de l'autre ;
7) la déshydratation d'un composé répondant à la formule 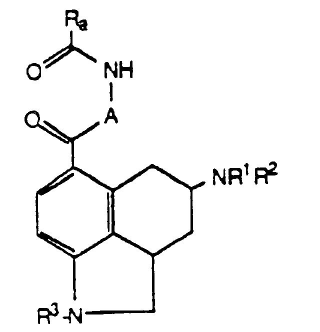 dans laquelle R¹, R² et R³ sont tels que présentés ci-dessus, A représente un groupe C-Rₐ ou NH et chaque Rₐ représente indépendamment un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 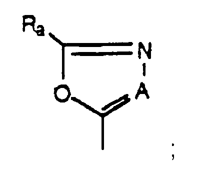
8) la cyclisation et la déshydratation d'un composé répondant à la formule 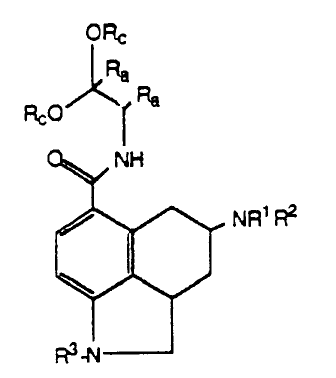 dans laquelle R¹, R² et R³ sont tels que définis ci-dessus, Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle et R_{c} représente un atome d'hydrogène ou un groupe alkyle en C₁-C₃, de façon à obtenir un composé répondant à la formule 1, dans laquelle HET représente un groupe 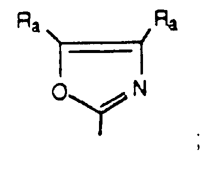
9) la réaction d'un composé répondant à la formule 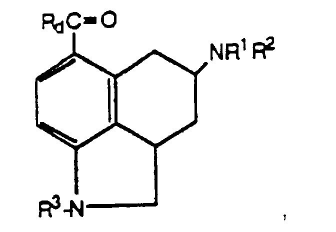 dans laquelle R¹, R² et R³ sont tels que présentés ci-dessus et R_{d} représente un groupe OH, un groupe O(alkyle en C₁-C₃), un groupe O(phényle), un groupe O((alkyle en C₁-C₃)phényle), un atome d'halogène, un groupe S(alkyle en C₁-C₃), un groupe S(phényle), un groupe S((alkyle en C₁-C₃)phényle), un groupe NH₂, un groupe NH(alkyle en C₁-C₃), un groupe N(alkyle en C₁-C₃)₂, un groupe OCO(alkyle en C₁-C₃), un groupe OCO(phényle) ou un groupe OCO((alkyle en C₁-C₃)phényle, avec 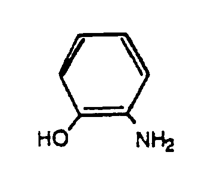 de façon à obtenir un composé répondant à la formule 1 dans laquelle HET représente un groupe 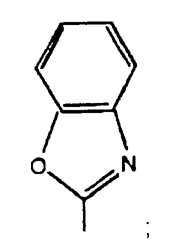
10) la cyclisation et la déshydratation d'un composé répondant à la formule 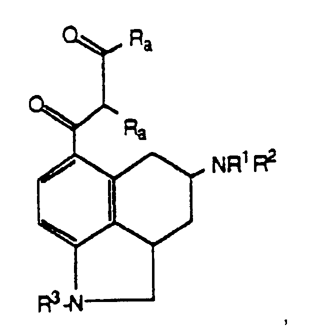 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 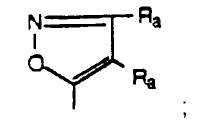
11) la cyclisation et la déshydratation d'un composé répondant à la formule 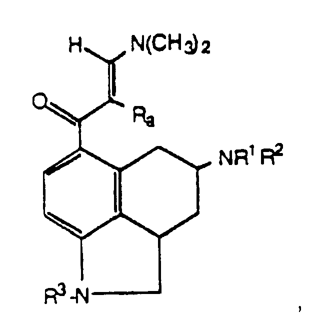 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 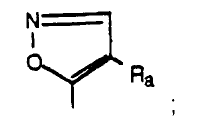
12) la cyclisation et la déshydratation d'un composé répondant à la formule 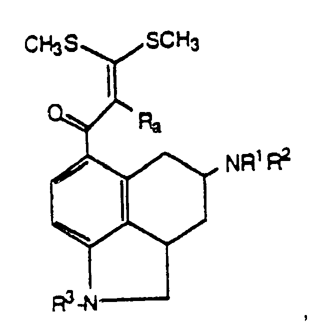 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 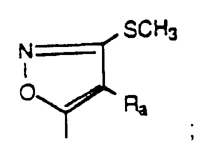
13) la cyclisation et la déshydratation d'un dianion répondant à la formule 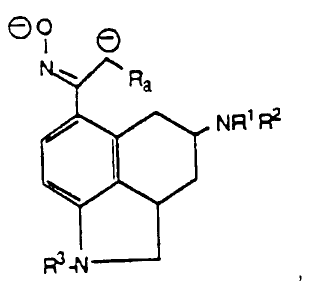 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un dérivé carbonyle répondant à la formule RₐCOOR_{c} (où Rₐ est tel défini ci-dessus et R_{c} représente un atome d'hydrogène ou un groupe alkyle en C₁-C₃) ou RₐCON(CH₃)₂ (où Rₐ est tel que défini ci-dessus) de façon à obtenir un composé répondant à la formule 1 dans laquelle HET représente un groupe 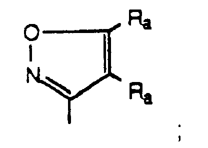
14) la cyclisation et la déshydratation d'un composé répondant à la formule 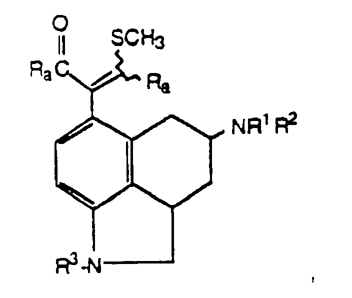 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 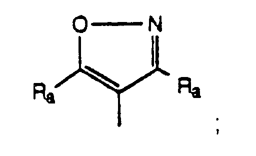
15) la réaction d'un composé répondant à la formule 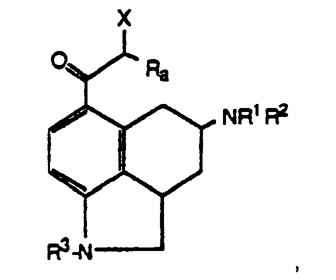 dans laquelle R¹, R² et R³ sont tels que définis ci-dessus, X représente un atome d'halogéne et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec 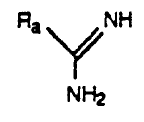 (où Rₐ est tel que présenté ci-dessus)
de façon à obtenir un composé répondant à la formule 1, dans laquelle HET représente un groupe 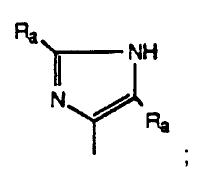
16) la réaction d'un composé répondant à la formule 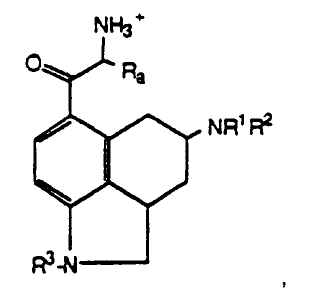 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du KSCN, et ensuite du R_{c}X (où R_{c} représente un atome d'hydrogène ou un groupe alkyle en C₁-C₃ et X représente un atome d'halogène) en présence d'une base de façon à obtenir un composé répondant à la formule 1, dans laquelle HET représente un groupe 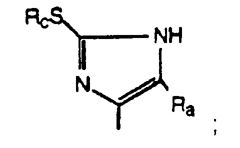
17) la déshydratation d'un composé répondant à la formule 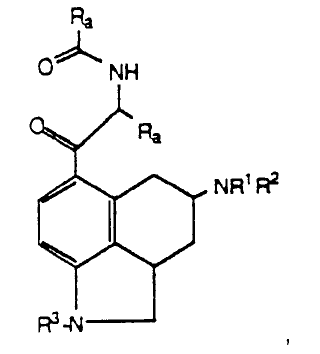 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, en présence d'ammoniac ou d'hydroxyde d'ammonium de façon à obtenir un composé répondant à la formule 1, dans laquelle HET représente un groupe 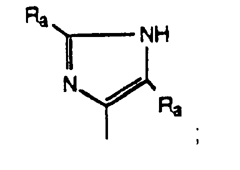
18) la réaction d'un composé répondant à la formule 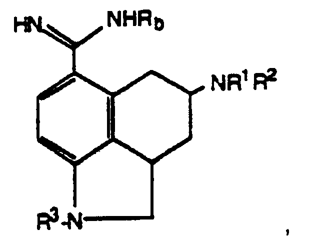 dans laquelle R¹, R² et R³ sont tels que définis ci-dessus et R_{b} représente un atome d'hydrogène, un groupe alkyle en C₁-C₃, un groupe phényle ou un groupe (alkyle en C₁-C₃)phényle, avec un composé répondant à la formule 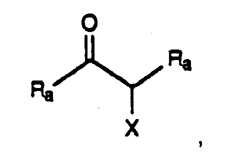 dans laquelle Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle et X représente un atome d'halogène, de façon à obtenir un composé répondant à la formule 1 dans laquelle HET représente un groupe 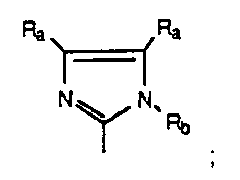
19) la déshydratation d'un composé répondant à la formule 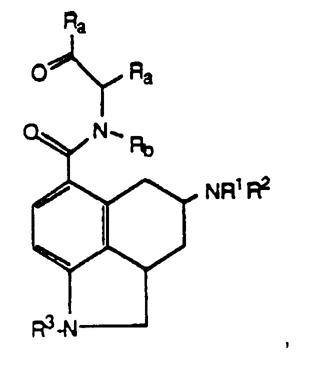 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, et R_{b} représente un atome d'hydrogène, un groupe alkyle en C₁-C₃, un groupe phényle ou un groupe (alkyle en C₁-C₃)phényle, en présence d'ammoniac ou d'hydroxyde d'ammonium de façon à obtenir un composé répondant à la formule 1, dans laquelle HET représente un groupe 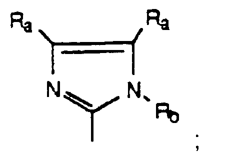
20) la réaction d'un composé répondant à la formule 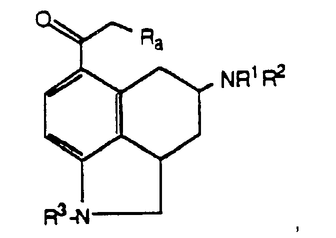 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un azide répondant à la formule R_{b}N₃, où R_{b} représente un atome d'hydrogène, un groupe alkyle en C₁-C₃, un groupe phényle ou un groupe (alkyle en C₁-C₃)phényle, suivi par la déshydratation du composé résultant de façon à obtenir un composé répondant à la formule 1, dans laquelle HET représente un groupe 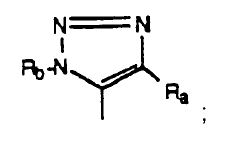
21) la cyclisation et la déshydratation d'un composé répondant à la formule 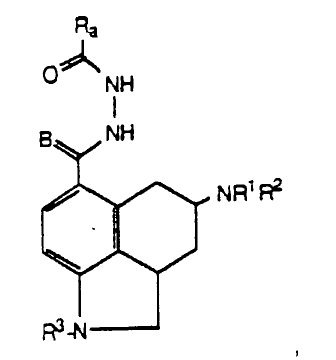 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, et B représente O ou NH, de façon à obtenir un composé répondant à la formule 1 dans laquelle HET représente un groupe 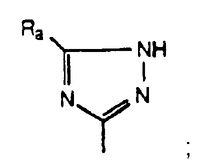 pour autant que lorsque B représente O, ladite réaction de cyclisation est effectuée en présence d'ammoniac ou d'hydroxyde d'ammonium ;
22) la réaction d'un composé répondant à la formule 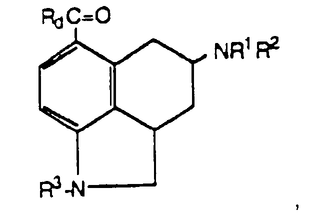 dans laquelle R¹, R² et R³ sont tels que présentés ci-dessus et R_{d} représente un groupe OH, un groupe O(alkyle en C₁-C₃), un groupe O(phényle), un groupe O((alkyle en C₁-C₃)phényle), un atome d'halogène, un groupe S(alkyle en C₁-C₃), un groupe S(phényle), un groupe S((alkyle en C₁-C₃)phényle), un groupe NH₂, un groupe NH(alkyle en C₁-C₃), un groupe N(alkyle en C₁-C₃)₂, un groupe OCO(alkyle en C₁-C₃), un groupe OCO(phényle) ou un groupe OCO((alkyle en C₁-C₃)phényle, avec un composé répondant à la formule 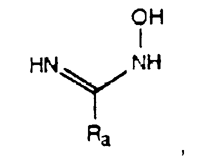 dans laquelle Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 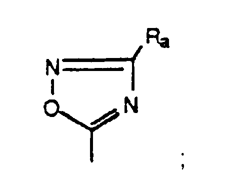
23) la réaction d'un composé répondant à la formule 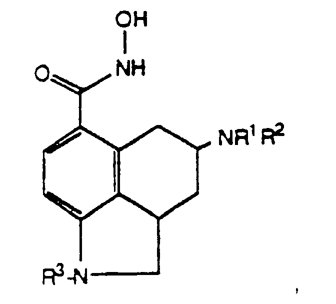 dans laquelle R¹, R² et R³ sont tels que définis ci-dessus, avec RₐCN, où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 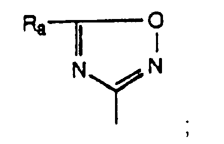
24) la cyclisation et la déshydratation d'un composé répondant à la formule 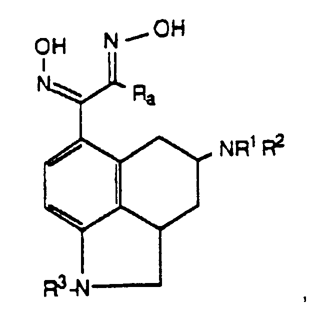 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 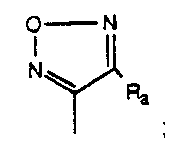
25) la réaction d'un composé répondant à la formule 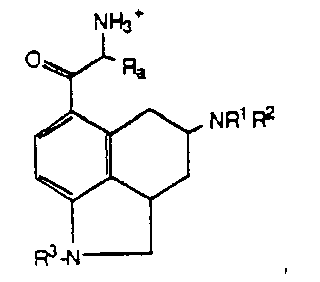 laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 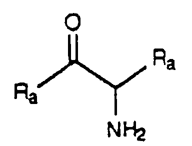 (où Rₐ est tel que présenté ci-dessus)
en présence d'un agent oxydant, de façon à obtenir un composé répondant à la formule 1 dans laquelle HET représente un groupe 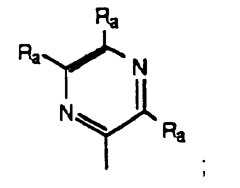
26) la réaction d'un composé répondant à la formule 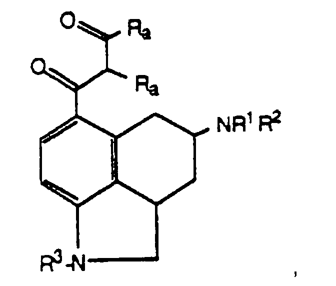 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 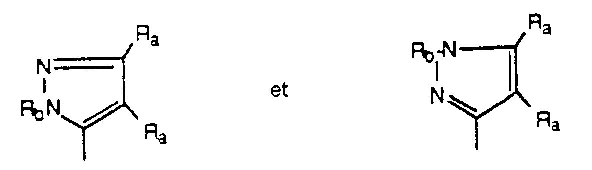 et ensuite, facultativement, la séparation des composés l'un de l'autre ;
27) la réaction d'un composé répondant à la formule 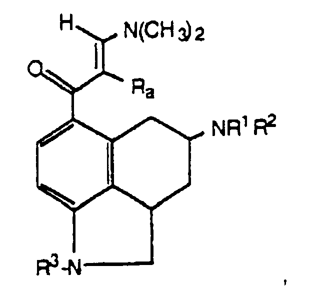 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 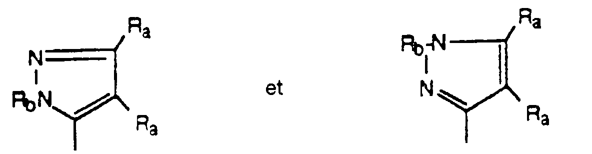 et ensuite, facultativement, la séparation des composés l'un de l'autre ;
28) la réaction d'un composé répondant à la formule 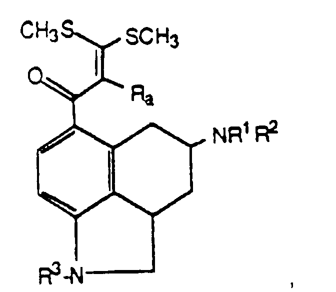 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 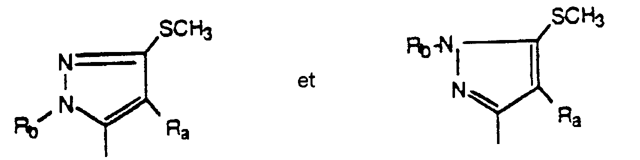 et ensuite, facultativement, la séparation des composés l'un de l'autre ;
29) la réaction d'un composé répondant à la formule 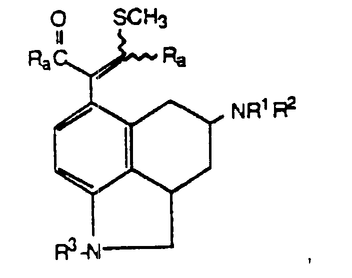 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente un groupe 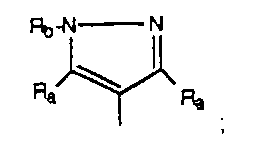
30) la réaction d'un composé répondant à la formule 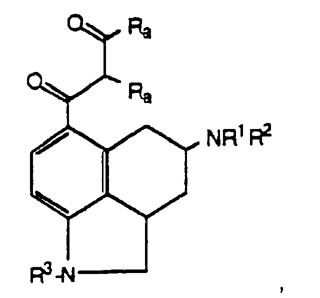 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 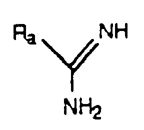 (où Rₐ est tel que présenté ci-dessus)
de façon à obtenir un composé répondant à la formule 1, dans laquelle HET représente un groupe 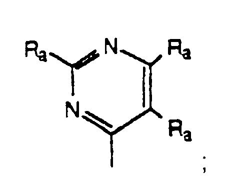
31) la réaction d'un composé répondant à la formule 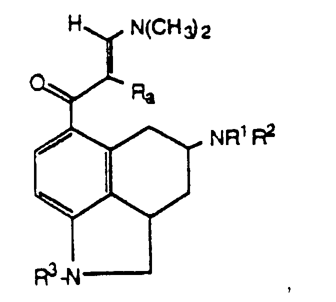 dans laquelle R¹, R² et R³ sont tels que définis ci-dessus et Rₐ représente un atome d'hydrogène, un groupe alkyle en C₁-C₃, un atome d'halogène un groupe OH, un groupe O(alkyle en C₁-C₃), un groupe S(alkyle en C₁-C₃), un groupe NH₂, un groupe CN ou un groupe phényle, avec un composé répondant à la formule 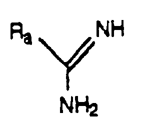 (où Rₐ est tel que présenté ci-dessus)
de façon à obtenir un composé répondant à la formule 1, dans laquelle HET représente un groupe 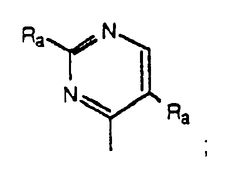
32) la réaction d'un composé répondant à la formule 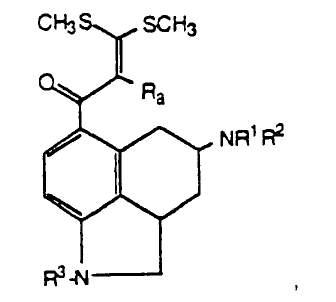 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 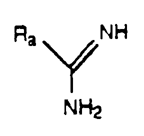 (où Rₐ est tel que présenté ci-dessus)
de façon à obtenir un composé répondant à la formule 1, dans laquelle HET représente un groupe 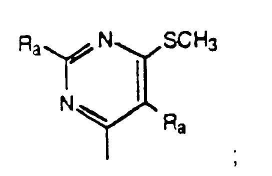
33) la réaction d'un composé répondant à la formule 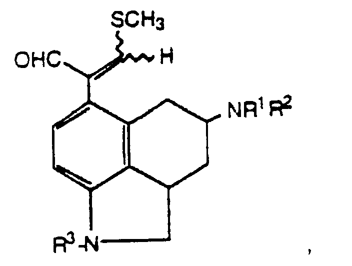 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 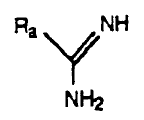 (où Rₐ est tel que présenté ci-dessus)
de façon à obtenir un composé répondant à la formule 1, dans laquelle HET représente un groupe 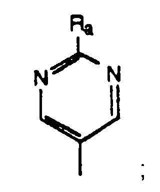
34) la réaction d'un composé répondant à la formule 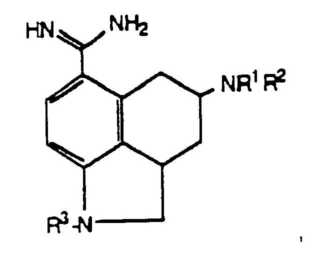 dans laquelle R¹, R² et R³ sont tels que définis ci-dessus, avec un composé répondant à la formule 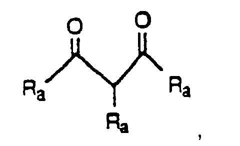 où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle de façon à obtenir un composé répondant à la formule 1, dans laquelle HET représente un groupe 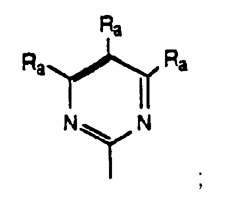
35) la réaction d'un composé répondant à la formule 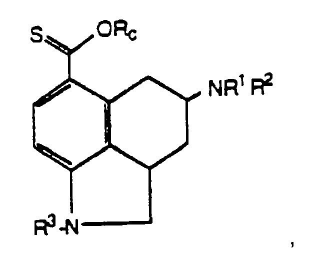 dans laquelle R¹, R² et R³ sont tels que définis ci-dessus et R_{c} représente un atome d'hydrogène ou un groupe alkyle en C₁-C₃, avec un composé répondant à la formule RₐCH₂NNH, où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1, dans laquelle HET représente un groupe 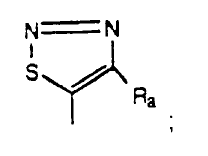
36) la réaction d'un composé répondant à la formule 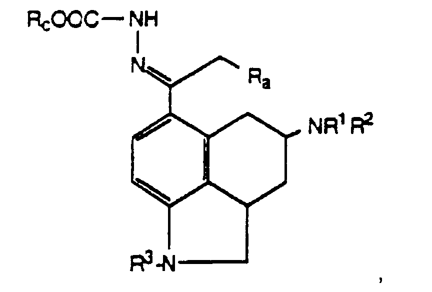 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1, dans laquelle HET représente un groupe 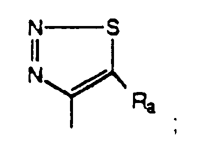
37) la réaction d'un composé répondant à la formule 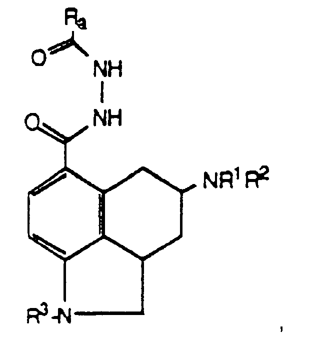 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du P₂S₅, de façon à obtenir un composé répondant à la formule 1, dans laquelle HET représente un groupe 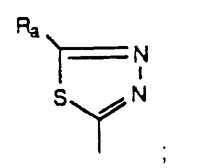
38) la réaction d'un composé répondant à la formule 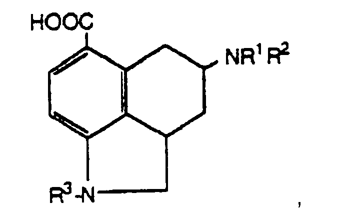 dans laquelle R¹, R² et R³ sont tels que définis ci-dessus, avec un composé répondant à la formule H₂NNHCSNH₂, en présence d'acide polyphosphorique, de façon à obtenir un composé répondant à la formule 1, dans laquelle HET représente un groupe 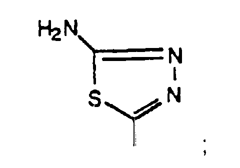
39) la réaction d'un composé répondant à la formule 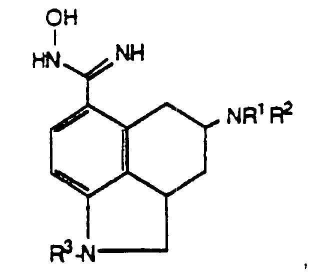 dans laquelle R¹, R² et R³ sont tels que définis ci-dessus, avec du disulfure de carbone, de façon à obtenir un composé répondant à la formule 1, dans laquelle HET représente un groupe 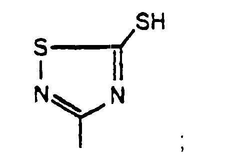
40) la réaction d'un composé répondant à la formule 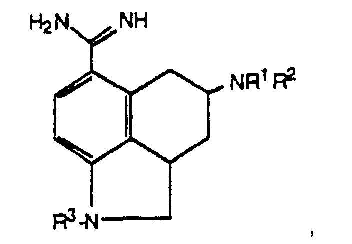 dans laquelle R¹, R² et R³ sont tels que définis ci-dessus, avec du (CNS)₂, de façon à obtenir un composé répondant à la formule 1, dans laquelle HET représente un groupe 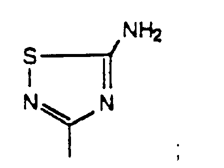
41) l'oxydation d'un composé répondant à la formule 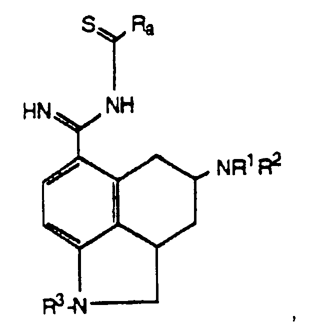 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 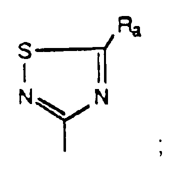
42) la réaction d'un composé répondant à la formule 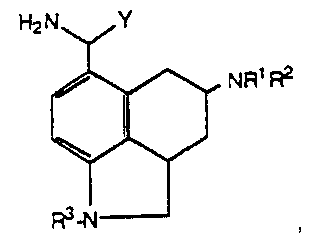 dans laquelle R¹, R² et R³ sont tels que définis ci-dessus et Y représente un groupe -CN ou un groupe -C(O)NH₂, avec un agent oxydant, tel que le SOCl₂, le SCl₂, le S₂Cl₂ ou le SO₂Cl₂, de façon à obtenir un composé répondant à la formule 1 dans laquelle HET représente un groupe 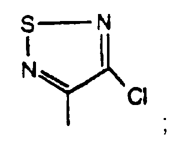
43) la réaction d'un composé répondant à la formule 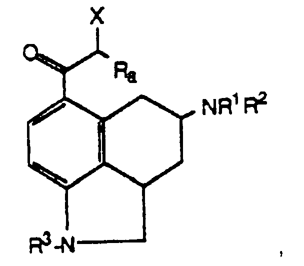 dans laquelle R¹, R² et R³ sont tels que définis ci-dessus, X représente un atome d'halogéne et Rₐ représente un atome d'hydrogène, un groupe alkyle en C₁-C₃, un atome d'hydrogène, un groupe OH, un groupe O(alkyle en C₁-C₃), un groupe S(alkyle en C₁-C₃), un groupe NH₂, un groupe CN ou un groupe phényle, avec du RₐCSNH₂, où Rₐ est tel que présenté ci-dessus, de façon à obtenir un composé répondant à la formule 1, dans laquelle HET représente un groupe 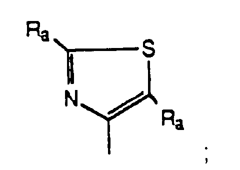
44) la réaction d'un composé répondant à la formule 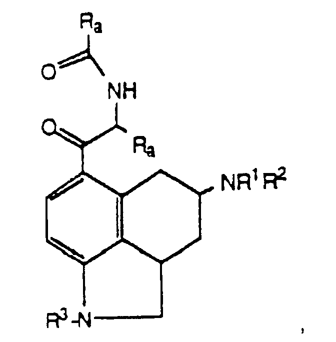 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du P₂S₅, de façon à obtenir un composé répondant à la formule 1, dans laquelle HET représente un groupe 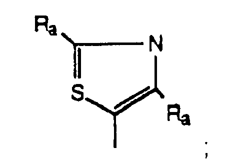
45) la réaction d'un composé répondant à la formule 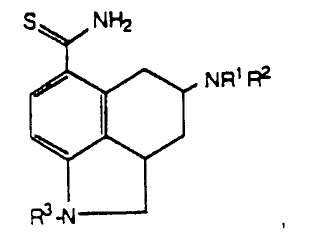 dans laquelle R¹, R² et R³ sont tels que définis ci-dessus, avec un composé répondant à la formule 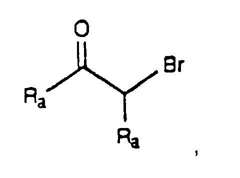 où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 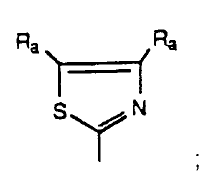
46) la réaction d'un composé répondant à la formule 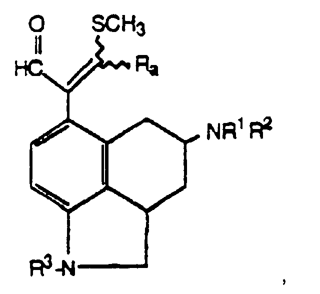 dans laquelle R¹, R² et R³ sont tels que définis ci-dessus, et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du RₐCSNH₂, où Rₐ est tel que présenté ci-dessus, de façon à obtenir un composé répondant à la formule 1, dans laquelle HET représente un groupe 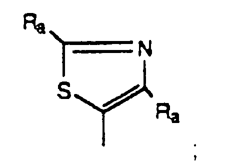
47) la réaction d'un composé répondant à la formule 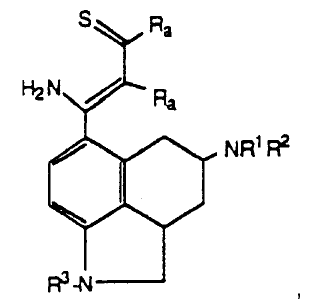 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à préparer un composé répondant à la formule 1 dans laquelle HET représente un groupe 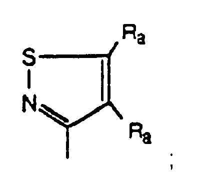
48) la réaction d'un composé répondant à la formule 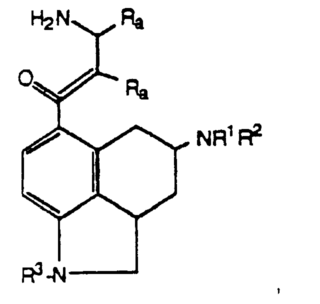 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à obtenir un composé répondant à la formule 1 dans laquelle HET représente un groupe 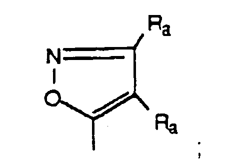
49) la réaction d'un composé répondant à la formule 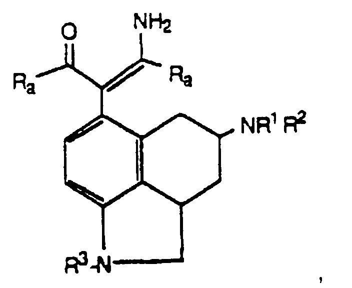 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à obtenir un composé répondant à la formule 1 dans laquelle HET représente un groupe 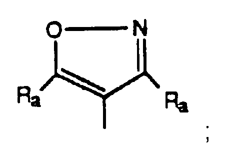
50) la réaction d'un composé répondant à la formule 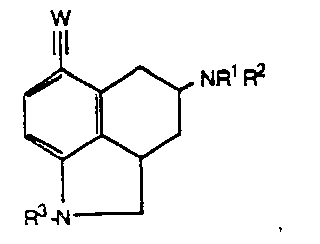 dans laquelle R¹, R² et R³ sont tels que définis ci-dessus et W représente un atome d'hydrogène ou un groupe CHO, avec du KSCN ou du NaHSSO₃ et ensuite la réaction de l'intermédiaire formé de cette façon avec l'ammoniac ou l'hydroxyde d'ammonium, de façon à obtenir un composé répondant à la formule 1 dans laquelle HET représente un groupe 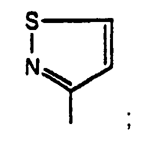
51) la réaction d'un composé répondant à la formule 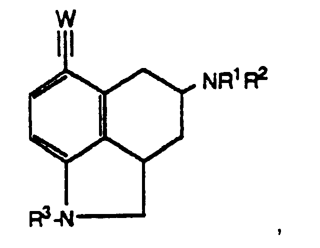 dans laquelle R¹, R² et R³ sont tels que définis ci-dessus et W représente un atome d'hydrogène ou un groupe CHO, avec du H₂NSSO₃K et ensuite la réaction de l'intermédiaire formé de cette façon avec une base, de façon à obtenir un composé répondant à la formule 1 dans laquelle HET représente un groupe 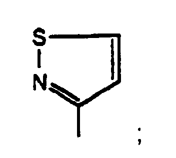
52) la réaction d'un composé répondant à la formule 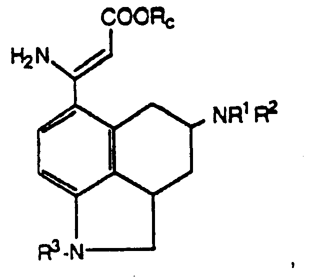 dans laquelle R¹, R² et R³ sont tels que définis ci-dessus et R_{c} représente un atome d'hydrogène ou un groupe alkyle en C₁-C₃, avec du CSCl₂, de façon à obtenir un composé répondant à la formule 1 dans laquelle HET représente un groupe 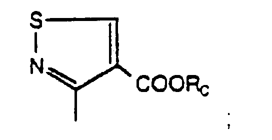
53) la réaction d'un composé répondant à la formule 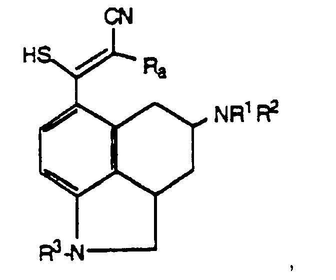 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du NCS de façon à obtenir un composé répondant à la formule 1 dans laquelle HET représente un groupe 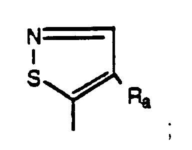
54) la réaction d'un composé répondant à la formule 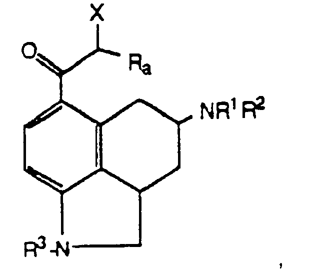 dans laquelle R¹, R² et R³ sont tels que définis ci-dessus, X représente un atome d'halogène et Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, avec 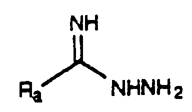 (où Rₐ est tel que présenté ci-dessus)
de façon à obtenir un composé répondant à la formule 1 dans laquelle HET représente un groupe 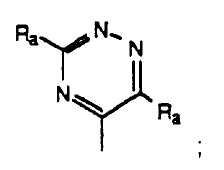
55) la réaction d'un composé répondant à la formule 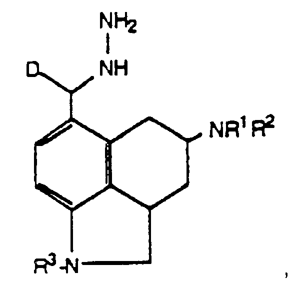 dans laquelle R¹, R² et R³ sont tels que définis ci-dessus et D représente =NH ou =O, avec un composé répondant à la formule 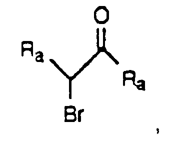 dans laquelle Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1, dans laquelle HET représente un groupe 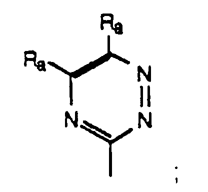
56) la réaction d'un composé répondant à la formule 1 dans laquelle HET est substitué par au moins un groupe hydroxyle, avec du POX₃, du PX₃, du SOX₂, du P(phényle)₃.X₂ ou du P(alcoxy en C₁-C₃)₃.X₂, où chaque X représente un atome d'halogéne, de façon à convertir le groupe hydroxyle en substituant halogène ;
57) la réaction d'un composé répondant à la formule 1 dans laquelle HET est substitué par au moins un groupe amino, avec du HONO de façon à former l'ion diazonium correspondant, suivi par la conversion de cet ion en composé correspondant substitué par un atome d'halogéne, avec du CuX (où X représente un atome d'halogène) du KI ou du HBF₄, par application de chaleur ;
58) la réaction d'un composé répondant à la formule 1 dans laquelle HET est substitué par au moins un substituant halogène, avec un anion alcoxyde répondant à la formule
(alkyle en C₁-C₃)-O^{⊖}
de façon à convertir le substituant halogène en substituant alcoxy ;
59) la réaction d'un composé répondant à la formule 1 dans laquelle HET est substitué par au moins un groupe hydroxyle, avec un halogénure d'alkyle en C₁-C₃ de façon à convertir le groupe hydroxyle en substituant alcoxy ;
60) la réaction d'un composé répondant à la formule 1 dans laquelle HET est substitué par au moins un substituant hydroxyle, avec un composé diazo répondant à la formule (alkyle en C₁-C₃)N₂ de façon à convertir le groupe hydroxyle en substituant alcoxy ;
61) la réaction d'un composé répondant à la formule 1 dans laquelle HET est substitué par au moins un groupe amino, avec du HONO de façon à former l'ion diazonium correspondant, suivi par la conversion de cet on en composé correspondant substitué par un groupe hydroxyle en utilisant de l'eau ou un acide ;
62) la réaction d'un composé répondant à la formule 1 dans laquelle HET est substitué par au moins un groupe alcoxy en C₁-C₃, avec de l'acide iodhydrique concentré, de l'acide bromhydrique concentré ou un acide de Lewis de façon à convertir le groupe alcoxy en substituant hydroxyle ;
63) la réaction d'un composé répondant à la formule 1 dans laquelle HET est substitué par au moins un groupe amino, avec du HONO de façon à former l'ion diazonium correspondant, suivi par la conversion de cet ion en composé correspondant substitué par un groupe nitrile en utilisant du CuCN ;
64) la réaction d'un composé répondant à la formule 1 dans laquelle HET est substitué par au moins un groupe amino, avec du HONO de façon à former l'ion diazonium correspondant, suivi par la conversion de cet ion en composé correspondant substitué par un groupe (alkyle en C₁-C₃)thio en utilisant un (alkyle en C₁-C₃)mercaptan ;
65) la réaction d'un composé répondant à la formule 1 dans laquelle HET est substitué par au moins un substituant halogène, avec un anion alkylthio répondant à la formule
(alkyle en C₁-C₃)-S^{⊖}
de façon à convertir le substituant halogéne en une fraction alkylthio ;
66) la réaction d'un composé répondant à la formule 1 dans laquelle HET est substitué par au moins une fraction -SH, avec un halogénure d'alkyle en C₁-C₃ de façon à obtenir le composé correspondant répondant à la formule 1 présentant une fraction alkylthio ;
67) la réduction d'un composé répondant à la formule 1, dans laquelle HET est substitué par au moins un substituant nitro, de façon à obtenir un composé répondant à la formule 1, dans laquelle le substituant nitro est converti en fraction amino ;
68) la réduction d'un composé répondant à la formule 1 dans laquelle HET est substitué par au moins un substituant halogène, de façon à convertir le substituant halogène en atome d'hydrogène ;
69) la réduction d'un composé répondant à la formule 1 dans laquelle HET est substitué par au moins un groupe hydroxyle, de façon à convertir le substituant hydroxyle en atome d'hydrogène ;
70) la réaction d'un composé répondant à la formule 1 dans laquelle HET est substitué par au moins une fraction amino, avec du HONO, de façon à former l'ion diazonium correspondant, suivi par la conversion de cet ion en composé non-substitué correspondant en utilisant du H₃PO₂ ;
71) la réduction d'un composé répondant à la formule 1 dans laquelle HET est substitué sur un des atomes d'azote dans ce noyau hétérocyclique par un groupe (alkyle en C₁-C₃)phényle de façon à obtenir le composé correspondant répondant à la formule 1, dans laquelle l'atome d'azote est non-substitué ;
72) la réduction d'un composé répondant à la formule 1 dans laquelle HET est substitué par au moins une fraction (alkyle en C₁-C₃)thio de façon à obtenir le composé non-substitué correspondant répondant à la formule 1 ou
73) la réaction d'un composé répondant à la formule 1 avec un acide organique ou inorganique pharmaceutiquement acceptable de façon à former un sel d'addition acide pharmaceutiquement acceptable de ce composé.

## Revendications (Revendications pour l'(les) Etat(s) contractant(s) suivant(s): ES, GR)

1. Procédé de préparation d'un composé répondant à la formule 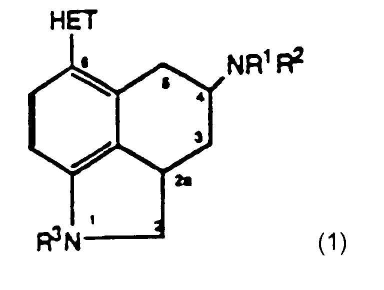 dans laquelle
R¹ représente un atome d'hydrogène, un groupe alkyle en C₁-C₄, un groupe alcényle en C₃-C₄, un groupe cyclopropylméthyle, un groupe phényl(alkyle en C₁-C₄), un groupe phényl(alkyle en C₁-C₄) substitué par un ou deux substituants choisis parmi un groupe alcoxy en C₁-C₃, un atome d'halogène, un groupe hydroxyle, un groupe thio(alkyle en C₁-C₃), un groupe nitro, un groupe alkyle en C₁-C₃, et un groupe trifluorométhyle, un groupe -(CH₂)ₙS(alkyle en C₁-C₄), un groupe -C(O)R⁴, un groupe -(CH₂)ₙC(O)NR⁵R⁶ ;
R² représente un atome d'hydrogène, un groupe alkyle en C₁-C₄, un groupe cyclopropylméthyle ou un groupe alcényle en C₃-C₄ ;
R³ représente un atome d'hydrogène, un groupe alkyle en C₁-C₄ ou un groupe amino-protecteur ;
n vaut 1 à 4 ;
R⁴ représente un atome d'hydrogène un groupe alkyle en C₁-C₄, un groupe halogénoalkyle en C₁-C₄, un groupe alcoxy en C₁-C₄ ou un groupe phényle ;
R⁵ et R⁶ représentent, indépendamment, un atome d'hydrogène, un groupe alkyle en C₁-C₄ ou un groupe cycloalkyle en C₅-C₈, pour autant que lorsqu'un des groupes R⁵ ou R⁶ représente un groupe cycloalkyle, l'autre représente un atome d'hydrogène ;
HET représente un noyau hétérocyclique aromatique à 5 ou à 6 membres, ledit noyau présentant de 1 à 3 hétéroatomes qui sont identiques ou différents et qui sont choisis parmi le groupe constitué du soufre, de l'oxygène et de l'azote, pour autant que le noyau hétérocyclique à 6 membres ne puisse contenir que du carbone et de l'azote et en outre pour autant que le noyau à 5 membres puisse contenir au plus un atome d'oxygène ou un atome de soufre et non simultanément un atome d'oxygène et un atome de soufre, facultativement substitué sur un ou deux atomes de carbone par un groupe alkyle en C₁-C₃, un atome d'halogène, un groupe hydroxyle, un groupe alcoxy en C₁-C₃, un groupe thio(alkyle en C₁-C₃), un groupe NH₂, un groupe CN ou un groupe phényle ; ou un sel pharmaceutiquement acceptable de celui-ci, qui comprend :
1) la réaction d'un hexahydrobenz[cd]indole 4-amino-6-métallo-substitué répondant à la formule 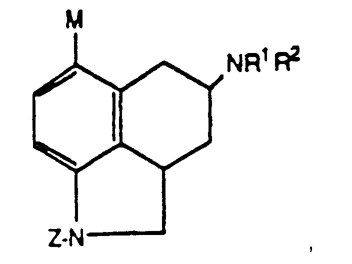 dans laquelle R¹ et R² sont tels que présentés ci-dessus ; Z représente un groupe amino-protecteur et M représente une fraction métallo, avec un composé hétérocyclique répondant à la formule
HET-L,
dans laquelle HET est tel que défini ci-dessus et L représente un groupe sortant ;
2) la déprotection d'un composé répondant à la formule 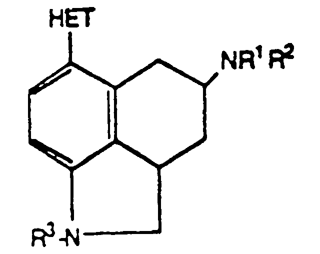 dans laquelle HET, R¹ et R² sont tels que définis ci-dessus et R³ représente un groupe amino-protecteur, de façon à obtenir un composé répondant à la formule 1, dans laquelle R³ représente un atome d'hydrogène ;
3) la réaction d'un hexahydrobenz[cd]indole 4-amino-6-halogéno-substitué répondant à la formule 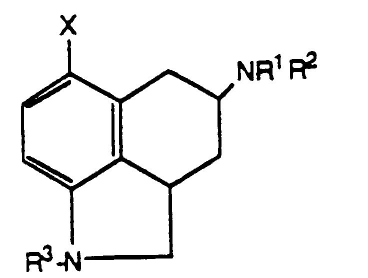 dans laquelle R¹, R² et R³ sont tels que définis ci-dessus et X représente un atome d'halogène, avec un dérivé organo-métallique répondant à la formule
M-HET
dans laquelle HET est tel que défini ci-dessus et M représente le lithium, le magnésium, le zinc, l'étain, le mercure ou l'acide borique ;
4) la réaction d'un composé répondant à la formule 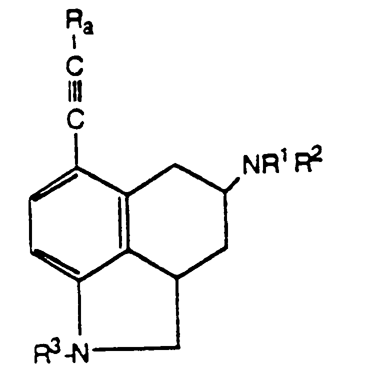 dans laquelle R¹, R² et R³ sont tels que définis ci-dessus et Rₐ représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle, avec un dipôle 1,3 répondant à la formule
⁺T=U-V⁻
dans laquelle T, U et V peuvent être choisis parmi la liste suivante
| T | U | V |
|---|---|---|
| CRₐ | N | CHRₐ |
| CRₐ | N | NR_{b} |
| CRₐ | N | O |
| N | N | O |
| CRₐ | CRₐ' | NR_{b} |
| CRₐ | CRₐ' | O |
| N | CRₐ' | CHRₐ |
| N | CRₐ' | NR_{b} |
| N | CRₐ' | O |
dans laquelle Rₐ est tel que présenté ci-dessus, Rₐ' représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle et R_{b} représente un atome d'hydrogène, un groupe alkyle en C₁-C₃, un groupe phényle ou un groupe (alkyle en C₁-C₃)phényle, de façon à obtenir un composé répondant à la formule 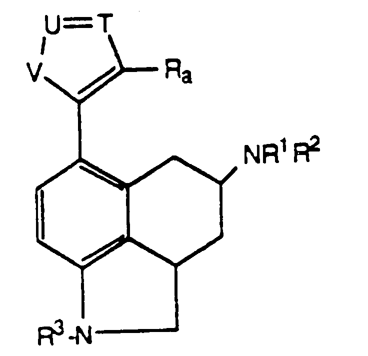 dans laquelle R₁, R₂, R₃, Rₐ, T, U et V sont tels que présentés ci-dessus ;
5) la réaction d'un composé répondant à la formule 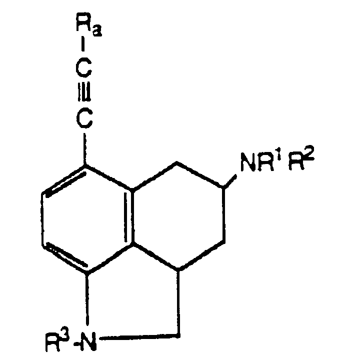 dans laquelle R¹, R² et R³ sont tels que définis ci-dessus et Rₐ représente un atome d'hydrogène, un groupe alkyle en C₁-C₃, un atome d'halogène, un groupe O(alkyle en C₁-C₃), un groupe S(alkyle en C₁-C₃), un groupe CN ou un groupe phényle, avec un dipôle 1,3 répondant à la formule
⁺T=U-V⁻
dans laquelle T, U et V peuvent être choisis parmi la liste suivante
| T | U | V |
|---|---|---|
| CHRₐ | N | N |
| NR_{b} | N | N |
dans laquelle Rₐ est tel que présenté ci-dessus et R_{b} représente un atome d'hydrogène, un groupe alkyle en C₁-C₃, un groupe phényle ou un groupe (alkyle en C₁-C₃)phényle, de façon à obtenir un composé répondant à la formule 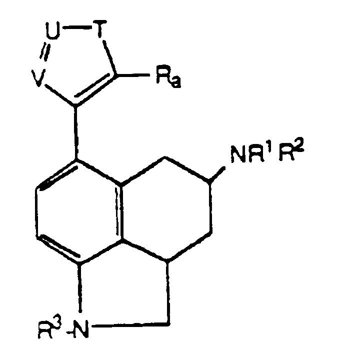 dans laquelle R₁, R₂, R₃, Rₐ, V, U et T sont tels que définis ci-dessus ;
6) la réaction d'un composé répondant à la formule 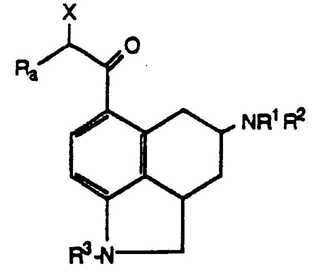 dans laquelle R₁, R₂ et R₃ sont tels que présentés ci-dessus, X représente un atome d'halogène et Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, avec
NH₄⁺RₐCOO⁻ (où Rₐ est tel que défini ci-dessus)
de façon à obtenir un mélange de composés répondant à la formule 1, dans laquelle HET représente les groupes 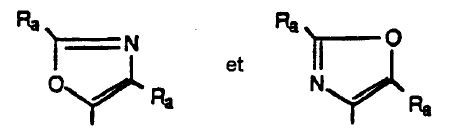 et ensuite, facultativement, la séparation des composés l'un de l'autre ;
7) la déshydratation d'un composé répondant à la formule 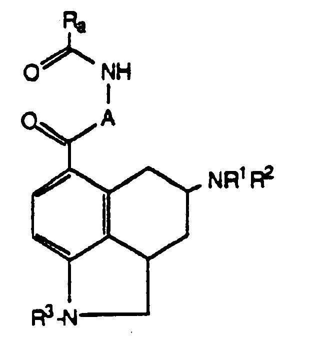 dans laquelle R¹, R² et R³ sont tels que présentés ci-dessus, A représente un groupe C-Rₐ ou NH et chaque Rₐ représente indépendamment un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 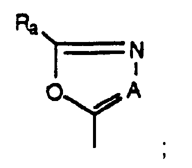
8) la cyclisation et la déshydratation d'un composé répondant à la formule 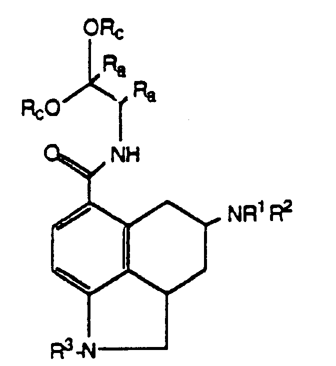 dans laquelle R¹, R² et R³ sont tels que définis ci-dessus, Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle et R_{c} représente un atome d'hydrogène ou un groupe alkyle en C₁-C₃, de façon à obtenir un composé répondant à la formule 1, dans laquelle HET représente un groupe 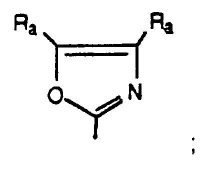
9) la réaction d'un composé répondant à la formule 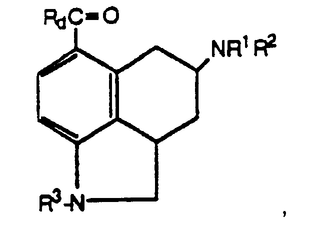 dans laquelle R¹, R² et R³ sont tels que présentés ci-dessus et R_{d} représente un groupe OH, un groupe O(alkyle en C₁-C₃), un groupe O(phényle), un groupe O((alkyle en C₁-C₃)phényle), un groupe NH₂, un atome d'halogéne, un groupe S(alkyle en C₁-C₃), un groupe S(phényle), un groupe S((alkyle en C₁-C₃)phényle), un groupe NH(alkyle en C₁-C₃), un groupe N(alkyle en C₁-C₃)₂, un groupe OCO(alkyle en C₁-C₃), un groupe OCO(phényle) ou un groupe OCO((alkyle en C₁-C₃)phényle, avec 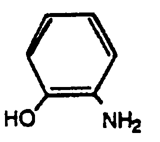 de façon à obtenir un composé répondant à la formule 1, dans laquelle HET représente un groupe 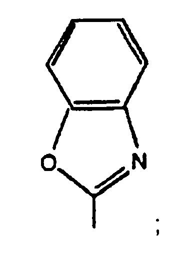
10) la cyclisation et la déshydratation d'un composé répondant à la formule 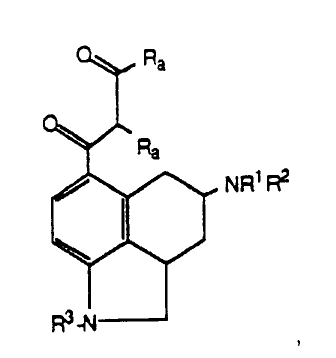 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 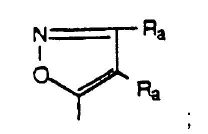
11) la cyclisation et la déshydratation d'un composé répondant à la formule 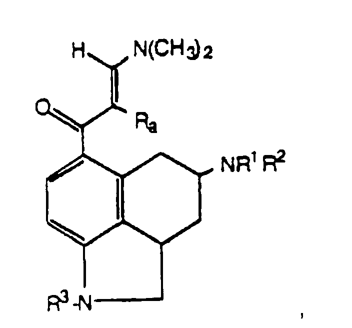 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 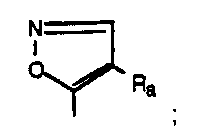
12) la cyclisation et la déshydratation d'un composé répondant à la formule 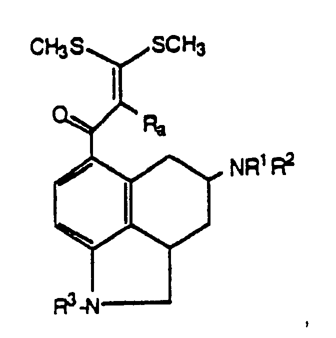 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 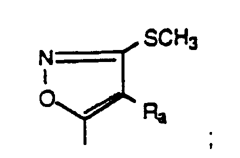
13) la cyclisation et la déshydratation d'un dianion répondant à la formule 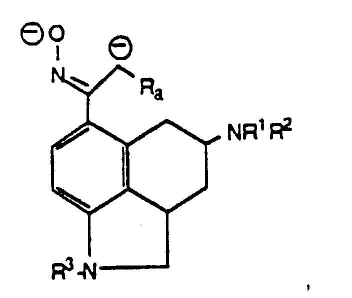 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un dérivé carbonyle répondant à la formule RₐCOOR_{c} (où Rₐ est tel défini ci-dessus et R_{c} représente un atome d'hydrogène ou un groupe alkyle en C₁-C₃) ou RₐCON(CH₃)₂ (où Rₐ est tel que défini ci-dessus) de façon à obtenir un composé répondant à la formule 1 dans laquelle HET représente un groupe 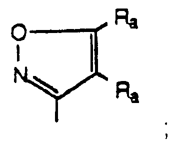
14) la cyclisation et la déshydratation d'un composé répondant à la formule 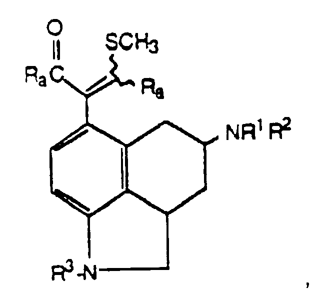 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec l'hydroxylamine de façon à obtenir un composé répondant à la formule 1 dans laquelle HET représente un groupe 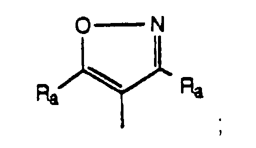
15) la réaction d'un composé répondant à la formule 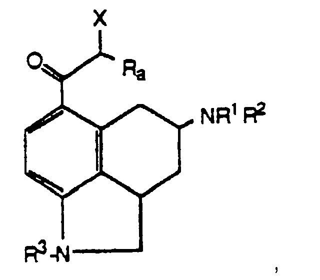 dans laquelle R¹, R² et R³ sont tels que définis ci-dessus, X représente un atome d'halogène et Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, avec 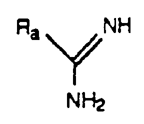 (où Rₐ est tel que présenté ci-dessus)
de façon à obtenir un composé répondant à la formule 1, dans laquelle HET représente un groupe 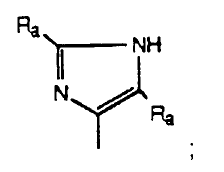
16) la réaction d'un composé répondant à la formule 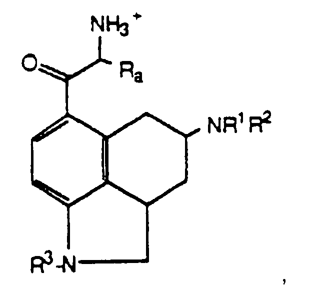 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du KSCN, et ensuite du R_{c}X (où R_{c} représente un atome d'hydrogène ou un groupe alkyle en C₁-C₃ et X représente un atome d'halogène) en présence d'une base de façon à obtenir un composé répondant à la formule 1, dans laquelle HET représente un groupe 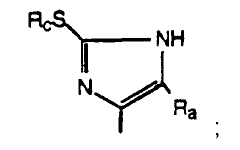
17) la déshydratation d'un composé répondant à la formule 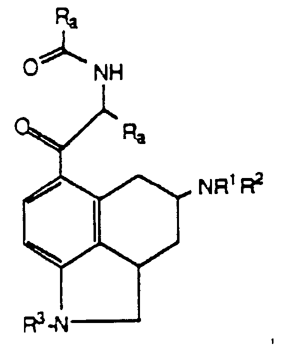 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, en présence d'ammoniac ou d'hydroxyde d'ammonium de façon à obtenir un composé répondant à la formule 1, dans laquelle HET représente un groupe 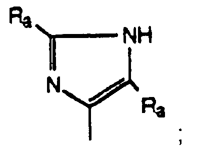
18) la réaction d'un composé répondant à la formule 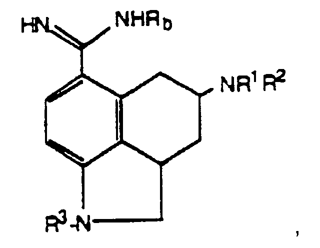 dans laquelle R¹, R² et R³ sont tels que définis ci-dessus et R_{b} représente un atome d'hydrogène, un groupe alkyle en C₁-C₃, un groupe phényle ou un groupe (alkyle en C₁-C₃)phényle, avec un composé répondant à la formule 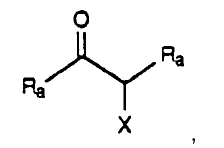 dans laquelle Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle et X représente un atome d'halogéne, de façon à obtenir un composé répondant à la formule 1 dans laquelle HET représente un groupe 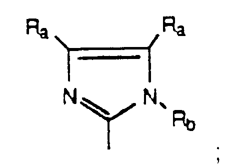
19) la déshydratation d'un composé répondant à la formule 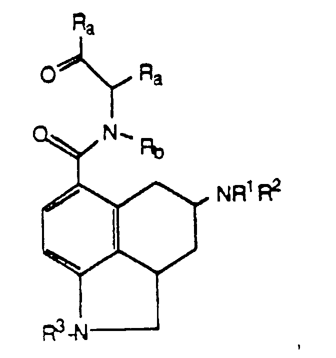 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, et R_{b} représente un atome d'hydrogène, un groupe alkyle en C₁-C₃, un groupe phényle ou un groupe (alkyle en C₁-C₃)phényle, en présence d'ammoniac ou d'hydroxyde d'ammonium de façon à obtenir un composé répondant à la formule 1, dans laquelle HET représente un groupe 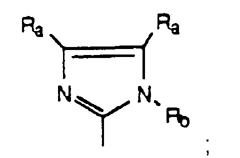
20) la réaction d'un composé répondant à la formule 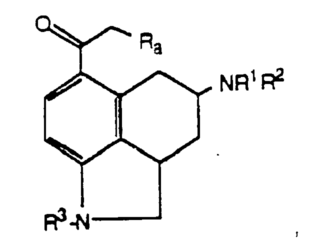 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un azide répondant à la formule R_{b}N₃, où R_{b} représente un atome d'hydrogène, un groupe alkyle en C₁-C₃, un groupe phényle ou un groupe (alkyle en C₁-C₃)phényle, suivi par la déshydratation du composé résultant de façon à obtenir un composé répondant à la formule 1, dans laquelle HET représente un groupe 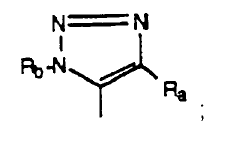
21) la cyclisation et la déshydratation d'un composé répondant à la formule 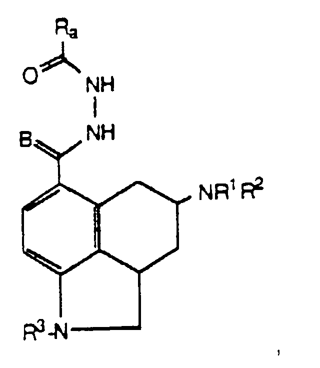 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, et B représente O ou NH, de façon à obtenir un composé répondant à la formule 1 dans laquelle HET représente un groupe 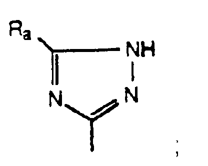 pour autant que lorsque B représente O, ladite réaction de cyclisation est effectuée en présence d'ammoniac ou d'hydroxyde d'ammonium ;
22) la réaction d'un composé répondant à la formule 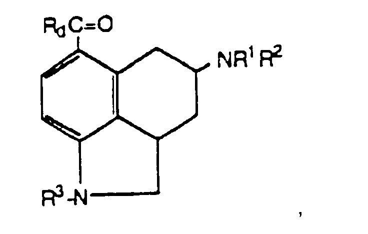 dans laquelle R¹, R² et R³ sont tels que présentés ci-dessus et R_{d} représente un groupe OH, un groupe O(alkyle en C₁-C₃), un groupe O(phényle), un groupe O((alkyle en C₁-C₃)phényle), un atome d'halogène, un groupe S(alkyle en C₁-C₃), un groupe S(phényle), un groupe S((alkyle en C₁-C₃)phényle), un groupe NH₂, un groupe NH(alkyle en C₁-C₃), un groupe N(alkyle en C₁-C₃)₂, un groupe OCO(alkyle en C₁-C₃), un groupe OCO(phényle) ou un groupe OCO((alkyle en C₁-C₃)phényle, avec un composé répondant à la formule 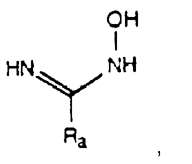 dans laquelle Rₐ représente un atome d'hydrogène un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 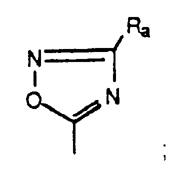
23) la réaction d'un composé répondant à la formule 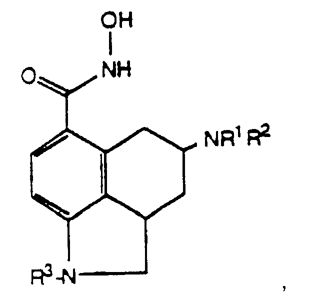 dans laquelle R¹, R² et R³ sont tels que définis ci-dessus, avec RₐCN, où Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 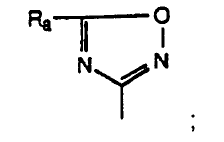
24) la cyclisation et la déshydratation d'un composé répondant à la formule 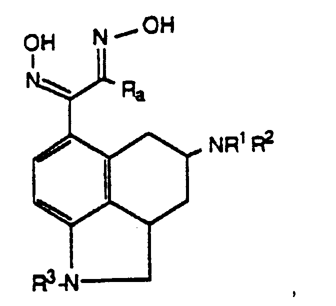 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 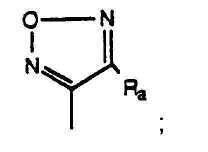
25) la réaction d'un composé répondant à la formule 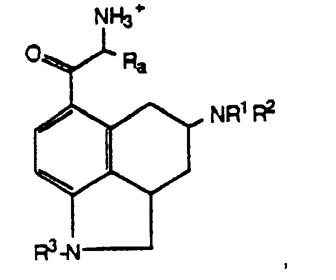 laquelle R¹, R² et R³ sont tels que définis ci-dessus et Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, avec un composé répondant à la formule 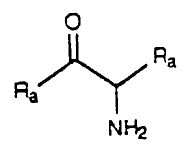 (où Rₐ est tel que présenté ci-dessus)
en présence d'un agent oxydant, de façon à obtenir un composé répondant à la formule 1 dans laquelle HET représente un groupe 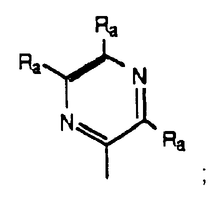
26) la réaction d'un composé répondant à la formule 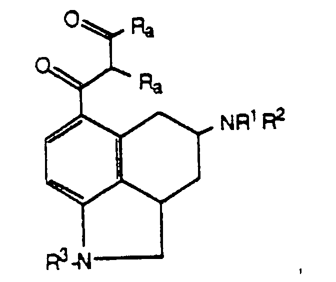 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 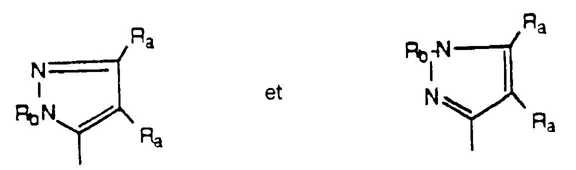 et ensuite, facultativement, la séparation des composés l'un de l'autre ;
27) la réaction d'un composé répondant à la formule 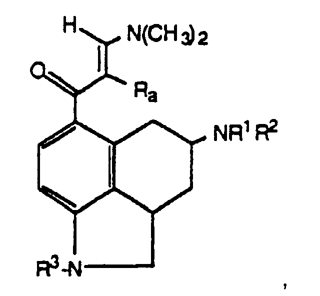 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 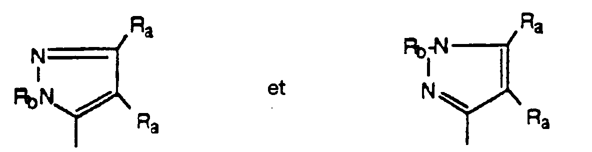 et ensuite, facultativement, la séparation des composés l'un de l'autre ;
28) la réaction d'un composé répondant à la formule 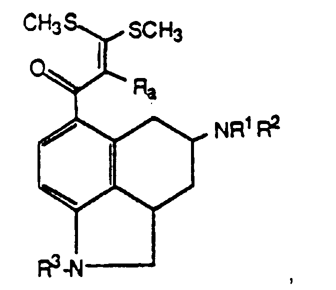 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente les groupes 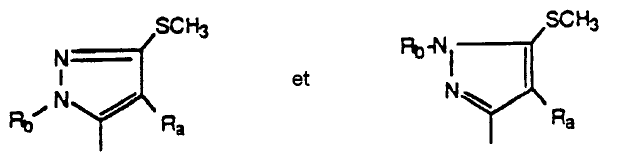 et ensuite, facultativement, la séparation des composés l'un de l'autre ;
29) la réaction d'un composé répondant à la formule 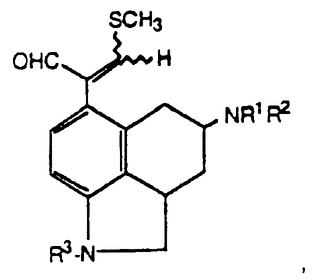 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule H₂NNHR_{b}, où R_{b} représente un atome d'hydrogène, un groupe alkyle en C₁-C₃, un groupe phényle ou un groupe (alkyle en C₁-C₃)phényle, de façon à obtenir des composés répondant à la formule 1 dans laquelle HET représente un groupe 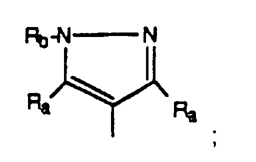
30) la réaction d'un composé répondant à la formule 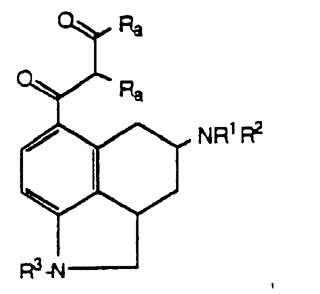 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 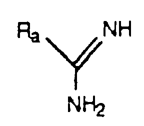 (où Rₐ est tel que présenté ci-dessus)
de façon à obtenir un composé répondant à la formule 1, dans laquelle HET représente un groupe 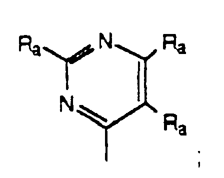
31) la réaction d'un composé répondant à la formule 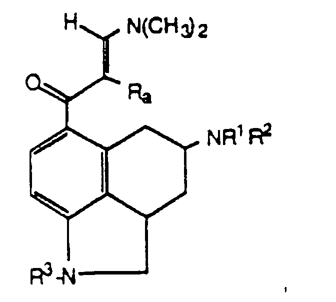 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 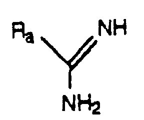 (où Rₐ est tel que présenté ci-dessus)
de façon à obtenir un composé répondant à la formule 1, dans laquelle HET représente un groupe 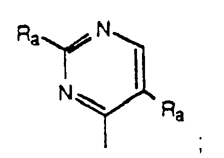
32) la réaction d'un composé répondant à la formule 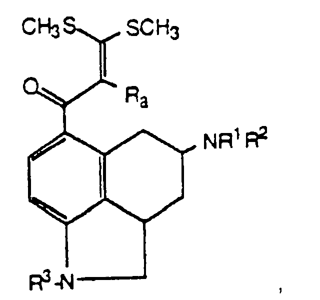 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 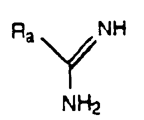 (où Rₐ est tel que présenté ci-dessus)
de façon à obtenir un composé répondant à la formule 1, dans laquelle HET représente un groupe 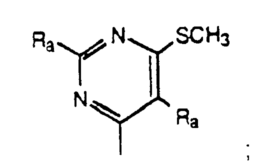
33) la réaction d'un composé répondant à la formule 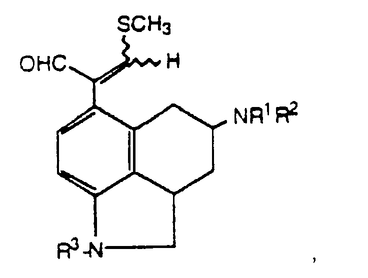 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un composé répondant à la formule 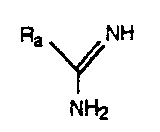 (où Rₐ est tel que présenté ci-dessus)
de façon à obtenir un composé répondant à la formule 1, dans laquelle HET représente un groupe 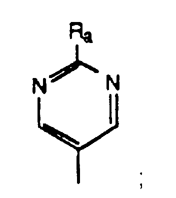
34) la réaction d'un composé répondant à la formule 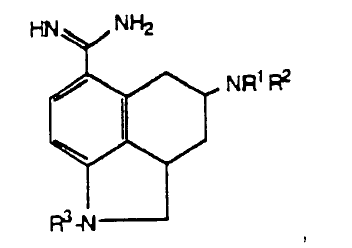 dans laquelle R¹, R² et R³ sont tels que définis ci-dessus, avec un composé répondant à la formule 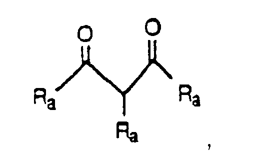 où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle de façon à obtenir un composé répondant à la formule 1, dans laquelle HET représente un groupe 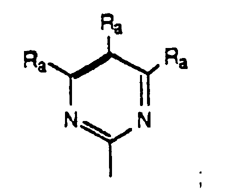
35) la réaction d'un composé répondant à la formule 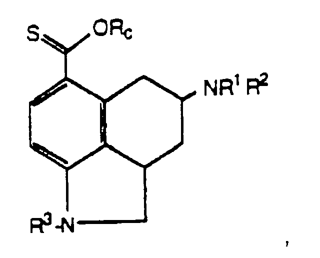 dans laquelle R¹, R² et R³ sont tels que définis ci-dessus et R_{c} représente un atome d'hydrogène ou un groupe alkyle en C₁-C₃, avec un composé répondant à la formule RₐCH₂NNH, où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1, dans laquelle HET représente un groupe 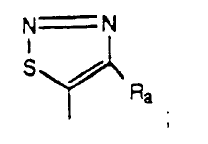
36) la réaction d'un composé répondant à la formule 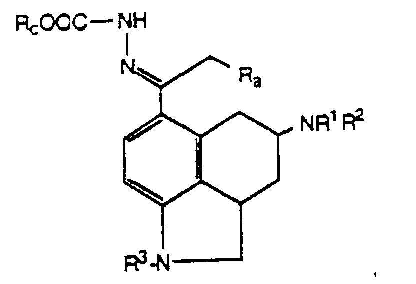 dans laquelle R¹, R² et R³ sont tels que définis ci-dessus et Rₐ représente un atome d'hydrogène un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1, dans laquelle HET représente un groupe 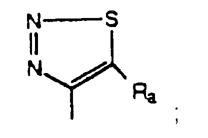
37) la réaction d'un composé répondant à la formule 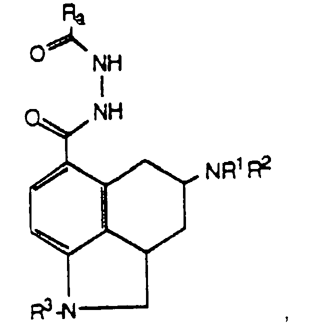 dans laquelle R¹, R² et R³ sont tels que définis ci-dessus et Rₐ représente un atome d'hydrogène, un groupe alkyle en C₁-C₃, un atome d'halogène un groupe OH, un groupe O(alkyle en C₁-C₃), un groupe S(alkyle en C₁-C₃), un groupe NH₂, un groupe CN ou un groupe phényle, avec du P₂S₅, de façon à obtenir un composé répondant à la formule 1, dans laquelle HET représente un groupe 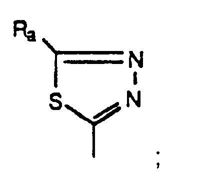
38) la réaction d'un composé répondant à la formule 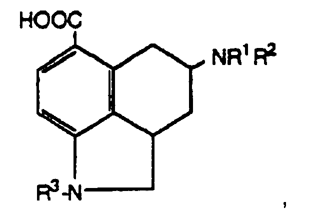 dans laquelle R¹, R² et R³ sont tels que définis ci-dessus, avec un composé répondant à la formule H₂NNHCSNH₂, en présence d'acide polyphosphorique, de façon à obtenir un composé répondant à la formule 1, dans laquelle HET représente un groupe 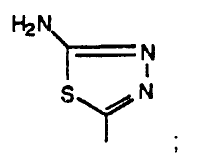
39) la réaction d'un composé répondant à la formule 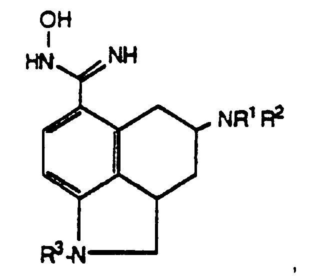 dans laquelle R¹, R² et R³ sont tels que définis ci-dessus, avec du disulfure de carbone, de façon à obtenir un composé répondant à la formule 1, dans laquelle HET représente un groupe 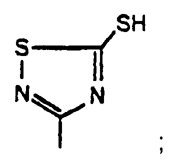
40) la réaction d'un composé répondant à la formule 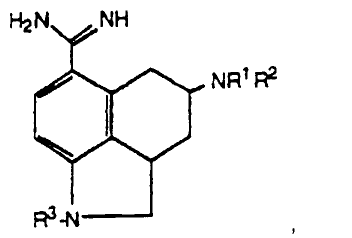 dans laquelle R¹, R² et R³ sont tels que définis ci-dessus, avec du (CNS)₂, de façon à obtenir un composé répondant à la formule 1, dans laquelle HET représente un groupe 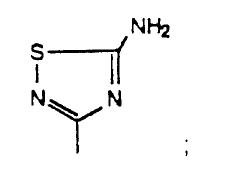
41) l'oxydation d'un composé répondant à la formule 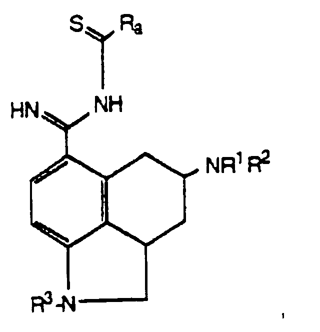 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 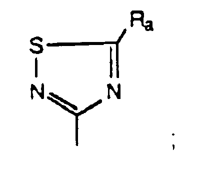
42) la réaction d'un composé répondant à la formule 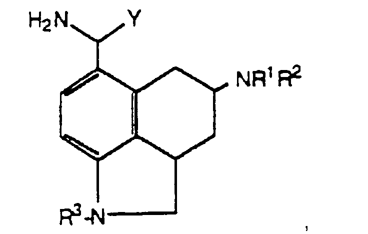 dans laquelle R¹, R² et R³ sont tels que définis ci-dessus et Y représente un groupe -CN ou un groupe -C(O)NH₂, avec un agent oxydant, tel que le SOCl₂, le SCl₂, le S₂Cl₂ ou le SO₂Cl₂, de façon à obtenir un composé répondant à la formule 1, dans laquelle HET représente un groupe 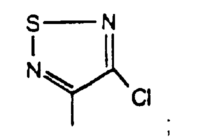
43) la réaction d'un composé répondant à la formule 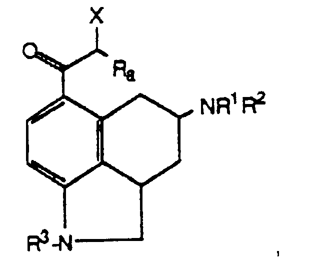 dans laquelle R¹, R² et R³ sont tels que définis ci-dessus, X représente un atome d'halogéne et Rₐ représente un atome d'hydrogène, un groupe alkyle en C₁-C₃, un atome d'halogéne, un groupe OH, un groupe O(alkyle en C₁-C₃), un groupe S(alkyle en C₁-C₃), un groupe NH₂, un groupe CN ou un groupe phényle, avec du RₐCSNH₂, où Rₐ est tel que présenté ci-dessus, de façon à obtenir un composé répondant à la formule 1, dans laquelle HET représente un groupe 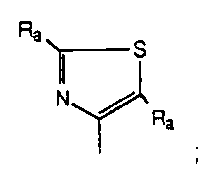
44) la réaction d'un composé répondant à la formule 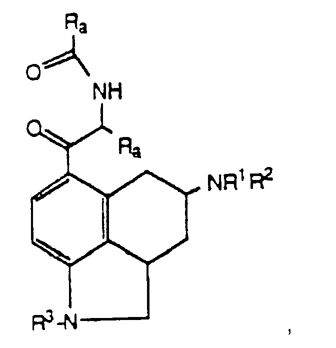 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du P₂S₅, de façon à obtenir un composé répondant à la formule 1, dans laquelle HET représente un groupe 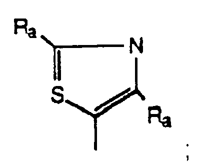
45) la réaction d'un composé répondant à la formule 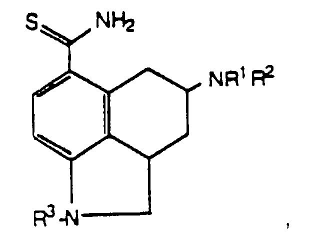 dans laquelle R¹, R² et R³ sont tels que définis ci-dessus, avec un composé répondant à la formule 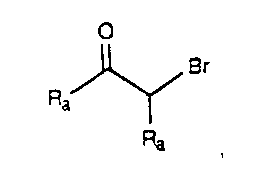 où Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, de façon à obtenir un composé répondant à la formule 1 dans laquelle HET représente un groupe 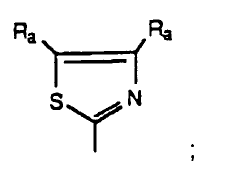
46) la réaction d'un composé répondant à la formule 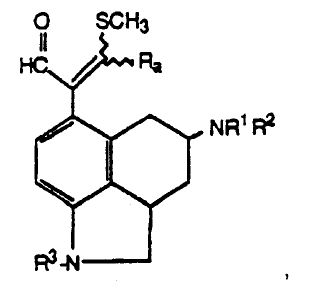 dans laquelle R¹, R² et R³ sont tels que définis ci-dessus, et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du RₐCSNH₂, où Rₐ est tel que présenté ci-dessus, de façon à obtenir un composé répondant à la formule 1, dans laquelle HET représente un groupe 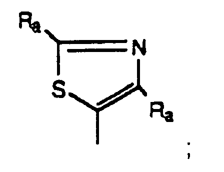
47) la réaction d'un composé répondant à la formule 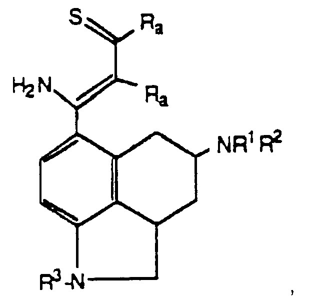 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à préparer un composé répondant à la formule 1 dans laquelle HET représente un groupe 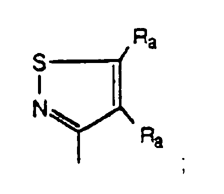
48) la réaction d'un composé répondant à la formule 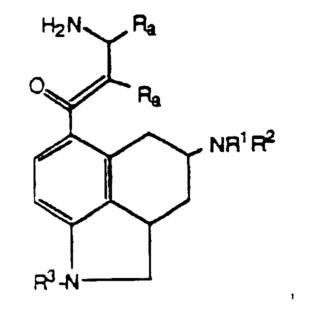 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à obtenir un composé répondant à la formule 1 dans laquelle HET représente un groupe 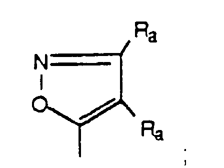
49) la réaction d'un composé répondant à la formule 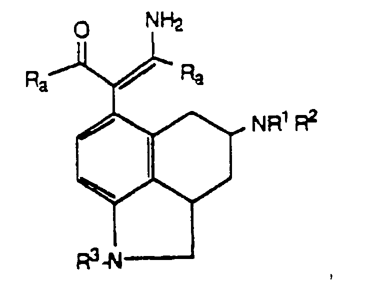 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec un agent oxydant de façon à obtenir un composé répondant à la formule 1 dans laquelle HET représente un groupe 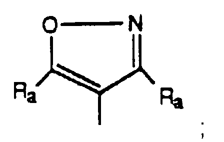
50) la réaction d'un composé répondant à la formule 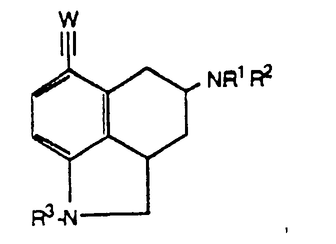 dans laquelle R¹, R² et R³ sont tels que définis ci-dessus et W représente un atome d'hydrogène ou un groupe CHO, avec du KSCN ou du NaHSSO₃ et ensuite la réaction de l'intermédiaire formé de cette façon avec l'ammoniac ou l'hydroxyde d'ammonium, de façon à obtenir un compose répondant à la formule 1 dans laquelle HET représente un groupe 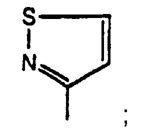
51) la réaction d'un composé répondant à la formule 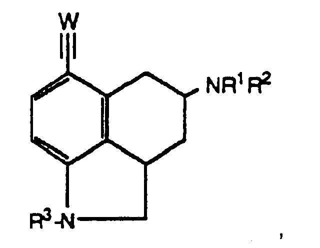 dans laquelle R¹, R² et R³ sont tels que définis ci-dessus et W représente un atome d'hydrogène ou un groupe CHO, avec du H₂NSSO₃K et ensuite la réaction de l'intermédiaire formé de cette façon avec une base, de façon à obtenir un composé répondant à la formule 1 dans laquelle HET représente un groupe 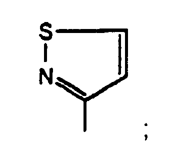
52) la réaction d'un composé répondant à la formule 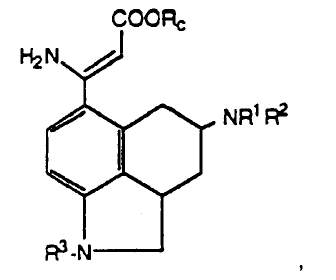 dans laquelle R¹, R² et R³ sont tels que définis ci-dessus et R_{c} représente un atome d'hydrogène ou un groupe alkyle en C₁-C₃, avec du CSCl₂, de façon à obtenir un composé répondant à la formule 1 dans laquelle HET représente un groupe 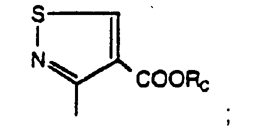
53) la réaction d'un composé répondant à la formule 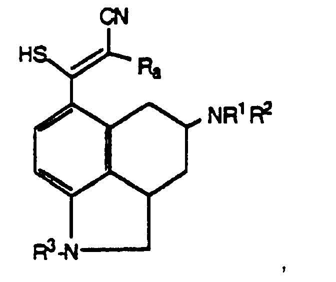 dans laquelle R¹, R² et R³ sont tels que définis ci-dessus et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec du NCS de façon à obtenir un composé répondant à la formule 1 dans laquelle HET représente un groupe 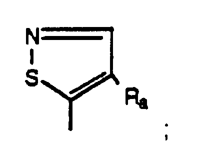
54) la réaction d'un composé répondant à la formule 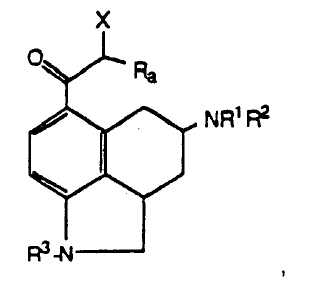 dans laquelle R¹, R² et R³ sont tels que définis ci-dessus, X représente un atome d'halogène et Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle, avec 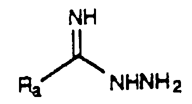 (où Rₐ est tel que présenté ci-dessus)
de façon à obtenir un composé répondant à la formule 1 dans laquelle HET représente un groupe 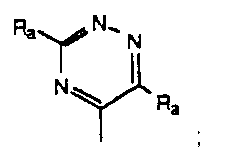
55) la réaction d'un composé répondant à la formule 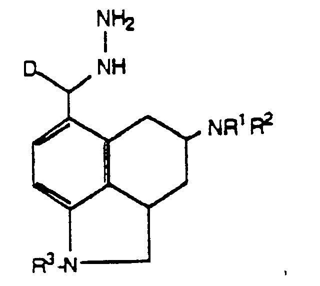 dans laquelle R¹, R² et R³ sont tels que définis ci-dessus et D représente =NH ou =O, avec un composé répondant à la formule 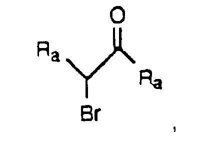 dans laquelle Rₐ représente un atome d'hydrogène, un groupe alkyle en C₁-C₃, un atome d'halogène, un groupe OH, un groupe O(alkyle en C₁-C₃), un groupe S(alkyle en C₁-C₃), un groupe NH₂, un groupe CN ou un groupe phényle de façon à obtenir un composé répondant à la formule 1, dans laquelle HET représente un groupe 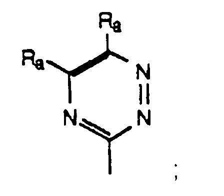
56) la réaction d'un composé répondant à la formule 1 dans laquelle HET est substitué par au moins un groupe hydroxyle, avec du POX₃, du PX₃, du SOX₂, du P(phényle)₃.X₂ ou du P(alcoxy en C₁-C₃)₃.X₂, où chaque X représente un atome d'halogène, de façon à convertir le groupe hydroxyle en substituant halogène ;
57) la réaction d'un composé répondant à la formule 1 dans laquelle HET est substitué par au moins un groupe amino, avec du HONO de façon à former l'ion diazonium correspondant, suivi par la conversion de cet ion en composé correspondant substitué par un atome d'halogène, avec du CuX (où X représente un atome d'halogéne), du KI ou du HBF₄, par application de chaleur ;
58) la réaction d'un composé répondant à la formule 1 dans laquelle HET est substitué par au moins un substituant halogène, avec un anion alcoxyde répondant à la formule
(alkyle en C₁-C₃)-O^{⊖}
de façon à convertir le substituant halogène en substituant alcoxy ;
59) la réaction d'un composé répondant à la formule 1 dans laquelle HET est substitué par au moins un groupe hydroxyle, avec un halogénure d'alkyle en C₁-C₃ de façon à convertir le groupe hydroxyle en substituant alcoxy ;
60) la réaction d'un composé répondant à la formule 1 dans laquelle HET est substitué par au moins un substituant hydroxyle, avec un composé diazo répondant à la formule (alkyle en C₁-C₃)N₂ de façon à convertir le groupe hydroxyle en substituant alcoxy ;
61) la réaction d'un composé répondant à la formule 1 dans laquelle HET est substitué par au moins un groupe amino, avec du HONO de façon à former l'ion diazonium correspondant, suivi par la conversion de cet on en composé correspondant substitué par un groupe hydroxyle en utilisant de l'eau ou un acide ;
62) la réaction d'un composé répondant à la formule 1 dans laquelle HET est substitué par au moins un groupe alcoxy en C₁-C₃, avec de l'acide iodhydrique concentré, de l'acide bromhydrique concentré ou un acide de Lewis de façon à convertir le groupe alcoxy en substituant hydroxyle ;
63) la réaction d'un composé répondant à la formule 1 dans laquelle HET est substitué par au moins un groupe amino, avec du HONO de façon à former l'ion diazonium correspondant, suivi par la conversion de cet ion en composé correspondant substitué par un groupe nitrile en utilisant du CuCN ;
64) la réaction d'un composé répondant à la formule 1 dans laquelle HET est substitué par au moins un groupe amino, avec du HONO de façon à former l'ion diazonium correspondant, suivi par la conversion de cet on en composé correspondant substitué par un groupe (alkyle en C₁-C₃)thio en utilisant un (alkyle en C₁-C₃)mercaptan ;
65) la réaction d'un composé répondant à la formule 1 dans laquelle HET est substitué par au moins un substituant halogène, avec un anion alkylthio répondant à la formule
(alkyle en C₁-C₃)-S^{⊖}
de façon à convertir le substituant halogène en une fraction alkylthio ;
66) la réaction d'un composé répondant à la formule 1 dans laquelle HET est substitué par au moins une fraction -SH, avec un halogénure d'alkyle en C₁-C₃ de façon à obtenir le composé correspondant répondant à la formule 1 présentant une fraction alkylthio ;
67) la réduction d'un composé répondant à la formule 1, dans laquelle HET est substitué par au moins un substituant nitro, de façon à obtenir un composé répondant à la formule 1, dans laquelle le substituant nitro est converti en fraction amino ;
68) la réduction d'un composé répondant à la formule 1 dans laquelle HET est substitué par au moins un substituant halogène, de façon à convertir le substituant halogéne en atome d'hydrogène ;
69) la réduction d'un composé répondant à la formule 1 dans laquelle HET est substitué par au moins un groupe hydroxyle, de façon à convertir le substituant hydroxyle en atome d'hydrogène ;
70) la réaction d'un composé répondant à la formule 1 dans laquelle HET est substitué par au moins une fraction amino, avec du HONO, de façon à former l'ion diazonium correspondant, suivi par la conversion de cet ion en composé non-substitué correspondant en utilisant du H₃PO₂ ;
71) la réduction d'un composé répondant à la formule 1 dans laquelle HET est substitué sur un des atomes d'azote dans ce noyau hétérocyclique par un groupe (alkyle en C₁-C₃)phényle de façon à obtenir le composé correspondant répondant à la formule 1, dans laquelle l'atome d'azote est non-substitué ;
72) la réduction d'un composé répondant à la formule 1 dans laquelle HET est substitué par au moins une fraction (alkyle en C₁-C₃)thio de façon à obtenir le composé non-substitué correspondant répondant à la formule 1 ou
73) la réaction d'un composé répondant à la formule 1 avec un acide organique ou inorganique pharmaceutiquement acceptable de façon à former un sel d'addition acide pharmaceutiquement acceptable de ce composé.

2. Procédé selon la revendication 1, dans lequel HET représente un groupe isoxazole, un groupe pyrazole, un groupe pyridine, un groupe thiazole, un groupe furane, un groupe thiophène, un groupe oxadiazole, ou un sel pharmaceutiquement acceptable de celui-ci.

3. Procédé selon la revendication 1 ou 2, dans lequel R¹ et R² représentent indépendamment un groupe alkyle en C₁-C₃, ou un sel pharmaceutiquement acceptable de celui-ci.

4. Procédé selon l'une quelconque des revendications 1 à 3, dans lequel R³ représente un atome d'hydrogène, ou un sel pharmaceutiquement acceptable de celui-ci.

5. Procédé selon la revendication 1 ou 2, dans lequel R¹ représente un groupe -(CH₂)ₙC(O)NR⁵R⁶, dans lequel n vaut 2, R⁵ représente un atome d'hydrogène, R⁶ représente un groupe cyclohexyle, R² représente un groupe alkyle en C₁-C₃ et R³ représente un atome d'hydrogène ou un groupe alkyle en C₁-C₄, ou un sel pharmaceutiquement acceptable de celui-ci.

6. Procédé de préparation d'un stéréo-isomère essentiellement pur d'un composé selon la revendication 1, ou d'un sel pharmaceutiquement acceptable de celui-ci, qui comprend le procédé selon l'une quelconque des revendications 1 à 5.

7. Procédé selon la revendication 6, dans lequel la configuration du stéréo-isomère est S à la position 2a et R à la position 4, ou un sel pharmaceutiquement acceptable de celui-ci.

8. Procédé selon la revendication 1, qui est utilisé pour préparer un composé choisi parmi le groupe constitué du 6-(3-isoxazolyl)-4-(di-n-propylamino)-1,2,2a,3,4,5-hexahydrobenz[cd]indole ; du 6-(5-isoxazolyl)-4-(di-n-propylamino)-1,2,2a,3,4,5-hexahydrobenz[cd]indole ; du 6-(3-pyrazolyl)-4-(di-n-propylamino)-1,2,2a,3,4,5-hexahydrobenz[cd]indole ; du 6(4-pyrazolyl)-4-(diméthylamino)-1,2,2a,3,4,5-hexahydrobenz[cd]indole ; du 6-(4-pyridinyl)-4-(di-n-propylamino)-1,2,2a,3,4,5-hexahydrobenz[cd]indole; du 6-(2-pyridinyl)-4-(di-n-propylamino)-1,2,2a,3,4,5-hexahydrobenz[cd]indole ; du 6-(3-pyridinyl)-4-(di-n-propylamino)-1,2,2a,3,4,5-hexahydrobenz[cd]indole ; du 6-(2-thiazolyl)-4-(di-n-propylamino)-1,2,2a,3,4,5-hexahydrobenz[cd]indole ; du 6-(5-thiazolyl)-4-(di-n-propylamino)-1,2,2a,3,4,5-hexahydrobenz[cd]indole ; du 6-(2-oxadiazolyl)-4-(di-n-propylamino)-1,2,2a,3,4,5-hexahydrobenz[cd]indole ; du 6-(3-furyl)-4-(di-n-propylamino)-1,2,2a,3,4,5-hexahydrobenz[cd]indole, ou un sel pharmaceutiquement acceptable de celui-ci.

9. Procédé de préparation d'une formulation pharmaceutique, qui comprend le mélange d'un composé répondant à la formule 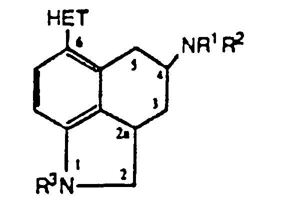 dans laquelle
R¹ représente un atome d'hydrogène, un groupe alkyle en C₁-C₄, un groupe alcényle en C₃-C₄, un groupe cyclopropylméthyle, un groupe phényl(alkyle en C₁-C₄), un groupe phényl(alkyle en C₁-C₄) substitué par un ou deux substituants choisis parmi un groupe alcoxy en C₁-C₃, un atome d'halogène, un groupe hydroxyle, un groupe thio(alkyle en C₁-C₃), un groupe nitro, un groupe alkyle en C₁-C₃ et un groupe trifluorométhyle, un groupe -(CH₂)ₙS(alkyle en C₁-C₄), un groupe -C(O)R⁴, un groupe -(CH₂)ₙC(O)NR⁵R⁶ ;
R² représente un atome d'hydrogène, un groupe alkyle en C₁-C₄, un groupe cyclopropylméthyle ou un groupe alcényle en C₃-C₄ ;
R³ représente un atome d'hydrogène, un groupe alkyle en C₁-C₄ ou un groupe amino-protecteur ;
n vaut 1 à 4 ;
R⁴ représente un atome d'hydrogène, un groupe alkyle en C₁-C₄, un groupe halogénoalkyle en C₁-C₄, un groupe alcoxy en C₁-C₄ ou un groupe phényle ;
R⁵ et R⁶ représentent, indépendamment, un atome d'hydrogène, un groupe alkyle en C₁-C₄ ou un groupe cycloalkyle en C₅-C₈, pour autant que lorsqu'un des groupes R⁵ ou R⁶ représente un groupe cycloalkyle, l'autre représente un atome d'hydrogène ;
HET représente un noyau hétérocyclique aromatique à 5 ou à 6 membres, ledit noyau présentant de 1 à 3 hétéroatomes qui sont identiques ou différents et qui sont choisis parmi le groupe constitué du soufre, de l'oxygène et de l'azote, pour autant que le noyau hétérocyclique à 6 membres ne puisse contenir que du carbone et de l'azote et en outre pour autant que le noyau à 5 membres puisse contenir au plus un atome d'oxygène ou un atome de soufre et non simultanément un atome d'oxygène et un atome de soufre, facultativement substitué sur un ou deux atomes de carbone par un groupe alkyle en C₁-C₃, un atome d'halogène, un groupe hydroxyle, un groupe alcoxy en C₁-C₃, un groupe thio(alkyle en C₁-C₃), un groupe NH₂, un groupe CN ou un groupe phényle, ou un sel pharmaceutiquement acceptable de celui-ci, avec un ou davantage de supports, diluants ou excipients pharmaceutiquement acceptables pour celui-ci.
